(19) Europäisches Patentamt
European Patent Office
Office européen des brevets

(11) EP 1 912 979 B1

(12) **EUROPEAN PATENT SPECIFICATION**

(45) Date of publication and mention of the grant of the patent:
**22.06.2011 Bulletin 2011/25**

(21) Application number: **06779899.1**

(22) Date of filing: **17.05.2006**

(51) Int Cl.:
*C07D 413/14* (2006.01) *C07D 401/14* (2006.01)
*A61K 31/4245* (2006.01) *A61K 31/415* (2006.01)
*A61P 25/22* (2006.01) *A61P 25/18* (2006.01)
*A61P 25/32* (2006.01) *A61P 25/34* (2006.01)
*A61P 25/36* (2006.01)

(86) International application number:
**PCT/IB2006/002047**

(87) International publication number:
**WO 2006/123257 (23.11.2006 Gazette 2006/47)**

(54) **PHENYL-{3-(3-(1H-PYRROL-2-YL)-[1,2,4]OXADIAZOL-5-YL]PIPERIDIN-1-YL}-METHANONE DERIVATIVES AND RELATED COMPOUNDS AS POSITIVE ALLOSTERIC MODULATORS OF METABOTROPIC GLUTAMATE RECEPTORS**

PHENYL-{3-[3-(1H-PYRROL-2-YL)-[1,2,4]OXADIAZOL-5-YL]PIPERIDIN-1-YL}-METHANON-DERIVATE UND VERWANDTE VERBINDUNGEN ALS POSITIVE ALLOSTERISCHE MODULATOREN METABOTROPER GLUTAMATREZEPTOREN

DERIVES DE PHÉNYL-{3-[3-(1H-PYRROL-2-YL)-[1,2,4]OXADIAZOL-5-YL]PIPÉRIDIN-1-YL}-METHANON ET COMPOSÉS SIMILAIRES EN TANT QUE MODULATEURS ALLOSTERIQUES POSITIFS DES RECEPTEURS DE GLUTAMATE METABOTROPIQUE

(84) Designated Contracting States:
**AT BE BG CH CY CZ DE DK EE ES FI FR GB GR HU IE IS IT LI LT LU LV MC NL PL PT RO SE SI SK TR**

(30) Priority: **18.05.2005 GB 0510141**

(43) Date of publication of application:
**23.04.2008 Bulletin 2008/17**

(73) Proprietor: **ADDEX Pharma S.A.**
**1228 Plan-lès-Ouates (Geneva) (CH)**

(72) Inventors:
- **GAGLIARDI, Stefania**
  **NiKem Research Srl**
  **I-20021 Baranzante (Milan) (IT)**
- **LE POUL, Emmanuel**
  **Addex Pharma SA**
  **I-1228 Plan les Ouates (FR)**
- **LINGARD, Lain**
  **NiKem Research Srl**
  **I-20021 Baranzate (GB)**
- **PALOMBI, Giovanni**
  **NiKem Research Srl**
  **I-20021 Baranzate (IT)**
- **POLI, Sonia, Maria**
  **Addex Pharma SA**
  **CH-1228, Plan-les-Ouates (CH)**
- **ROCHER, Jean-Philippe**
  **Addex Pharma SA**
  **CH-1228 Plan les Ouates (CH)**

(74) Representative: **Davies, Jonathan Mark**
**Reddie & Grose**
**16 Theobalds Road**
**London WC1X 8PL (GB)**

(56) References cited:
**EP-A1- 1 300 396** **WO-A-03/027080**
**WO-A-03/037888** **WO-A-03/093236**
**WO-A-2004/029044** **WO-A-2004/048334**
**WO-A-2004/058754** **WO-A-2005/009988**
**WO-A-2005/044797** **WO-A-2005/074934**
**WO-A-2006/048771** **WO-A-2006/123249**
**WO-A-2006/123255** **WO-A-2006/123257**
**WO-A2-03/087304** **WO-A2-03/093297**
**WO-A2-2004/014902** **WO-A2-2005/115389**
**WO-A2-2006/036015** **WO-A2-2006/044509**
**WO-A2-2006/065601**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 1 912 979 B1

- DATABASE CA [Online] CHEMICAL ABSTRACTS SERVICE, COLUMBUS, OHIO, US; 1989, VASVARI-DEBRECZY, LELLE ET AL: "Nitrogen bridgehead compounds. Part 74. Cyclization of 2-[(2-pyridylamino)methylene]succinates in ethanolic sodium ethoxide. Part 2. Michael addition of pyridyldihydropyrrolones" XP002413099 retrieved from STN Database accession no. 1989:75220 & JOURNAL OF THE CHEMICAL SOCIETY, PERKIN TRANSACTIONS 1: ORGANIC AND BIO-ORGANIC CHEMISTRY (1972-1999) , (7), 2019-22 CODEN: JCPRB4; ISSN: 0300-922X, 1988,

## Description

FIELD OF THE INVENTION

The present invention provides new compounds of formula I-B as defined in Claim I as positive allosteric modulators of metabotropic receptors - subtype 5 ("mGluR5") which are useful for the treatment or prevention of central nervous system disorders such as for example, cognitive decline, both positive and negative symptoms in schizophrenia as well as other central or peripheral nervous system disorders in which the mGluR5 subtype of glutamate metabotropic receptor is involved. The invention is also directed to use of compounds and pharmaceutical compositions in the prevention or treatment of such diseases in which mGluR5 is involved.

BACKGROUND OF THE INVENTION

Glutamate, the major amino-acid transmitter in the mammalian central nervous system (CNS), mediates excitatory synaptic neurotransmission through the activation of ionotropic glutamate receptors receptor-channels (iGluRs, namely NMDA, AMPA and kainate) and metabotropic glutamate receptors (mGluRs). iGluRs are responsible for fast excitatory transmission (Nakanishi S et al., (1998) Brain Res Brain Res Rev., 26:230-235) while mGluRs have a more modulatory role that contributes to the fine-tuning of synaptic efficacy. Glutamate performs numerous physiological functions such as long-term potentiation (LTP), a process believed to underlie learning and memory but also cardiovascular regulation, sensory perception, and the development of synaptic plasticity. In addition, glutamate plays an important role in the pathophysiology of different neurological and psychiatric diseases, especially when an imbalance in glutamatergic neurotransmission occurs.

The mGluRs are seven-transmembrane G protein-coupled receptors. The eight members of the family are classified into three groups (Groups I, II & III) according to their sequence homology and pharmacological properties (Schoepp DD et al. (1999) Neuropharmacology, 38:1431-1476). Activation of mGluRs lead to a large variety of intracellular responses and activation of different transductional cascades. Among mGluR members, the mGluR5 subtype is of high interest for counterbalancing the deficit or excesses of neurotransmission in neuropsychatric diseases. mGluR5 belongs to Group I and its activation initiates cellular responses through G-protein mediated mechanisms. mGluR5 is coupled to phospholipase C and stimulates phosphoinositide hydrolysis and intracellular calcium mobilization.

mGluR5 proteins have been demonstrated to be localized in post-synaptic elements adjacent to the post-synaptic density (Lujan R et al. (1996) Eur J Neurosci. 8:1488-500; Lujan R et al. (1997) J Chem Neuroanat., 13:219-41) and are rarely detected in the pre-synaptic elements (Romano C et al. (1995) J Comp Neurol. 355:455-69). mGluR5 receptors can therefore modify the post-synaptic responses to neurotransmitter or regulate neurotransmitter release.

In the CNS, mGluR5 receptors are abundant mainly throughout the cortex, hippocampus, caudate-putamen and nucleus accumbens. As these brain areas have been shown to be involved in emotion, motivational processes and in numerous aspects of cognitive function, mGluR5 modulators are predicted to be of therapeutic interest.

A variety of potential clinical indications have been suggested to be targets for the development of subtype selective mGluR modulators. These include epilepsy, neuropathic and inflammatory pain, numerous psychiatric disorders (eg anxiety and schizophrenia), movement disorders (eg Parkinson disease), neuroprotection (stroke and head injury), migraine and addiction/drug dependency (for reviews, see Brauner-Osbome H et al. (2000) J Med Chem. 43:2609-45; Bordi F and Ugolini A. (1999) Prog Neurobiol. 59:55-79; Spooren W et al. (2003) Behav Pharmacol: 14:257-77).

The hypothesis of hypofunction of the glutamatergic system as reflected by NMDA receptor hypofunction as a putative cause of schizophrenia has received increasing support over the past few years (Goff DC and Coyle JT (2001) Am J Psychiatry, 158:1367-1377; Carlsson A et al. (2001) Annu Rev Pharmacol Toxicol., 41:237-260 for a review). Evidence implicating dysfunction of glutamatergic neurotransmission is supported by the finding that antagonists of the NMDA subtype of glutamate receptor can reproduce the full range of symptoms as well as the physiologic manifestation of schizophrenia such as hypofrontality, impaired prepulse inhibition and enhanced subcortical dopamine release. In addition, clinical studies have suggested that mGluR5 allele frequency is associated with schizophrenia among certain cohorts (Devon RS et al. (2001) Mol Psychiatry. 6:311-4) and that an increase in mGluR5 message has been found in cortical pyramidal cells layers of schizophrenic brain (Ohnuma T et al. (1998) Brain Res Mol Brain Res. 56:207-17).

The involvement of mGluR5 in neurological and psychiatric disorders is supported by evidence showing that in vivo activation of group I mGluRs induces a potentiation of NMDA receptor function in a variety of brain regions mainly through the activation of mGluR5 receptors (Mannaioni G et al. (2001) Neurosci. 21:5925-34; Awad H et al. (2000) J Neurosci 20:7871-7879; Pisani A et al (2001) Neuroscience 106:579-87; Benquet P et al (2002) J Neurosci. 22:9679-86).

The role of glutamate in memory processes also has been firmly established during the past decade (Martin SJ et al. (2000) Annu. Rev. Neurosci. 23:649-711; Baudry M and Lynch G. (2001) Neurobiol Learn Mem., 76:284-297). The use of mGluR5 null mutant mice have strongly supported a role of mGluR5 in learning and memory. These mice show a selective loss in two tasks of spatial learning and memory, and reduced CA1 LTP (Lu et al. (1997) J. Neurosci.,

17:5196-5205; Schulz B et al. (2001) Neuropharmacology. 41:1-7; Jia Z et al. (2001) Physiol Behav., 73:793-802; Rodrigues et al. (2002) J Neurosci., 22:5219-5229).

The finding that mGluR5 is responsible for the potentiation of NMDA receptor mediated currents raises the possibility that agonists of this receptor could be useful as cognitive-enhancing agents, but also as novel antipsychotic agents that act by selectively enhancing NMDA receptor function.

The activation of NMDARs could potentiate hypofunctional NMDARs in neuronal circuitry relevant to schizophrenia. Recent in vivo data strongly suggest that mGluR5 activation may be a novel and efficacious approach to treat cognitive decline and both positive and negative symptoms in schizophrenia (Kinney GG et al. (2002) 43:292).

mGluR5 receptor is therefore being considered as a potential drug target for treatment of psychiatric and neurological disorders including treatable diseases in this connection are Anxiety Disorders, Attentional disorders, Eating Disorders, Mood Disorders, Psychotic Disorders, Cognitive Disorders, Personality Disorders and Substance-related disorders.

Most of the current modulators of mGluR5 function have been developed as structural analogues of glutamate, quisqualate or phenylglycine (Schoepp DD et al. (1999) Neuropharmacology, 38:1431-1476) and it has been very challenging to develop in vivo active and selective mGluR5 modulators acting at the glutamate binding site. A new avenue for developing selective modulators is to identify molecules that act through allosteric mechanisms, modulating the receptor by binding to site different from the highly conserved orthosteric binding site.

Positive allosteric modulators of mGluRs have emerged recently as novel pharmacological entities offering this attractive alternative. This type of molecule has been discovered for mGluR1, mGluR2, mGluR4, and mGluR5 (Knoflach F et al. (2001) Proc Natl Acad Sci U S A. 98:13402-13407; O'Brien JA et al. (2003) Mol Pharmacol. 64:731-40 ; Johnson K et al. (2002) Neuropharmacology 43:291; Johnson MP et al. (2003) J Med Chem. 46:3189-92; Marino MJ et al. (2003) Proc Natl Acad Sci U S A. 100(23):13668-73; for a review see Mutel V (2002) Expert Opin. Ther. Patents 12:1-8; Kew JN (2004) Pharmacol Ther. 104(3):233-44; Johnson MP et al (2004) Biochem Soc Trans. 32:881-7). DFB and related molecules were described as in vitro mGluR5 positive allosteric modulators but with low potency (O'Brien JA et al. (2003) Mol. Pharmacol. 64:731-40). Benzamide derivatives have been patented (WO 2004/087048; O'Brien JA (2004) J. Pharmacol. Exp. Ther. 309:568-77) and recently aminopyrazole derivatives have been disclosed as mGluR5 positive allosteric modulators (Lindsley et al. (2004) J. Med. Chem. 47:5825-8; WO 2005/087048). Among aminopyrazole derivatives, CDPPB has shown in vivo activity antipsychotic-like effects in rat behavioral models (Kinney GG et al. (2005) J Pharmacol Exp Ther 313:199-206). This report is consistent with the hypothesis that allosteric potentiation of mGluR5 may provide a novel approach for development of antipsychotic agents.. Recently a novel series of positive allosteric modulators of mGluR5 receptors has been disclosed (WO 2005/044797). International publication WO 99/45006 by Pfizer Inc. discloses oxadiazolyl piperidine derivatives as rotamase enzyme inhibitors. Several classes of aryl and heteroaryloxadiazole compounds have been disclosed: US 04/106607, WO 03/056823, WO 02/72570, GB 1164572, FR 6671).

None of the specifically disclosed compounds are structurally related to the compounds of the present invention.

PRIOR ART

The following documents have been cited in the International and European search reports:

D1: WO 2005/009988 A (Euro Celtique SA)
D2: WO 2004/058754 A (Euro Celtique SA)
D3: WO 2004/029044 A (Euro Celtique SA)
D4: WO 2004/014902 A2 (Astrazeneca AB)
D5: WO 03/093236 A (Euro Celtique SA)
D6: WO 2004/048334 A (Pfizer Prod Inc)
D7: WO 03/093297 A2 (Exelixis Inc)
D8: WO 03/037888 A (Mitsubishi Pharma Corp; Sanofi Synthelabo)
D9: WO 03/027080 A (Mitsubishi Pharma Corp; Sanofi Synthelabo)
D10 EP-A1-1 300 396 (Eisai Co Ltd)
D11: WO 03/087304 A2 (Biogen Inc)
D12: Database CA [Online] Chemical Abstracts Service, Columbus, Ohio, US; 1989, Vasvari-Debreczy, Lelle et al: "Nitrogen bridgehead compounds. Part 74. Cyclization of 2-[(2-pyridylamino)methylene]succinates in ethanolic sodium ethoxide. Part 2. Michael addition of pyridyldihydropyrrolones" XP002413099 retrieved from STN Database accession no. 1989:75220
D13: WO 2006/048771 A (Addex Pharmaceuticals SA) P-document
D14: WO 2005/044797 A (Addex Pharmaceuticals SA) P-document
D15: WO 2006/036015 A2 (Mitsubishi Pharma Corp; Sanofi Aventis) P-document
D16: WO 2005/074934 A (Merck Patent GmbH) P-document

D17: WO 2005/115389 A2 (Pfizer Prod Inc; Kehrli Marcus Eugene Jr) P-document
D18: WO 2006/044509 A2 (Biogen Idec Inc) P-document
D19: WO 2006/123249 A (Addex Pharmaceuticals SA) E-document
D20: WO 2006/123255 A (Addex Pharmaceuticals SA) E-document
D21: WO 2006/123257 A (Addex Pharmaceuticals SA) E-document
D22: WO 2006/065601 A2 (Merck & Co Inc) E-document

FIGURES

Figure 1 shows the effect of 10 μM of the example #1 of the present invention on primary cortical mGluR5-expressing cell cultures in the absence or in the presence of 300nM glutamate.

DETAILED DESCRIPTION OF THE INVENTION

According to the present invention, there are provided new compounds of the general formula I-B

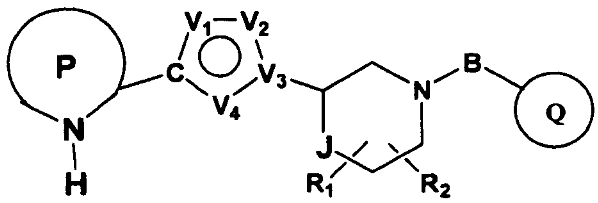

I-B

Or pharmaceutically acceptable salts, hydrates or solvates of such compounds Wherein $R_1$, $R_2$, P, Q, $V_1$, $V_2$, $V_3$, $V_4$, B and J are defined in claim 1.

The present invention includes both possible stereoisomers and includes not only racemic compounds but the individual enantiomers as well.

For the avoidance of doubt it is to be understood that in this specification "($C_1$-$C_6$)" means a carbon group having 1, 2, 3, 4, 5 or 6 carbon atoms. "($C_0$-$C_6$)" means a carbon group having 0, 1, 2, 3, 4, 5 or 6 carbon atoms. In this specification "C" means a carbon atom.

In the above definition, the term "($C_1$-$C_6$)alkyl" includes group such as methyl, ethyl, propyl, isopropyl, butyl, isobutyl, sec-butyl, tert-butyl, pentyl, isopentyl, neopentyl, tert-pentyl, hexyl or the like.

"($C_2$-$C_6$)alkenyl" includes group such as ethenyl, 1-propenyl, allyl, isopropenyl, 1-butenyl, 3-butenyl, 4-pentenyl and the like.

"($C_2$-$C_6$)alkynyl" includes group such as ethynyl, propynyl, butynyl, pentynyl and the like.

"Halogen" includes atoms such as fluorine, chlorine, bromine and iodine.

"Cycloalkyl" refers to an optionally substituted carbocycle containing no heteroatoms, includes mono-, bi-, and tricyclic saturated carbocycles, as well as fused ring systems. Such fused ring systems can include on ring that is partially or fully unsaturated such as a benzene ring to form fused ring systems such as benzo fused carbocycles. Cycloalkyl includes such fused ring systems as spirofused ring systems. Examples of cycloalkyl include cyclopropyl, cyclobutyl, cyclopentyl, cyclohexyl, decahydronaphthalene, adamantane, indanyl, fluorenyl, 1,2,3,4-tetrahydronaphthalene and the like.

"Heterocycloalkyl" refers to an optionally substituted carbocycle containing at least one heteroatom selected independently from O, N, S. It includes mono-, bi-, and tricyclic saturated carbocycles, as well as fused ring systems. Such fused ring systems can include one ring that is partially or fully unsaturated such as a benzene ring to form fused ring systems such as benzo fused carbocycles. Examples of heterocycloalkyl include piperidine, piperazine, morpholine, tetrahydrothiophene, indoline, isoquinoline and the like.

"Aryl" includes ($C_6$-$C_{10}$)aryl group such as phenyl, 1-naphtyl, 2-naphtyl and the like.

"Arylalkyl" includes ($C_6$-$C_{10}$)aryl-($C_1$-$C_3$)alkyl group such as benzyl group, 1-phenylethyl group, 2-phenylethyl group, 1-phenylpropyl group, 2-phenylpropyl group, 3-phenylpropyl group, 1-naphtylmethyl group, 2-naphtylmethyl group or the like.

"Heteroaryl" includes 5-10 membered heterocyclic group containing 1 to 4 heteroatoms selected from oxygen, nitrogen or sulphur to form a ring such as furyl (furan ring), benzofuranyl (benzofuran ring), thienyl (thiophene ring), benzothiophenyl (benzothiophene ring), pyrrolyl (pyrrole ring), imidazolyl (imidazole ring), pyrazolyl (pyrazole ring),

thiazolyl (thiazole ring), isothiazolyl (isothiazole ring), triazolyl (triazole ring), tetrazolyl (tetrazole ring), pyridil (pyridine ring), pyrazynyl (pyrazine ring), pyrimidinyl (pyrimidine ring), pyridazinyl (pyridazine ring), indolyl (indole ring), isoindolyl (isoindole ring), benzoimidazolyl (benzimidazole ring), purinyl group (purine ring), quinolyl (quinoline ring), phtalazinyl (phtalazine ring), naphtyridinyl (naphtyridine ring), quinoxalinyl (quinoxaline ring), cinnolyl (cinnoline ring), pteridinyl (pteridine ring), oxazolyl (oxazole ring), isoxazolyl (isoxazole ring), benzoxazolyl (benzoxazole ring), benzothiazolyly (benzothiaziole ring), furazanyl (furazan ring) and the like.

"Heteroarylalkyl" includes heteroaryl-($C_1$-$C_3$-alkyl) group, wherein examples of heteroaryl are the same as those illustrated in the above definition, such as 2-furylmethyl group, 3-furylmethyl group, 2-thienylmethyl group, 3-thienylmethyl group, 1-imidazolylmethyl group, 2-imidazolylmethyl group, 2-thiazolylmethyl group, 2-pyridylmethyl group, 3-pyridylmethyl group, 1-quinolylmethyl group or the like.

"Solvate" refers to a complex of variable stoechiometry formed by a solute (e.g. a compound of formula I) and a solvent. The solvent is a pharmaceutically acceptable solvent as water preferably; such solvent may not interfere with the biological activity of the solute.

"Optionally" means that the subsequently described event(s) may or may not occur, and includes both event (s), which occur, and events that do not occur.

The term "substituted" refers to substitution with the named substituent or substituents, multiple degrees of substitution being allowed unless otherwise stated.

Preferred compounds of the present invention are compounds of formula I-C, I-D, I-E, II-A, II-B and II-C as defined in the claims or pharmaceutically acceptable salts, hydrates or solvates of such compounds.

Specifically preferred compounds are:

(4-Fluoro-phenyl)-{(S)-3-[3-(1H-pyrrol-2-yl)-[1,2,4]oxadiazol-5-yl]-piperidin-1-yl}-methanone
(2,4-Difluoro-phenyl)-{(S)-3-[3-(1H-pyrrol-2-yl)-[1,2,4]oxadiazol-5-yl]-piperidin-1-yl}-methanone
(3,4-Difluoro-phenyl)-{(S)-3-[3-(1H-pyrrol-2-yl)-[1,2,4]oxadiazol-5-yl]-piperidin-1-yl}-methanone
(6-Fluoro-pyridin-3-yl)-{(S)-3-[3-(1H-pyrrol-2-yl)-[1,2,4]oxadiazol-5-yl]-piperidin-1-yl}-methanone
(3,4-Difluoro-phenyl)-{3-[5-(1H-pyrrol-2-yl)-[1,2,4]oxadiazol-3-yl]-piperidin-1-yl}-methanone
(2,4-Difluoro-phenyl)-{3-[5-(1H-pyrrol-2-yl)-[1,2,4]oxadiazol-3-yl]-piperidin-1-yl}-methanone
(4-Fluoro-phenyl)-{3-[5-(1H-pyrrol-2-yl)-[1,2,4]oxadiazol-3-yl]-piperidin'-1-yl}-methanone
(6-Fluoro-pyridin-3-yl)-{3-[5-(1H-pyrrol-2-yl)-[1,2,4]oxadiazol-3-yl]-piperidin-1-yl}-methanone
(4-Fluoro-2-methyl-phenyl)-{3-[5-(1H-pyrrol-2-yl)-[1,2,4]oxadiazol-3-yl]-piperidin-1-yl}-methanone
(3,4-Difluoro-phenyl)-{(S)-3-[5-(1H-pyrrol-2-yl)-[1,2,4]oxadiazol-3-yl]-piperidin-1-yl}-methanone
(4-Fluoro-phenyl)-{3-[5-(1H-indol-2-yl)-[1,2,4]oxadiazol-3-yl]-piperidin-1-yl}-methanone
(2,4-Difluoro-phenyl)-{(S)-3-[3-(1H-indol-2-yl)-[1,2,4]oxadiazol-5-yl]-piperidin-1-yl}-methanone
(4-Fluoro-phenyl)-{3-[5-(2H-pyrazol-3-yl)-[1,2,4]oxadiazol-3-yl]-piperidin-1-yl}-methanone
(3,4-Difluoro-phenyl)-{3-[5-(2H-pyrazol-3-yl)-[1,2,4]oxadiazol-3-yl]-piperidin-1-yl}-methanone
(4-Fluoro-phenyl)-{3-[5-(1H-imidazol-2-yl)-[1,2,4]oxadiazol-3-yl]-piperidin-1-yl}-methanone
(3,4-Difluoro-phenyl)-{(S)-3-[3-(1H-indol-2-yl)-[1,2,4]oxadiazol-5-yl]-piperidin-1-yl}-methanone
(4-Fluoro-phenyl)-{(S)-3-[3-(1H-indol-2-yl)-[1,2,4]oxadiazol-5-yl]-piperidin-1-yl}-methanone
(3,4-diFluoro-phenyl)-{3-[5-(1H-imidazol-2-yl)-[1,2,4]oxadiazol-3-yl]-piperidin-1-yl}-methanone.
{(S)-3-[3-(1H-Indol-2-yl)-[1,2,4]oxadiazol-5-yl]-piperidin-1-yl}-(5-methyl-isoxazol-4-yl)-methanone
(5-Methyl-isoxazol-4-yl)-{(S)-3-[3-(1H-pyrrol-2-yl)-[1,2,4]oxadiazol-5-yl]-piperidin-1-yl}-methanone
(6-Fluoro-pyridin-3-yl)-{(S)-3-[5-(1H-pyrrol-2-yl)-[1,2,4]oxadiazol-3-yl]-piperidin-1-yl}-methanone
(4-Fluoro-phenyl)-{(S)-3-[5-(1H-pyrrol-2-yl)-[1,2,4]oxadiazol-3-yl]-piperidin-1-yl}-methanone
(6-Fluoro-pyridin-3-yl)-{3-[5-(1H-indol-2-yl)-[1,2,4]oxadiazol-3-yl]-piperidin-1-yl}-methanone
(4-Fluoro-phenyl)-{(S)-3-[3-(1H-imidazol-2-yl)-[1,2,4]oxadiazol-5-yl]-piperidin-1-yl}-methanone
(3,4-Difluoro-phenyl)-{(S)-3-[3-(1H-imidazol-2-yl)-[1,2,4]oxadiazol-5-yl]-piperidin-1-yl}-methanone
{3-[5-(1H-Indol-2-yl)-[1,2,4]oxadiazol-3-yl]-piperidin-1-yl}-(5-methyl-isoxazol-4-yl)-methanone
(4-Fluoro-phenyl)-{(S)-3-[5-(4-methyl-1H-pyrrol-2-yl)-[1,2,4]oxadiazol-3-yl]-piperidin-1-yl}-methanone
(6-Fluoro-pyridin-3-yl)-{(S)-3-[5-(4-methyl-1H-pyrrol-2-yl)-[1,2,4]oxadiazol-3-yl]-piperidin-1-yl}-methanone
(5-Methyl-isoxazol-4-yl)-{(S)-3-[5-(4-methyl-1H-pyrrol-2-yl)-[1,2,4]oxadiazol-3-yl]-piperidin-1-yl}-methanone
(2-Fluoro-pyridin-4-yl)-{(S)-3-[5-(4-methyl-1H-pyrrol-2-yl)-[1,2,4]oxadiazol-3-yl]-piperidin-1-yl}-methanone
(4-Fluoro-phenyl)-{(S)-3-[3-(4-methyl-1H-pyrrol-2-yl)-[1,2,4]oxadiazol-5-yl]-piperidin-1-yl}-methanone
(3,4-Difluoro-phenyl)-{(S)-3-[3-(4-methyl-1H-pyrrol-2-yl)-[1,2,4]oxadiazol-5-yl]-piperidin-1-yl}-methanone
(6-Fluoro-pyridin-3-yl)-{(S)-3-[3-(4-methyl-1H-pyrrol-2-yl)-[1,2,4]oxadiazol-5-yl]-piperidin-1-yl}-methanone
(2-Fluoro-pyridin-4-yl)-{(S)-3-[3-(4-methyl-1H-pyrrol-2-yl)-[1,2,4]oxadiazol-5-yl]-piperidin-1-yl}-methanone
(5-Methyl-isoxazol-4-yl)-{(S)-3-[3-(4-methyl-1H-pyrrol-2-yl)-[1,2,4]oxadiazol-5-yl]-piperidin-1-yl}-methanone
(4-Fluoro-phenyl)-{(S)-3-[5-(4-nitro-1H-pyrrol-2-yl)-[1,2,4]oxadiazol-3-yl]-piperidin-1-yl}-methanone
(4-Fluoro-phenyl)-{(R)-3-[3-(1H-pyrrol-2-yl)-[1,2,4]oxadiazol-5-yl]-piperidin-1-yl}-methanone

(4-Fluoro-phenyl)-{(S)-3-[5-(5-methyl-1H-pyrrol-2-yl)-[1,2,4]oxadiazol-3-yl]-piperidin-1-yl}-methanone
{(S)-3-[5-(4-Chloro-1H-pyrrol-2-yl)-[1,2,4]oxadiazol-3-yl]-piperidin-1-yl}-(4-fluorophenyl)-methanone
{(S)-3-[5-(4-Chloro-1H-pyrrol-2-yl)-[1,2,4]oxadiazol-3-yl]-piperidin-1-yl}-(6-fluoro-pyridin-3-yl)-methanone
{(S)-3-[5-(4-Chloro-1H-pyrrol-2-yl)-[1,2,4]oxadiazol-3-yl]-piperidin-1-yl}-(2-fluoro-pyridin-4-yl)-methanone
{(S)-3-[5-(4-Chloro-1H-pyrrol-2-yl)-[1,2,4]oxadiazol-3-yl]-pipefidin-1-yl}}-(5-methyl-isoxazol-4-yl)-methanone
{(S)-3-[3-(4-Chloro-1H-pyrrol-2-yl)-[1,2,4]oxadiazol-5-yl]-piperidin-1-yl}-(4-fluorophenyl)-methanone
{(S)-3-[5-(4-Bromo-1H-pyrrol-2-yl)-[1,2,4]oxadiazol-3-yl]-piperidin-1-yl}-(6-fluoro-pyridin-3-yl)-methanone
{(S)-3-[3-(4-Bromo-1H-pyrrol-2-yl)-[1,2,4]oxadiazol-5-yl]-piperidin-1-yl}-(4-fluorophenyl)-methanone
{(S)-3-[3-(4-Bromo-1H-pyrrol-2-yl)-[1,2,4]oxadiazol-5-yl]-piperidin-1-yl}-(6-fluoro-pyridin-3-yl)-methanone
(4-Fluoro-phenyl)-{3-fluoro-3-[3-(1H-pyrrol-2-yl)-[1,2,4]oxadiazol-5-yl]-piperidin-1-yl}-methanone
(4-Fluoro-phenyl)-{(S)-3-[3-(4-fluoro-1H-pyrrol-2-yl)-[1,2,4]oxadiazol-5-yl]-piperidin-1-yl}-methanone
(3,4-Difluoro-phenyl)-{(S)-3-[3-(4-fluoro-1H-pyrrol-2-yl)-[1,2,4]oxadiazol-5-yl]-piperidin-1-yl}-methanone
(6-Fluoro-pyridin-3-yl)-{(S)-3-[3-(4-fluoro-1H-pyrrol-2-yl)-[1,2,4]oxadiazol-5-yl]-piperidin-1-yl}-methanone
(2-Fluoro-pyridin-4-yl)-{(S)-3-[3-(4-fluoro-1H-pyrrol-2-yl)-[1,2,4]oxadiazol-5-yl]-piperidin-1-yl}-methanone
(4-Fluoro-phenyl)-{(S)-3-[5-(11H-pyrrol-2-yl)-tetrazol-2-yl]-piperidin-1-yl}-methanone
(4-Fluoro-phenyl)-{(S)-3-[5-(4-trifluoromethyl-1H-imidazol-2-yl)-[1,2,4]oxadiazol-3-yl]-piperidin-1-yl}-methanone
(6-Fluoro-pyridin-3-yl)-{(S)-3-[5-(4-isopropyl-1H-pyrrol-2-yl)-[1,2,4]oxadiazol-3-yl]-piperidin-1-yl}-methanone
(3-Fluoro-pyridin-4-yl)-{(S)-3-[5-(4-methyl-1H-pyrrol-2-yl)-[1,2,4]oxadiazol-3-yl]-piperidin-1-yl}-methanone
{(S)-3-[5-(4-Chloro-1H-pyrrol-2-yl)-[1,2,4]oxadiazol-3-yl]-piperidin-1-yl}-(3-fluoro-pyridin-4-yl)-methanone
(2-Fluoro-pyridin-4-yl)-{(S)-3-[5-(4-fluoro-1H-pyrrol-2-yl)-[1,2,4]oxadiazol-3-yl]-piperidin-1-yl}-methanone
{(S)-3-[5-(4-Bromo-1H-pyrrol-2-yl)-[1,2,4]oxadiazol-3-yl]-piperidin-1-yl}-(3-fluoro-pyridin-4-yl)-methanone
(3-Fluoro-pyridin-4-yl)-{(S)-3-[5-(4-fluoro-1H-pyrrol-2-yl)-[1,2,4]oxadiazol-3-yl]-piperidin-1-yl}-methanone
(4-Fluoro-phenyl)-{(S)-3-[5-(4-fluoro-1H-pyrrol-2-yl)-[1,2,4]oxadiazol-3-yl]-piperidin-1-yl}-methanone
(6-Fluoro-pyridin-3-yl)-{(S)-3-[5-(4-fluoro-1H-pyrrol-2-yl)-[1,2,4] oxadiazol-3-yl]-piperidin-1-yl}-methanone
{(S)-3-[3-(4-Chloro-1H-pyrrol-2-yl)-[1,2,4]oxadiazol-5-yl]-piperidin-1-yl}-(6-fluoro-pyridin-3-yl)-methanone
{(S)-3-[3-(4-Chloro-1H-pyrrol-2-yl)-[1,2,4]oxadiazol-5-yl]-piperidin-1-yl}-(2-fluoro-pyridin-4-yl)-methanone
{(S)-3-[3-(4-Chloro-1H-pyrrol-2-yl)-[1,2,4]oxadiazol-5-yl]-piperidin-1-yl}-(3-fluoro-pyridin-4-yl)-methanone
{(S)-3-[3-(4-Chloro-1H-pyrrol-2-yl)-[1,2,4]oxadiazol-5-yl]-piperidin-1-yl}-(5-methyl-isoxazol-4-yl)-methanone
{(S)-3-[3-(4-Bromo-1H-pyrrol-2-yl)-[1,2,4]oxadiazol-5-yl]-piperidin-1-yl}-(3-fluoro-pyridin-4-yl)-methanone
(3-Fluoro-pyridin-4-yl)-{(S)-3-[3-(4-fluoro-1H-pyrrol-2-yl)-[1,2,4]oxadiazol-5-yl]-piperidin-1-yl} -methanone
(3-Fluoro-pyridin-4-yl)-{(S)-3-[3-(4-methyl-1H-pyrrol-2-yl)-[1,2,4]oxadiazol-5-yl]-piperidin-1-yl}-methanone
(4-Fluoro-phenyl)-{(S)-3-[5-(4-trifluoromethyl-1H-pyrrol-2-yl)-[1,2,4] oxadiazol-3-yl]-piperidin-1-yl}-methanone
(3-Fluoro-pyridin-4-yl)-{(S)-3-[5-(4-trifluoromethyl-1H-pyrrol-2-yl)[1,2,4]oxadiazol-3-yl]-piperidin-1-yl}-methanone
(6-Fluoro-pyridin-3-yl)-{(S)-3-[5-(4-trifluoromethyl-1H-pyrrol-2-yl)-[1,2,4]oxadiazol-3-yl]-piperidin-1-yl}-methanone
(3,4-Difluoro-phenyl)-{(S)-3-[3-(4-methyl-1H-imidazol-2-yl)-[1,2,4]oxadiazol-5-yl]-piperidin-1-yl}-methanone
{(S)-3-[5-(4-Chloro-1H-pyrrol-2-yl)-[1,2,4]oxadiazol-3-yl]-piperidin-1-yl}-pyridin-4-yl-methanone
(6-Fluoro-pyridin-3-yl)-{(S)-3-[3-(4-trifluoromethyl-1H-pyrrol-2-yl)-[1,2,4]oxadiazol-5-yl]-piperidin-1-yl}-methanone.

The present invention relates to the pharmaceutically acceptable acid addition salts of compounds of the formula I or pharmaceutically acceptable carriers or excipients.

The present invention relates to compounds and compositions treating or preventing a condition in a mammal, including a human, the treatment or prevention of which is affected or facilitated by the neuromodulatory effect of mGluR5 allosteric modulators and particularly positive allosteric modulators.

The present invention relates to compounds and compositions useful for treating or preventing peripheral and central nervous system disorders such as tolerance or dependence, anxiety, depression, psychiatric disease such as psychosis, inflammatory or neuropathic pain, memory impairment, Alzheimer's disease, ischemia, drug abuse and addiction.

The present invention relates to pharmaceutical compositions which provide from about 0.01 to 1000 mg of the active ingredient per unit dose. The compositions may be administered by any suitable route. For example orally in the form of capsules, parenterally in the form of solutions for injection, topically in the form of onguents or lotions, ocularly in the form of eye-lotion, rectally in the form of suppositories.

The pharmaceutical formulations of the invention may be prepared by conventional methods in the art; the nature of the pharmaceutical composition employed will depend on the desired route of administration. The total daily dose usually ranges from about 0.05 - 2000 mg.

METHODS OF SYNTHESIS

Compounds of general formula I-B may be prepared by methods known in the art of organic synthesis as set

forth in part by the following synthesis schemes. In all of the schemes described below, it is well understood that protecting groups for sensitive or reactive groups are employed where necessary in accordance with general principles of chemistry. Protecting groups are manipulated according to standard methods of organic synthesis (Green T.W. and Wuts P.G.M. (1991) Protecting Groups in Organic Synthesis, John Wiley et Sons ). These groups are removed at a convenient stage of the compound synthesis using methods that are readily apparent to those skilled in the art. The selection of process as well as the reaction conditions and order of their execution shall be consistent with the preparation of compounds of formula I-B.

The compound may be represented as a mixture of enantiomers, which may be resolved into the individual pure *R*- or *S*-enantiomers. If for instance, a particular enantiomer of the compound is desired, it may be prepared by asymmetric synthesis, or by derivation with a chiral auxiliary, where the resulting diastereomeric mixture is separated and the auxiliary group cleaved to provide the pure desired enantiomers. Alternatively, where the molecule contains a basic functional group such as amino, or an acidic functional group such as carboxyl, this resolution may be conveniently performed by fractional crystallization from various solvents, of the salts of the compounds with optical active acid or by other methods known in the literature, e.g. chiral column chromatography.

Resolution of the final product, an intermediate or a starting material may be performed by any suitable method known in the art as described by Eliel E.L., Wilen S.H. and Mander L.N. (1984) Stereochemistry of Organic Compounds, Wiley-Interscience.

Many of the heterocyclic compounds of formula I-B can be prepared using synthetic routes well known in the art (Katrizky A.R. and. Rees C.W. (1984) Comprehensive Heterocyclic Chemistry, Pergamon Press).

The product from the reaction can be isolated and purified employing standard techniques, such as extraction, chromatography, crystallization, distillation, and the like.

The compounds of formula **I-B** wherein W is a 3-substituted piperidine ring may be prepared according to the synthetic sequences illustrated in the Schemes 1-4.

Wherein

P is an heterocyclic ring with an N-H function as defined above
Q is aryl or heteroaryl as described above
B represents -C(=O)-($C_0$-$C_2$)alkyl-.

The starting material amidoxime can be prepared by methods known in the art of organic synthesis as set forth in part by the following synthesis Scheme 1.

Scheme 1

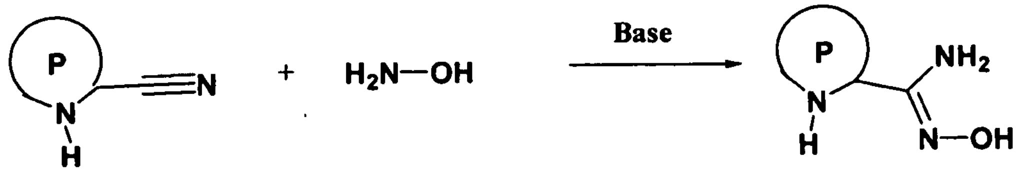


In turn, a nitrile derivative (for example 4-fluoro-benzylnitrile) is reacted with hydroxylamine under neutral or basic conditions such as triethylamine, diisopropyl-ethylamine, sodium carbonate, sodium hydroxide and the like in a suitable solvent (*e.g.* methyl alcohol, ethyl alcohol). The reaction typically proceeds by allowing the reaction temperature to warm slowly from ambient temperature to a temperature range of 70°C up to 80°C inclusive for a time in the range of about 1 hour up to 48 hours inclusive (see for example Lucca, George V. De; Kim, Ui T.; Liang, Jing; Cordova, Beverly; Klabe, Ronald M.; et al; J.Med.Chem.; EN; 41; 13; 1998; 2411-2423, Lila, Christine; Gloanec, Philippe; Cadet, Laurence; Herve, Yolande; Fournier, Jean; et al.; Synth.Commun.; EN; 28; 23; 1998; 4419-4430 and see: Sendzik, Martin; Hui, Hon C.; Tetrahedron Lett.; EN; 44; 2003; 8697-8700 and references therein for reaction under neutral conditions).

Scheme 2

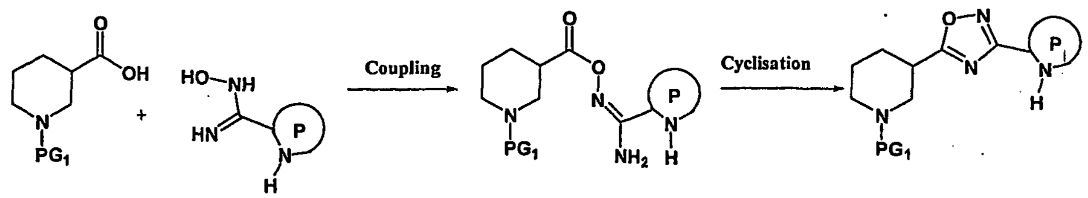


The substituted amidoxime derivative (described in the Scheme 1) may be converted to an acyl-amidoxime derivative using the approach outlined in the Scheme 2. In the Scheme 2, $PG_1$ is an amino protecting group such as tert-Butyloxycarbonyl, Benzyloxycarbonyl, Ethoxycarbonyl, Benzyl and the like. The coupling reaction may be promoted by coupling agents known in the art of organic synthesis such as EDCI (1-(3-Dimethylaminopropyl)-3-ethylcarbodiimide), DCC (N,N'-Dicyclohexylcarbodiimide), in the presence of a suitable base such as triethylamine, diisopropyl-ethylamine, in a suitable solvent (e.g. tetrahydrofuran, dichloromethane, N,N-dimethylformamide, dioxane). Typically, a co-catalyst such as HOBT (Hydroxy-benzotriazole), HOAT (1-Hydroxy-7-azabenzotriazole) may also be present in the reaction mixture. The reaction typically proceeds at a temperature in the range of ambient temperature up to 60°C inclusive for a time in the range of about 2 hoursup to 12 hours to produce the intermediate acyl-amidoxime. The cyclisation reaction may be effected thermally in a temperature range of about 80°C up to about 150°C for a time in the range of about 2 hours up to 18 hours (see for example Suzuki, Takeshi; Iwaoka, Kiyoshi; Imanishi, Naoki; Nagakura, Yukinori; Miyata, Keiji; et al.; Chem.Pharm.Bull.; EN; 47; 1; 1999; 120 - 122). The product from the reaction can be isolated and purified employing standard techniques, such as extraction, chromatography, crystallization, distillation, and the like.

The final step may be effected either by a process described in the Scheme 3 or by a process described in the Scheme 4.

Scheme 3

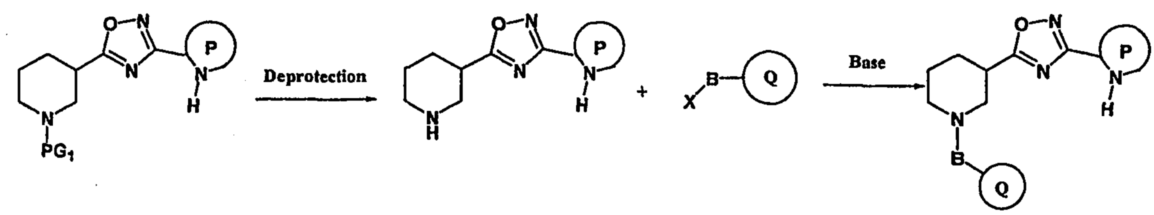


As shown in the Scheme 3, protecting groups $PG_1$ are removed using standard methods. In the Scheme 3, B is as defined above, X is halogen, for example the piperidine derivative is reacted with an aryl or heteroaryl acyl chloride using method that are readily apparent to those skilled in the art. The reaction may be promoted by a base such as triethylamine, diisopropylamine, pyridine in a suitable solvent (*e.g.* tetrahydrofuran, dichloromethane). The reaction typically proceeds by allowing the reaction temperature to warm slowly from 0°C up to ambient temperature for a time in the range of about 4 up to 12 hours.

Scheme 4

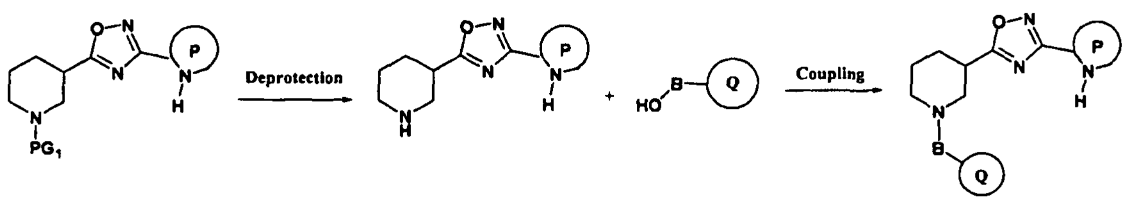


As shown in the Scheme 4, protecting groups $PG_1$ are removed using standard methods. The coupling reaction may be promoted by coupling agents known in the art of organic synthesis such as EDCI (1-(3-Dimethylaminopropyl)-3-ethylcarbodiimide), DCC (N,N'-Dicyclohexyl-carbodiimide) or by polymer-supported coupling agents such as polymer-supported carbodiimide (PS-DCC, ex Argonaut Technologies), in the presence of a suitable base such as triethylamine, diisopropyl-ethylamine, in a suitable solvent (e.g. tetrahydrofuran, dichloromethane, N,N-dimethylformamide, dioxane). Typically, a co-catalyst such as HOBT (1-Hydroxy-benzotriazole), HOAT (1-Hydroxy-7-azabenzotriazole) and the like may also be present in the reaction mixture. The reaction typically proceeds at ambient temperature for a time in the range of about 2 hours up to 12 hours.

The compounds of formula **II-B** wherein J is a CH2 and R1, R2 are H may be prepared according to the synthetic sequences illustrated in Scheme 5.

Wherein

P is a heterocyclic ring with an N-H function as defined above
Q is aryl or heteroaryl as described above
B represents -C(=O)-($C_0$-$C_2$)alkyl-.

The oxadiazole ring described below is prepared following synthetic routes well known in the art (Katrizky A.R. and Rees C.W. (1984) Comprehensive Heterocyclic Chemistry, Pergamon Press).

Scheme 5

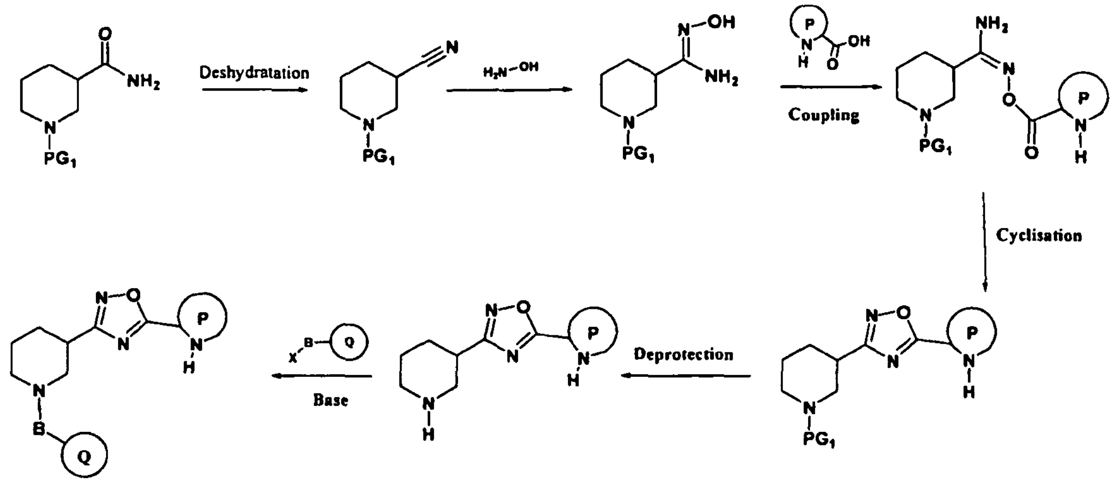


The starting nitrile derivative is reacted with hydroxylamine under neutral or basic conditions such as triethylamine, diisopropyl-ethylamine, sodium carbonate, sodium hydroxide and the like in a suitable solvent (*e.g.* methyl alcohol, ethyl alcohol). The reaction typically proceeds by allowing the reaction temperature to warm slowly from ambient temperature to a temperature range of 70°C up to 80°C inclusive for a time in the range of about 1 hour up to 48 hours inclusive (see for example. Lucca, George V. De; Kim, Ui T.; Liang, Jing; Cordova, Beverly; Klabe, Ronald M.; et al; J.Med.Chem.; EN; 41; 13; 1998; 2411-2423, Lila, Christine; Gloanec, Philippe; Cadet, Laurence; Herve, Yolande; Fournier, Jean; et al.; Synth.Commun.; EN; 28; 23; 1998; 4419-4430 and see: Sendzik, Martin; Hui, Hon C.; Tetrahedron Lett.; EN; 44; 2003; 8697-8700 and references therein for reaction under neutral conditions).

The substituted amidoxime derivative (described in the Scheme 5) may be converted to an acyl-amidoxime derivative using the approach outlined in the Scheme 1. In the Scheme 1, $PG_1$ is an amino protecting group such as *tert*-Butyloxycarbonyl, Benzyloxycarbonyl, Ethoxycarbonyl, Benzyl and the like. The coupling reaction may be promoted by coupling agents known in the art of organic synthesis such as EDCI (1-(3-Dimethylaminopropyl)-3-ethylcarbodiimide), DCC (N,N'-Dicyclohexylcarbodiimide), in the presence of a suitable base such as triethylamine, diisopropyl-ethylamine, in a suitable solvent (e.g. tetrahydrofuran, dichloromethane, N,N-dimethylformamide, dioxane). Typically, a co-catalyst such as HOBT (Hydroxy-benzotriazole), HOAT (1-Hydroxy-7-azabenzotriazole) may also be present in the reaction mixture. The reaction typically proceeds at a temperature in the range of ambient temperature up to 60°C inclusive for a time in the range of about 2 hoursup to 12 hours to produce the intermediate acyl-amidoxime. The cyclisation reaction may be performed thermically by warming the reaction mixture without the purification of the acyl-amidoxime intermediate in a temperature range of about 80°C up to about 150°C for a time in the range of about 2 hours up to 18 hours (see for example Suzuki, Takeshi; Iwaoka, Kiyoshi; Imanishi, Naoki; Nagakura, Yukinori; Miyata, Keiji; et al.; Chem.Pharm.Bull.; EN; 47; 1; 1999; 120 - 122). Otherwise the acyl-amidoxime can be isolated and purified employing standard techniques and then cyclised. The cyclization reaction is tipically carried out under basic condition such as triethylamine, diisopropyl-ethylamine, sodium carbonate, sodium hydroxide and the like in a suitable solvent (*e.g.* acetonitrile, dioxane). The reaction typically proceeds in temperature range of about 80°C up to about 150°C for a time in the range of about 2 hours up to 18 hours.

The product from the reaction can be isolated and purified employing standard techniques, such as extraction, chromatography, crystallization, distillation, and the like.

Then, the protecting group $PG_1$ is removed using standard methods. In the Scheme 5, B is as defined above, X is halogen or hydroxyl; for example the piperidine derivative is reacted with an aryl or heteroaryl acyl chloride using method that are readily apparent to those skilled in the art. The reaction may be promoted by a base such as triethylamine, diisopropylamine, pyridine in a suitable solvent (*e.g.* tetrahydrofuran, dichloromethane). The reaction typically proceeds by allowing the reaction temperature to warm slowly from 0°C up to ambient temperature for a time in the range of about 4 up to 12 hours.

When X is OH, the coupling reaction may be promoted by coupling agents known in the art of organic synthesis such as EDCI (1-(3-dimethylaminopropyl)-3-ethylcarbodiimide), DCC (N,N'-dicyclohexyl-carbodiimide) or by polymer-supported coupling agents such as polymer-supported carbodiimide (PS-DCC, ex Argonaut Technologies), in the presence of a suitable base such as triethylamine, diisopropyl-ethylamine, in a suitable solvent (e.g. tetrahydrofuran, dichloromethane, N,N-dimethylformamide, dioxane). Typically, a co-catalyst such as HOBT (1-hydroxy-benzotriazole), HOAT (1-hydroxy-7-azabenzotriazole) and the like may also be present in the reaction mixture. The reaction typically proceeds at ambient temperature for a time in the range of about 2 hours up to 12 hours.

The compounds of the invention which are basic in nature can form a wide variety of different pharmaceutically acceptable salts with various inorganic and organic acids. These salts are readily prepared by treating the base compounds with a substantially equivalent amount of the chosen mineral or organic acid in a suitable organic solvent such as methanol, ethanol or isopropanol (see Stahl P.H., Wermuth C.G., Handbook of Pharmaceuticals Salts, Properties, Selection and Use, Wiley, 2002).

The following examples illustrate the invention. The physical data given for the compounds exemplified is consistent with the assigned structure of those compounds.

EXAMPLES

Unless otherwise noted, all starting materials were obtained from commercial suppliers and used without further purification.

Specifically, the following abbreviation may be used in the examples and throughout the specification.

| | |
|---|---|
| g (grams) | rt (room temperature) |
| mg (milligrams) | MeOH (methanol) |
| mL (millilitres) | |
| μl (microliters) | Hz (Hertz) |
| M (molar) | LCMS (Liquid Chromatography Mass Spectrum) |
| MHz (megahertz) | HPLC (High Pressure Liquid Chromatography) |
| mmol (millimoles) | NMR (Nuclear Magnetic Resonance) |
| Min (minutes) | 1H (proton) |
| AcOEt (ethyl acetate) | $Na_2SO_4$ (sodium sulphate) |
| $K_2CO_3$ (potassium carbonate) | $MgSO_4$ (magnesium sulphate) |
| $CDCl_3$ (deuteriated chloroform) | HOBT (1-hydroxybenzotriazole) |
| EDCI.HCl (1-3(Dimethylaminopropyl)-3-ethylcarbodiimide, hydrochloride) | RT (Retention Time) |
| EtOH (ethyl alcohol) | NaOH (sodium hydroxide) |
| % (percent) | h (hour) |
| DCM (dichloromethane) | HCl (hydrochloric acid) |
| DIEA (diisopropyl ethyl amine) | n-BuLi (n-butyllithium) |
| Mp (melting point) | THF (tetrahydrofuran) |

All references to brine refer to a saturated aqueous solution of NaCI. Unless otherwise indicated, all temperatures are expressed in °C (degrees Centigrade). All reactions are conducted under an inert atmosphere at room temperature unless otherwise noted.

$^1$H NMR spectra were recorded on a Brucker 500MHz or on a Brucker 300MHz. Chemical shifts are expressed in parts of million (ppm, δ units). Coupling constants are in units of herts (Hz) Splitting patterns describe apparent multiplicities and are designated as s (singlet), d (doublet), t (triplet), q (quadruplet), quint (quintuplet), m (multiple).

LCMS were recorded under the following conditions:

Method A) Waters Alliance 2795 HT Micromass ZQ. Column Waters XTerra MS C 18 (50x4.6 mm, 2.5μm). Flow rate 1 mL/min Mobile phase: A phase = water/$CH_3CN$ 95/5 + 0.05% TFA, B phase = water/$CH_3CN$ = 5/95 + 0.05% TFA. 0-1 min (A: 95%, B: 5%), 1-4 min (A: 0%, B: 100%), 4-6 min (A: 0%, B: 100%), 6-6.1 min (A: 95%, B: 5%). T= 35°C; UV detection: Waters Photodiode array 996, 200-400nm.

Method B) Waters Alliance 2795 HT Micromass ZQ. Column Waters XTerra MS C18 (50x4.6 mm, 2.5μm). Flow rate 1.2 mL/min Mobile phase: A phase = water/$CH_3CN$ 95/5 + 0.05% TFA, B phase = water/$CH_3CN$ = 5/95 + 0.05% TFA. 0-0.8 min (A: 95%, B: 5%), 0.8-3.3 min (A: 0%, B: 100%), 3.3-5 min (A: 0%, B: 100%), 5-5.1 min (A: 95%, B: 5%). T= 35°C; UV detection: Waters Photodiode array 996, 200-400nm.

Method C): Pump 515, 2777 Sample Manager, Micromass ZQ Single quadrupole (Waters). Column 2.1x50mm stainless steel packed with 3.5μm SunFire RP C-18 (Waters); flow rate 0.25 mL/min splitting ratio MS :waste/ 1:4; mobile phase: A phase = water/acetonitrile 95/5 + 0.1% TFA, B phase = water/acetonitrile 5/95 + 0.1% TFA. 0-1.0min (A: 98%, B: 2%), 1.0-5.0min (A: 0%, B: 100%), 5.0-9.0min (A: 0%, B: 100%), 9.1-12min (A: 98%, B: 2%); UV detection wavelength 254 nm; Injection volume: 5μl

Method D) Waters Alliance 2795 HT Micromass ZQ. Column Waters Symmetry C18 (75x4.6 mm, 3.5μm). Flow rate 1.5 ml/min. Mobile phase: A phase = water/$CH_3CN$ 95/5 + 0.05% TFA, B phase = water/$CH_3CN$ = 5/95 + 0.05% TFA. 0-0.5 min (A: 95%, B: 5%), 0.5-7 min (A: 0%, B: 100%), 7-8 min (A: 0%, B: 100%), 8-8.1 min (A: 95%, B: 5%). T= 35°C; UV detection: Waters Photodiode array 996, 200-400nm.

Method E) Waters Alliance 2795 HT Micromass ZQ. Column Waters Symmetry C18 (75x4.6 mm, 3.5 μm). Flow rate 1.5 ml/min. Mobile phase: A phase = water/$CH_3CN$ 95/5 + 0.05% TFA, B phase = water/$CH_3CN$ = 5/95 + 0.05% TFA. 0-0.1 min (A: 95%, B: 5%), 6 min (A: 0%, B: 100%), 6-8 min (A: 0%, B: 100%), 8.1 min (A: 95%, B: 5%). T= 35°C; UV detection: Waters Photodiode array 996, 200-400nm.

Method F) Waters Alliance 2795 HT Micromass ZQ. Column Waters Symmetry C18 (75x4.6 mm, 3.5 μm). Flow rate 1.0 ml/min. Mobile phase: A phase = water/$CH_3CN$ 95/5 + 0.05% TFA, B phase = water/$CH_3CN$ = 5/95 + 0.05% TFA. 0-1 min (A: 95%, B: 5%), 11 min (A: 0%, B: 100%), 11-12 min (A: 0%, B: 100%), 12.1 min (A: 95%, B: 5%). T= 35°C; UV detection: Waters Photodiode array 996, 200-400nm.

Method G) Waters Alliance 2795 HT Micromass ZQ. Column Waters Atlantis C18 (75x4.6 mm, 3.0 μm). Flow rate 1.5 ml/min. Mobile phase: A phase = water/$CH_3CN$ 95/5 + 0.05% TFA, B phase = water/$CH_3CN$ = 5/95 + 0.05% TFA. 0-0.5 min (A: 95%, B: 5%), 5.5 min (A: 0%, B: 100%), 5.5-8 min (A: 0%, B: 100%), 8.1 min (A: 95%, B: 5%). T= 35°C; UV detection: Waters Photodiode array 996, 200-400nm.

Method H): UPLC system Waters Acquity, Micromass ZQ2000 Single quadrupole (Waters). Column 2.1*50mm stainless steel packed with 1.7μm Acquity UPLC-BEH; flow rate 0.50 ml/min; mobile phase: A phase = water/acetonitrile 95/5 + 0.05% TFA, B phase = water/acetonitrile 5/95 + 0.05% TFA. 0-0.1min (A: 95%, B: 5%), 1.6min (A: 0%, B: 100%), 1.6-1.9min (A: 0%, B: 100%), 2.4min (A: 95%, B: 5%); UV detection wavelenght 254 nm.

Method I): UPLC system Waters Acquity, Micromass ZQ2000 Single quadrupole (Waters). Column 2.1*50mm stainless steel packed with 1.7μm Acquity UPLC-BEH; flow rate 0.50 ml/min; mobile phase: A phase = water/acetonitrile 95/5 + 0.05% TFA, B phase = water/acetonitrile 5/95 + 0.05% TFA. 0-0.3min (A: 95%, B: 5%), 3.3min (A: 0%, B: 100%), 3.3-3.9min (A: 0%, B: 100%), 4.4min (A: 95%, B: 5%); UV detection wavelenght 254 nm.

Method L): UPLC system Waters Acquity, Micromass ZQ2000 Single quadrupole (Waters). Column 2.1*50mm stainless steel packed with 1.7μm Acquity UPLC-BEH; flow rate 0.50 ml/min; mobile phase: A phase = water/acetonitrile 95/5 + 0.05% TFA, B phase = water/acetonitrile 5/95 + 0.05% TFA. 0-0.1min (A: 95%, B: 5%), 3.1min (A: 0%, B: 100%), 3.1-3.9min (A: 0%, B: 100%), 4.4min (A: 95%, B: 5%); UV detection wavelenght 254 nm.

Method M) Waters Alliance 2795 HT Micromass ZQ. Column Waters Symmetry C18 (75x4.6 mm, 3.5μm). Flow rate 1.0 ml/min. Mobile phase: A phase = water/$CH_3CN$ 95/5 + 0.05% TFA, B phase = water/$CH_3CN$ = 5/95 + 0.05% TFA. 0-0.1 min (A: 95%, B: 5%), 9 min (A: 0%, B: 100%), 9-12 min (A: 0%, B: 100%), 12.1 min (A: 95%, B: 5%). T= 35°C; UV detection: Waters Photodiode array 996, 200-400nm.

Method N): HPLC system: Waters Acquity, MS detector: Waters ZQ2000. Column: Acquity UPLC-BEH C18 50x2,1mmx1.7um; flow rate 0.4 ml/min; mobile phase: A phase = water/acetonitrile 95/5 + 0.1 % TFA, B phase = water/acetonitrile 5/95 + 0.1 % TFA. 0-0.25min (A: 98%, B: 2%), 0.25-4.0min (A: 0%, B: 100%), 4.0-5.0min (A: 0%, B: 100%), 5.1-6min (A: 98%, B: 2%); UV detection wavelenght 254 nm. Method O): HPLC system: Waters Acquity, MS detector: Waters ZQ2000. Column: Acquity UPLC-BEH C18 50x2.1mmx1.7um; flow rate 0.6 ml/min; mobile

phase: A phase = water/acetonitrile 95/5 + 0.1% TFA, B phase = water/acetonitrile 5/95 + 0.1% TFA. 0-0.25min (A: 98%, B: 2%), 3.30min (A: 0%, B: 100%), 3.3-4.0min (A: 0%, B: 100%), 4.1min (A: 98%, B: 2%); UV detection wavelenght 254 nm.

Method P): HPLC system: Waters Acquity, MS detector: Waters ZQ2000. Column: Acquity UPLC-BEH C18 50x2.1mmx1.7um; flow rate 0.3 ml/min; mobile phase: A phase = water/acetonitrile 95/5 + 0.1% TFA, B phase = water/acetonitrile 5/95 + 0.1% TFA. 0-0.5min (A: 98%, B: 2%), 2.0min (A: 20%, B: 80%), 6.0min (A: 0%, B: 100%), 6.0-9.5min (A: 0%, B: 100%), 9.6min (A: 98%, B: 2%), 9.6-11.0min (A: 98%, B: 2%); UV detection wavelenght 254 nm.

Method Q): Pump 1525u (Waters), 2777 Sample Manager, Micromass ZQ2000 Single quadrupole (Waters); PDA detector: 2996 (Waters). Column 2.1*30mm stainless steel packed with 3.0μm Luna C18; flow rate 0.25 ml/min splitting ratio MS :waste/ 1:4; mobile phase: A phase = water/acetonitrile 95/5 + 0.1% TFA, B phase = water/acetonitrile 5/95 + 0.1% TFA. 0-1.0min (A: 98%, B: 2%), 1.0-5.0min (A: 0%, B: 100%), 5.0-9.0min (A: 0%, B: 100%), 9.1-12min (A: 98%, B: 2%); UV detection wavelenght 254 nm; Injection volume: 5μl.

Method R): Pump 1525u (Waters), 2777 Sample Manager, Micromass ZQ2000 Single quadrupole (Waters); PDA detector: 2996 (Waters). Column. Fusion RP-C18, 20x2mm x2um; flow rate 0.25 ml/min splitting ratio MS :waste/ 1:4; mobile phase: A phase = water/acetonitrile 95/5 + 0.1% TFA, B phase = water/acetonitrile 5/95 + 0.1% TFA. 0-1.0min (A: 98%, B: 2%), 1.0-5.0min (A: 0%, B: 100%), 5.0-9.0min (A: 0%, B: 100%), 9.1-12min (A: 98%, B: 2%); UV detection wavelenght 254 nm; Injection volume: 5μl.

Method S): Pump 1525u (Waters), 2777 Sample Manager, Micromass ZQ2000 Single quadrupole (Waters); PDA detector: 2996 (Waters). Column: Acquity UPLC-BEH C18 50x2.1mmx1.7um; flow rate 0.25 ml/min splitting ratio MS :waste/ 1:4; mobile phase: A phase = water/acetonitrile 95/5 + 0.1% TFA, B phase = water/acetonitrile 5/95 + 0.1% TFA. 0-1.0min (A: 98%, B: 2%), 1.0-5.0min (A: 0%, B: 100%), 5.0-9.0min (A: 0%, B: 100%), 9.1-12min (A: 98%, B: 2%); UV detection wavelenght 254 nm; Injection volume: 5μl.

Method T): Pump 1525u (Waters), 2777 Sample Manager, Micromass ZQ2000 Single quadrupole (Waters); PDA detector: 2996 (Waters).Column: Ascentis 100x2.1mm x 3um; flow rate 0.3 ml/min; mobile phase: A phase = water/acetonitrile 95/5 + 0.1% TFA, B phase = water/acetonitrile 5/95 + 0.1% TFA. 0-0.5min (A: 98%, B: 2%), 2.0min (A: 20%, B: 80%), 6.0min (A: 0%, B: 100%), 6.0-9.5min (A: 0%, B: 100%), 9.6min (A: 98%, B: 2%), 9.6-11.0min (A: 98%, B: 2%); UV detection wavelenght 254 nm.

All mass spectra were taken under electrospray ionisation (ESI) methods.

Most of the reaction were monitored by thin-layer chromatography on 0.25mm Macherey-Nagel silica gel plates (60F-2254), visualized with UV light. Flash column chromatography was performed on silica gel (220-440 mesh, Fluka).

Melting point determination was performed on a Buchi B-540 apparatus.

The microwave oven used is an apparatus from Biotage (Optimizer™) equipped with an internal probe that monitors reaction temperature and pressure, and maintains the desired temperature by computer control.

Example 1

(4-Fluoro-phenyl)-{(S)-3-[3-(1H-pyrrol-2-yl)-[1,2,4]oxadiazol-5-yl]-piperidin-1-yl}-methanone

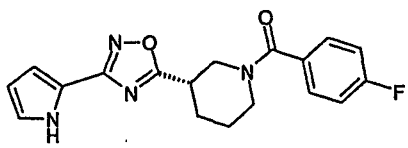

1 (A) (S)-3-[3-(1H-Pyrrol-2-yl)-[1,2,4]oxadiazol-5-yl]-piperidine-1-carboxylic acid tert-butyl ester

To a solution of 1H-Pyrrole-2-carbonitrile (0.110 mL, 1.3 mmol) in EtOH (2 mL), hydroxylamine (50% wt. aqueous solution, 0.318 mL, 5.2 mmol) was added at room temperature and the solution was stirred under reflux for 2 hours. The solvent was removed under reduced pressure to afford N-Hydroxy-1H-pyrrole-2-carboxamidine that was used immediately for the next step.

A mixture of N-Hydroxy-1H-pyrrole-2-carboxamidine (1.3 mmol), *S*-1-Boc-piperidine-3-carboxylic acid (0.3 g, 1.3 mmol), EDCI.HCl (0.374 g, 1.95 mmol) and HOBT (0.2 g, 1.3 mmol) in dioxane (6 mL) was stirred for 2h at room temperature, under nitrogen atmosphere, then the reaction mixture was heated under reflux for 7h. The solvent was evaporated under reduced pressure. The residue was diluted with water (20 mL) and DCM (20 mL), the phases were separated and the organic layer was washed sequentially with water (20 mL x 2 times) and with NaOH 1N (20 mL x 2

times). The organic layer was dried over $Na_2SO_4$ and concentrated under reduced pressure. Purification of the crude by flash chromatography (silica gel, eluent: DCM/MeOH/ 99/1/) gave 0.11 g of (S)-3-[3-(1H-Pyrrol-2-yl)-[1,2,4]oxadiazol-5-yl]-piperidine-1-carboxylic acid tert-butyl ester.
Yield: 26%; (brown oil); LCMS (RT): 5.45 min (Method A); MS (ES+) gave m/z: 318.2 (MH+).

1(B) (S)-3-[3-(1H-Pyrrol-2-yl)-[1,2,4]oxadiazol-5-yl]-piperidine hydrochloride

(S)-3-[3-(1H-Pyrrol-2-yl)-[1,2,4]oxadiazol-5-yl]-piperidine-1-carboxylic acid tert-butyl ester (0.11 g, 0.35 mmol) was dissolved in dioxane (2 mL) and 2 mL of HCl 4N (dioxane solution) were added dropwise at 0°C. The resulting mixture was stirred at room temperature for 1h. The solvent was evaporated under reduced pressure to afford 76 mg (yield: quantitative) of (S)-3-[3-(1H-Pyrrol-2-yl)-[1,2,4]oxadiazol-5-yl]-piperidine hydrochloride as a white solid.
Yield: quantitative; (brown solid); LCMS (RT): 0.65 min (Method A); MS (ES+) gave m/z: 218.2 (MH+).

1(C) (4-Fluoro-phenyl)-{(S)-3-[3-(1H-pyrrol-2-yl)-[1,2,4]oxadiazol-5-yl]-piperidin-1-yl}-methanone

To a suspension of (S)-3-[3-(1H-Pyrrol-2-yl)-[1,2,4]oxadiazol-5-yl]-piperidine hydrochloride (76 mg, 0.35 mmol) in dry dichloromethane (15 mL), triethylamine (0.12 mL, 0.87 mmol) and 4-fluorobenzoyl chloride (0.045 mL, 0.38 mmol) were added dropwise at 0°C. The reaction mixture was allowed to warm at room temperature and stirred under nitrogen atmosphere overnight. The solution was then treated with NaOH 1N (10 mL) and the phases were separated. The organic layer was washed with water (5 mL) and with brine (5 mL), then was dried over $Na_2SO_4$ and evaporated under reduced pressure. The crude was purified by flash chromatography (silica gel, eluent: DCM/MeOH/$NH_4OH$ 98:2:0.2) to give 80 mg of the title compound.
Yield: 58% (white powder); mp = 130-135°C; $[\alpha]_D^{20}$ = +118.13 (c=1.02, MeOH); LCMS (RT): 6.63 min (Method O); MS (ES+) gave m/z: 341.2 (MH+).
$^1$H-NMR (DMSO-$d_6$), δ (ppm): 11.52 (s br, 1H); 7.47 (dd, 2H); 7.23 (dd, 2H); 6.97 (m, 1H); 6.74 (m, 1H); 6.21 (m, 1H); 4.22 (m, 1H); 3.77 (m, 1H); 3.50 (dd, 1H); 3.35 (ddd, 1H); 3.27 (ddd, 1H); 2.24 (m, 1H); 1.96 (m, 1H); 1.82 (m, 1H); 1.63 (m, 1H).

Example 2

(2,4-Difluoro-phenyl)-{(S)-3-[3-(1H-pyrrol-2-yl)-[1,2,4]oxadiazol-5-yl]-piperidin-1-yl}-methanone

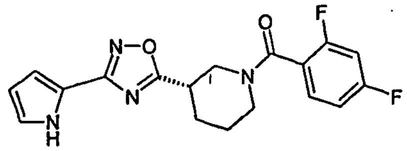

The compound was prepared following the procedure described in the Example 1(C), starting from (S)-3-[3-(1H-Pyrrol-2-yl)-[1,2,4]oxadiazol-5-yl]-piperidine hydrochloride (prepared as described in the Example 1(B)). The final compound was purified by preparative HPLC.
Yield 20% (brown oil); LCMS (RT): 6.59 min (Method Q); MS (ES+) gave m/z: 359.1 (MH+).
$^1$H-NMR (DMSO-$d_6$), δ (ppm): 11.53 (s br, 1H); 7.46 (ddd, 1H); 7.25 (ddd, 1H); 7.14 (ddd, 1H); 6.97 (m, 1H); 6.74 (m, 1H); 6.22 (m, 1H); 4.35 (s br, 1H); 3.91 (s br, 1H); 3.52 (dd, 1H); 3.40-3.18 (m, 2H); 2.24 (m, 1H); 1.97 (m, 1H); 1.82 (m, 1H); 1.62 (m, 1H).

Example 3

(3,4-Difluoro-phenyl)-{(S)-3-[3-(1H-pyrrol-2-yl)-[1,2,4]oxadiazol-5-yl]-piperidin-1-yl}-methanone

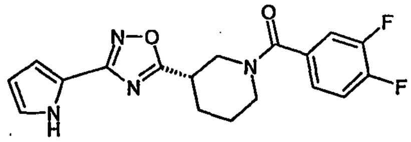

The compound was prepared following the procedure described in the Example 1(C), starting from (S)-3-[3-(1H-Pyrrol-2-yl)-[1,2,4]oxadiazol-5-yl]-piperidine hydrochloride (prepared as described in the.Example 1(B)). The final compound was purified by preparative HPLC.
Yield: 25% (brown oil); LCMS (RT): 6.65 min (Method Q); MS (ES+) gave m/z: 359.1 (MH+).
$^1$H-NMR (DMSO-$d_6$), δ (ppm): 11.54 (s br, 1H); 7.46 (m, 2H); 7.27 (m, 1H); 6.97 (m, 1H); 6.74 (m, 1H); 6.21 (m, 1H); 4.20 (m, 1H); 3.74 (m, 1H); 3.51 (dd, 1H); 3.41-3.23 (m, 2H); 2.24 (m, 1H); 1.95 (m, 1H); 1.82 (m, 1H); 1.64 (m, 1H).

Example 4

(6-Fluoro-pyridin-3-yl)-{(S)-3-[3-(1H-pyrrol-2-yl)-[1,2,4]oxadiazol-5-yl]-piperidin-1-yl}-methanone

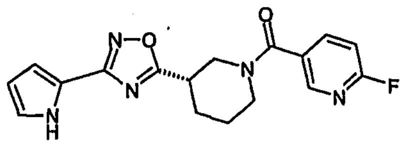

A mixture of (S)-3-[3-(1H-Pyrrol-2-yl)-[1,2,4]oxadiazol-5-yl]-piperidine hydrochloride (0.1 g, 0.39mmol, prepared as described in the Example 1(B)), 6-Fluoronicotinic acid (66 mg, 0.47 mmol), HOAT (80 mg, 0.59 mmol), PS-DCC (ex Argonaut Technologies, 0.66 g, 0.79 mmol, loading: 1.2 mmol/g) and TEA (0.14 mL, 1 mmol) in dry dichloromethane (10 mL) was kept overnight under orbital shaking (IKA Vibrax VXR). The resin was filtered off and washed repeatedly with dichloromethane; the filtrate was washed with HCl 1N (10 mL x 2 times), with NaOH 1N (aq.) (10 mL x 2 times) and with brine, then was dried over sodium sulphate and evaporated under reduced pressure. The crude was purified by flash chromatography (silica gel, eluent: AcOEt/ Hexane 7/3) to give 28 mg of (6-Fluoro-pyridin-3-yl)-{(S)-3-[3-(1H-pyrrol-2-yl)-[1,2,4]oxadiazol-5-yl]-piperidin-1-yl}-methanone.
Yield: 23% (white solid); mp= 131-132°C; $[\alpha]_D^{20}$= +45.54 (c=0.67, MeOH); LCMS (RT): 7.04 min (Method Q); MS (ES+) gave m/z: 342.2 (MH+).
$^1$H-NMR (DMSO-$d_6$), δ (ppm): 11.54 (s br, 1H); 8.32 (m, 1H); 8.03 (ddd, 1H); 7.22 (ddd, 1H); 6.97 (m, 1H); 6.74 (m, 1H); 6.22 (m, 1H); 4.22 (m, 1H); 3.76 (m, 1H); 3.55 (dd, 1H); 3.44-3.28 (m, 2H); 2.24 (m, 1H); 1.98 (m, 1H); 1.81 (m, 1H); 1.67 (m, 1H).

Example 5

(3,4-Difluoro-phenyl)-{3-[5-(1H-pyrrol-2-yl)-[1,2,4]oxadiazol-3-yl]-piperidin-1-yl}-methanone

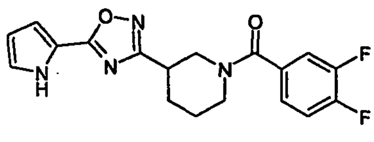

5(A) 3-Carbamoyl=piperidine-1-carboxylic acid tert-butyl ester

Triethylamine (0.96 mL, 6.89 mmol) and then ethyl chloroformate (0.69 mL, 7.23 mmol) were added dropwise at 0°C to a solution of 1-Boc-piperidine-3-carboxylic acid (1.58 g, 6.89 mmol) in chloroform (10 mL), under nitrogen atmosphere. After stirring 10 min at 0°C, $NH_3$ (gas) was bubbled into the solution for 1h. The reaction mixture was then stirred at room temperature for 3h, 5% $NaHCO_3$ (aq) was added and the phases were separated. The organic layer was

dried over sodium sulphate and evaporated under reduced pressure to afford the title compound, which was used for the next step without further purification.
Yield: quantitative; LCMS (RT): 3.31 min (Method A); MS (ES+) gave m/z: 229.0 (MH+).

5(B) 3-Cyano-piperidine-1-carboxylic acid tert-butyl ester

Phosphorus oxychloride (0.64 mL, 6.89 mmol) was added dropwise at 0°C to a solution of 3-carbamoyl-piperidine-1-carboxylic acid tert-butyl ester (1.58 g, 6.89 mmol) in pyridine (15 mL), under nitrogen atmosphere. After stirring overnight at room temperature, ethyl acetate was added and the solution was washed with 10% HCl (2 times). The phases were separated and the organics were dried over sodium sulphate and evaporated to dryness under reduced pressure.
The title compound was used for the next step without further purification.
Yield: quantitative; LCMS (RT): 4.48 min (Method A); MS (ES+) gave m/z: 211.1 (MH+).

5(C) 3-(N-Hydroxycarbamimidoyl)-piperidine-1-carboxylic acid tert-butyl ester

A solution of 3-cyano-piperidine-1-carboxylic acid tert-butyl ester (1.4 g, 6.89 mmol) and aqueous hydroxylamine (50% in water, 1.7 mL, 27.5 mmol) in ethanol (15 mL) was refluxed for 2h. The solvent was evaporated under reduced pressure to afford the title compound that was used for the next step without further purification. Yield: quantitative; LCMS (RT): 2.71 min (Method A); MS (ES+) gave m/z: 244.0 (MH+).

5(D) 3-[5-(1H-Pyrrol-2-yl)-[1,2,4]oxadiazol-3-yl]-piperidine-1-carboxylic acid tert-butyl ester

A mixture of 3-(N-hydroxycarbamimidoyl)-piperidine-1-carboxylic acid tert-butyl ester (0.4 g, 1.6 mmol), 1H-pyrrole-2-carboxylic acid (182 mg, 1.6 mmol), HOBT (248 mg, 1.6 mmol), EDCI.HCl (0.47 g, 2.5 mmol) and dry triethylamine (0.461 mL, 3.29 mmol) in dry dioxane (4 mL) was kept under stirring at ambient temperature for 20h, under nitrogen atmosphere. The reaction mixture was then refluxed for 5h and the solvent was evaporated under reduced pressure. The residue was diluted with water (15 mL) and ethyl acetate (15 mL), the phases were separated and the organic layer was washed sequentially with water (10 mL, twice), $Na_2CO_3$ 1N (10 mL, twice) and with brine. The organic layer was dried over sodium sulphate and the solvent was removed under vacuum to give a residue that was purified by flash chromatography (silica gel, eluent: petroleum ether/ethyl acetate 4:1) to give the pure title compound (110 mg).
Yield: 38%; LCMS (RT): 5.54 min (Method A); MS (ES+) gave m/z: 319.1 (MH+).

5(E) 3-[5-(1H-Pyrrol-2-yl)-[1,2,4]oxadiazol-3-yl]-piperidine hydrochloride

To a solution of 3-[5-(1H-Pyrrol-2-yl)-[1,2,4]oxadiazol-3-yl]-piperidine-1-carboxylic acid tert-butyl ester (0.110 g, 0.35 mmol) in dichloromethane (5 mL), 1.5 mL of HCl 4N (dioxane solution) were added at 0°C and the reaction mixture was allowed to warm at room temperature and stirred for 20 h. The solvent was evaporated under reduced pressure to give the title compound as a white solid, which was used for the next step without further purification.
Yield: quantitative; LCMS (RT): 2.25 min (Method A); MS (ES+) gave m/z: 219.1 (MH+).

5(F) (3,4-Difluoro-phenyl)-{3-[5-(1H-pyrrol-2-yl)-[1,2,4]oxadiazol-3-yl]-piperidin-1-yl}-methanone

To a suspension of 3-[5-(1H-Pyrrol-2-yl)-[1,2,4]oxadiazol-3-yl]-piperidinehydrochloride (88 mg, 0.35 mmol) in dry dichloromethane (5 mL), triethylamine (145 μL, 1 mmol) and 3,4-difluorobenzoyl chloride (52 μL, 0.4 mmol) were added dropwise at 0°C. The reaction mixture was allowed to warm at room temperature and stirred for 30 minutes under nitrogen atmosphere. The solution was then treated with water (5 mL) and the phases were separated. The organic layer was washed subsequently with HCl 0.5 N (10 mL, 2 times), 5% $NaHCO_3$ (10 mL, twice), then was dried over $Na_2SO_4$ and evaporated under reduced pressure. The crude was purified by flash chromatography (silica gel, eluent petroleum ether: AcOEt 1:1) to afford 49 mg of the title compound.
Yield: 70% (white solid); mp= 177°C; LCMS (RT): 6.88 min (Method Q); MS (ES+) gave m/z: 359.1 (MH+).
H-NMR (DMSO-$d_6$), δ (ppm): 12.02 (s br, 1H); 7.44 (m, 2H); 7.26 (m, 1H); 7.12 (dd, 1H); 6.96 (dd, 1H); 6.30 (dd, 1H); 4.22 (m, 1H); 3.80 (m, 1H); 3.34 (dd, 1H); 3.22 (ddd, 1H); 3.10 (m, 1H); 2.19 (m, 1H); 1.96-1.76 (m, 2H); 1.64 (m, 1H).

Example 6

(2,4-Difluoro-phenyl)-{3-[5-(1H-pyrrol-2-yl)-[1,2,4]oxadiazol-3-yl]-piperidin-1-yl}-methanone

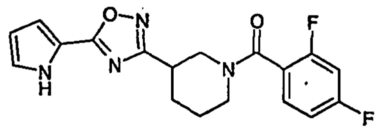

The compound was prepared following the procedure described in the Example 5(F), starting from 3-[5-(1H-pyrrol-2-yl)-[1,2,4]oxadiazol-3-yl]-piperidine hydrochloride (prepared as described in the Example 5(E)). Purification of the final compound was performed by flash chromatography on silica gel (eluent: Hexane: AcOEt 1:1)
Yield: 61% (white solid); mp= 151°C; LCMS (RT): 7.11 min (Method Q); MS (ES+) gave m/z: 359.1.
$^1$H-NMR (DMSO-$d_6$), δ (ppm): 12.02 (s br, 1H); 7.45 (m, 1H); 7.22 (m, 1H); 7.12 (m, 2H); 6.96 (d, 1H); 6.30 (dd, 1H); 4.57 (m br, 1H); 3.95 (m br, 1H); 3.44-3.13 (m, 2H); 3.05 (m, 1H); 2.19 (m, 1H); 1.96-1.74 (m, 2H); 1.59 (m, 1H).

Example 7

(4-Fluoro-phenyl)-{3-[5-(1H-pyrrol-2-yl)-[1,2,4]oxadiazol-3-yl]-piperidin-1-yl}-methanone

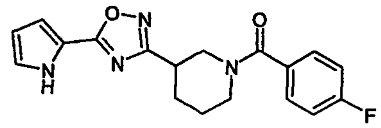

The compound was prepared following the procedure described in the Example 5(F), starting from 3-[5-(1H-pyrrol-2-yl)-[1,2,4]oxadiazol-3-yl]-piperidine hydrochloride (prepared as described in the Example 5(E)). Purification of the final compound was performed by flash chromatography on silica gel (eluent: Hexane: AcOEt 1:1)
Yield: 52% (white solid); mp= 158°C; LCMS (RT): 6.88 min (Method Q); MS (ES+) gave m/z: 341.2 (MH+).
$^1$H-NMR (DMSO-$d_6$), δ (ppm): 12.03 (s br, 1H); 7.47 (dd, 2H); 7.22 (dd, 2H); 7.12 (dd, 1H); 6.96 (dd, 1H); 6.30 (dd, 1H); 4.26 (m, 1H); 3.83 (m, 1H); 3.32 (dd, 1H); 3.19 (ddd, 1H); 3.08 (m, 1H); 2.19 (m, 1H); 1.96-1.76 (m, 2H); 1.63 (m, 1H).

Example 8

(6-Fluoro-pyridin-3-yl)-{3-[5-(1H-pyrrol-2-yl)-[1,2,4]oxadiazol-3-yl]-piperidin-1-yl}-methanone

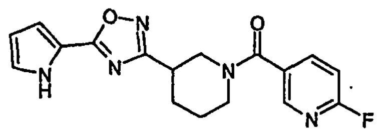

A mixture of 3-[5-(1H-pyrrol-2-yl)-[1,2,4]oxadiazol-3-yl]-piperidine hydrochloride (50 mg, 0.2 mmol; prepared as described in the Example 5(E)), 6-Fluoro-nicotinic acid (32 mg, 0.23 mmol), EDCI.HCl (56 mg, 0.3 mmol), HOBT (44 mg, 0.3 mmol) and TEA (0.083 mL, 0.59 mmol) in DCM (3 mL) was stirred overnight at room temperature, under nitrogen atmosphere. The solvent was evaporated under reduced pressure. The residue was diluted with water (5 mL) and ethyl acetate (10 mL), the phases were separated and the organic layer was washed with $Na_2CO_3$ 2N (5 mL x 2 times) and dried over $Na_2SO_4$. Evaporation of the solvent under reduced pressure gave a crude solid that was purified by flash chromatography on silica gel eluent petroleum ether/ethyl acetate 1:1).
Yield: 56% (white solid); mp= 143°C; LCMS (RT): 6.44 min (Method Q); MS (ES+) gave m/z: 342.1 (MH+).

$^1$H-NMR (DMSO-$d_6$), δ (ppm): 12.03 (s br, 1H); 8.31 (m, 1H); 8.02 (ddd, 1H); 7.21 (ddd, 1H); 7.13 (dd, 1H); 6.96 (dd, 1H); 6.30 (dd, 1H); 4.24 (m, 1H); 3.81 (m, 1H); 3.46-3.21 (m, 2H); 3.13 (m, 1H); 2.19 (m, 1H); 1.97-1.76 (m, 2H); 1.65 (m, 1H).

Example 9

(4-Fluoro-2-methyl-phenyl)-{3-[5-(1H-pyrrol-2-yl)-[1,2,4]oxadiazol-3-yl]-piperidin-1-yl}-methanone

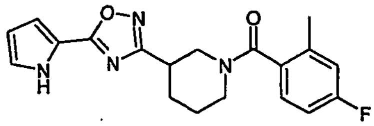

The compound was prepared following the procedure described in the Example 8, using 4-fluoro-2-methyl-benzoic acid as acid of choice and starting from 3-[5-(1H-pyrrol-2-yl)-[1,2,4]oxadiazol-3-yl]-piperidine hydrochloride (prepared as described in the Example 5(E)). Purification of the final compound was performed by flash chromatography on silica gel (eluent petroleum ether/ethyl acetate 1:1)
Yield: 43% (white solid); mp= 203°C; LCMS (RT): 6.68 min (Method Q); MS (ES+) gave m/z: 355.2 (MH+).
$^1$H-NMR (DMSO-$d_6$), δ (ppm): 12.02 (s br, 1H);. 7.22 (m, 1H); 7.15-6.92 (m, 4H); 6.30 (dd, 1H); 4.56 (m br, 1H); 3.79 (m br, 1H); 3.32 (dd, 1H); 3.21-2.99 (m, 2H); 2.24 (s, 3H); 2.19 (m, 1H); 1.96-1.72 (m, 2H); 1.58 (m, 1H).

Example 10

(3,4-Difluorophenyl)-{3-[5-(1H-pyrrol-2-yl)-[1,2,4]oxadiazol-3-yl]-piperidin-1-yl}-methanone

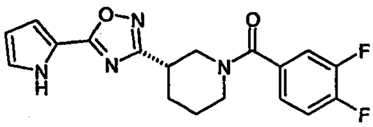

10(A) (S)-3-Carbamoyl-piperidine-1-carboxylic acid tert-butyl ester

Triethylamine (1.21mL, 8.72 mmol) and then ethyl chloroformate (0.8 mL, 8.30 mmol) were added dropwise at 0°C to a solution of (S)-1-Boc-piperidine-3-carboxylic acid (2 g, 8.72 mmol) in chloroform (40 mL), under nitrogen atmosphere. After stirring 10 min at 0°C, $NH_3$ (gas) was bubbled into the solution for 1h. The reaction mixture was then stirred at room temperature for 3h, 5% $NaHCO_3$ (aq) was added and the phases were separated. The organic layer was dried over sodium sulphate and evaporated under reduced pressure to afford the title compound, which was used for the next step without further purification.
Yield: quantitative; LCMS (RT): 3.31 min (Method A); MS (ES+) gave m/z: 229.0 (MH+).

10(B) (S)-3-Cyano-piperidine-1-carboxylic acid tert-butyl ester

Phosphorus oxychloride (812 μL, 8.72 mmol) was added dropwise at 0°C to a solution of (S)-3-carbamoyl-piperidine-1-carboxylic acid tert-butyl ester (2 g, 8.72 mmol) in pyridine (20 mL), under nitrogen atmosphere. After stirring overnight at room temperature, ethyl acetate was added and the solution was washed with 10% HCl (2 times). The phases were separated and the organics were dried over sodium sulphate and evaporated to dryness under reduced pressure. The title compound was used for the next step without further purification.
Yield: quantitative; LCMS (RT): 4.48 min (Method A); MS (ES+) gave m/z: 211.1 (MH+).

10(C) (S)-3-(N-Hydroxycarbamimidoyl)-piperidine-1-carboxylic acid tert-butyl ester

A solution of (S)-3-cyano-piperidine-1-carboxylic acid tert-butyl ester (1.8 g, 8.72 mmol) and aqueous hydroxylamine (50% in water, 2.1 mL, 34.88 mmol) in ethanol (20 mL) was refluxed for 2h. The solvent was evaporated under reduced pressure to afford the title compound that was used for the next step without further purification.
Yield: quantitative; LCMS (RT): 2.71 min (Method A); MS (ES+) gave m/z: 244.0 (MH+).

10(D) (S)-3-[5-(1H-Pyrrol-2-yl)-[1,2,4]oxadiazol-3-yl]-piperidine-1-carboxylic acid tert-butyl ester

A mixture of (S)-3-(N-hydroxycarbamimidoyl)-piperidine-1-carboxylic acid tert-butyl ester (0.4 g, 1.6 mmol), prepared as described in Example 10(C), 1H-pyrrole-2-carboxylic acid (182 mg, 1.6 mmol), HOBT (248 mg, 1.6 mmol), EDCI.HCl (0.47 g, 2.5 mmol) and dry triethylamine (0.461 mL, 3.29 mmol) in dry dioxane (4 mL) was kept under stirring at ambient temperature for 20h, under nitrogen atmosphere. The reaction mixture was then refluxed for 5h and the solvent was evaporated under reduced pressure. The residue was diluted with water (15 mL) and ethyl acetate (15 mL), the phases were separated and the organic layer was washed sequentially with water (10 mL, twice), 1N $Na_2CO_3$ (10 mL, twice) and with brine. The organic layer was dried over sodium sulphate and the solvent was removed under vacuum to give a residue that was purified by flash chromatography (silica gel, eluent: petroleum ether/ethyl acetate 4:1) to give the pure title compound (110 mg).
Yield: 35%; LCMS (RT): 5.55 min (Method A); MS (ES+) gave m/z: 319.1 (MH+).

10(E) (S)-3-[5-(1H-Pyrrol-2-yl)-[1,2,4]oxadiazol-3-yl]-piperidine hydrochloride

To a solution of (S)-3-[5-(1H-pyrrol-2-yl)-[1,2,4]oxadiazol-3-yl]-piperidine-1-carboxylic acid tert-butyl ester (0.110 g, 0.35 mmol) in dichloromethane (5 mL), 1.5 mL of 4N HCl (dioxane solution) were added at 0°C and the reaction mixture was allowed to warm at room temperature and stirred for 20 h. The solvent was evaporated under reduced pressure to give the title compound as a white solid, which was used for the next step without further purification.
Yield: quantitative; LCMS (RT): 2.25 min (Method A); MS (ES+) gave m/z: 219.1 (MH+).

10(F) (3,4-Difluoro-phenyl)-{(S)-3-[5-(1H-pyrrol-2-yl)-[1,2,4]oxadiazol-3-yl]-piperidin-1-yl}-methanone

To a suspension of (S)-3-[5-(1H-pyrrol-2-yl)-[1,2,4]oxadiazol-3-yl]-piperidine hydrochloride (88 mg, 0.35 mmol) in dry dichloromethane (5 mL), triethylamine (145 μL, 1 mmol) and 3,4-difluorobenzoyl chloride (52 μL, 0.4 mmol) were added dropwise at 0°C. The reaction mixture was allowed to warm at room temperature and stirred for 30 minutes under nitrogen atmosphere. The solution was then treated with water (5 mL) and the phases were separated. The organic layer was washed subsequently with 0.5 N HCl (10 mL, 2 times), 5% $NaHCO_3$ (10 mL, twice), then was dried over $Na_2SO_4$ and evaporated under reduced pressure. The crude was purified by flash chromatography (silica gel, eluent petroleum ether: AcOEt 1:1) to afford 49 mg of the title compound.
Yield: 48% (white solid); mp= 168°C; LCMS (RT): 6.42 min (Method Q); MS (ES+) gave m/z: 359.2 (MH+).
H-NMR (DMSO-$d_6$), δ (ppm): 12.02 (s br, 1H); 7.50-7.38 (m, 2H); 7.27 (m, 1H); 7.12 (dd, 1H); 6.96 (dd, 1H); 6.30 (dd, 1H); 4.22 (m, 1H); 3.80 (m, 1H); 3.34 (dd, 1H); 3.22 (ddd, 1H); 3.10 (ddd, 1H); 2.19 (m, 1H); 1.97-1.76 (m, 2H); 1.63 (m, 1H).

Example 11

(4-Fluoro-phenyl)-(3-[5-(1H-indol-2-yl)-[1,2,4]oxadiazol-3-yl]-piperidin-1-yl)-methanone

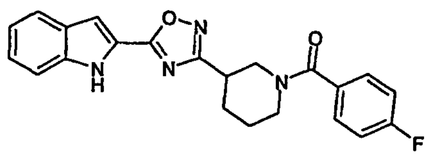

11(A) 3-[5-(1H-Indol-2-yl)-[1,2,4]oxadiazol-3-yl]-piperidine-1-carboxylic acid tert-butyl ester

A mixture of 3-(N-hydroxycarbamimidoyl)-piperidine-1-carboxylic acid tert-butyl ester (0.3 g, 1.2 mmol, prepared as described in Example 5(C)), 1H-indole-2-carboxylic acid (0.2 g, 1.2 mmol), HOBT (0.17 g, 1.2 mmol), EDCI.HCl (0.71

g, 3.7 mmol) and dry DIEA (0.631 mL, 3.7 mmol) in dry acetonitrile (10 mL) was warmed at 130°C for 30 minutes in a microwave oven. The solvent was evaporated under reduced pressure and then the residue was diluted with water (15 mL) and ethyl acetate (15 mL), the phases were separated and the organic layer was washed sequentially with water (10 mL, twice), 1N $Na_2CO_3$ (10 mL, twice) and with brine. The organic layer was dried over sodium sulphate and the solvent was removed under vacuum to give a residue that was purified by flash chromatography (silica gel, eluent: petroleum ether:ethyl acetate 4:1) to give the pure title compound (120 mg).
Yield: 27%; LCMS (RT): 6.47 min (Method A); MS (ES+) gave m/z: 369.1 (MH+).

11(B) 2-(3-Piperidin-3-yl-[1,2,4]oxadiazol-5-yl)-1H-indole hydrochloride

The compound was prepared following the procedure described in the Example 10(E) starting from 3-[5-(1H-indol-2-yl)-[1,2,4]oxadiazol-3-yl]-piperidine-1-carboxylic acid tert-butyl ester (prepared as described in Example 11(A)).
Yield: quantitative (white powder); LCMS (RT): 3.06 min (Method A); MS (ES+) gave m/z: 269.1 (MH+).

11(C) (4-Fluoro-phenyl)-{3-[5-(1H-indol-2-yl)-[1,2,4]oxadiazol-3-yl]-piperidin-1-yl}-methanone

The compound was prepared following the procedure described in the Example 10(F), using 2-(3-piperidin-3-yl-[1,2,4]oxadiazol-5-yl)-1H-indole hydrochloride (prepared as described in the Example 11(B)). Purification of the final compound was performed by flash chromatography on silica gel (eluent: Hexane: AcOEt 6:4)
Yield: 64% (white solid); mp= 199-201 °C; LCMS (RT): 7.28 min (Method Q); MS (ES+) gave m/z: 391.2 (MH+).
$^1$H-NMR (DMSO-$d_6$), δ (ppm): 12.04 (s br, 1H); 7.70 (dd, 1H); 7.53 (dd, 1H); 7.48 (dd, 2H); 7.34 (dd, 1H); 7.30 (ddd, 1H); 7.23 (dd, 2H); 7.13 (ddd, 1H); 4.31 (m, 1H); 3.85 (m, 1H); 3.38 (dd, 1H); 3.27-3.11 (m, 2H); 2.25 (m, 1H); 2.00-1.78 (m, 2H); 1.65 (m, 1H).

Example 12

(2,4-Difluoro-phenyl)-{(S)-3-[3-(1H-indol-2-yl)-[1,2,4]oxadiazol-5-yl]-piperidin-1-yl}-methanone

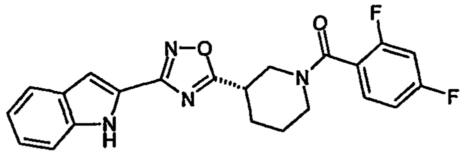

12 (A) (S)-3-[3-(1H-Indol-2-yl)-[1,2,4]oxadiazol-5-yl]-piperidine-1-carboxylic acid tert-butyl ester

The compound was prepared following the procedure described in the Example 1 (A), starting from 1H-indole-2-carbonitrile.
(S)-3-[3-(1H-Indol-2-yl)-[1,2,4]oxadiazol-5-yl]-piperidine-1-carboxylic acid tert-butyl ester was used without further purification.
Yield: quantitative (brown oil); LCMS (RT): 6.41 min (Method A); MS (ES+) gave m/z: 369.1 (MH+).

12 (B) 2-((S)-5-Piperidin-3-yl-[1,2,4]oxadiazol-3-yl)-1H-indole hydrochloride

The compound was prepared following the procedure described in the Example 1 (B), starting from (S)-3-[3-(1H-indol-2-yl)-[1,2,4]oxadiazol-5-yl]-piperidine-1-carboxylic acid tert-butyl ester.
Yield: quantitative (brown solid); LCMS (RT): 2.63 min (Method B); MS (ES+) gave m/z: 269.1 (MH+).

12 (C) (2,4-Difluoro-phenyl)-{(S)-3-[3-(1H-indol-2-yl)-[1,2,4]oxadiazol-5-yl]-piperidin-1-yl}-methanone

The compound was prepared following the procedure described in the Example 1 (C), starting from 2-((S)-5-piperidin-3-yl-[1,2,4]oxadiazol-3-yl)-1H-indole hydrochloride.
(2,4-difluoro-phenyl)-{(S)-3-[3-(1H-indol-2-yl)-[1,2,4]oxadiazol-5-yl]-piperidin-1-yl}-methanone was obtained pure after flash column chromatography (silica gel, eluent: AcOEt: petroleum ether 3:7). ,
Yield: 3% (white solid); LCMS (RT): 7.13 min (Method Q); MS (ES+) gave m/z: 409.3 (MH+).

$^1$H-NMR (DMSO-$d_6$), δ (ppm): 11.67 (s br, 1H); 7.65 (d, 1H); 7.52-7.43 (m, 2H); 7.30-7.03 (m, 5H); 4.41 (m, 1H); 3.98 (m, 1H); 3.58 (dd, 1H); 3.45-3.19 (m, 2H); 2.29 (m,1H); 2.02 (m, 1H); 1.84 (m, 1H); 1.65 (m, 1H).

Example 13

(4-Fluoro-phenyl)-{3-[5-(2H-pyrazol-3-yl)-[1,2,4]oxadiazol-3-yl]-piperidin-1-yl}-methanone

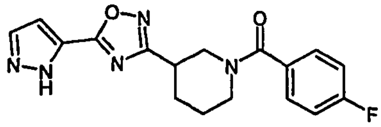

13(A) 3-[5-(2H-Pyrazol-3-yl)-[1,2,4]oxadiazol-3-yl]-piperidine-1-carboxylic acid tert-butyl ester

A mixture of 3-(N-hydroxycarbamimidoyl)-piperidine-1-carboxylic acid tert-butyl ester (0.5 g, 2.05 mmol, prepared as described in 5 (C)), 2H-pyrazole-3-carboxylic acid (0.23 mg, 2.05 mmol), HOBT (0.31 mg, 2.05 mmol), EDCI.HCl (0.59 g, 3.08 mmol) and dry triethylamine (1.1 mL, 4 mmol) in dry dioxane (8 mL) was kept under stirring at ambient temperature for 5h, under nitrogen atmosphere. The reaction mixture was then diluted with DCM and washed with 5% $NaHCO_3$ and brine. The organic layer was dried over $Na_2SO_4$ and concentrated. The crude was purified on silica gel (eluent: DCM: MeOH 20:1.5) to afford 520 mg of 3-{[(hydroxyimino]-[(2H-pyrazole-3-carbonyl)-amino]-methyl}-piperidine-1-carboxylic acid tert-butyl ester (yield: 75%; LCMS (RT): 3.18 min (Method A); MS (ES+) gave m/z: 338.06).

A solution of 3-{[(hydroxyimino]-[(2H-pyrazole-3-carbonyl)-amino]-methyl}-piperidine-1-carboxylic acid tert-butyl ester (0.52 g, 1.54 mmol) and triethylamine (0.43 mL, 3.086 mmol) in dioxane (4 mL) was refluxed for 14h and then the solvent was partially removed under vacuo. The solid precipitated was filtered to afford 360 mg of the title compound
Yield: 73% (white solid); LCMS (RT): 3.5 min (Method A); MS (ES+) gave m/z: 320.1 (MH+).

13(B) 3-[5-(2H-Pyrazol-3-yl)-[1,2,4]oxadiazol-3-yl]-piperidine hydrochloride

The compound was prepared following the procedure described in the Example 5(E) starting from 3-[5-(2H-pyrazol-3-yl)-[1,2,4]oxadiazol-3-yl]-piperidine-1-carboxylic acid tert-butyl ester (prepared as described in Example 13(A))
Yield: quantitative (white powder); LCMS (RT): 1.1 min (Method C); MS (ES+) gave m/z: 220.1 (MH+).

13(C) (4-Fluoro-phenyl)-{3-[5-(2H-pyrazol-3-yl)-[1,2,4]oxadiazol-3-yl]-piperidin-1-yl}-methanone

The compound was prepared following the procedure described in the Example 5(F), using 3-[5-(2H-pyrazol-3-yl)-[1,2,4]oxadiazol-3-yl]-piperidine hydrochloride (prepared as described in the Example 13(B)). Purification of the final compound was performed by flash chromatography on silica gel (eluent: AcOEt: Hexane 3: 1)
Yield: 62% (amorphous white solid); LCMS (T.R.): 6.90 min (Method Q); MS (ES+) gave m/z: 342.2 (MH+).
$^1$H-NMR (DMSO-$d_6$), δ (ppm): 13.60 (s br, 1H); 7.93 (d, 1H); 7.47 (dd, 2H); 7.23 (dd, 2H); 6.92 (d, 1H); 4.23 (m, 1H); 3.83 (m, 1H); 3.37 (dd, 1H); 3.27-3.09 (m, 2H); 2.20 (m, 1H); 1.98-1.76 (m, 2H); 1.63 (m, 1H).

Example 14

(3,4-Difluoro-phenyl)-{3-[5-(2H-pyrazol-3-yl)-[1,2,4]oxadiazol-3-yl]-piperidin-1-yl}-methanone

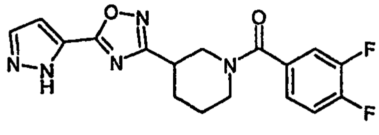

The compound was prepared following the procedure described in the Example 5(F), using 3-[5-(2H-pyrazol-3-yl)-[1,2,4]oxadiazol-3-yl]-piperidine hydrochloride (prepared as described in the Example 13(B)). Purification of the final compound was performed by flash chromatography on silica gel (eluent: AcOEt: petroleum ether 3:1)
Yield: 54% (amorphous white solid); LCMS (RT): 7.07 min (Method Q); MS (ES+) gave m/z: 360.2 (MH+).
$^1$H-NMR (DMSO-$d_6$), δ (ppm): 13.61 (s br, 1H); 7.93 (d, 1H); 7.51-7.39 (m, 2H); 7.27 (m, 1H); 6.92 (d, 1H); 4.19 (m, 1H); 3.79 (m, 1H); 3.39 (dd, 1H); 3.30-3.11 (m, 2H); 2.19 (m, 1H); 2.00-1.76 (m, 2H); 1.64 (m, 1H).

Example 15

(3,4-Difluoro-phenyl)-{(S)-3-[3-(1H-indol-2-yl)-[1,2,4]oxadiazol-5-yl]-piperidin-1-yl}-methanone

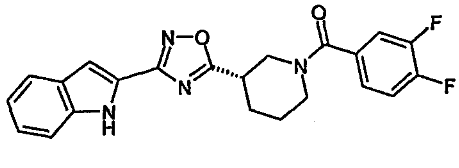

The compound was prepared following the procedure described in the Example 1 (C), starting from 2-((S)-5-piperidin-3-yl-[1,2,4]oxadiazol-3-yl)-1H-indole hydrochloride (prepared as described in Example 12 (B)).
(3,4-Difluoro-phenyl)-{(S)-3-[3-(1H-indol-2-yl)-[1,2,4]oxadiazol-5-yl]-piperidin-1-yl}-methanone was obtained pure after flash column chromatography (silica gel, eluent: AcOEt: petroleum ether 3:7).
Yield: 13% (white solid); mp = 93-94°C ; LCMS (RT): 7.11 min (Method Q); MS (ES+) gave m/z: 409.2 (MH+). ,
$^1$H-NMR (DMSO-$d_6$), δ (ppm): 11.70 (s br, 1H); 7.64 (d, 1H); 7.53-7.42 (m, 3H); 7.28 (m, 1H); 7.22 (dd, 1H); 7.12 (s br, 1H); 7.07 (dd, 1H); 4.24 (m, 1H); 3.73 (m, 1H); 3.57 (dd, 1H); 3.45 (m, 1H); 3.31 (m, 1H); 2.26 (m, 1H); 2.02 (m, 1H); 1.82 (m, 1H); 1.67(m, 1H).

Example 16

(4-Fluoro-phenyl)-{(S)-3-[3-(1H-indol-2-yl)-[1,2,4]oxadiazol-5-yl]-piperidin-1-yl}-methanone

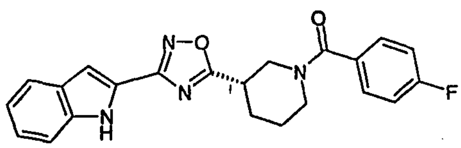

The compound was prepared following the procedure described in the Example 1 (C), starting from 2-((S)-5-piperidin-3-yl-[1,2,4]oxadiazol-3-yl)-1H-indole hydrochloride (prepared as described in Example 12 (B)).
(4-Fluoro-phenyl)-{(S)-3-[3-(1H-indol-2-yl)-[1,2,4]oxadiazol-5-yl]-piperidin-1-yl}-methanone was obtained pure after flash column chromatography (silica gel, eluent: AcOEt: petroleum ether 3:7).
Yield: 18% (white solid); LCMS (RT): 6.99 min (Method Q); MS (ES+) gave m/z: 391.2 (MH+).
$^1$H-NMR (DMSO-$d_6$), δ (ppm): 11.70 (s, 1H); 7.64 (d, 1H); 7.48 (m, 3H); 7.28-7.18 (m, 3H); 7.12 (m, 1H); 7.07 (dd, 1H); 4.27 (m, 1H); 3.78 (m, 1H); 3.56 (dd, 1H); 3.43 (m, 1H); 3.30 (ddd, 1H); 2.30 (m, 1H); 2.01 (m, 1H); 1.84 (m, 1H); 1.67 (m, 1H).

Example 17

(4-Fluoro-phenyl)-{3-[5-(1H-imidazol-2-yl)-[1,2,4]oxadiazol-3-yl]-piperidin-1-yl}-methanone

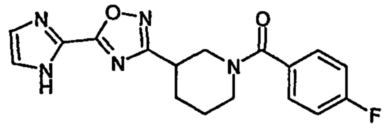

17(A) 3-[5-(1H-Imidazol-2-yl)-[1,2,4]oxadiazol-3-yl]-piperidine-1-carboxylic acid tert-butyl ester

A mixture of 3-(N-hydroxycarbamidoyl)-piperidine-1-carboxylic acid tert-butyl ester (0.25 g, 1.03 mmol, prepared as described in Example 5 (C)), 1H-imidazole-2-carboxylic acid (116 mg, 1.03 mmol), HOBT (161 mg, 1.05 mmol), EDCI.HCl (0.3 g, 1.55 mmol) and dry triethylamine (0.29 mL, 2.05 mmol) in dry DCM (10 mL) was stirred for 4h at ambient temperature, under nitrogen atmosphere. The solution was then concentrated under vacuum and the crude was purified on silica gel (eluent: DCM: MeOH 20:1) to afford 100 mg of 3-{[(hydroxyimino]-[(1H-imidazole-2-carbonyl)-amino]-methyl}-piperidine-1-carboxylic acid tert-butyl ester (yield: 29%; LCMS (RT): 2.54 min (Method B); MS (ES+) gave m/z: 357.95 (MH+).).

A solution of 3-{[(hydroxyimino]-[(1H-imidazole-2-carbonyl)-amino]-methyl}-piperidine-1-carboxylic acid tert-butyl ester (0.1 g, 0.3 mmol) and DIEA (0.043 mL, 0.3 mmol) in MeCN (4 mL) was heated for 30 min at 150° in a sealed tube under microwave irradiation. Upon cooling a white solid precipitated which was collected by filtration to afford 43 mg of the title compound.
Yield: 45% (white solid); LCMS (RT): 2.97 min (Method B); MS (ES+) gave m/z: 320.1(MH+).

17(B) 3-[5-(1H-imidazol-2-yl)-[1,2,4]oxadiazol-3-yl]-piperidine trifluoroacetate.

3-[5-(1H-Iimidazole-2-yl)-[1,2,4]oxadiazol-3-yl]-piperidine-1-carboxylic acid tert-butyl ester (40 mg, 0.125 mmol), prepared as described in Example 17 (A), was dissolved in DCM (1mL) and TFA (1mL) was added. The solution was stirred for 30 min and then the solvent was removed under vacuum to give the title compound as a colourless gum, which was used without further purification.
Yield: quantitative (colourless gum); LCMS (RT): 0.65 min (Method B); MS (ES+) gave m/z: 220.1 (MH+).

17(C) (4-Fluoro-phenyl)-{3-[1H-imidazol-2-yl)-[1,2,4]oxadiazol-3-yl]-piperidin-1-yl}-methanone.

4-Fluorobenzoyl chloride (16 μL, 0-13 mmol) was added to a stirred solution of 3-5-(1H-imidazol-2-yl)-[1,2,4] oxadiazol-3-yl]-piperidine trifluoroacetate (prepared as described in example 19 (B)) and triethylamine (35 μL, 0.25 mmol) in dry DCM (2 mL). The solution was stirred under nitrogen atmosphere for 2h and then concentrated under vacuum. Purification of the crude was performed by flash chromatography on silica gel (eluent: DCM:MeOH 20:1). The title compound was obtained as a white solid (35 mg) Yield: 81% (amorphous white solid); LCMS (RT): 5.58 min (Method Q); MS (ES+) gave m/z: 342.1 (MH+).

$^1$H-NMR (DMSO-$d_6$, 343K), δ (ppm): 8.80 (s br, 1H); 7.47 (dd, 2H); 7.36 (s, 2H); 7.23 (dd, 2H); 4.29 (m, 1H); 3.83 (m, 1H); 3.34 (dd, 1H); 3.25-3.08 (m, 2H); 2.22 (m, 1H); 1.98-1.77 (m, 2H); 1.64 (m, 1H).

Example 18

(3,4-Difluoro-phenyl)-{3-[5-(1H-imidazol-2-yl)-[1,2,4]oxadiazol-3-yl]-piperidin-1-yl}-methanone

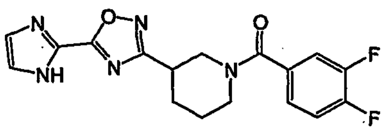

The title compound was obtained following the experimental procedure described in Example 17(C), starting from 3-[5-(1H-imidazol-2-yl)-[1,2,4]oxadiazol-3-yl]-piperidine trifluoroacetate (prepared as described in Example 17(B)) and 3,4-difluorobenzoyl chloride. Purification was performed by trituration from diethyl ether to afford (3,4-difluoro-phenyl)-{3-[5-(1H-imidazol-2-yl)-[1,2,4]oxadiazol-3-yl]-piperidin-1-yl}-methanone as a white solid.
Yield: 60% (white solid); mp=148.5-148.9°C; LCMS (RT): 6.73 min (Method Q); MS (ES+) gave m/z: 360.2 (MH+).

$^1$H-NMR (DMSO-$d_6$, 343K), δ (ppm): 13.51 (s br, 1H); 7.52-7.38 (m, 3H); 7.32-7.20 (m, 2H); 4.21 (m, 1H); 3.79 (m, 1H); 3.45-3.08 (m, 3H); 2.29-2.14 (m, 1H); 2.12-1.46 (m, 3H).

Example 19

{(S)-3-[3-(1H-Indol-2-yl)-[1,2,4]oxadiazol-5-yl]-piperidin-1-yl}-(5-methyl-isoxazol-4-yl)-methanone

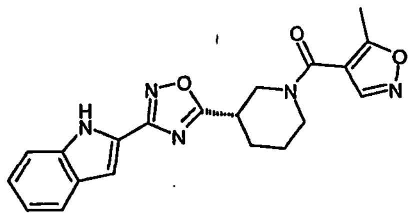

The compound was prepared following the procedure described in the Example 4, starting from 2-((S)-5-Piperidin-3-yl-[1,2,4]oxadiazol-3-yl)-1H-indole hydrochloride (prepared as described in Example 12 (B)) and using 5-Methyl-isoxazole-4-carboxylic acid as the acid of choice.
{(S)-3-[3-(1H-Indol-2-yl)-[1,2,4]oxadiazol-5-yl]-piperidin-1-yl}-(5-methyl-isoxazol-4-yl)-methanone was obtained pure after flash column chromatography (silica gel, eluent: DCM).
Yield: 5% (White powder); mp=163-164 °C; LCMS (RT): 6.63 min (Method Q); MS (ES+) gave m/z: 378.2 (MH+).
$^1$H-NMR (DMSO-$d_6$, 373K), δ (ppm): 11.48 (s br, 1H); 8.54 (s, 1H); 7.64 (d, 1H); 7.51 (d, 1H); 7.22 (dd, 1H); 7.12 (m, 1H); 7.07 (dd, 1H); 4.27 (dd, 1H); 3.80 (ddd, 1H); 3.63 (dd, 1H); 3.48-3.33 (m, 2H); 2.48 (s, 3H); 2.30 (m, 1H); 2.04 (m, 1H); 1.88 (m, 1H); 1.68 (m, 1H).

Example 20

(5-Methyl-isoxazol-4-yl)-{(S)-3-[3-(1H-pyrrol-2-yl)-[1,2,4]oxadiazol-5-yl]-piperidin-1-yl}-methanone

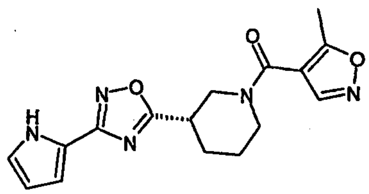

The compound was prepared following the procedure described in the Example 4, starting from (S)-3-[3-(1H-Pyrrol-2-yl)-[1,2,4]oxadiazol-5-yl]-piperidine hydrochloride (prepared as described in Example 1 (B)) and using 5-Methylisoxazole-4-carboxylic acid as the acid of choice.
(5-Methyl-isoxazol-4-yl)-{(S)-3-[3-(1H-pyrrol-2-yl)-[1,2,4]oxadiazol-5-yl]-piperidin-1-yl}-methanone was obtained pure after flash column chromatography (silica gel, eluent: hexane/ethyl acetate 3:7).
Yield: 60% (White powder); mp=125-127°C; $[\alpha]_D^{20}$ = +47.8 (c=0.68, MeOH); LCMS (RT): 6.01 min (Method Q); MS (ES+) gave m/z: 328.1 (MH+).
$^1$H-NMR (DMSO-$d_6$, 373K), δ (ppm): 11.32 (s br, 1H); 8.52 (s, 1H); 6.97 (m, 1H); 6.74 (m, 1H); 6.22 (m, 1H); 4.22 (dd, 1H); 3.78 (ddd, 1H); 3.58 (dd, 1H); 3.36 (m, 2H); 2.46 (s, 3H); 2.24 (m, 1H); 2.06-1.79 (m, 2H); 1.66 (m, 1H).

Example 21

(6-Fluoro-pyridin-3-yl)-{(S)-3-[5-(1H-pyrrol-2-yl)-[1,2,4]oxadiazol-3-yl]-piperidin-1-yl}-methanone

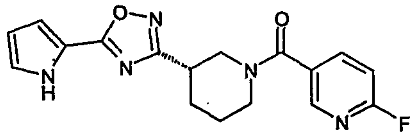

The compound was prepared following the procedure described in the Example 8, starting from ((S)-3-[5-(1H-pyrrol-2-yl)-[1,2,4]oxadiazol-3-yl]-piperidine hydrochloride (prepared as described in Example 10 (E)) and using 6-fluoro-pyridine-3-carboxylic acid as the acid of choice.
(6-Fluoro-pyridin-3-yl)-{(S)-3-[5-(1H-pyrrol-2-yl)-[1,2,4]oxadiazol-3-yl]-piperidin-1-yl}-methanone was obtained pure after flash column chromatography (silica gel, eluent: petroleum ether/ethyl acetate 1:1).
Yield: 49% (white solid); mp=147 °C; $[\alpha]_D^{20}$ = +118.45 (c=1.005, MeOH); LCMS (RT): 6.03 min (Method Q); MS (ES+) gave m/z: 342.1 (MH+).
$^1$H-NMR (DMSO-$d_6$, 343K), δ (ppm): 12.05 (s br, 1H); 8.32 (m, 1H); 8.03 (ddd, 1H); 7.21 (ddd, 1H); 7.13 (dd, 1H); 6.96 (dd, 1H); 6.30 (dd, 1H); 4.23 (m, 1H); 3.81 (m, 1H); 3.37 (dd, 1H); 3.26 (ddd, 1H); 3.13 (m, 1H); 2.19 (m, 1H); 1.97-1.76 (m, 2H); 1.66 (m, 1H).

Example 22

(4-Fluoro-phenyl)-{(S)-3-[S-(1H-pyrrol-2-yl)-[1,2,4]oxadiazol-3-yl]-piperidin-1-yl}-methanone

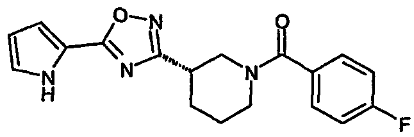

The compound was prepared following the procedure described in the Example 1(C), starting from ((S)-3-[5-(1H-pyrrol-2-yl)-[1,2,4]oxadiazol-3-yl]-piperidine hydrochloride (prepared as described in Example 10 (E)) and using 4-fluorobenzoyl chloride as the acylating agent.
(4-Fluoro-phenyl)-{(S)-3-[5-(1H-pyrrol-2-yl)-[1,2,4]oxadiazol-3-yl]-piperidin-1-yl}-methanone was obtained pure after flash column chromatography (silica gel, eluent: petroleum ether/ethyl acetate 1:1).
Yield: 54% (white solid); mp=181°C; $[\alpha]_D^{20}$ = +108.05 (c=0.975, MeOH); LCMS (RT): 6.41 min (Method Q); MS (ES+) gave m/z: 341.2 (MH+).
$^1$H-NMR (DMSO-$d_6$, 343K), δ (ppm): 12.04 (s br, 1H); 7.47 (dd, 2H); 7.22 (dd, 2H); 7.12 (m, 1H); 6.96 (m, 1H); 6.30 (dd, 1H); 4.26 (m, 1H); 3.83 (m, 1H); 3.31 (dd, 1H); 3.19 (ddd, 1H); 3.08 (m, 1H); 2.19 (m, 1H); 1.95-1.76 (m, 2H); 1.62 (m, 1H).

Example 23

(6-Fluoro-pyridin-3-yl)-{3-[5-(1H-indol-2-yl)-[1,2,4]oxadiazol-3-yl]-piperidin-1-yl}-methanone

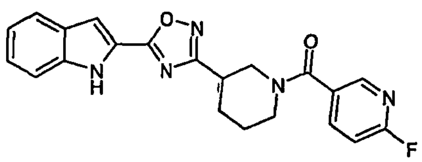

The compound was prepared following the procedure described in the Example 8, starting from 2-(3-piperidin-3-yl-[1,2,4]oxadiazol-5-yl)-1H-indole hydrochloride (prepared as described in Example 11 (B)) and using 6-fluoro-pyridine-3-carboxylic acid as the acid of choice.
Yield: 51% (white solid); mp=163.1-164.3°C; LCMS (RT): 7.52 min (Method Q); MS (ES+) gave m/z: 392.2 (MH+).
$^1$H-NMR (DMSO-d6, 343K), δ (ppm): 12.07 (s br, 1H); 8.33 (m, 1H); 8.05 (ddd, 1H); 7.70 (d, 1H); 7.53 (dd, 1H); 7.34 (d, 1H); 7.30 (ddd, 1H); 7.22 (dd, 1H); 7.12 (dd, 1H); 4.28 (m, 1H); 3.83 (m, 1H); 3.43 (dd, 1H); 3.34-3.16 (m, 2H); 2.24 (m,

1H); 1.94. (m, 1H); 1.85 (m, 1H); 1.70 (m, 1H).

Example 24

(4-Fluoro-phenyl)-{(S)-3-[3-(1H-imidazol-2-yl)-[1,2,4]oxadiazol-5-yl]-piperidin-1-yl}-methanone

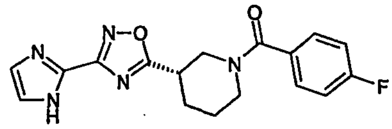

24(A) 1H-Imidazole-2-carboxylic acid amide

A solution of 1H-imidazole-2-carboxylic acid (200 mg, 1.78 mmol) and thionyl chloride (3 mL) was refluxed for 2h. The reaction mixture was cooled at room temperature and poured into toluene (5 mL), the resulting precipitate was collected by filtration and then washed with diethyl ether. The solid was dissolved in conc. $NH_4OH$ (aq) (3 mL) and stirred at 10°C for 1h, then the mixture was allowed to warm at RT. A solid precipitated out and was filtered, washed with water and dried in a vacuum oven at 40°C for 1 night to afford 72 mg of 1H-imidazole-2-carboxylic acid amide.
Yield: 36%; LCMS (RT): 0.62 min (Method D); MS (ES+) gave m/z: 112.0 (MH+).

24(B) N-Hydroxy-1H-imidazole-2-carboxamidine

A solution of 1H-imidazole-2-carboxylic acid amide (360 mg, 3.24 mmol) and phenyl dichlorophosphate (2 mL) was heated at 170°C for 8 min, in a microwaves oven. The reaction mixture was cooled at room temperature and poured into water (50 mL). The solution was cooled at 0°C and the pH was adjusted to 11 by addition of NaOH 10 M. Ethyl acetate was added and the phases were separated. The organic layer was dried over sodium sulphate and evaporated in vacuo to provide 1H-Imidazole-2-carbonitrile. A solution of 1H-imidazole-2-carbonitrile and hydroxylamine (50% sol. in water, 794 μL, 13 mmol) in ethanol (15 mL) was refluxed for 4h. The solvent was removed and the crude N-hydroxy-1H-imidazole-2-carboxamidine was used for the next step without further purification.
Yield: quantitative; LCMS (RT): 0.62 min (Method D); MS (ES+) gave m/z: 127.0 (MH+).

24(C) (S)-3-[3-(1H-Imidazol-2-yl)-[1,2,4]oxadiazol-5-yl]-piperidine-1-carboxylic acid tert-butyl ester

A mixture of N-hydroxy-1H-imidazole-2-carboxamidine (3.24 mmol), S-1-Boc-piperidine-3-carboxylic acid (0.743 g, 3.24 mmol), EDCI.HCl (0.932 g, 4.86 mmol) and HOBT (0.438 g, 3.24 mmol) in DCM (10 mL) was stirred overnight at room temperature, under nitrogen atmosphere. The mixture was washed with $NaHCO_3$ (aq), the phases were separated and the organic layer was dried over $Na_2SO_4$ and concentrated under reduced pressure. Purification of the crude by flash chromatography (silica gel, eluent: DCM/MeOH 98/2) gave a solid that was dissolved in $CH_3CN$ (5 mL), triethylamine (450 μL, 3.24 mmol) was added and the resulting solution was heated at 150°C for 1h, in a microwaves oven. The solvent was removed and the crude was purified by flash chromatography (silica gel, eluent: DCM/MeOH 98/2) to give (S)-3-[3-(1H-imidazol-2-yl)-[1,2,4]oxadiazol-5-yl]-piperidine-1-carboxylic acid tert-butyl ester (50 mg).
Yield: 5%; LCMS (RT): 3.21 min (Method D); MS (ES+) gave m/z: 342.11 (MH+).

24(D) (S)-3-[3-(1H-Imidazol-2-yl)-[1,2,4]oxadiazol-5-yl]-piperidine hydrochloride

To a solution of (S)-3-[3-(1H-imidazol-2-yl)-[1,2,4]oxadiazol-5-yl]-piperidine-1-carboxylic acid tert-butyl ester (50 mg, 0.157 mmol) in dichloromethane (1 mL), 1 mL of HCl 4N (dioxane solution) was added at 0°C and the reaction mixture was allowed to warm at room temperature and stirred for 2 h. The solvent was evaporated under reduced pressure to give the title compound as a white solid, which was used for the next step without further purification.
Yield: quantitative.

24(E) (4-Fluoro-phenyl)-{(S)-3-[3-(1H-imidazol-2-yl)-[1,2,4]oxadiazol-5-yl]-piperidin-1-yl}-methanone

The title compound was obtained following the experimental procedure described in Example 1(C), starting

from (S)-3-[3-(1H-imidazol-2-yl)-[1,2,4]oxadiazol-5-yl]-piperidine hydrochloride and using 4-fluorobenzoyl chloride as the acylating agent. Purification by preparative HPLC gave (4-fluoro-phenyl)-{(S)-3-[3-(1H-imidazol-2-yl)-[1,2,4]oxadiazol-5-yl]-piperidin-1-yl}-methanone as a colourless oil. Yield: 12% (colourless oil); LCMS (RT): 5.34 min (Method Q); MS (ES+) gave m/z: 342.2 (MH+).

$^1$H-NMR (DMSO-$d_6$ 343K), δ (ppm): 7.48 (dd, 2H); 7.30 (s, 2H); 7.24 (dd, 2H); 4.27 (m, 1H); 3.79 (m, 1H); 3.51 (dd, 1H); 3.42 (ddd, 1H); 3.26 (ddd, 1H); 2.27 (m, 1H); 2.05-1.78 (m, 2H); 1.66 (m, 1H).

Example 25

(3,4-Difluoro-phenyl)-{(S)-3-[3-(1H-imidazol-2-yl)-[1,2,4]oxadiazol-5-yl]-piperidin-1-yl}-methanone

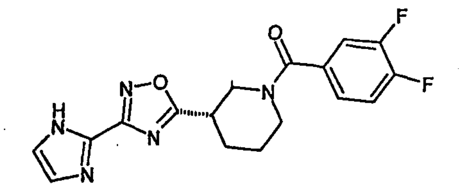

The title compound was obtained following the same experimental procedure described in Example 4, starting from (S)-3-[3-(1H-imidazol-2-yl)-[1,2,4]oxadiazol-5-yl]-piperidine hydrochloride (prepared as described in Example 24 (D)) and using 3,4-difluorobenzoic acid as the acid of choice.
Purification was performed by flash chromatography (silica gel, eluent: DCM/MeOH 98:2).
Yield: 19% (White powder); mp=156-157°C; $[\alpha]_D^{20}$= +90.0 (c=0.50, MeOH). LCMS (RT): 5.31 min (Method Q); MS (ES+) gave m/z: 360.2 (MH+).
$^1$H-NMR (DMSO-$d_6$, 343K), δ (ppm): 12.91 (s br, 1H); 7.53-7.40 (m, 2H); 7.34-7.13 (m, 3H); 4.23 (m, 1H); 3.76 (m, 1H); 3.53 (dd, 1H); 3.43 (ddd, 1H); 3.29 (ddd, 1H); 2.29 (m, 1H); 1.98 (m, 1H); 1.83 (m, 1H); 1.66 (m, 1H).

Example 26

{3-[5-(1H-Indol-2-yl)-[1,2,4]oxadiazol-3-yl]-piperidin-1-yl}-(5-methyl-isoxazol-4-yl)-methanone

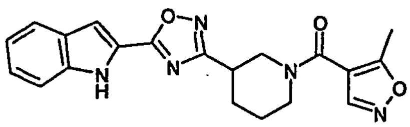

The compound was prepared following the procedure described in the Example 8, starting from 2-(3-piperidin-3-yl-[1,2,4]oxadiazol-5-yl)-1H-indole hydrochloride (prepared as described in Example 11 (B)) and using 5-methyl-isoxazole-4-carboxylic acid as the acid of choice.
Yield: 97% (white solid); mp=175.6-177.2°C; LCMS (RT): 8.01 min (Method Q); MS (ES+) gave m/z: 378.2 (MH+).
$^1$H-NMR (DMSO-$d_6$, 343K), δ (ppm): 12.08 (s br, 1H); 8.60 (s, 1H); 7.70 (d, 1H); 7.53 (dd, 1H); 7.35 (dd, 1H); 7.30 (ddd, 1H); 7.13 (ddd, 1H); 4.31 (m, 1H); 3.87 (m, 1H); 3.42 (dd, 1H); 3.28 (ddd, 1H); 3.17 (m, 1H); 2.48 (d, 3H); 2.23 (m, 1H); 2.03-1.79 (m, 2H); 1.66 (m, 1H).

Example 27

(4-Fluoro-phenyl)-{(S)-3-[5-(4-methyl-1H-pyrrol-2-yl)-[1,2,4]oxadiazol-3-yl]-piperidin-1-yl}-methanone

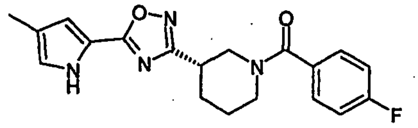

27 (A) (S)-3-Carbamoyl-piperidine-1-carboxylic acid tert-butyl ester

Triethylamine (1.21mL, 8.72 mmol) and then ethyl chloroformate (0.8 mL, 8.30 mmol) were added dropwise at 0°C to a solution of (S)-1-Boc-piperidine-3-carboxylic acid (2 g, 8.72 mmol) in chloroform (40 mL), under nitrogen atmosphere. After stirring 10 min at 0°C, $NH_3$ (gas) was bubbled into the solution for 1h. The reaction mixture was then stirred at room temperature for 3h, 6% $NaHCO_3$ (aq) was added and the phases were separated. The organic layer was dried over sodium sulphate and evaporated under reduced pressure to afford the title compound, which was used for the next step without further purification.
Yield: quantitative; LCMS (RT): 3.31 min (Method A); MS (ES+) gave m/z: 229.0 (MH+).

27 (B) (S)-3-Cyano-piperidine-1-carboxylic acid tert-butyl ester

Phosphorus oxychloride (812 μL, 8.72 mmol) was added dropwise at 0°C to a solution of (S)-3-carbamoyl-piperidine-1-carboxylic acid tert-butyl ester (2 g, 8.72 mmol) in pyridine (20 mL), under nitrogen atmosphere. After stirring overnight at room temperature, ethyl acetate was added and the solution was washed with 10% HCl (2 times). The phases were separated and the organics were dried over sodium sulphate and evaporated to dryness under reduced pressure.
The title compound was used for the next step without further purification.
Yield: quantitative; LCMS (RT): 4.48 min (Method A); MS (ES+) gave m/z: 211.1 (MH+).

27 (C) (S)-1-(4-Fluoro-benzoyl)-piperidine-3-carbonitrile

(S)-3-Cyano-piperidine-1-carboxylic acid tert-butyl ester (1.5 g, 7.14 mmol), was dissolved in dioxane (15 mL) and 10 mL of 4N HCl (dioxane solution) were added dropwise at 0°C. The resulting mixture was stirred at room temperature for 5h. The solvent was evaporated under reduced pressure to afford (S)-piperidine-3-carbonitrile hydrochloride as a white solid, that was used for the next step without further purification.

To a suspension of (S)-piperidine-3-carbonitrile hydrochloride (7.14 mmol) in dry dichloromethane (100 mL), triethylamine (3 mL, 21.4 mmol) and 4-fluorobenzoyl chloride (930 μL, 7.85 mmol) were added dropwise at 0°C. The reaction mixture was allowed to warm at room temperature and stirred for 3h under nitrogen atmosphere. The solution was then treated with 5% $NaHCO_3$ (50 mL, twice) and the phases were separated. The organic layer was washed with 1N HCl (50 mL) and with brine (50 mL), then was dried over $Na_2O_4$ and evaporated under reduced pressure. The crude was purified by flash chromatography (silica gel, eluent gradient: from petroleum ether/ethyl acetate 7:3 to petroleum ether/ethyl acetate 1:1) to give 1.01g of the title compound.
Yield: 61% (yellow oil); LCMS (RT): 3.7 min (Method D); MS (ES+) gave m/z: 233.1 (MH+).

27 (D) (S)-1-(4-Fluoro-benzoyl)-N-hydroxy-piperidine-3-carboxamidine

A solution of (S)-1-(4-fluoro-benzoyl)-piperidine-3-carbonitrile (1.01 g, 4.35 mmol) and aqueous hydroxylamine (50% in water, 1.1 mL, 17.4 mmol) in ethanol (10 mL) was refluxed for 4h. The solvent was evaporated under reduced pressure to afford the title compound (1.15 g) that was used for the next step without further purification.
Yield: quantitative; $^1$H-NMR (DMSO-$d_6$, 343K), δ (ppm): 8.61 (s br, 1H); 7.44 (dd, 2H); 7.22 (dd, 2H); 5.12 (s br, 2H); 4.00 (m, 2H); 3.17-2.82 (m, 3H); 2.23 (m, 1H); 1.98 (m, 1H); 1.78-1.55 (m, 2H).

27 (E) (4-Fluoro-phenyl)-{(S)-3-[5-(4-methyl-1H-pyrrol-2-yl)-[1,2,4]oxadiazol-3-yl]-piperidin-1-yl}-methanone

A mixture of (S)-1-(4-fluoro-benzoyl)-N-hydroxy-piperidine-3-carboxamidine (150 mg, 0.56 mmol), 4-methyl-pyrrole-2-carboxylic acid (70 mg, 0.56 mmol), EDCI.HCl (162 mg, 0.85 mmol) and HOBT (85 mg, 0.56 mmol) in dioxane (2 mL) was stirred at 40°C for 2h, then at 90°C for 20h, then under reflux for 24h, under nitrogen atmosphere. The mixture was diluted with ethyl acetate and washed with 1N $Na_2CO_3$ (aq), the phases were separated and the organic layer was dried over $Na_2SO_4$ and concentrated under reduced pressure. Purification of the crude by flash chromatography (silica gel, eluent: petroleum ether/ethyl acetate 3:2) gave a solid that was triturated from ethyl acetate/diethyl ether 1:1. (4-Fluoro-phenyl)-{(S)-3-[5-(4-methyl-1H-pyrrol-2-yl)-[1,2,4]oxadiazol-3-yl]-piperidin-1=yl}-methanone was obtained (20

mg).
Yield: 10% (White solid); mp=183 °C; LCMS (RT): 6.69 min (Method Q); MS (ES+) gave m/z: 355.1 (MH+).
$^1$H-NMR (DMSO-$d_6$, 343K), δ (ppm): 11.61 (s br, 1H); 7.46 (dd, 2H); 7.21 (dd, 2H); 6.89 (m, 1H); 6.76 (m, 1H); 4.24 (m, 1H); 3.84 (m, 1H); 3.31 (dd, 1H); 3.18 (ddd, 1H); 3.ops (m, 1H); 2.18 (m, 1H); 2.09 (s, 3H); 1.95-1.73 (m, 2H); 1.70-1.51 (m, 1H).

Example 28

(6-Fluoro-pyridin-3-yl)-{(S)-3-[S-(4-methyl-1H-pyrrol-2-yl)-[1,2,4]oxadiazol-3-yl]-piperidin-1-yl}-methanone

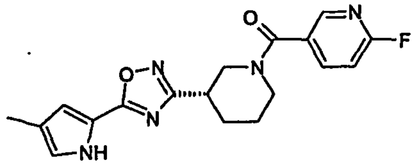

28(A) (S)-3-[5-(4-Methyl-1H-pyrrol-2-yl)-[1,2,4]oxadiazol-3-yl]-piperidine-1-carboxylic acid tert-butyl ester

A mixture of 4-methyl-pyrrole-2-carboxylic acid (412 mg, 3.28 mmol), HOAT (448 mg, 3.28 mmol), EDCI.HCl (948 mg, 4.92 mmol) in dry dioxane (12 mL) was kept under stirring at 50°C for 1h, under nitrogen atmosphere, then (S)-3-(N-hydroxycarbamimidoyl)-piperidine-1-carboxylic acid tert-butyl ester (0.8 g, 3.28 mmol), prepared as described in Example 10(C), was added and the reaction mixture was stirred at 50°C for 2h. The solvent was evaporated under reduced pressure. The residue was diluted with water (15 mL) and ethyl acetate (15 mL), the phases were separated and the organic layer was washed sequentially 5% $NaHCO_3$ (aq) (10 mL, twice) and with brine. The organic layer was dried over sodium sulphate and the solvent was removed under vacuum to give a residue that was purified by flash chromatography (silica gel, eluent: petroleum ether/ethyl acetate 1:1) to give 950 mg of a solid. The solid was dissolved in acetonitrile (10 mL), activated 4A molecular sieves were added and the mixture was heated at 120°C for 2h in a microwaves oven. Ethyl acetate was added and the molecular sieves were filtered off. The filtrate was evaporated under reduced pressure and the crude was purified by flash chromatography (silica gel, eluent: petroleum ether/ethyl acetate 2:1) to give (S)-3-[5-(4-methyl-1H-pyrrol-2-yl)-[1,2,4]oxadiazol-3-yl]-piperidine-1-carboxylic acid tert-butyl ester (464 mg) as a yellow oil.
Yield: 43% (yellow oil); LCMS (RT): 5.3 min (Method E); MS (ES+) gave m/z: 333.2 (MH+).

28(B) (S)-3-[5-(4-Methyl-1H-pyrrol-2-yl)-[1,2,4]oxadiazol-3-yl]-piperidine hydrochloride

To a solution of (S)-3-[5-(4-methyl-1H-pyrrol-2-yl)-[1,2,4]oxadiazol-3-yl]-piperidine-1-carboxylic acid tert-butyl ester (0.46 g, 1.38 mmol) in dichloromethane (20 mL), 3.45 mL of HCl 4N (dioxane solution) were added at 0°C and the reaction mixture was allowed to warm at room temperature and stirred for 3 h. The solvent was evaporated under reduced pressure to give the title compound as a brown solid, which was used for the next step without further purification.
Yield: quantitative.

28(C) (6-Fluoro-pyridin-3-yl)-{(S)-3-[5-(4-methyl-1H-pyrrol-2-yl)-[1,2,4]oxadiazol-3-yl]-piperidin-1-yl}-methanone

A mixture of 6-fluoro-nicotinic acid (63 mg, 0.44 mmol), HOAT (76 mg, 0.55 mmol), EDCI.HCl (107 mg, 0.55 mmol) and triethylamine (156 μL, 1.11 mmol) in dry DCM (10 mL) was kept under stirring at RT for 15 min, under nitrogen atmosphere, then (S)-3-[5-(4-methyl-1H-pyrrol-2-yl)-[1,2,4]oxadiazol-3-yl]-piperidine hydrochloride (0.1 g, 0.37 mmol), was added and the reaction mixture was stirred at RT for 2h. The mixture was diluted with DCM and was washed sequentially with 5% $NaHCO_3$ (aq) (10 mL, twice) and with brine. The organic layer was dried over sodium sulphate and the solvent was removed under vacuum to give a residue that was purified by flash chromatography (silica gel, eluent: petroleum ether/ethyl acetate 1.5:1) to give 59 mg of a solid. The solid was then crystallised from EtOH/iPrOH to give 44 mg of (6-fluoro-pyridin-3-yl)-{(S)-3-[5-(4-methyl-1H-pyrrol-2-yl)-[1,2,4]oxadiazol-3-yl]-piperidin-1-yl}-methanone.
Yield: 33% (White solid); $[\alpha]_D^{20}$ = +124.5° (c=0.90, MeOH); LCMS (RT): 2.61 min (Method N); MS (ES+) gave m/z: 356.4 (MH+).
$^1$H-NMR (DMSO-$d_6$, 353K), δ (ppm): 11.70 (s br, 1H); 8.31 (m, 1H); 8:02 (ddd, 1H); 7.20 (dd, 1H); 6.90 (m, 1H); 6.77 (m, 1H); 4.23 (m, 1H); 3.81 (m, 1H); 3.37 (dd, 1H); 3.26 (ddd, 1H); 3.12 (m, 1H); 2.18 (m, 1H); 2.09 (s, 3H); 1.96-1.76

(m, 2H); 1.65 (m, 1H).

Example 29

(5-Methyl-isoxazol-4-yl)-{(S)-3-[5-(4-methyl-1H-pyrrol-2-yl)-[1,2,4]oxadiazol-3-yl]-piperidin-1-yl}-methanone

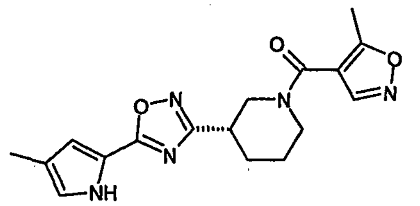

The title compound was prepared following the experimental procedure described in Example 28(C), starting from (S)-3-[5-(4-methyl-1H-pyrrol-2-yl)-[1,2,4]oxadiazol-3-yl]-piperidine hydrochloride, prepared as described in Example 28 (B), and using 5-methyl-isoxazole-4-carboxylic acid as the acid of choice.
Purification was performed by flash chromatography (silica gel, eluent: petroleum ether/ethyl acetate 1.5:1).
Yield: 36% (White solid); $[\alpha]_D^{20}$ = +95.0 (c=1.01; MeOH); LCMS (RT): 2.56 min (Method N); MS (ES+) gave m/z: 342.4 (MH+).
$^1$H-NMR (DMSO-$d_6$ 353K), δ (ppm): 11.69 (s br, 1H); 8.57 (m, 1H); 6.90 (m, 1H); 6.77 (m, 1H); 4.24 (m, 1H); 3.85 (m, 1H); 3.36 (dd, 1H); 3.26 (ddd, 1H); 3.07 (m, 1H); 2.47 (d, 3H); 2.18 (m, 1H); 2.09(m, 3H); 1.97-1.77 (m, 2H); 1.63 (m, 1H).

Example 30

(2-Fluoro-pyridin-4-yl)-{(S)-3-[5-(4-inethyl-1H-pyrrol-2-yl)-[1,2,4]oxadiazol-3-yl]-piperidin-1-yl}-methanone

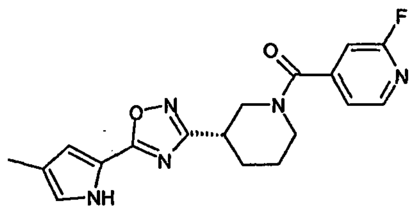

The title compound was prepared following the experimental procedure described in Example 28(C), starting from (S)-3-[5-(4-methyl-1H-pyrrol-2-yl)-[1,2,4]oxadiazol-3-yl]-piperidine hydrochloride, prepared as described in Example 28 (B), and using 2-fluoro-pyridine-4-carboxylic acid as the acid of choice. Purification was performed by flash chromatography (silica gel, eluent: petroleum ether/ethyl acetate 2:1).

Yield: 49% (White solid); $[a]_D^{20}$ = +100.1 (c=0.82, MeOH); LCMS (RT): 2.64 min (Method N); MS (ES+) gave m/z: 356.4 (MH+).
$^1$H-NMR (DMSO-$d_6$, 353K), δ (ppm): 11.69 (s br, 1H); 8.31 (d, 1H); 7.34 (ddd, 1H); 7.16 (m, 1H); 6.90 (m, 1H); 6.77 (m, 1H); 4.60-3.53 (m br, 2H); 3.41-3.07 (m, 3H); 2.18 (m, 1H); 2.10 (s, 3H); 1.96-1.74 (m, 2H); 1.65 (m, 1H).

Example 31

(4-Fluoro-phenyl)-{(S)-3-[3-(4-methyl-1H-pyrrol-2-yl)-[1,2,4]oxadiazol-5-yl]-piperidin-1-yl}-methanone

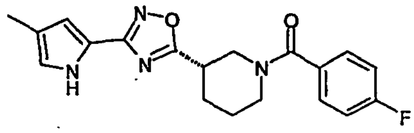

31(A) 4-Methyl-1H-pyrrole-2-carboxylic acid amide

A solution of 4-methyl-pyrrole-2-carboxylic acid (250 mg, 2 mmol) and carbonyl-diimidazole (356 mg, 2.2 mmol) in acetonitrile (10 mL) was stirred at room temperature for 2h, then conc. $NH_4OH$ (2 mL) was added and the mixture was heated at 80°C for 3h. The solvent was removed, the residue was dissolved in water and treated with 1N HCl to adjust the pH to 1. Ethyl acetate was then added, the phases were separated and the organic layer was dried over magnesium sulphate and evaporated under vacuum. The crude residue was purified by flash chromatography (silica gel cartridge, eluent gradient: from hexane/ethyl acetate 100:0 to hexane/ethyl acetate 0:100) to give 215 mg.
Yield: 87%; LCMS (RT): 2.01 min (Method D); MS (ES+) gave m/z: 125.1 (MH+).

31(B) 4-Methyl-1H-pyrrole-2-carbonitrile

A solution of 4-methyl-1H-pyrrole-2-carboxylic acid amide (210 mg, 1.7 mmol) in phosphorus oxychloride (5 mL) was heated at 100°C for 5 minutes, then the mixture was cooled, ice was added and conc. $NH_4OH$ was added to adjust the pH to 10. Extraction with ethyl acetate was performed, the organic layer was dried over magnesium sulphate and evaporated under vacuum. The crude residue was purified by flash chromatography (silica gel cartridge, eluent gradient: from hexane/ethyl acetate 100:0 to hexane/ethyl acetate 60:40) to give 180 mg.
Yield: 100%; LCMS (RT): 2.74 min (Method B); MS (ES+) gave m/z: 107.0 (MH+).

31(C) N-Hydroxy-4-methyl-1H-pyrrole-2-carboxamidine

A solution of 4-methyl-1H-pyrrole-2-carbonitrile (180 mg, 1.7 mmol) and aqueous hydroxylamine (50% in water, 460 μL, 7 mmol) in ethanol (10 mL) was refluxed for 1h. The solvent was evaporated under reduced pressure and the crude residue was purified by flash chromatography (silica gel cartridge, eluent gradient: from hexane/ethyl acetate 100:0 to hexane/ethyl acetate 0:100) to give 240 mg.
Yield: 100%; LCMS (RT): 0.63 min (Method B); MS (ES+) gave m/z: 140.1 (MH+).

31(D) (S)-3-[3-(4-Methyl-1H-pyrrol-2-yl)-[1,2,4]oxadiazol-5-yl]-piperidine-1-carboxylic acid tert-butyl ester

A mixture of (S)-N-Boc-nipecotic acid (460 mg, 2 mmol), HOAT (272 mg, 2 mmol), EDCI.HCl (480 mg, 2.5 mmol) in dry DCM (10 mL) was kept under stirring at ambient temperature for 10 minutes, under nitrogen atmosphere, then N-hydroxy-4-methyl-1H-pyrrole-2-carboxamidine (240 mg, 1.7 mmol) was added and stirring at RT was maintained overnight. The solvent was removed under vacuum to give a residue that was purified by flash chromatography (silica gel cartridge, eluent gradient: from hexane/ethyl acetate 100:0 to hexane/ethyl acetate 60:40). The solid thus obtained was dissolved in acetonitrile (2 mL) and heated in a sealed tube at 80°C for 2h20, in a microwaves oven. Solvent was removed and the crude residue was purified by flash chromatography (silica gel cartridge, eluent gradient: from hexane/ethyl acetate 100:0 to hexane/ethyl acetate 80:20) to give (S)-3-[3-(4-methyl-1H-pyrrol-2-yl)-[1,2,4]oxadiazol-5-yl]-piperidine-1-carboxylic acid tert-butyl ester. Yield: 12%; LCMS (RT): 5.84 min (Method D); MS (ES+) gave m/z: 333.1 (MH+).

31(E) (S)-3-[3-(4-Methyl-1H-pyrrol-2-yl)-[1,2,4]oxadiazol-5-yl]-piperidine trifluoroacetate

To a solution of (S)-3-[3-(4-methyl-1H-pyrrol-2-yl)-[1,2,4]oxadiazol-5-yl]-piperidine-1-carboxylic acid tert-butyl ester (50 mg, 0.15 mmol) in dichloromethane (2 mL), 0.5 mL of TFA were added at 0°C and the reaction mixture was stirred at 0°C for 1 h, in the dark. The solvent was evaporated under reduced pressure to give the title compound, which was used for the next step without further purification.
Yield: quantitative; LCMS (RT): 2.6 min (Method D); MS (ES+) gave m/z: 233.2 (MH+).

31(F) (4-Fluoro-phenyl)-{(S)-3-[3-(4-methyl-1H-pyrrol-2-yl)-[1,2,4]oxadiazol-5-yl]-piperidin-1-yl}-methanone

The compound was prepared following the procedure described in the Example 1(C), starting from (S)-3-[3-(4-methyl-1H-pyrrol-2-yl)-[1,2,4]oxadiazol-5-yl]-piperidine trifluoroacetate and using 4-fluorobenzoyl chloride as the acylating agent. The final compound was purified by flash chromatography (silica gel cartridge, eluent gradient: from hexane/ethyl acetate 100:0 to hexane/ethyl acetate 60:40).
Yield: 60% (off-white solid); $[\alpha]_D^{20}$= +114 (c=0.4, MeOH); mp= 188-190°C; LCMS (RT): 7.01 min (Method C); MS (ES+) gave m/z: 355.2 (MH+).
$^1$H-NMR (DMSO-$d_6$, 343K), δ (ppm): 11.15 (s br, 1H); 7.46 (dd, 2H); 7.23 (dd, 2H); 6.73 (m, 1H); 6.55 (m, 1H); 4.21 (m, 1H); 3.76 (m, 1H); 3.48 (dd, 1H); 3.38-3.19 (m, 2H); 2.23 (m, 1H); 2.07 (s, 3H); 2.01-1.76 (m, 2H); 1.64 (m, 1H).

Example 32

(3,4-Difluoro-phenyl)-{(S)-3-[3-(4-methyl-1H-pyrrol-2-yl)-[1,2,4]oxadiazol-5-yl]-piperidin-1-yl}-methanone

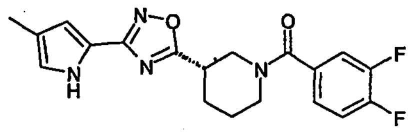

The compound was prepared following the procedure described in the Example 1(C), starting from (S)-3-[3-(4-methyl-1H-pyrrol-2-yl)-[1,2,4]oxadiazol-5-yl]-piperidine trifluoroacetate, prepared as described in Example 31 (E), and using 3,4-difluorobenzoyl chloride as the acylating agent. The final compound was purified by flash chromatography (silica gel, eluent gradient: from hexane/ethyl acetate 100:0 to hexane/ethyl acetate 40:60).
Yield: 77% (white solid); $[\alpha]_D^{20}$ = +107 (c=0.5, MeOH); mp= 166-167°C; LCMS (RT): 3.02 min (Method N); MS (ES+) gave m/z: 373.1 (MH+).
$^1$H-NMR (DMSO-$d_6$, 353K), δ (ppm): 11.09 (s br, 1H); 7.51-7.38 (m, 2H); 7.26 (m, 1H); 6.73 (m, 1H); 6.56 (m, 1H); 4.18 (m, 1H); 3.73 (dt, 1H); 3.51 (dd, 1H); 3.40-3.24 (m, 2H); 2.23 (m, 1H); 2.08 (s, 3H); 2.02-1.75 (m, 2H); 1.65 (m, 1H).

Example 33

(6-Fluoro-pyridin-3-yl)-{(S)-3-[3-(4-methyl-1H-pyrrol-2-yl)-[1,2,4]oxadiazol-5-yl]-piperidin-1-yl}-methanone

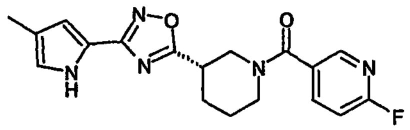

The compound was prepared following the procedure described in the Example 28 (C), starting from (S)-3-[3-(4-methyl-1H-pyrrol-2-yl)-[1,2,4]oxadiazol-5-yl]-piperidine trifluoroacetate, prepared as described in Example 31 (E), and using 6-fluoro-nicotinic acid as the acid of choice. The final compound was purified by flash chromatography (silica gel, eluent gradient: from hexane/ethyl acetate 100:0 to hexane/ethyl acetate 0:100).
Yield: 93% (white solid); $[\alpha]_D^{20}$ = +131 (c=0.5, MeOH); LCMS (RT): 2.58 min (Method N); MS (ES+) gave m/z: 356.1 (MH+).
$^1$H-NMR (DMSO-$d_6$, 353K), δ (ppm): 11.16 (s br, 1H); 8.31 (m, 1H); 8.02 (ddd, 1H); 7.22 (dd, 1H); 6.74 (m, 1H); 6.56 (m, 1H); 4.21 (m, 1H); 3.76 (m, 1H); 3.54 (dd, 1H); 3.43-3.27 (m, 2H); 2.22 (m, 1H); 2.08 (s, 3H); 2.03-1.75 (m, 2H); 1.66 (m, 1H).

Example 34

(2-Fluoro-pyridin-4-yl)-{(S)-3-[3-(4-methyl-1H-pyrrol-2-yl)-[1,2,4]oxadiazol-5-yl]-piperidin-1-yl}-methanone

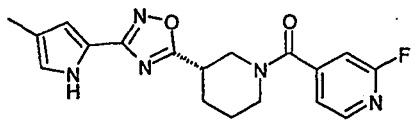

The compound was prepared following the procedure described in the Example 28 (C), starting from (S)-3-[3-(4-methyl-1H-pyrrol-2-yl)-[1,2,4]oxadiazol-5-yl]-piperidine trifluoroacetate, prepared as described in Example 31 (E), and using 2-fluoro-isonicotinic acid as the acid of choice. The final compound was purified by flash chromatography (silica gel, eluent gradient: from hexane/ethyl acetate 100:0 to hexane/ethyl acetate 1:1).
Yield: 49% (white glass); $[\alpha]_D^{20}$= +113 (c=0.67, MeOH); LCMS (RT): 3.68 min (Method P); MS (ES+) gave m/z: 356.4 (MH+).
$^1$H-NMR (DMSO-$d_6$, 353K), δ (ppm): 11.15 (s br, 1H); 8.32 (m, 1H); 7.34 (ddd, 1H); 7.16 (m, 1H); 6.74 (m, 1H); 6.56 (m, 1H); 4.18 (m br, 1H); 3.69 (m br, 1H); 3.53 (dd, 1H); 3.43-3.24 (m, 2H); 2.22 (m, 1H); 2.08 (s, 3H); 2.03-1.75 (m, 2H); 1.67 (m, 1H).

Example 35

(5-Methyl-isoxazol-4-yl)-{(S)-3-[3-(4-methyl-1H-pyrrol-2-yl)-[1,2,4]oxadiazol-5-yl]-piperidin-1-yl}-methanone

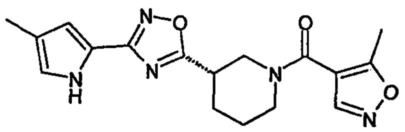

The compound was prepared following the procedure described in the Example 28 (C), starting from (S)-3-[3-(4-methyl-1H-pyrrol-2-yl)-[1,2,4]oxadiazol-5-yl]-piperidine trifluoroacetate, prepared as described in Example 31 (E), and using 5-methyl-isoxazole-4-carboxylic acid as the acid of choice. The final compound was purified by flash chromatography (silica gel, eluent gradient: from hexane/ethyl acetate 100:0 to hexane/ethyl acetate 1:1). Yield: 68% (colourless gum); $[\alpha]_D^{20}$ = +102.5 (c=0.62, MeOH); LCMS (RT): 2.5 min (Method N); MS (ES+) gave m/z: 342.3 (MH+).

$^1$H-NMR (DMSO-$d_6$, 353K), δ (ppm): 11.15 (s br, 1H); 8.58 (m, 1H); 6.74 (m, 1H); 6.56 (m, 1H); 4.22 (m, 1H); 3.78 (dt, 1H); 3.54 (dd, 1H); 3.42-3.27 (m, 2H); 2.46 (d, 3H); 2.22 (m, 1H); 2.08 (m, 3H); 2.03-1.76 (m, 2H); 1.65 (m, 1H).

Example 36

(4-Fluoro-phenyl)-{(S)-3-[5-(4-nitro-1H-pyrrol-2-yl)-[1,2,4]oxadiazol-3-yl]-piperidin-1-yl}-methanone

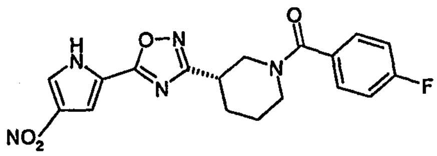

A mixture of 4-nitro-pyrrole-2-carboxylic acid (200 mg, 1.28 mmol), EDCI.HCl (370 mg, 1.92 mmol) and HOAT (175 mg, 1.28 mmol) in dioxane (70 mL) was stirred at 50°C for 1h, then (S)-1-(4-fluoro-benzoyl)-N-hydroxy-piperidine-3-carboxamidine (340 mg, 1.28 mmol), prepared as described in Example 27 (D), was added and the mixture was stirred at 80°C overnight, then for a weekend at room temperature and then under reflux for 20h. Solvent was removed. The residue was diluted with ethyl acetate and water, the phases were separated and the organic layer was washed with $Na_2CO_3$ (aq), dried over $Na_2SO_4$ and concentrated under reduced pressure. Purification of the crude by flash chromatography (silica gel, eluent gradient: from DCM/MeOH 99:1 to DCM/MeOH 97:3) gave a solid that was triturated from diisopropylether.

Yield: 34% (White powder); $[\alpha]_D^{20}$ = +92.8 (c=0.91 MeOH); mp= 157-158°C; LCMS (RT): 6.47 min (Method Q); MS (ES+) gave m/z: 386.1 (MH+).
$^1$H-NMR (DMSO-$d_6$, 368K), δ (ppm): 13.10 (s br, 1H); 8.02 (d, 1H); 7.45 (dd, 2H); 7.43 (m, 1H); 7.20 (dd, 2H); 4.26 (m, 1H); 3.82 (m, 1H); 3.38 (dd, 1H); 3.23 (ddd, 1H); 3.14 (m, 1H); 2.27-2.16 (m, 1H); 1.99-1.77 (m, 2H); 1.71-1.55 (m, 1H).

Example 37

(4-Fluoro-phenyl)-{(R)-3-[3-(1H-pyrrol-2-yl)-[1,2,4]oxadiazol-5-yl]-piperidin-1-yl}-methanone

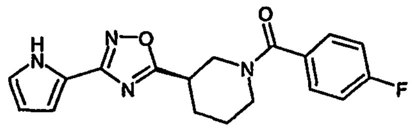

The title compound was prepared following the experimental procedure described in Example 1, starting from 1H-pyrrole-2-carbonitrile and using (R)-N-Boc-nipecotic acid. Purification of the final compound was performed by flash chromatography (silica gel, eluent gradient: from hexane/ethyl acetate 7:3 to hexane/ethyl acetate 1:1). The resulting colourless oil was triturated with diisopropylether to give (4-fluorophenyl)-{(R)-3-[3-(1H-pyrrol-2-yl)-[1,2,4]oxadiazol-5-yl]-piperidin-1-yl}-methanone as a white solid.
Yield: 47% (White powder); $[\alpha]_D^{20}$ = -125.7 (c=0.98, MeOH); mp= 132-133°C; LCMS (RT): 6.71 min (Method C); MS (ES+) gave m/z: 341.1 (MH+).
$^1$H-NMR (DMSO-$d_6$), δ (ppm): 11.54 (s br, 1H); 7.46 (dd, 2H); 7.23 (dd, 2H); 6.97 (m, 1H); 6.74 (m, 1H); 6.21 (m, 1H); 4.22 (m, 1H); 3.77 (m, 1H); 3.50 (dd, 1H); 3.39-3.21 (m, 2H); 2.24 (m, 1H); 2.02-1.75 (m, 2H); 1.63 (m, 1H).

Example 38

(4-Fluoro-phenyl)-{(S)-3-[5-(5-methyl-1H-pyrrol-2-yl)-[1,2,4]oxadiazol-3-yl]-piperidin-1-yl}-methanone

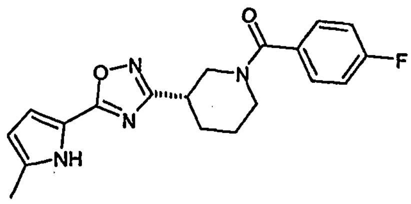

38(A) 5-Methyl-1H-pyrrole-2-carboxylic acid

A solution of 5-methyl-1H-pyrrole-2-carboxylic acid ethyl ester (400 mg, 2.61 mmol), prepared as described in Curran, T.; Keaney, M.; J. Org. Chem., 61 (25), 1996, 9068-9069, and sodium hydroxide (520 mg, 13 mmol) in dioxane/water/ethanol (10 mL/1 mL/2 mL) was refluxed for 3h. The solvent was removed and the crude was partitioned between water and DCM. 1N HCl was added to adjust the pH to 1 and the phases were separated. The organic layer was dried over sodium sulphate and evaporated under vacuum to give a solid that was used for the next step without further purification.
Yield: quantitative; LCMS (RT): 2.51 min (Method D); MS (ES+) gave m/z: 126.03 (MH+).

38(B) (4-Fluoro-phenyl)-{(S)-3-[5-(5-methyl-1H-pyrrol-2-yl)-[1,2,4]oxadiazol-3-yl]-piperidin-1-yl}-methanone

A mixture of 5-methyl-1H-pyrrole-2-carboxylic acid (236 mg, 1.89 mmol), (S)-1-(4-fluoro-benzoyl)-N-hydroxy-piperidine-3-carboxamidine (500 mg, 1.89 mmol), prepared as described in Example 27 (D), EDCI.HCl (543 mg, 2.84 mmol) and HOAT (257 mg, 1.89 mmol) in DCM (15 mL) was stirred at room temperature overnight, then the solvent was removed and the residue was dissolved in dioxane and refluxed for 24h. Solvent was removed and the residue was diluted with ethyl acetate and water, the phases were separated and the organic layer was washed with $Na_2CO_3$ (aq),

then with 1N HCl, dried over $Na_2SO_4$ and concentrated under reduced pressure. Purification of the crude was performed by preparative HPLC.
Yield: 1% (black oil); LCMS (RT): 7.41 min (Method C); MS (ES+) gave m/z: 355.2 (MH+).
$^1$H-NMR (DMSO-$d_6$ 343K), δ (ppm): 10.86 (s br, 1H); 8.15 (dd" 2H); 7.44 (dd, 2H); 6.39 (m, 1H); 5.82 (m, 1H); 4.56 (m, 1H); 4.23 (m, 1H); 3.44-3.18 (m, 2H); 3.09 (m, . 1H); 2.24 (m, 1H); 2.20 (s, 3H); 1.99-1.80 (m, 2H); 1.61 (m, 1H).

Example 39

((S)-3-[5-(4-Chloro-1H-pyrrol-2-yl)-[1,2,4]oxadiazol-3-yl]-piperidin-1-yl)-(4-fluorophenyl)-methanone

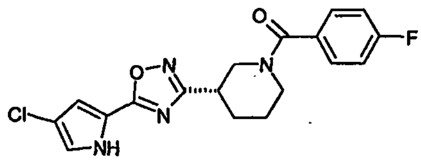

39(A) 4-Chloro-1H-pyrrole-2-carboxylic acid

A mixture of 2,2,2-trichloro-1-(4-chloro-1H-pyrrol-2-yl)-ethanone (14.12 mmol), prepared as described in Belanger; Tetrahedron Lett.; 1979; 2505-2508, and 5 mL of 10% NaOH (aq) in THF (10 mL) was stirred at room temperature for 1h. The solvent was removed and the crude was partitioned between water and ethyl acetate, then 10% HCl was added to adjust the pH to 5. The phases were separated, the aqueous layer was re-extracted with ethyl acetate, the combined organics were dried over magnesium sulphate. After evaporation, 4-Chloro-1H-pyrrole-2-carboxylic acid was obtained as a solid, which was used for the next step without further purification.
Yield: quantitative; LCMS (RT): 3.3 min (Method D); MS (ES+) gave m/z: 145.9 and 147.9 (MH+).

39(B) (S)-3-[5-(4-Chloro-1H-pyrrol-2-yl)-[1,2,4]oxadiazol-3-yl]-piperidine-1-carboxylic acid tert-butyl ester

A mixture of 4-chloro-1H-pyrrole-2-carboxylic acid (769 mg, 5.28 mmol), (S)-3-(N-hydroxycarbamimidoyl)-piperidine-1-carboxylic acid tert-butyl ester (4.8 mmol), prepared as described in Example 10 (C), EDCI.HCl (1.38 g, 7.2 mmol) and HOAT (653 mg, 4.8 mmol) in dioxane (15 mL) was stirred at room temperature overnight. Solvent was removed and the residue was diluted with ethyl acetate and water, the phases were separated and the organic layer was washed with 1M NaOH (aq), then dried over $Na_2SO_4$ and concentrated under reduced pressure.
The residue was dissolved in acetonitrile (2 mL), in the presence of few 4A molecular sieves, and heated at 100°C for 50 min, in a sealed tube, in a microwaves oven. The solvent was removed and the crude was passed through a silica gel short pad (eluent: petroleum ether/ethyl acetate 2:1) to afford (S)-3-[5-(4-chloro-1H-pyrrol-2-yl)-[1,2,4]oxadiazol-3-yl]-piperidine-1-carboxylic acid tert-butyl ester (250 mg).
Yield: 73% (yellow oil); LCMS (RT): 5.42 min (Method E); MS (ES+) gave m/z: 353.08 (MH+).

39(C) (S)-3-[5-(4-Chloro-1H-pyrrol-2-yl)-[1,2,4]oxadiazol-3-yl]-piperidine hydrochloride

To a solution of (S)-3-[5-(4-methyl-1H-pyrrol-2-yl)-[1,2,4]oxadiazol-3-yl]-piperidine-1-carboxylic acid tert-butyl ester (0.25 g, 0.71 mmol) in dichloromethane (10 mL), 1.7 mL of 4N HCl (dioxane solution) were added at 0°C and the reaction mixture was allowed to warm at room temperature and stirred for 3 h. The solvent was evaporated under reduced pressure to give the title compound, which was used for the next step without further purification.
Yield: 92%; LCMS (RT): 3.0 min (Method D); MS (ES+) gave m/z: 253.1 (MH+).

39(D) {(S)-3-[5-(4-Chloro-1H-pyrrol-2-yl)-[1,2,4]oxadiazol-3-yl]-piperidin-1-yl}-(4-fluoro-phenyl)-methanone

The compound was prepared following the procedure described in the Example 1(C), starting from (S)-3-[5-(4-chloro-1H-pyrrol-2-yl)-[1,2,4]oxadiazol-3-yl]-piperidine hydrochloride, prepared as described in Example 39 (C), and using 4-fluorobenzoyl chloride as the acylating agent. The final compound was purified by flash chromatography (silica gel, eluent: petroleum ether/ethyl acetate 1:2).
Yield: 79% (white solid); LCMS (RT): 3.00 min (Method N); MS (ES+) gave m/z: 375.2 (MH+).
$^1$H-NMR (DMSO-$d_6$ 353K), δ (ppm): 7.46 (dd, 2H); 7.22 (dd, 2H); 7.20 (m, 1H); 6.94 (d, 1H); 4.25 (m, 1H); 3.83 (m, 1H);

3.33 (dd, 1H); 3.20 (ddd, 1H); 3.09 (m, 1H); 2.19 (m, 1H); 1.96-1.76 (m, 2H); 1.62 (m, 1H).

Example 40

{(S)-3-[5-(4-Chloro-1H-pyrrol-2-yl)-[1,2,4]oxadiazol-3-yl]-piperidin-1-yl}-(6-fluoro-pyridin-3-yl)-methanone

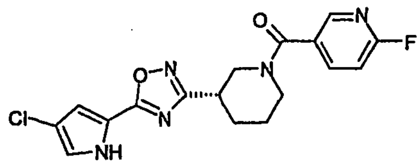

The title compound was prepared following the experimental procedure described in Example 28(C), starting from (S)-3-[5-(4-chloro-1H-pyrrol-2-yl)-[1,2,4]oxadiazol-3-yl]-piperidine hydrochloride, prepared as described in Example 39 (C), and using 6-fluoro-nicotinic acid as the acid of choice.
Purification was performed by flash chromatography (silica gel, eluent: petroleum ether/ethyl acetate 1:2).
Yield: 82% (White solid); $[\alpha]_D^{20}$ = +109.8 (c=1.08, MeOH); LCMS (RT): 2.69 min (Method N); MS (ES+) gave m/z: 376.3 (MH+).
$^1$H-NMR (DMSO-$d_6$ 353K), δ (ppm): 12.37 (s br, 1H); 8.31 (m, 1H); 8.02 (ddd, 1H); 7.23-7.18 (m, 2H); 6.94 (d, 1H); 4.24 (m, 1H); 3.81 (m, 1H); 3.38 (dd, 1H); 3.27 (ddd, 1H); 3.14 (m, 1H); 2.20 (m, 1H); 1.98-1.76 (m, 2H); 1.66 (m, 1H).

Example 41

((S)-3-[5-(4-Chloro-1H-pyrrol-2-yl)-[1,2,4]oxadiazol-3-yl]-piperidin-1-yl)-(2-fluoro-pyridin-4-yl)-methanone

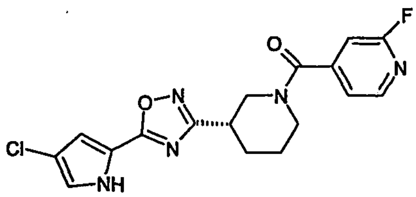

The title compound was prepared following the experimental procedure described in Example 28(C), starting from (S)-3-[5-(4-chloro-1H-pyrrol-2-yl)-[1,2,4]oxadiazol-3-yl]-piperidine hydrochloride, prepared as described in Example 39 (C), and using 2-fluoro-pyridine-4-carboxylic acid as the acid of choice. Purification was performed by flash chromatography (silica gel, eluent: petroleum ether/ethyl acetate 1:2).

Yield: 86% (White solid); $[\alpha]_D^{20}$ = +94.5 (c=0.92, MeOH); LCMS (RT): 2.69 min (Method N); MS (ES+) gave m/z: 376.2 (MH+).
$^1$H-NMR (DMSO-$d_6$ 373K), δ (ppm): 12.24 (s br, 1H); 8.31 (m, 1H); 7.32 (ddd, 1H); 7.18 (d, 1H); 7.13 (m, 1H); 6.93 (d, 1H); 4.19 (m, 1H); 3.74 (m, 1H); 3.39 (dd, 1H); 3.26 (ddd, 1H); 3.15 (m, 1H); 2.20 (m, 1H); 1.98-1.76 (m, 2H); 1.67 (m, 1H).

Example 42

((S)-3-[5-(4-Chloro-1H-pyrrol-2-yl)-[1,2,4]oxadiazol-3-yl]-piperidin-1-yl)-(5-methyl-isoxazol-4-yl)-methanone

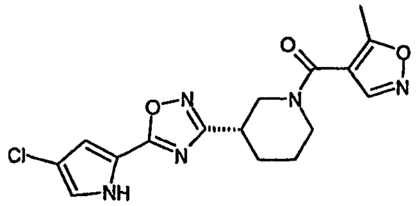

The title compound was prepared following the experimental procedure described in Example 28(C), starting from (S)-3-[5-(4-chloro-1H-pynol-2-yl)-[1,2,4]oxadiazol-3-yl]-piperidine hydrochloride, prepared as described in Example 39 (C), and using 5-methyl-isoxazole-4-carboxylic acid as the acid of choice.
Purification was performed by flash chromatography (silica gel, eluent: petroleum ether/ethyl acetate 1:2).
Yield: 91% (White solid); $[\alpha]_D{}^{20}$ = +90.2 (c = 1.05, MeOH); LCMS (RT): 2.63 min (Method N); MS (ES+) gave m/z: 362.2 (MH+).
$^1$H-NMR (DMSO-$d_6$ 373K), δ (ppm): 12.27 (s br, 1H); 8.53 (m, 1H); 7.18 (d, 1H); 6.94 (d, 1H); 4.25 (m, 1H); 3.84 (m, 1H); 3.39 (dd, 1H); 3.28 (ddd, 1H); 3.10 (m, 1H); 2.47 (d, 3H); 2.20 (m, 1H); 1.98-1.79 (m, 2H); 1.64 (m, 1H).

Example 43

{(S)-3-[3-(4-Chloro-1H-pyrrol-2-yl)-[1,2,4]oxadiazol-5-yl]-piperidin-1-yl}-(4-fluorophenyl)-methanone

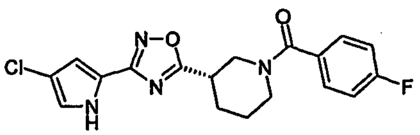

43(A) 4-Chloro-1H-pyrrole-2-carboxylic acid amide

A solution of 2,2,2-trichloro-1-(4-chloro-1H-pyrrol-2-yl)-ethanone (7.6 mmol), prepared as described in Belanger; Tetrahedron Lett.; 1979; 2505-2508, and conc. $NH_4OH$ (15 mL) in acetonitrile (15 mL) was refluxed for 10 min. The solvent was removed and the crude was partitioned between water and ethyl acetate, the organic layer was then dried over sodium sulphate and evaporated under reduced pressure. The crude was purified by flash chromatography (silica gel, eluent: petroleum ether/ethyl acetate 4:6).
Yield: 100%; LCMS (RT): 2.37 min (Method D); MS (ES+) gave m/z: 145.17 (MH+).

43(B) 4-Chloro-1H-pyrrole-2-carbonitrile

A solution of 4-chloro-1H-pyrrole-2-carboxylic acid amide (570 mg, 3.94 mmol) and phosphorus oxychloride (370 μL, 3.94 mmol) in pyridine (10 mL) was stirred at room temperature overnight, then the mixture was diluted with ethyl acetate and washed with 10% HCl (twice). The organic layer was dried over sodium sulphate and evaporated under reduced pressure to give a crude that was purified by flash chromatography (silica gel, eluent: petroleum ether/ethyl acetate 9:1).
Yield: 22%; LCMS (RT): 3.97 min (Method D); MS (ES+) gave m/z: 127.13 (MH+).

43(C) 4-Chloro-N-hydroxy-1H-pyrrole-2-carboxamidine

The compound was prepared following the same experimental procedure described in Example 31 (C), starting from 4-chloro-1H-pyrrole-2-carbonitrile.
Yield: 100%; LCMS (RT): 0.71 min (Method D); MS (ES+) gave m/z: 160.21 (MH+).

43(D) (S)-3-[3-(4-Chloro-1H-pyrrol-2-yl)-[1,2,4]oxadiazol-5-yl]-piperidine-1-carboxylic acid tert-butyl ester

A mixture of (S)-N-Boc-nipecotic acid (199 mg, 0.87 mmol), 4-chloro-N-hydroxy-1H-pyrrole-2-carboxamidine (0.87 mmol), HOAT (119 mg, 0.87 mmol), EDCI.HCl (250 mg, 1.305 mmol) in dry dioxane (10 mL) was heated at 80°C for 16 h, under nitrogen atmosphere. The solvent was removed under vacuum, the residue was partitioned between

water and ethyl acetate, the phases were separated. The organic layer was dried over sodium sulphate to give a residue that was purified by flash chromatography (silica gel, eluent: petroleum ether/ethyl acetate 8:2).
Yield: 20%; LCMS (RT): 6.03 min (Method D); MS (ES+) gave m/z: 353.0 (MH+).

43(E) (S)-3-[3-(4-Chloro-1H-pyrrol-2-yl)-[1,2,4]oxadiazol-5-yl]-piperidine hydrochloride

To a solution of (S)-3-[3-(4-chloro-1H-pyrrol-2-yl)-[1,2,4]oxadiazol-5-yl]-piperidine-1-carboxylic acid tert-butyl ester (60 mg, 0.17 mmol) in dichloromethane (2 mL), 1.0 mL of 4N HCl (dioxane solution) was added at 0°C and the reaction mixture was allowed to warm at room temperature and stirred for 1 h. The solvent was evaporated under reduced pressure to give the title compound, which was used for the next step without further purification.
Yield: quantitative; LCMS (RT): 2.68 min (Method D); MS (ES+) gave m/z: 253.28 (MH+).

43(F) {(S)-3-[3-(4-Chloro-1H-pyrrol-2-yl)-[1,2,4]oxadiazol-5-yl]-piperidin-1-yl}-(4-fluoro-phenyl)-methanone

The compound was prepared following the procedure described in the Example 1(C), starting from (S)-3-[3-(4-chloro-1H-pyrrol-2-yl)-[1,2,4]okadiazol-5-yl]-piperidine hydrochloride, prepared as described in Example 43 (E), and using 4-fluorobenzoyl chloride as the acylating agent. The final compound was purified by preparative HPLC.
Yield: 31% (pink solid); $[\alpha]_D^{20}$ = +114.1 (c=0.80, $CH_3OH$); LCMS (RT): 6.01 min (Method R); MS (ES+) gave m/z: 375.1 (MH+).
$^1$H-NMR (DMSO-$d_6$ 353K), δ (ppm): 11.83 (s br, 1H); 7.45 (dd, 2H); 7.22 (dd, 2H); 7.03 (dd, 1H); 6.69 (dd, 1H); 4.22 (m, 1H); 3.75 (m, 1H); 3.51 (dd, 1H); 3.41-3.19 (m, 2H); 2.24 (m, 1H); 2.04-1.75 (m, 2H); 1.64 (m, 1H).

Example 44

{(S)-3-[5-(4-Bromo-1H-pyrrol-2-yl)-[1,2,4]oxadiazol-3-yl]-piperidin-1-yl}-(6-fluoro-pyridin-3-yl)-methanone

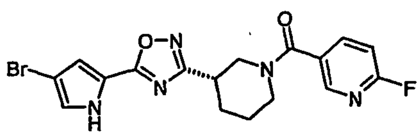

44 (A) (S)-1-(6-Fluoro-pyridine-3-carbonyl)-N-hydroxy-piperidine-3-carboxamidine

(S)-3-Cyano-piperidine-1-carboxylic acid tert-butyl ester (2.33 g, 11.1 mmol), prepared as described in Example 27 (B), was dissolved in DCM (15 mL) and 9 mL of HCl 4N (dioxane solution) were added dropwise at 0°C. The resulting mixture was stirred at room temperature for 1.5h. The solvent was evaporated under reduced pressure to afford (S)-piperidine-3-carbonitrile hydrochloride as a white solid, that was used for the next step without further purification.
A mixture of (S)-piperidine-3-carbonitrile hydrochloride (11.1 mmol) 6-fluoro-nicotinic acid (1.6 g, 11.1 mmol), HOBT (2.24 g, 16.6 mmol), EDCI.HCl (2.13 g, 11.1 mmol) and triethylamine (3.1 mL, 22.2 mmol) in dry DCM (20 mL) was kept under stirring at RT overnight, under nitrogen atmosphere. The mixture was diluted with DCM and was washed sequentially with 5% $Na_2CO_3$ (aq) (10 mL, twice) and with brine. The organic layer was dried over sodium sulphate and the solvent was removed under vacuum to give a residue that was purified by flash chromatography (silica gel, eluent: DCM/MeOH 98:2) to give 1.36 g of (S)-1-(6-fluoro-pyridine-3-carbonyl)-piperidine-3-carbonitrile.
A solution of (S)-1-(6-fluoro-pyridine-3-carbonyl)-piperidine-3-carbonitrile (150 mg, 0.64 mmol) and aqueous hydroxylamine (50% in water, 160 uL, 2.6 mmol) in ethanol (5 mL) was refluxed for 4h. The solvent was evaporated under reduced pressure to afford the title compound that was used for the next step without further purification. Yield: quantitative; HPLC (RT): 1.48 min (Method F).

44 (B) 4-Bromo-1H-pyrrole-2-carboxylic acid

A solution of 1-(4-bromo-1H-pyrrol-2-yl)-2,2,2-trichloro-ethanone (4.7 mmol), prepared as described in Belanger; Tetrahedron Lett.; 1979; 2505-2508, and 1 mL of 10% NaOH (aq) in THF (5 mL) was stirred at room temperature for 1h. The solvent was removed and the crude was partitioned between water and ethyl acetate, then 10% HCl was added to adjust the pH to 5. The phases were separated, the aqueous layer was re-extracted with ethyl acetate, the combined organics were dried over magnesium sulphate. After evaporation, 4-bromo-1H-pyrrole-2-carboxylic acid was

obtained as a solid, which was used for the next step without further purification. Yield: 64%; LCMS (RT): 2.74 min (Method B); MS (ES+) gave m/z: 191 and 193.

44 (C) {(S)-3-[5-(4-Bromo-1H-pyrrol-2-yl)-[1,2,4]oxadiazol-3.7yl]-piperidin-1-yl}-(6-fluoro-pyridin-3-yl)-methanone

A solution of 4-bromo-1H-pyrrole-2-carboxylic acid (134 mg, 0.704 mmol), (S)-1-(6-fluoro-pyridine-3-carbonyl)-N-hydroxy-piperidine-3-carboxamidine (0.64 mmol), EDC (184 mg, 0.96 mmol), HOAT (87 mg, 0.64 mmol) in dioxane (5 mL) was stirred at room temperature overnight. The solvent was removed, the crude was diluted with DCM and washed with 1N NaOH, the organic layer was dried over sodium sulphate and evaporated under reduced pressure to give a solid that was purified by flash chromatography (silica gel, eluent: DCM/MeOH 9:1). The solid obtained after this purification was dissolved in acetonitrile and heated at 110°C for 6h, in a sealed tube, in a microwaves oven, then another heating cycle was performed (6h, 130°C, microwaves). The solvent was evaporated under reduced pressure and the crude was purified by preparative HPLC.
Yield: 11% (yellow oil); $[\alpha]_D^{20}$ = +95.19 (c=1.2, $CH_3OH$); LCMS (RT): 2.80 min (Method N); MS (ES+) gave m/z: 420.0 (MH+).
$^1$H-NMR (DMSO-$d_6$ 353K), δ (ppm): 12.36 (s br, 1H); 8.30 (m, 1H); 8.01 (ddd, 1H); 7.22 (d, 1H); 7.19 (dd, 1H); 6.99 (d, 1H); 4.23 (m, 1H); 3.80 (m, 1H); 3.39 (dd, 1H); 3.27 (ddd, 1H); 3.14 (m, 1H); 2.20 (m, 1H); 1.98-1.76 (m, 2H); 1.66 (m, 1H).

Example 45

{(S)-3-[3-(4-Bromo-1H-pyrrol-2-yl)-[1,2,4]oxadiazol-5-yl]-piperidin-1-yl}-(4-fluorophenyl)-methanone

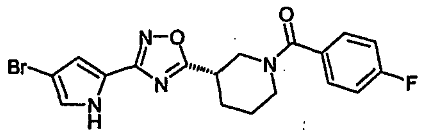

45 (A) (S)-3-[3-(4-Bromo-1H-pyrrol-2-yl)-[1,2,4]oxadiazol-5-yl]-piperidine hydrochloride
The compound was prepared starting from 1-(4-bromo-1H-pyrrol-2-yl)-2,2,2-trichloro-ethanone (prepared as described in Belanger; Tetrahedron Lett.; 1979; 2505-2508) according to the experimental procedures described in Examples 43 (A), 43 (B), 43 (C), 43 (D) and 43 (E).
LCMS (RT): 2.93 min (Method D); MS (ES+) gave m/z: 297.17 (MH+).

45 (B) {(S)-3-[3-(4-Bromo-1H-pyrrol-2-yl)-[1,2,4]oxadiazol-5-yl]-piperidin-1-yl}-(4-fluoro-phenyl)-methanone

The compound was prepared following the procedure described in the Example 1(C), starting from (S)-3-[3-(4-bromo-1H-pyrrol-2-yl)-[1,2,4]oxadiazol-5-yl]-piperidine hydrochloride, prepared as described in Example 45 (A), and using 4-fluorobenzoyl chloride as the acylating agent. The final compound was purified by flash chromatography (silica gel, eluent: petroleum ether/ethyl acetate 7:3) and then by preparative HPLC.
Yield: 26% (white solid); $[\alpha]_D^{20}$ = +123.3 (c=0.73, $CH_3OH$); LCMS (RT): 6.08 min (Method R); MS (ES+) gave m/z: 419.1 (MH+).
$^1$H-NMR (DMSO-$d_6$ 353K), δ (ppm): 11.89 (s br, 1H); 7.45 (dd, 2H); 7.22 (dd, 2H); 7.06 (d, 1H); 6.75 (d, 1H); 4.22 (m, 1H); 3.75 (m, 1H); 3.51 (dd, 1H); 3.41-3.21 (m, 2H); 2.24 (m, 1H); 2.04-1.76 (m, 2H); 1.63 (m, 1H).

Example 46

{(S)-3-[3-(4-Bromo-1H-pyrrol-2-yl)-[1,2,4]oxadiazol-5-yl]-piperidin-1-yl}-(6-fluoro-pyridin-3-yl)-methanone

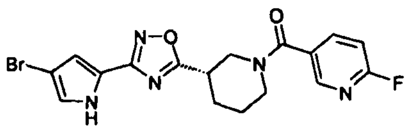

The title compound was prepared following the experimental procedure described in Example 28(C), starting from (S)-3-[3-(4-bromo-1H-pyrrol-2-yl)-[1,2,4]oxadiazol-5-yl]-piperidine hydrochloride, prepared as described in Example 45 (A), and using 6-fluoro-nicotinic acid as the acid of choice. The final compound was purified by flash chromatography (silica gel, eluent: DCM/MeOH 99:1) and then by preparative HPLC.

Yield: 30% (white gummy solid); LCMS (RT): 2.72 min (Method N); MS (ES+) gave m/z: 419.9 (MH+).
$^1$H-NMR (DMSO-$d_6$ 353K), δ (ppm): 11.91 (s br, 1H); 8.30 (m, 1H); 8.01 (dd, 1H); 7.21 (dd, 1H); 7.06 (dd, 1H); 6.75 (dd, 1H); 4.23 (m, 1H); 3.76 (m, 1H); 3.55 (dd, 1H); 3.45-3.27 (m, 2H); 2.25 (m, 1H); 2.05-1.76 (m, 2H); 1.67 (m, 1H).

Example 47 (DELETED)

Example 49

{3,3-Dimethyl-5-[3-(1H-pyrrol-2-yl)-[1,2,4]oxadiazol-5-yl]-piperidin-1-yl}-(4-fluorophenyl)-methanone

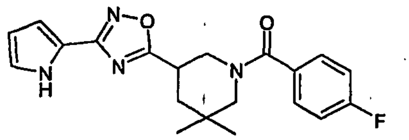

49 (A) 3,3-Dimethyl-4-oxo-piperidine-1-carboxylic acid tert-butyl ester

A solution of 4-oxo-piperidine-1-carboxylic acid tert-butyl ester (500 mg, 4.2 mmol) in dry THF (10 mL) was cooled to 10°C under nitrogen atmosphere. NaH (403 mg, 9.2 mmol) and $CH_3I$ (664 μL, 10.5 mmol) were added and the mixture was stirred at 10°C for 30 min. The solvent was removed under reduced pressure and the crude was partitioned between diethyl ether and brine. The two layers were separated and the organic layer was dried over $Na_2SO_4$. The solvent was removed under reduced pressure and the crude 3,3-dimethyl-4-oxo-piperidine-1-carboxylic acid tert-butyl ester was used in the next step without further purification
Yield: 73%; $^1$H-NMR ($CDCl_3$, 300MHz): 1.05 (s, 6H), 1.45 (s, 9H), 2.50 (t, 2H), 3.40 (s, 2H), 3.75 (t, 2H).

49 (B) 5,5-Dhnethyl-4-oxo-piperidine-1,3-dicarboxylic acid 1-tert-butyl ester 3-methyl ester

A solution of 3,3-dimethyl-4-oxo-piperidine-1-carboxylic acid tert-butyl ester (1.8 g, 7.9 mmol) in dry THF (30 mL) was cooled to -78°C under nitrogen atmosphere. LHMDS (1M in THF, 9.5 mL, 9.5 mmol) was added, stirring was maintained at -78°C for 1h, then $CNCO_2Me$ (752 μL, 9.5 mmol) was slowly added. The mixture was stirred at -78°C for 10 min, then $H_2O$ (30 mL) was added. The reaction was allowed to warm to room temperature. THF was removed under reduced pressure, then the aqueous phase was extracted with ethyl acetate (3x30 mL). The combined organic layers were dried over $Na_2SO_4$, then the solvent was removed under reduced pressure and the crude 5,5-dimethyl-4-oxo-piperidine-1,3-dicarboxylic acid 1-tert-butyl ester 3-methyl ester was used in the next step without further purification
Yield: 100%; LCMS (RT): 6.39 min (Method D); MS (ES+) gave m/z: 286.2 (MH+).

49 (C) 1-(4-Fluoro-benzoyl)-5,5-dimethyl-4-oxo-piperidine-3-carboxylic acid methyl ester

A solution of 5,5-dimethyl-4-oxo-piperidine-1,3-dicarboxylic acid-1-tert-butyl ester-3-methyl ester (200 mg, 0.70 mmol) in DCM (5 mL) was cooled at 0°C. HCl (4M in dioxane, 1.5 mL, 6 mmol) was added and the mixture was stirred at room temperature for 1h. The solvent was removed under reduced pressure and the crude was dissolved in DCM (5 mL). Triethylamine (293 μL, 2.1 mmol) and 4-fluorobenzoyl chloride (99 μL, 0.84 mmol) were added and the mixture was stirred at room temperature for 2h. The organic layer was washed with 1M HCl (2x5 mL), with $NaHCO_3$ (2x5 mL), then it was dried over $Na_2SO_4$. The solvent was removed under reduced pressure and the crude was purified by flash chromatography (silica gel, eluent: hexane/ethyl acetate 10:1) to yield 1-(4-fluoro-benzoyl)-5,5-dimethyl-4-oxo-piperidine-3-carboxylic acid methyl ester.
Yield: 21%; LCMS (RT): 5.28 min (Method D); MS (ES+) gave m/z: 308.16 (MH+).

49 (D) 1-(4-Fluoro-benzoyl)-4-hydroxy-5,5-dimethyl-piperidine-3-carboxylic acid methyl ester

To a solution of 1-(4-fluoro-benzoyl)-5,5-dimethyl-4-oxo-piperidine-3-carboxylic acid methyl ester (80 mg, 0.26 mmol) in MeOH (1 mL), $NaBH_4$ (10 mg, 0.26 mmol) was added. The mixture was stirred at room temperature for 15 min, then acetone (5 mL) was added. The solvent was removed under reduced pressure, the crude was dissolved in ethyl acetate and washed with 1M HCl (2x5 mL). The crude 1-(4-fluoro-benzoyl)-4-hydroxy-5,5-dimethyl-piperidine-3-carboxylic acid methyl ester was used in the next step without further purification.
Yield: 100%; LCMS (RT): 3.73 min (Method D); MS (ES+) gave m/z: 310.29 (MH+).

49 (E) 1-(4-Fluoro-benzoyl)-5,5-dimethyl-1,2,5,6-tetrahydro-pyridine-3-carboxylic acid methyl ester

A solution of 1-(4-fluoro-benzoyl)-4-hydroxy-5,5-dimethyl-piperidine-3-carboxylic acid methyl ester (280 mg, 0.91 mmol) in DCM (10 mL) was cooled at 0°C, then triethylamine (380 μL, 2.73 mmol) and MsC1 (106 μL, 1.37 mmol) were added. The mixture was stirred at room temperature for 3h, then the solution was washed with $H_2O$ (2x10 mL) and dried over $Na_2SO_4$. The solvent was removed under reduced pressure and the crude was dissolved in toluene (5 mL). DBU (272 μL, 1.82 mmol) was added and the mixture was heated at 80°C for 30 min. The solution was diluted with DCM and washed with 1M HCl (2x15 mL). The organic layer was dried over $Na_2SO_4$ then the solvent was removed under reduced pressure. The crude was purified by flash chromatography (silica gel, eluent: DCM/Methanol 100:1) to yield 1-(4-fluoro-benzoyl)-5,5-dimethyl-1,2,5,6-tetrahydro-pyridine-3-carboxylic acid methyl ester.
Yield: 48%; LCMS (RT): 4.86 min (Method D) MS (ES+) gave m/z: 292.24 (MH+).

49 (F) 1-(4-Fluoro-benzoyl)-5,5-dimethyl-piperidine-3-carboxylic acid methyl ester

To a suspension of 10% Pd/C (20 mg) in EtOH (10 mL), 1-(4-fluoro-benzoyl)-5,5-dimethyl-1,2,5,6-tetrahydro-pyridine-3-carboxylic acid methyl ester (125 mg, 0.43 mmol) was added. The mixture was hydrogenated (40 psi, room temperature) overnight. The mixture was then filtered over a celite pad, the solvent was removed under reduced pressure and the crude was purified by flash chromatography (silica gel, eluent: hexane/ethyl acetate 80:20) to yield 1-(4-fluoro-benzoyl)-5,5-dimethyl-piperidine-3-carboxylic acid methyl ester.
Yield: 37%; LCMS (RT): 4.88 min (Method D); MS (ES+) gave m/z: 294.25 (MH+).

49 (G) Lithium 1-(4-fluoro-benzoyl)-5,5-dimethyl-piperidine-3-carboxylate

To a solution of 1-(4-fluoro-benzoyl)-5,5-dimethyl-piperidine-3-carboxylic acid methyl ester (43 mg, 0.15 mmol) in THF/MeOH 1:1 (5 mL), LiOH (4 mg, 0.15 mmol) and $H_2O$ (100 μL) were added. The mixture was stirred overnight at room temperature, then the solvent was removed under reduced pressure and the crude Lithium 1-(4-fluoro-benzoyl)-5,5-dimethyl-piperidine-3-carboxylate was used in the next step without further purification.
Yield: 100%; LCMS (RT): 4.02 min (Method D); MS (ES+) gave m/z: 280.26 (MH+).

49 (H) {3,3-Dimethyl-5-[3-(1H-pyrrol-2-yl)-[1,2,4]oxadiazol-5-yl]-piperidin-1-yl}-(4-fluoro-phenyl)-methanone

A mixture of lithium 1-(4-fluoro-benzoyl)-5,5-dimethyl-piperidine-3-carboxylate (42 mg, 0.15 mmol), HOAT (20 mg, 0.15 mmol) and EDCI.HCl (43 mg, 0.23 mmol) in dioxane (2 mL) was stirred at room temperature for 10 min. N-Hydroxy-1H-pyrrole-2-carboxamidine (19 mg, 0.15 mmol, prepared as described in Example 1(A)) and triethylamine (41 μL, 0.30 mmol) were added. The mixture was stirred for 3 days at room temperature, then at 80°C for 4h. The solvent was removed under reduced pressure then the crude was dissolved in DCM and washed with 5% $Na_2CO_3$ (aq) (2x5 mL). The organic layer was dried over $Na_2SO_4$ then the solvent was removed under reduced pressure and the crude was purified by flash chromatography (silica gel; eluent: DCM/methanol 98:2).
Yield: 60% (white solid); LCMS (RT): 3.09 min (Method N); MS (ES+) gave m/z: 369.2 (MH+).
$^1$H-NMR (DMSO-$d_6$ 353K), δ (ppm): 11.42 (s br, 1H); 7.50 (dd, 2H); 7.25 (dd, 2H); 6.96 (dd, 1H); 6.73 (dd, 1H); 6.21 (dd, 1H); 4.47 (m, 1H); 3.71 (m, 1H); 3.46 (m, 1H); 3.21-3.04 (m, 2H); 2.00 (m, 1H); 1.74 (dd, 1H); 0.99 (s, 3H); 0.96 (s, 3H).

Example 50

(4-Fluoro-phenyl)-{(S)-3-[3-(4-fluoro-1H-pyrrol-2-yl)-[1,2,4]oxadiazol-5-yl]-piperidin-1-yl}-methanone

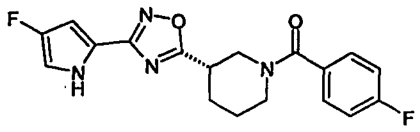

50(A) (S)-4-Oxo-*N*-Boc-pyrrolidine-2-carboxylic acid methyl ester

A solution of DMSO (1.38 mL, 19.5 mmol) in dry DCM (30 mL) was cooled to -78°C and oxalyl chloride (1.65 mL, 18 mmol) was added. After stirring at -78°C under $N_2$ for 15 min, *N*-Boc-*trans*-4-hydroxy proline methyl ester (3.07g, 12.5 mmol) was added and the resulting solution stirred for 4 hours at -50°C under $N_2$. Triethylamine (5 mL, 36 mmol) was added, and the solution allowed to warm slowly to room temperature, then stirred overnight. The solution was diluted with approx 50 mL of DCM then washed twice with 10% citric acid aqueous solution, then with water and with brine. The solution was dried over sodium sulphate and the solvent removed to give the product as a pale yellow oil.
Yield: 100%; LCMS (RT): 3.53min (Method A); MS (ES+) gave m/z: 244 (MH+).

50(B) (S)-4,4-Difluoro-*N*-Boc-pyrrolidine-2-carboxylic acid methyl ester

A solution of (S)-4-oxo-*N*-Boc-pyrrolidine-2-carboxylic acid methyl ester (1 g, 4.1 mmol) in dry DCM (10 mL) was cooled to -78°C under $N_2$, and then diethylamino sulfur trifluoride (1.95 mL, 16 mmol) was added. The mixture was stirred at -78°C for 10 minutes then allowed to warm to room temperature and stirred under $N_2$ for 2 hours. Ice was added and the solution was then basified with 5% $NaHCO_3$ (aq) and extracted three times with DCM. The combined organic extracts were washed with 5% $NaHCO_3$ (aq) solution, water and brine, dried over sodium sulphate and the solvent removed to give the required product as a yellow oil.
Yield: 99%; LCMS (RT): 5.03 min (Method D); MS (ES+) gave m/z: 266 (MH+).

50(C) (S)-4,4-Difluoropyrrolidine-2-carboxylic acid methyl ester trifluoroacetate

(S)-4,4-Difluoro-*N*-Boc-pyrrolidine-2-carboxylic acid methyl ester (1.08 g, 4.07 mmol) was dissolved in TFA (5 mL) and stirred under $N_2$ for 30 min. The solvent was removed under vacuum and the residue dissolved in MeOH, loaded onto an SCX ion exchange column, washed with MeOH and DCM then eluted with 5% $NH_3$ in MeOH. The solvent was removed to give the product as a pale brown oil.
Yield: 77%; LCMS (RT): 0.63 min (Method A); MS (ES+) gave m/z: 166 (MH+).

50(D) (S)-4,4-Difluoro-*N*-tosyl-pyrrolidine-2-carboxylic acid methyl ester

Tosyl chloride (667 mg, 3.5 mmol) and triethylamine (550 μL, 4 mmol) were added to a solution of (S)-4,4-difluoropyrrolidine-2-carboxylic acid methyl ester trifluoroacetate (520 mg, 3.15 mmol) in DCM and the resulting mixture was stirred for two days. The solution was washed twice with 10% citric acid solution, then with 5% $NaHCO_3$ solution and with brine, dried and the solvent removed. The residue was purified by flash chromatography (silica gel cartridge, eluent gradient: from hexane/ethyl acetate 100:0 to hexane/ethyl acetate 70:30) to give the product as a colourless oil which solidified on standing.
Yield: 76%; LCMS (RT): 5.2 min (Method D); MS (ES+) gave m/z: 320 (MH+).

50(E) 4-Fluoro-1H-pyrrole-2-carboxylic acid methyl ester

Sodium (830 mg, 35 mmol) was dissolved in dry MeOH (10 mL) under $N_2$ and then added to a solution of (S)-4,4-difluoro-*N*-tosyl-pyrrolidine-2-carboxylic acid methyl ester (765 mg, 2.4 mmol) in dry MeOH (10 mL). The solution was stirred under $N_2$ for 2 hours and then the solvent was removed under vacuum. 10% Citric acid aqueous solution (30 mL) was added and the solution extracted three times with EtOAc. The combined organic extracts were dried over sodium sulphate and the solvent removed. The residue was purified by flash chromatography (silica gel cartridge, eluent gradient: from hexane/ethyl acetate 100:0 to hexane/ethyl acetate 75:25) to give the product as a white solid.
Yield: 77%; LCMS (RT): 3.7 min (Method D); MS (ES+) gave m/z: 112 $[M-OMe]^+$

50(F) 4-Fluoro-1H-pyrrole-2-carboxylic acid

4-Fluoro-1H-pyrrole-2-carboxylic acid methyl ester (264 mg, 1.85 mmol) and NaOH (75 mg, 1.9 mmol) were dissolved in 1:1 dioxane/water (10 mL) and stirred overnight. The solvent was removed, 10% citric acid aqueous solution

(20 mL) added and the solution extracted three times with EtOAc. The combined organic extracts were washed with brine, dried over sodium sulphate and the solvent removed to give the product as a white solid.
Yield: 97% LCMS (RT): 2.7 min (Method D); MS (ES+) gave m/z: 130 (MH+).

50(G) 4-Fluoro-1H-pyrrole-2-carboxylic acid amide

Carbonyl diimidazole (340 mg, 2.1 mmol) was added to a solution of 4-fluoro-1H-pyrrole-2-carboxylic acid (230 mg, 1.78 mmol) in MeCN (10 mL) and stirred for 90 min. Concentrated $NH_4OH$ solution (2 mL) was added and the resulting mixture refluxed for 90 min. The solvent was removed, 10% citric acid solution (10 mL) was added and the solution extracted three times with EtOAc. The organic extracts were combined, dried over sodium sulphate and the solvent removed to give the product as a white solid.
Yield: 100% LCMS (RT): 2.1 min (Method G); MS (ES+) gave m/z: 129 (MH+).

50(H) 4-Fluoro-1H-pyrrole-2-carbonitrile

A solution of 4-fluoro-1H-pyrrole-2-carboxylic acid amide (210 mg, 1.7 mmol) in phosphorus oxychloride (5 mL) was heated at 100°C for 5 minutes, cooled, ice was added, basified with conc. $NH_4OH$ solution then extracted three times with EtOAc. The organic extracts were combined, dried and the solvent removed to give the product as a pale brown oil
Yield: 90% LCMS (RT): 3.5 min (Method G); MS (ES+) gave m/z: 111 (MH+).

50(I) 4-Fluoro-*N*-hydroxy-1H-pyrrole-2-carboxamidine

50% Hydroxylamine solution in water (1.2 mL, 20 mmol) was added to a solution of 4-fluoro-1H-pyrrole-2-carbonitrile (176 mg, 1.6 mmol) in ethanol (3 mL) and heated under reflux for 1h. The solvent was removed under vacuum and the residue purified by flash chromatography (silica gel cartridge, eluent gradient: from hexane/ethyl acetate 100:0 to hexane/ethyl acetate 0:100) to give the product as a white solid.
Yield: 95% LCMS (RT): 1.4 min (Method G); MS (ES+) gave m/z: 144 (MH+).

50(J) (S)-3-[3-(4-Fluoro-1H-pyrrol-2-yl)-[1,2,4]oxadiazol-5-yl]-piperidine-1-carboxylic acid tert-butyl ester

A mixture of (S)-N-Boc-nipecotic acid (229 mg, 1 mmol), HOAT (163 mg, 1.2 mmol), EDCI.HCl (230 mg, 1.2 mmol) in dry DCM (10 mL) was stirred under $N_2$ for 10 minutes, then 4-fluoro-*N*-hydroxy-1H-pyrrole-2-carboxamidine (131 mg, 0.92 mmol) was added and the solution stirred overnight. The solution was washed with water, 10% citric acid solution and 5% $NaHCO_3$ solution, dried over sodium sulphate and the solvent removed to give a residue that was purified by flash chromatography (silica gel cartridge, eluent gradient: from hexane/ethyl acetate 100:0 to hexane/ethyl acetate 80:20). The solid thus obtained was dissolved in acetonitrile (2 mL) and heated in a sealed tube at 75°C for 90 min in a microwaves reactor. The solvent was removed and the crude residue was purified by flash chromatography (silica gel cartridge, eluent gradient: from hexane/ethyl acetate 100:0 to hexane/ethyl acetate 80:20) to give the product as a white solid.
Yield: 64%; LCMS (RT): 5.8 min (Method D); MS (ES+) gave m/z: 337 (MH+).

50(K) (S)-3-[3-(4-Fluoro-1H-pyrrol-2-yl)-[1,2,4]oxadiazol-5-yl]-piperidine trifluoroacetate salt

(S)-3-[3-(4-Fluoro-1H-pyrrol-2-yl)-[1,2,4]oxadiazol-5-yl]-piperidine-1-carboxylic acid tert-butyl ester (200 mg, 0.59 mmol) was dissolved in DCM (5 mL) and trifluoroacetic acid (2 mL) added. The solution was stirred for 30 min and then the solvent removed and dried under high vacuum.
Yield: 100%; LCMS (RT): 2.6 min (Method D); MS (ES+) gave m/z: 237 (MH+).

50(L) (4-Fluoro-phenyl)-{(S)-3-[3-(4-fluoro-1H-pyrrol-2-yl)-[1,2,4]oxadiazol-5-yl]-piperidin-1-yl}-methanone

(S)-3-[3-(4-Fluoro-1H-pyrrol-2-yl)-[1,2,4]oxadiazol-5-yl]-piperidine trifluoroacetate salt (104 mg, 0.3 mmol) was dissolved in DCM (5 mL) and 4-fluorobenzoyl chloride (49 $\mu$L, 0.4 mmol) was added followed by triethylamine (125 $\mu$L, 0.9 mmol). The solution was stirred for 1hour, then washed with 0.1 M HCl solution, with 0.1 M NaOH and the solvent removed. The residue was purified by flash chromatography (silica gel cartridge, eluent gradient: from hexane/ethyl acetate 100:0 to hexane/ethyl acetate 30:70) to give the product as a white solid.
Yield: 68%; $[\alpha]_D^{20}$ = +116.6 (c=0.5, MeOH); mp= 146.5-147.2°C; LCMS (RT): 2.84 min (Method N); MS (ES+) gave m/z: 359.1 (MH+). ,

$^1H$-NMR (DMSO-$d_6$ 353K), δ (ppm): 11.38 (s br, 1H); 7.46 (dd, 2H); 7.42 (dd, 2H); 6.83 (m, 1H); 6.53 (m, 1H); 4.22 (dd, 1H); 3.76 (dt, 1H); 3.50 (dd, 1H); 3.40-3.21 (m, 2H); 2.24 (m, 1H); 2.03-1.76 (m, 2H); 1.64 (m, 1H).

Example 51

(3,4-Difluoro-phenyl)-{(S)-3-[3-(4-fluoro-1H-pyrrol-2-yl)-[1,2,4]oxadiazol-5-yl]-piperidin-1-yl}-methanone

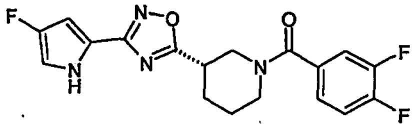

(S)-3-[3-(4-Fluoro-1H-pyrrol-2-yl)-[1,2,4]oxadiazol-5-yl]-piperidine trifluoroacetate salt (104 mg, 0.3 mmol) (prepared as described in example 50(K)) was dissolved in DCM (5 mL) and 3,4-difluorobenzoyl chloride (50 μL, 0.4 mmol) was added followed by triethylamine (125 μL, 0.9 mmol). The solution was stirred for 1hour then washed with 0.1 M HCl solution, with 0.1 M NaOH and then the solvent was removed. The residue was purified by flash chromatography (silica gel cartridge, eluent gradient: from hexane/ethyl acetate 100:0 to hexane/ethyl acetate 30:70) to give the product as a white solid.
Yield: 63%; $[\alpha]_D^{20}$ = +111.2 (c=0.5, MeOH); mp= 147.5-148.2°C; LCMS (RT): 2.91 min (Method N); MS (ES+) gave m/z: 377.0 (MH+).
$^1H$-NMR (DMSO-$d_6$ 353K), δ (ppm): 11.39 (s br, 1H); 7.50-7.39 (m, 2H); 7.25 (m, 1H); 6.84 (m, 1H); 6.53 (m, 1H); 4.20 (dd, 1H); 3.74 (dt, 1H); 3.51 (dd, 1H); 3.42-3.23 (m, 2H); 2.23 (m, 1H); 2.02-1.75 (m, 2H); 1.65 (m, 1H).

Example 52

(6-Fluoro-pyridin-3-yl)-{(S)-3-[3-(4-fluoro-1H-pyrrol-2-yl)-[1,2,4]oxadiazol-5-yl]-piperidin-1-yl}-methanone

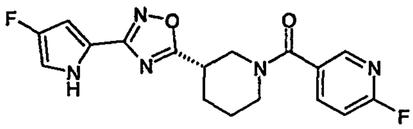

52(A) (S)-3-[3-(4-Fluoro-1H-pyrrol-2-yl)-[1,2,4]oxadiazol-5-yl]-piperidine hydrochloride salt

(S)-3-[3-(4-Fluoro-1H-pyrrol-2-yl)-[1,2,4]oxadiazol-5-yl]-piperidine-1-carboxylic acid tert-butyl ester (120 mg, 0.36 mmol) (prepared as described in example 50(J)) was dissolved in DCM (1 mL) and 4M HCl in dioxane (2 mL) added. The solution was stirred for 30 min at room temperature and then the solvent removed and dried under high vacuum.
Yield: 100%; LCMS (RT): 2.6 min (Method D); MS (ES+) gave m/z: 237 (MH+).

52(B) (6-Fluoro-pyridin-3-yl)-{(S)-3-[3-(4-fluoro-1H-pyrrol-2-yl)-[1,2,4]oxadiazol-5-yl]-piperidin-1-yl}-methanone

A mixture of 6-fluoro nicotinic acid (56 mg, 0.4 mmol), HOAT (68 mg, 0.5 mmol), EDCI.HCl (96 mg, 0.5 mmol) in dry DCM (10 mL) was stirred under $N_2$ for 10 minutes at room temperature, then (S)-3-[3-(4-fluoro-1H-pyrrol-2-yl)-[1,2,4]oxadiazol-5-yl]-piperidine hydrochloride salt (98 mg, 0.36 mmol) and triethylamine (83 μL, 0.6 mmol) were added and the solution stirred for 1 hour at room temperature. The solution was washed with water and with 0.2M NaOH solution, dried and the solvent removed to give a residue that was purified by flash chromatography (silica gel cartridge, eluent gradient: from hexane/ethyl acetate 100:0 to hexane/ethyl acetate 30:70) to give the product as a colourless gum.
Yield: 77%; $[\alpha]_D^{20}$ = +72 (c=0.3, MeOH); LCMS (RT): 3.27 min (Method P); MS (ES+) gave m/z: 360.1 (MH+).
$^1H$-NMR (DMSO-$d_6$ 353K), δ (ppm): 11.45 (s br, 1H); 8.31 (m, 1H); 8.02 (ddd, 1H); 7.22 (dd, 1H); 6.85 (dd, 1H); 6.54 (d, 1H); 4.23 (m, 1H); 3.77 (m, 1H); 3.55 (dd, 1H); 3.46-3.26 (m, 2H); 2.23 (m, 1H); 2.04-1.75 (m, 2H); 1.67 (m, 1H).

Example 53

(2-Fluoro-pyridin-4-yl)-{(S)-3-[3-(4-fluoro-1H-pyrrol-2-yl)-[1,2,4]oxadiazol-5-yl]-piperidin-1-yl}-methanone

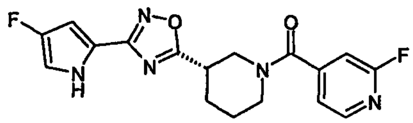

A mixture of 2-fluoro isonicotinic acid (42 mg, 0.3 mmol), HOAT (41 mg, 0.3 mmol), EDCI.HCl (58 mg, 0.3 mmol) in dry DCM (10 mL) was stirred at room temperature under $N_2$ for 10 minutes, then (S)-3-[3-(4-fluoro-1H-pyrrol-2-yl)-[1,2,4]oxadiazol-5-yl]-piperidine hydrochloride salt (63 mg, 0.23 mmol) (prepared as described in example 52(A)) and triethylamine (83 μL, 0.6 mmol) were added and the solution stirred overnight at room temperature. The solution was washed with water and with 0.2M NaOH solution, dried and the solvent removed to give a residue that was purified by flash chromatography (silica gel cartridge, eluent gradient: from hexane/ethyl acetate 100:0 to hexane/ethyl acetate 0:100) to give the product as a colourless gum.
Yield: 73%; $[\alpha]_D^{20}$ = +110 (c=0.7, MeOH); LCMS (RT): 2.50 min (Method N); MS (ES+) gave m/z: 360.3 (MH+).
$^1$H-NMR (DMSO-$d_6$ 353K), δ (ppm): 11.44 (s br, 1H); 8.32 (d, 1H); 7.33 (ddd, 1H); 7.15 (m, 1H); 6.86 (dd, 1H); 6.54 (d, 1H); 4.18 (m, 1H); 3.71 (m, 1H); 3.53 (dd, 1H); 3.45-3.22 (m, 2H); 2.22 (m, 1H); 2.04-1.75 (m, 2H); 1.67 (m, 1H).

Example 54

(4-Fluoro-phenyl)-{(S)-3-[5-(1H-pyrrol-2-yl)-tetrazol-2-yl]-piperidin-1-yl}-methanone

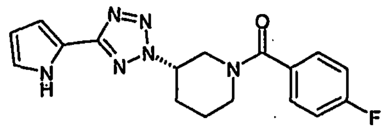

54(A) (4-Fluoro-phenyl)-((R)-3-hydroxy-piperidin-1-yl)-methanone

A mixture of (R)-3-hydroxy piperidine hydrochloride (0.2 g, 1.45 mmol), 4-fluoro benzoic acid (0.204 g, 1.45 mmol), EDC.HCl (0.42 g, 2.18 mmol), HOBT (0.196 g, 1.45 mmol), triethylamine (320 μL, 4.36 mmol) in dichloromethane (10 mL) was stirred under nitrogen atmosphere overnight at room temperature. The reaction mixture was diluted with dichloromethane (20 mL) and washed subsequently with 0.1N HCl (2 times), with 0.1N NaOH (2 times) and then with brine. The organic layer was dried over sodium sulphate and evaporated under reduced pressure to give a pale yellow oil (275 mg), which was used for the next step without further purification.
Yield: 85%; $[\alpha]_D^{20}$ = -8.7 (c=0.615, $CHCl_3$); LCMS (RT): 3.1 min (Method D); MS (ES+) gave m/z: 224.0 (MH+).
$^1$H-NMR ($CDCl_3$); δ (ppm): 7.43 (dd, 2H); 7.08 (dd, 2H); 4.00-3.14 (m br, 5H); 2.27 (s br, 1H); 1.98-1.76 (m, 2H); 1.74-1.55 (m, 2H).

54(B) 5-(1H-Pyrrol-2-yl)-2H-tetrazole

2-Cyanopyrrole (300 μL, 3.55 mmol), sodium azide (275 mg, 4.25 mmol) and ammonium chloride (134 mg, 4.25 mmol) were dissolved in DMF (1 mL) and heated in a sealed tube in a microwave reactor for 20 min at 120°C, then for 25 min at 160°C and then for 5 min at 180°C. After cooling, the tube was vented to release the pressure generated during the reaction and water was added. The solution was washed with EtOAc, acidified to about pH 3 with 1M HCl and then extracted three times with DCM. The combined organic extracts were dried and the solvent removed to give the product as a white solid.
Yield: 57%; LCMS (RT): 1.8 min (Method D); MS (ES+) gave m/z: 136 (MH+).
$^1$H-NMR (DMSO); δ (ppm): 11.92 (s br, 1H); 7.01 (d, 1H); 6.79 (d, 1H); 6.24 (dd, 1H).

54(C) (4-Fluoro-phenyl)-{(S)-3-[5-(1H-pyrrol-2-yl)-tetrazol-2-yl]-piperidin-1-yl}-methanone

Diisopropylazadicarboxylate (DIAD, 141 μL, 0.72 mmol) was added dropwise at 0°C with stirring to a mixture of 5-(1H-pyrrol-2-yl)-2H-tetrazole (95 mg, 0.7 mmol), (4-fluoro-phenyl)-((R)-3-hydroxy-piperidin-1-yl)-methanone (100 mg, 0.36 mmol) and solid supported triphenylphosphine (PS-$PPh_3$, ex Argonaut Technologies, loading 2.4 mmol/g, 420 mg, 1 mmol) in dichloromethane (4 mL). The mixture was heated in a sealed tube in a microwave reactor at 100°C for 30 min. The resin was filtered off and washed with DCM and MeOH. The combined solutions were concentrated under vacuum and the residue purified by flash chromatography (silica gel cartridge, eluent gradient: from DCM/MeOH 100:0 to DCM/MeOH 98:2) The crude material thus recovered was then dissolved in toluene and passed through a silica gel cartridge (Isolute Flash II 2 g, eluted with hexane, then with hexane/diethyl ether 75:25, then with hexane/diethyl ether 60:40,then with DCM/MeOH 98:2).
The title compound was obtained pure as a colourless gum.
Yield: 30%; LCMS (RT): 6.28 min (Method Q); MS (ES+) gave m/z: 341.2 (MH+).
$^1$H-NMR (DMSO-$d_6$ 368K), δ (ppm): 11.31 (s br, 1H); 7.45 (dd, 2H); 7.19 (dd, 2H); 6.93 (m, 1H); 6.70 (m, 1H); 6.21 (m, 1H); 4.99 (dddd, 1H); 4.31 (dd, 1H); 3.77 (dd, 1H); 3.71 (m, 1H); 3.42 (ddd, 1H); 2.47-2.23 (m, 2H); 2.03-1.90 (m, 1H); 1.73 (m, 1H).

Example 55

(4-Fluoro-phenyl)-{(S)-3-[5-(4-trifluoromethyl-1H-imidazol-2-yl)-[1,2,4]oxadiazol-3-yl]-piperidin-1-yl}-methanone

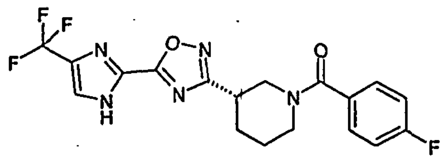

55(A) 4-Trifluoromethyl-1H-imidazole-2-carboxylic acid ethyl ester

3,3-Dibromo-1,1,1-trifluoropropanone (1g, 3.7 mmol) was added to a solution of sodium acetate trihydrate (1g, 7.4 mmol) in water (5 mL) and the mixture refluxed for 30 min. After cooling, a solution of ethyl glyoxalate (590 μL, 3 mmol) and conc. ammonia solution (500 μL) in MeOH (2 mL) was added and the mixture stirred for 24 hours at room temperature. The pH was adjusted to about 8 and the solution extracted three times with EtOAc. The combined organic extracts were dried and the solvent removed to give the product as a white solid.
Yield: 69%; LCMS (RT): 3.31 min (Method A); MS (ES+) gave m/z: 209 (MH+).

55(B) 4-Trifluoromethyl-1H-imidazole-2-carboxylic acid sodium salt

4-Trifluoromethyl-1H-imidazole-2-carboxylic acid ethyl ester (245 mg, 1.18 mmol) was dissolved in 5M NaOH solution (235 μL, 1.18 mmol) and heated for 12 hours at 70°C. The solvent was removed by azeotropic distillation with toluene to give the product as a white solid.
Yield: 100%; LCMS (RT): 2.32 min (Method D); MS (ES+) gave m/z: 181 (MH+).

55(C) (S)-3-[5-(4-Trifluoromethyl-1H-imidazol-2-yl)-[1,2,4]oxadiazol-3-yl]-piperidine-1-carboxylic acid tert-butyl ester

4-Trifluoromethyl-1H-imidazole-2-carboxylic acid (417 mg, 2.06 mmol) and (S)-3-(N-hydroxycarbamimidoyl)-piperidine-1-carboxylic acid tert-butyl ester (500 mg, 2.06 mmol) (prepared as described in Example 10(C)), were dissolved in dioxane (5 mL). HOAt (561 mg, 4.12 mmol) was added with stirring, followed by EDC.HCl (593 mg, 3.1 mmol). The solution was heated at 70°C for 9h, cooled, water was added and the solution was extracted three times with EtOAc. The combined organic extracts were dried and the solvent removed. The solid thus obtained was dissolved in acetonitrile (2 mL) and heated in a sealed tube at 80°C for 1 hour in a microwave reactor. The solvent was removed, the residue dissolved in EtOAc and washed twice with 5% citric acid solution, with 1M NaOH and with brine and the solvent removed. The residue was purified by flash chromatography (Biotage silica gel, eluted with EtOAc/hexane 10:90) to give the required product.
Yield: 10%; LCMS (RT): 4.18 min (Method A); MS (ES+) gave m/z: 389 (MH+).

55(D) (S)-3-[5-(4-Trifluoromethyl-1H-imidazol-2-yl)-[1,2,4]oxadiazol-3-yl]-piperidine hydrochloride salt

(S)-3-[5-(4-Trifluoromethyl-1H-imidazol-2-yl)-[1,2,4]oxadiazol-3-yl]-piperidine-1-arboxylic acid tert-butyl ester (83 mg, 0.214 mmol) was dissolved in a 2:1 mixture of DCM/ MeOH (3 mL) and 4M HCl in dioxane (1 mL) was added at 0°C. The solution was stirred under $N_2$ for 2 hours at room temperature, then the solvent was removed to give the product as a white solid.
Yield: 100%; LCMS (RT): 2.80 min (Method A); MS (ES+) gave m/z: 289 (MH+).

55(E) (4-Fluoro-phenyl)-{(S)-3-[5-(4-trifluoromethyl-1H-imidazol-2-yl)-[1,2,4]oxadiazol-3-yl]-piperidin-1-yl}-methanone

(S)-3-[5-(4-Trifluoromethyl-1H-imidazol-2-yl)-[1,2,4]oxadiazol-3-yl]-piperidine hydrochloride salt (70 mg, 0.214 mmol) was suspended in dry DCM (7 mL) at 0°C and triethylamine (63 μL, 0.45 mmol) added, followed by 4-fluorobenzoyl chloride (25 μL, 0.214 mmol). The mixture was stirred under $N_2$ at room temperature for 3 hours then washed with water, 5% citric acid solution and brine, dried and the solvent removed. The residue was purified by preparative HPLC to give the title compound.
Yield: 13%; LCMS (RT): 2.76 min (Method N); MS (ES+) gave m/z: 410.1 (MH+).
$^1$H-NMR (DMSO-$d_6$ 353K), δ (ppm): 7.97 (m, 1H); 7.47 (dd, 2H); 7.21 (dd, 2H); 4.28 (m, 1H); 3.83 (m, 1H); 3.38 (dd, 1H); 3.29-3.12 (m, 2H); 2.24 (m, 1H); 2.00-1.76 (m, 2H); 1.65 (m, 1H).

Example 56

(6-Fluoro-pyridin-3-yl)-{(S)-3-[5-(4-isopropyl-1H-pyrrol-2-yl)-[1,2,4]oxadiazol-3-yl]-piperidin-1-yl}-methanone

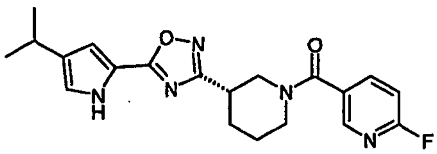

56 (A) 3-Methyl-2-methylene-butyraldehyde

The compound was prepared as described in Tetrahedron, 1996, 1231-1234. Yield: 37%; $^1$H-NMR ($CDCl_3$): 9.54 (s, 1H), 6.23 (d, 1H), 5.94 (s, 1H), 2.81 (m, 1H), 1.09 (d, 1H).

56 (B) (Toluene-4-sulfonylamino)-acetic acid methyl ester

To a solution of (toluene-4-sulfonylamino)-acetic acid (2 g, 8.72 mmol) in methanol (60 mL), conc. $H_2SO_4$ (1.5 mL) was added. The mixture was stirred at room temperature for 3h then the solvent was removed under reduced pressure. The crude was dissolved in DCM (20 mL) and the organic phase was washed with $H_2O$ (1x20 mL), 5% $Na_2CO_3$ (aq) (1x20 mL) and brine (1x20 mL). The organic layer was dried over $Na_2SO_4$ and the solvent was removed under reduced pressure. The crude (toluene-4-sulfonylamino)-acetic acid methyl ester was used in the next step without further purification.
Yield: 98%; LCMS (RT): 3.47 min (Method A); MS (ES+) gave m/z: 244.03 (MH+).

56 (C) 3-Hydroxy-4-isopropyl-1-(toluene-4-sulfonyl)-pyrrolidine-2-carboxylic acid methyl ester

To a solution of 3-methyl-2-methylene-butyraldehyde (850 mg, 8.72 mmol) and (toluene-4-sulfonylamino)-acetic acid methyl ester (2.09 g, 8.59 mmol) in THF (60 mL), DBU (2.90 mL, 19.18 mmol) was added. The mixture was stirred overnight at room temperature, then the solvent was removed under reduced pressure and the crude was dissolved in diethyl ether (50 mL). The organic layer was washed with 1N HCl (1x50 mL), 5% $NaHCO_3$ (aq) (1x50 mL) and $H_2O$ (1x50 mL), then it was dried over $Na_2SO_4$ and the solvent was removed under reduced pressure. The crude 3-hydroxy-4-isopropyl-1-(toluene-4-sulfonyl)-pyrrolidine-2-carboxylic acid methyl ester was used in the next step without further purification.
Yield: 99%; LCMS (RT): 3.94 min (Method A); MS (ES+) gave m/z: 341.00 (MH+).

56 (D) 4-Isopropyl-1-(toluene-4-sulfonyl)-4,5-dihydro-1H-pyrrole-2-carboxylic acid methyl ester

A solution of 3-hydroxy-4-isopropyl-1-(toluene-4-sulfonyl)-pyrrolidine-2-carboxylic acid ethyl ester (2.89 g, 8.46 mmol) in pyridine (30 mL) was cooled at 0°C. $POCl_3$ (2 mL) was added dropwise over 5 min and the mixture was stirred at room temperature for 3 days. The mixture was poured into ice and diluted with diethylether. The two layers were separated and the organic phase was washed with HCl 5% (2x20 mL), 5% $NaHCO_3$ (aq) (2x20 mL) and brine (1x20 mL). The organic layer was dried over $Na_2SO_4$ then the solvent was removed under reduced pressure to yield the crude 4-isopropyl-1-(toluene-4-sulfonyl)-4,5-dihydro-1H-pyrrole-2-carboxylic acid methyl ester, that was used in the next step without further purification.
Yield: 68%; LCMS (RT): 4.35 min (Method A); MS (ES+) gave m/z: 324.03 (MH+).

56 (E) 4-Isopropyl-1H-pyrrole-2-carboxylic acid methyl ester

To a solution of 4-isopropyl-1-(toluene-4-sulfonyl)-4,5-dihydro-1H-pyrrole-2-carboxylic acid (1.86 g, 5.75 mmol) in toluene (100 mL), DBU (1.72 mL, 11.50 mmol) was added. The mixture was refluxed for 4h, then was cooled to room temperature and diluted with diethyl ether. The organic layer was washed with 10% HCl (2x100 mL), 5% $NaHCO_3$ (aq) (2x100 mL) and brine (1x100 mL), then it was dried over $Na_2SO_4$ and the solvent was removed under reduced pressure to yield the crude 4-isopropyl-1H-pyrrole-2-carboxylic acid methyl ester that was used in the next step without further purification.
Yield: 65%; LCMS (RT): 3.94 min. (Method A); MS (ES+) gave m/z: 168.05 (MH+).

56 (F) 4-Isopropyl-1H-pyrrole-2-carboxylic acid

A mixture of 4-isopropyl-1H-pyrrole-2-carboxylic acid methyl ester (530 mg, 3.17 mmol) and NaOH (400 mg, 9.51 mmol) in dioxane/$H_2O$ 10/1 (110 mL) was refluxed for 4h, then stirred at room temperature overnight. The solvent was removed under reduced pressure. The crude residue was dissolved in $H_2O$ then 5%HCl was added to adjust the pH to 2. The aqueous phase was extracted with AcOEt (3x30 mL), then the combined organic layers were dried over $Na_2SO_4$ and the solvent was removed under reduced pressure. 4-isopropyl-1H-pyrrole-2-carboxylic acid was used in the next step without further purification.
Yield: 97%; LCMS (RT): 1.16 min (Method H); MS (ES+) gave m/z: 154.14 (MH+).

56 (G) (S)-3-[5-(4-Isopropyl-1H-pyrrol-2-yl)-[1,2,4]oxadiazol-3-yl]-piperidine-1-carboxylic acid tert-butyl ester

A mixture of 4-isopropyl-1H-pyrrole-2-carboxylic acid (200 mg, 1.31 mmol), HOAT (180 mg, 1.31 mmol), TDCI.HCl (380 mg, 1.96 mmol) in dioxane (30 mL) was stirred at 50°C for 2h, then (S)-3-(N-hydroxycarbamimidoyl)-piperidine-1-carboxylic acid tert-butyl ester (320 mg, 1.31 mmol, prepared as described in Example 10 (C)) was added. The mixture was stirred overnight at 80°C, then at room temperature for 24h. The solvent was removed under reduced pressure, the crude was dissolved in ethyl acetate and the organic layer was washed with 5% $Na_2CO_3$ (aq) (2x30 mL) and with brine (1x30 mL). The organic phase was dried over $Na_2SO_4$ and the solvent was removed under reduced pressure. The crude was dissolved in $CH_3CN$, triethylamine (182 $\mu$L, 1.3 mmol) was added and the mixture was heated at 130°C for 5h, in a sealed tube, in microwaves oven. The solvent was removed and the crude was purified through a silica gel cartridge (eluent: hexane/ethyl acetate 80:20) to yield (S)-3-[5-(4-isopropyl-1H-pyrrol-2-yl)-[1,2,4]oxadiazol-3-yl]-piperidine-1-carboxylic acid tert-butyl ester.
Yield: 100%; LCMS (RT): 4.72 min (Method A); MS (ES+) gave m/z: 261.14 (MH+).

56 (H) (6-Fluoro-pyridin-3-yl)-{(S)-3-[5-(4-isopropyl-1H-pyrrol-2-yl)-[1,2,4]oxadiazol-3-yl]-piperidin-1-yl}-methanone

A solution of (S)-3-[5-(4-isopropyl-1H-pyrrol-2-yl)-[1,2,4]oxadiazol-3-yl]-piperidine-1-carboxylic acid tert-butyl ester (500 mg, 1.31 mmol) in DCM (60 mL) was cooled at 0°C, then HCl (4M in dioxane, 2 mL, 8 mmol) was added. The mixture was stirred at room temperature for 15h then the solvent was removed under reduced pressure. The crude was dissolved in DCM (50 mL), then 6-fluoro-nicotinic acid (185 mg, 1.31 mmol), HOAT (180 mg, 1.31 mmol), EDCI.HCl (380 mg, 1.96 mmol) and triethylamine (580 $\mu$L, 3.93 mmol) were added. The mixture was stirred for 3 days at room temperature then the solvent was removed under reduced pressure. The crude was dissolved in ethyl acetate and the organic layer was washed with 5% $Na_2CO_3$ (aq) (2x20 mL) and brine (1x20 mL). The organic phase was dried over $Na_2SO_4$ and the solvent was removed under reduced pressure. The crude was purified by flash chromatography (silica gel, eluent: hexane/ethyl acetate 50:50) to yield (6-fluoro-pyridin-3-yl)-{(S)-3-[5-(4-isopropyl-1H-pyrrol-2-yl)-[1,2,4]oxadiazol-3-yl]-piperidin-1-yl}-methanone.
Yield: 26% (brown oil); $[\alpha_D]$= +90.8 (c=0.93, $CH_3OH$); LCMS (RT): 4.23 min (Method N); MS (ES+) gave m/z: 384.1 (MH+).

$^1$H-NMR (DMSO-$d_6$, 353K), δ (ppm): 11.71 (s br, 1H); 8.30 (m, 1H); 8.02 (ddd, 1H); 7.20 (dd, 1H); 6.92 (m, 1H); 6.84 (m, 1H); 4.23 (m, 1H); 3.81 (m, 1H); 3.38 (dd, 1H); 3.27 (ddd, 1H); 3.16-3.06 (m, 1H); 2.84 (sept, 1H); 2.19 (m, 1H); 1.97-1.75 (m, 2H); 1.66 (m, 1H); 1.21 (d, 6H).

Example 57 (DELETED)

Example 58

(3-Fluoro-pyridin-4-yl)-{(S)-3-[5-(4-methyl-1H-pyrrol-2-yl)-[1,2,4]oxadiazol-3-yl]-piperidin-1-yl}-methanone

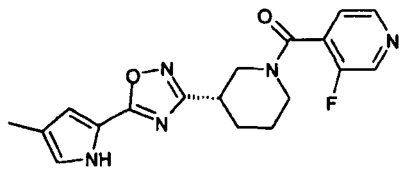

The title compound was prepared following the experimental procedure described in Example 28(C), starting from (S)-3-[5-(4-methyl-1H-pyrrol-2-yl)-[1,2,4]oxadiazol-3-yl]-piperidine hydrochloride, prepared as described in Example 28 (B), and using 3-fluoro-pyridine-4-carboxylic acid as the acid of choice.
Purification was performed by flash chromatography (silica gel, eluent: petroleum ether/ethyl acetate 1:2).
Yield: 98% (White amorphous solid); $[\alpha]_D^{20}$ = +101.8 (c = 0.94; MeOH); LCMS (RT): 1.91 min (Method S); MS (ES+) gave m/z: 356.1 (MH+).
$^1$H-NMR (DMSO-$d_6$ 373 K); δ (ppm): 11.57 (s br 1H); 8.61 (s 1H); 8.50 (dd 1H); 7.43 (dd 1H); 6.89 (s 1H); 6.67 (s 1H); 4.45 (m br 1H); 3.95 (m br 1H); 3.38 (m 1H); 3.30 (m 1H); 3.06 (m 1H); 2.20 (m 1H); 2.11 (s 3H); 1.99-1.79 (m 2H); 1.63 (m 1H).

Example 59

{(S)-3-[5-(4-Chloro-1H-pyrrol-2-yl)-[1,2,4]oxadiazol-3-yl]-piperidin-1-yl}-(3-fluoro-pyridin-4-yl)-methanone

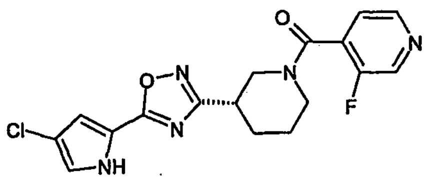

The title compound was prepared following the experimental procedure described in Example 28(C), starting from (S)-3-[5-(4-chloro-1H-pyrrol-2-yl)-[1,2,4]oxadiazol-3-yl]-piperidine hydrochloride, prepared as described in Example 39 (C), and using 3-fluoro-pyridine-4-carboxylic acid as the acid of choice.
Purification was performed by flash chromatography (silica gel, eluent: petroleum ether/ethyl acetate 2:8) and then by preparative HPLC.
Yield: 18%; LCMS (RT): 2.01 min (Method S); MS (ES+) gave m/z: 376.1 (MH+). $^1$H-NMR (DMSO-$d_6$ 373K), δ (ppm): 12.26 (s br 1H); 8.61 (s 1H); 8.50 (d 1H); 7.43 (dd 1H); 7.18 (d 1H); 6.93 (s 1H); 4.51 (m br 1H); 3.87 (m br 1H); 3.46 (m 1H); 3.27 (m 1H); 3.10 (m 1H); 2.21 (m 1H); 2.00-1.80 (m 2H); 1.64 (m 1H).

Example 60

(2-Fluoro-pyridin-4-yl)-{(s)-3-[5-(4-fluoro-1H-pyrrol-2-yl)-[1,2,4]oxadiazol-3-yl]-piperidin-1-yl}-methanone

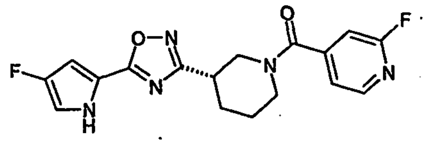

60(A) (S)-3-[5-(4-Fluoro-1H-pyrrol-2-yl)-[1,2,4]oxadiazol-3-yl]-piperidine-1-carboxylic acid tert-butyl ester

A mixture of 4-fluoro-1H-pyrrole-2-carboxylic acid (300 mg, 2.33 mmol), prepared as described in Example 50 (F), (S)-3-(N-hydroxycarbamimidoyl)-piperidine-1-carboxylic acid tert-butyl ester (567 mg, 2.33 mmol), prepared as described in Example 10 (C), EDCl.HCl (672 mg, 3.5 mmol) and HOBT (315 mg, 2.33 mmol) in dioxane (10 mL) was stirred at room temperature overnight, then at 80°C for 24h, in the presence of activated 3A molecular sieves. Molecular sieves were filtered off, then solvent was removed. Purification of the crude was performed by flash chromatography (silica gel, eluent: petroleum ether/ethyl acetate 8:2).
Yield: 38%; LCMS (RT): 5.91 min (Method D); MS (ES+) gave m/z: 337.0 (MH+).

60(B) (S)-3-[5-(4-Fluoro-1H-pyrrol-2-yl)-[1,2,4]oxadiazol-3-yl]-piperidine hydrochloride

A solution of (S)-3-[5-(4-fluoro-1H-pyrrol-2-yl)-[1,2,4]oxadiazol-3-yl]-piperidine-1-carboxylic acid tert-butyl ester (77 mg, 0.23 mmol) in DCM (3 mL) was cooled at 0°C, then 4M HC1 in dioxane (1 mL) was added. The mixture was stirred at room temperature for 2h then the solvent was removed under reduced pressure.
Yield: quantitative.

60(C) (2-Fluoro-pyridin-4-yl)-{(s)-3-[5-(4-fluoro-1h-pyrrol-2-yl)-[1,2,4]oxadiazol-3-yl]-piperidin-1-yl}-methanone

The title compound was prepared following the experimental procedure described in Example 28(C), starting from (S)-3-[5-(4-fluoro-1H-pyrrol-2-yl)-[1,2,4]oxadiazol-3-yl]-piperidine hydrochloride, prepared as described in Example 60 (B), and using 2-fluoro-pyridine-4-carboxylic acid as the acid of choice.
Purification was performed by flash chromatography (silica gel, eluent: petroleum ether/ethyl acetate 4:6).
Yield: 61% (white solid); LCMS (RT): 1.97 min (Method S); MS (ES+) gave m/z: 360.0 (MH+).
$^1$H-NMR (DMSO-$d_6$ 353K), δ (ppm): 11.97 (s br 1H); 8.32 (d 1H); 7.34 (m 1H); 7.16 (m 1H); 7.04 (dd 1H); 6.78 (m 1H); 4.24 (m br 1H); 3.76 (m br 1H); 3.46-3.05 (m 3H); 2.19 (m 1H); 1.96-1.76 (m 2H); 1.66 (m 1H).

Example 61

{(S)-3-[5-(4-Bromo-1H-pyrrol-2-yl)-[1,2,4]oxadiazol-3-yl]-piperidin-1-yl}-(3-fluoro-pyridin-4-yl)-methanone

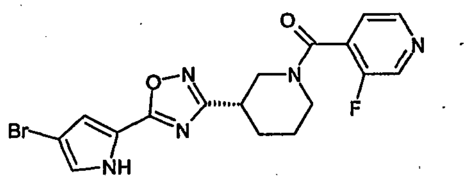

61(A) (S)-3-[5-(4-Bromo-1H-pyrrol-2-yl)-[1,2,4]oxadiazol-3-yl]-piperidine hydrochloride

The title compound was prepared following the experimental procedure described in Example 28(A) and 28 (B), starting from 4-bromo-1H-pyrrole-2-carboxylic acid, prepared as described in Example 44 (B).
Yield: 38%; LCMS (RT): 2.65 min (Method E); MS (ES+) gave m/z: 297.03 and 299.03.

61(B) {(S)-3-[5-(4-Bromo-1H-pyrrol-2-yl)-[1,2,4]oxadiazol-3-yl]-piperidin-1-yl}-(3-fluoro-pyridin-4-yl)-methanone

The title compound was prepared following the experimental procedure described in Example 28(C), starting from (S)-3-[5-(4-bromo-1H-pyrrol-2-yl)-[1,2,4]oxadiazol-3-yl]-piperidine hydrochloride, prepared as described in Example 61 (A), and using 3-fluoro-pyridine-4-carboxylic acid as the acid of choice.
Purification was performed by flash chromatography (silica gel, eluent: petroleum ether/ethyl acetate 1:2).

Yield: 79%; LCMS (RT): 3.12 min (Method P); MS (ES+) gave m/z: 419.9 (MH+). $^1$H-NMR (DMSO-$d_6$ 373K), δ (ppm): 12.34 (s br, 1H); 8.61 (s, 1H); 8.50 (m, 1H); 7.44 (dd, 1H); 7.22 (d, 1H); 6.99 (s, 1H); 4.98-3.86 (m br, 2H); 3.41 (m, 1H); 3.27 (m, 1H); 3.10 (m, 1H); 2.21 (m, 1H); 2.01-1.80 (m, 2H); 1.65 (m, 1H).

Example 62

(3-Fluoro-pyridin-4-yl)-{(S)-3-[5-(4-fluoro-1H-pyrrol-2-yl)-[1,2,4]oxadiazol-3-yl]-piperidin-1-yl}-methanone

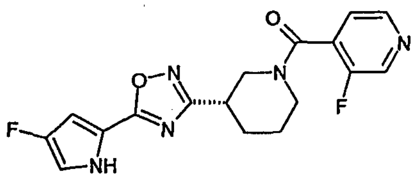

The title compound was prepared following the experimental procedure described in Example 28(C), starting from (S)-3-[5-(4-fluoro-1H-pyrrol-2-yl)-[1,2,4]oxadiazol-3-yl]-piperidine hydrochloride, prepared as described in Example 60 (B), and using 3-fluoro-pyridine-4-carboxylic acid as the acid of choice. Purification was performed by flash chromatography (silica gel, eluent: DCM/MeOH 99:1).
Yield: 64%; LCMS (RT): 1.83 min (Method S); MS (ES+) gave m/z: 360.1 (MH+).
$^1$H-NMR (DMSO-$d_6$ 373K), δ (ppm): 11.87 (s br, 1H); 8.62 (s, 1H); 8.51 (m, 1H); 7.43 (dd, 1H); 7.01 (m, 1H); 6.76 (s br, 1H); 4.75-4.20 (m br, 2H); 3.41 (m, 1H); 3.28 (m, 1H); 3.10 (m, 1H); 2.20 (m, 1H); 2.01-1.79 (m, 2H); 1.64 (m, 1H).

Example 63

(4-Fluoro-phenyl)-{(S)-3-[5-(4-fluoro-1H-pyrrol-2-yl)-[1,2,4]oxadiazol-3-yl]-piperidin-1-yl}-methanone

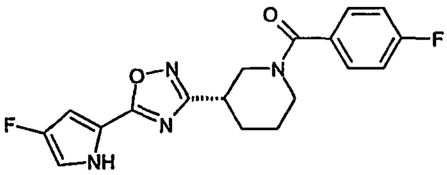

The title compound was prepared following the experimental procedure described in Example 1(C), starting from (S)-3-[5-(4-fluoro-1H-pyrrol-2-yl)-[1,2,4]oxadiazol-3-yl]-piperidine hydrochloride, prepared as described in Example 60 (B), and using 4-fluorobenzoyl chloride as the acylating agent. Purification was performed by flash chromatography (silica gel, eluent: DCM/MeOH 98:2).

Yield: 31%; LCMS (RT): 2.21 min (Method S); MS (ES+) gave m/z: 359.1 (MH+).
$^1$H-NMR (DMSO-$d_6$ 353K), δ (ppm): 12.01 (s br 1H); 7.47 (dd 2H); 7.23 (dd 2H); 7.04 (m.1H); 6.68 (m 1H); 4.25 (m 1H); 3.83(m 1H); 3.33 (dd 1H); 3.20 (ddd 1H); 3.09(m 1H); 2.20 (m 1H); 1.96-1.77 (m 2H); 1.64 (m 1H).

Example 64

(6-Fluoro-pyridin-3-yl)-{(S)-3-[5-(4-fluoro-1H-pyrrol-2-yl)-[1,2,4]oxadiazol-3-yl]-piperidin-1-yl}-methanone

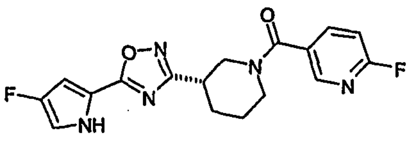

The title compound was prepared following the experimental procedure described in Example 28(C), starting from (S)-3-[5-(4-fluoro-1H-pyrrol-2-yl)-[1,2,4]oxadiazol-3-yl]-piperidine hydrochloride, prepared as described in Example 60 (B), and using 2-fluoro-pyridine-5-carboxylic acid as the acid of choice. Purification was performed by flash chromatography (silica gel, eluent: petroleum ether/ethyl acetate 4:6) and then by a second column flash chromatography (silica gel, eluent: DCM).

Yield: 7% (gummy white solid); LCMS (RT): 1.99 min (Method S); MS (ES+) gave m/z: 360.1 (MH+).
$^1$H-NMR (DMSO-$d_6$ 353K), δ (ppm): 11.99 (s br 1H); 8.31 (m 1H); 8.02 (ddd 1H); 7.21 (ddd 1H); 7.05 (dd 1H); 6.78 (m 1H); 4.24 (m 1H); 3.80 (m 1H); 3.38 (dd 1H); 3.27 (ddd 1H); 3.13 (m 1H); 2.20 (m 1H); 1.97-1.77 (m 2H); 1.76 (m 1H).

Example 65

{(S)-3-[3-(4-Chloro-1H-pyrrol-2-yl)-[1,2,4]oxadiazol-5-yl]-piperidin-1-yl}-(6-fluoro-pyridin-3-yl)-methanone

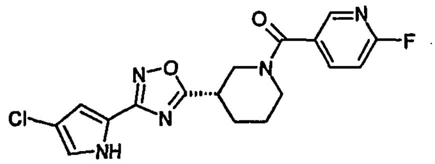

The title compound was prepared following the experimental procedure described in Example 28(C), starting from (S)-3-[3-(4-chloro-1H-pyrrol-2-yl)-[1,2,4]oxadiazol-5-yl]-piperidine hydrochloride, prepared as described in Example 43 (E), and using 2-fluoro-pyridine-5-carboxylic acid as the acid of choice. Purification was performed by flash chromatography (silica gel, eluent: DCM/MeOH 40:1).

Yield: 56% (white amorphous solid); $[\alpha_D]$= +125.0 (c = 0.98; MeOH); LCMS (RT): 2.12 min (Method S); MS (ES+) gave m/z: 376.1 (MH+).
$^1$H-NMR (DMSO-$d_6$ 353K), δ (ppm): 11.88 (s br 1H); 8.13 (m 1H); 8.02 (ddd 1H); 7.22 (dd 1H); 7.04 (d 1H); 6.70 (d 1H); 4.23 (m 1H); 3.76 (m 1H); 3.55 (dd 1H); 3.41 (ddd 1H); 3.33 (ddd 1H); 2.25 (m 1H); 1.97 (m 1H); 1.82 (m 1H); 1.68 (m 1H).

Example 66

{(S)-3-[3-(4-Chloro-1H-pyrrol-2-yl)-[1,2,4]oxadiazol-5-yl]-piperidin-1-yl}-(2-fluoro-pyridin-4-yl)-methanone

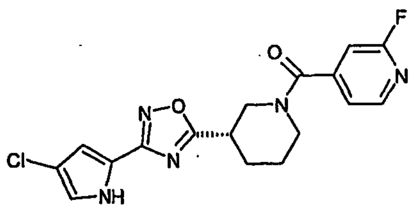

The title compound was prepared following the experimental procedure described in Example 28(C), starting from (S)-3-[3-(4-chloro-1H-pyrrol-2-yl)-[1,2,4]oxadiazol-5-yl]-piperidine hydrochloride, prepared as described in Example 43 (E), and using 2-fluoro-pyridine-4-carboxylic acid as the acid of choice.
Purification was performed by flash chromatography (silica gel, eluent: DCM/MeOH 40:1) and then by a successive column flash chromatography (silica gel, eluent: petroleum ether/ethyl acetate 2:1).
Yield: 66% (white amorphous solid); $[\alpha_D]$= +120.6 (c = 0.79; MeOH); LCMS (RT): 2.12 min (Method S); MS (ES+) gave m/z: 376.1 (MH+).
$^1$H-NMR (DMSO-$d_6$, 353K), δ (ppm): 11.90 (s br 1H); 8.33 (d 1H); 7.34 (m 1H); 7.16 (m 1H); 7.04 (d 1H); 6.70 (d 1H); 4.16 (m br 1H); 3.70 (m br 1H); 3.54 (dd 1H); 3.41 (m 1H); 3.30. (m 1H); 2.25 (m 1H); 1.96(m 1H); 1.82 (m 1H); 1.67 (m 1H).

Example 67

{(S)-3-[3-(4-Chloro-1H-pyrrol-2-yl)-[1,2,4]oxadiazol-5-yl]-piperidin-1-yl}-(3-fluoro-pyridin-4-yl)-methanone

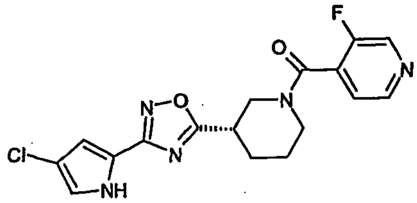

The title compound was prepared following the experimental procedure described in Example 28(C), starting from (S)-3-[3-(4-chloro-1H-pyrrol-2-yl)-[1,2,4]oxadiazol-5-yl]-piperidine hydrochloride, prepared as described in Example 43 (E), and using 3-fluoro-pyridine-4-carboxylic acid as the acid of choice. Purification was performed by flash chromatography (silica gel, eluent: petroleum ether/ethyl acetate 2:1).
Yield: 84% (white amorphous solid); [$\alpha_D$]= +107.7 (c = 1.09; MeOH); LCMS (RT): 2.00 min (Method S); MS (ES+) gave m/z: 376.1 (MH+).
$^1$H-NMR (DMSO-$d_6$, 353K), δ (ppm): 11.90 (s br 1H); 8.65 (s 1H); 8.52 (dd 1H); 7.44 (dd 1H); 7.04 (d 1H); 6.70 (m br 1H); 4.51 (m br 1H); 4.07 (m br 1H); 3.57 (dd 1H); 3.38 (m 2H); 2.25 (m 1H); 1.99(m 1H); 1.83(m 1H); 1.66 (m 1H).

Example 68

{(S)-3-[3-(4-Chloro-1H-pyrrol-2-yl)-[1,2,4]oxadiazol-5-yl]-piperidin-1-yl}-(5-methyl-isoxazol-4-yl)-methanone

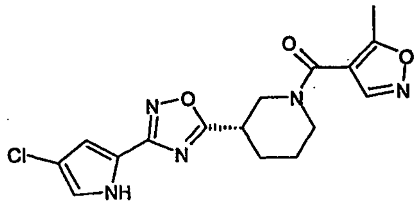

The title compound was prepared following the experimental procedure described in Example 28(C), starting from (S)-3-[3-(4-chloro-1H-pyrrol-2-yl)-[1,2,4]oxadiazol-5-yl]-piperidine hydrochloride, prepared as described in Example 43 (E), and using 5-methyl-isoxazole-4-carboxylic acid as the acid of choice. Purification was performed by flash chromatography (silica gel, eluent: DCM/MeOH 40:1).

Yield: 38% (white amorphous solid); [$\alpha_D$]= +95.1 (c = 0.54; MeOH); LCMS (RT): 2.09 min (Method S); MS (ES+) gave m/z: 362.1 (MH+).
$^1$H-NMR (DMSO-$d_6$, 373K), δ (ppm): 11.77 (s br 1H); 8.54 (s 1H); 7.02 (m 1H); 6.70 (m 1H); 4.23 (dd 1H); 3.79 (dd 1H); 3.57 (dd 1H); 3.37 (m 2H); 2.47 (d 3H); 2.25 (m 1H); 1.97(m 1H); 1.85 (m 1H); 1.66 (m 1H).

Example 69

{(S)-3-[3-(4-Bromo-1h-pyrrol-2-yl)-[1,2,4]oxadiazol-5-yl]-piperidin-1-yl}-(3-fluoro-pyridin-4-yl)-methanone

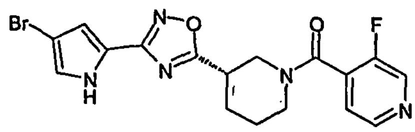

The title compound was prepared following the experimental procedure described in Example 28(C), starting from (S)-3-[3-(4-bromo-1H-pyrrol-2-yl)-[1,2,4]oxadiazol-5-yl]-piperidine hydrochloride, prepared as described in Example 45 (A), and using 3-fluoro-pyridine-4-carboxylic acid as the acid of choice. Purification was performed by flash chromatography (silica gel, eluent gradient: from hexane/ethyl acetate 100:0 to hexane/ethyl acetate 0:100).

Yield: 60% (white amorphous solid); [$\alpha_D$]= +100.3 (c= 0.525, MeOH); LCMS (RT): 5.20 min (Method T); MS (ES+) gave m/z: 419.9 (MH+).
$^1$H-NMR (DMSO-$d_6$ 353K), δ (ppm): 11.97 (s br 1H); 8.64 (s 1H); 8.52 (dd 1H); 7.45 (dd 1H); 7.08 (m 1H); 6.76 (m br 1H); 4.51 (s br 1H); 4.06 (m br 1H); 3.57 (dd 1H); 3.37 (m 2H); 2.25 (m 1H); 1.99 (m 1H); 1.81 (m 1H); 1.64 (m 1H).

Example 70

(3-Fluoro-pyridin-4-yl)-{(S)-3-[3-(4-fluoro-1H-pyrrol-2-yl)-[1,2,4]oxadiazol-5-yl]-piperidin-1-yl}-methanone

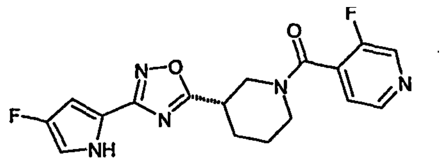

The title compound was prepared following the experimental procedure described in Example 28(C), starting from (S)-3-[3-(4-fluoro-1H-pyrrol-2-yl)-[1,2,4]oxadiazol-5-yl]-piperidine hydrochloride salt, prepared as described in Example 52 (A), and using 3-fluoro-pyridine-4-carboxylic acid as the acid of choice.
Yield: 40% (white solid); LCMS (RT): 1.83 min (Method S); MS (ES+) gave m/z: 360.1 (MH+).
$^1$H-NMR (DMSO-$d_6$ 353K), δ (ppm): 11.46 (s br 1H); 8.64 (s 1H); 8.52 (dd 1H); 7.45 (dd 1H); 6.86 (m 1H); 6.54 (m br 1H); 4.49 (m br 1H); 4.07 (m br 1H); 3.56 (dd 1H); 3.34 (m 2H); 2.25 (m 1H); 1.99 (m 1H); 1.82 (m 1H); 1.64 (m 1H).

Example 71

(3-Fluoro-pyridin-4-Yl)-{(S)-3-[3-(4-methyl-1H-pyrrol-2-yl)-[1,2,4]oxadiazol-5-Yl]-piperidin-1-yl}-methanone

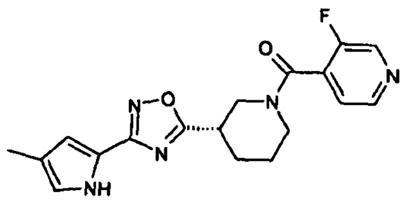

The title compound was prepared following the experimental procedure described in Example 28(C), starting from (S)-3-[3-(4-methyl-1H-pyrrol-2-yl)-[1,2,4]oxadiazol-5-yl]-piperidine trifluoroacetate, prepared as described in Example 31 (E), and using 3-fluoro-pyridine-4-carboxylic acid as the acid of choice. Purification was performed by flash chromatography (silica gel, eluent: petroleum ether/ethyl acetate 2:1).

Yield: 65% (white amorphous solid); [$\alpha_D$]= +112.1 (c = 0.80; MeOH); LCMS (RT): 1.89 min (Method S); MS (ES+) gave m/z: 356.1 (MH+).
$^1$H-NMR (DMSO-$d_6$, 353K), δ (ppm): 11.16 (s br 1H); 8.65 (s 1H); 8.52 (dd 1H); 7.45 (dd 1H); 6.74 (s 1H); 6.57 (m br 1H); 4.51 (m br 1H); 4.06 (m br 1H); 3.56 (dd 1H); 3.34 (m br 2H); 2.24 (m 1H); 2.08 (s 3H); 1.98(m 1H); 1.82 (m 1H); 1.64 (m 1H).

Example 72 (DELETED)

Example 76

(4-Fluoro-phenyl)-{(S)-3-[5-(4-trifluoromethyl-1H-pyrrol-2-yl)-[1,2,4]oxadiazol-3-yl]-piperidin-1-yl}-methanone

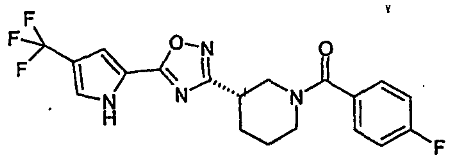

76(A) 4-Trifluoromethyl-pyrrole-1,2-dicarboxylic acid 2-benzyl ester 1-tert-butyl ester

The title compound was prepared according to the procedures reported in X. Qui, F. Qing, J. Org Chem. 2002, 67, 7162-7164; and X. Qui, F. Qing, J. Org. Chem. 2003, 68, 3614-3617.

76(B) (S)-3-[5-(4-Trifluoromethyl-1H-pyrrol-2-yl)-[1,2,4]oxadiazol-3-yl]-piperidine-1-carboxylic acid tert-buty 1 ester

4-Trifluoromethyl-pyrrole-1,2-dicarboxylic acid 2-benzyl ester 1-tert-butyl ester (498 mg, 1.35 mmol) was suspended in 4M HCl in dioxane (4 ml) and the mixture was stirred at room temperature for 6 hours. Then the solvent was removed affording a pale yellow solid, which was dissolved in EtOH (15 ml) and hydrogenolysed at 20 psi, at room temperature, in the presence of 10% Pd/C (40 mg) for 2 hours. Catalyst was filtered off and the filtrate was concentrated to dryness affording 220 mg of an off-white solid. A mixture of this product (163 mg, 0.91 mmol), HOAT (149 mg, 1.1 mmol), EDCI.HCl (211 mg, 1.1 mmol) in dry DCM (20 mL) was kept under stirring at ambient temperature for 30 minutes under nitrogen atmosphere. Then, (S)-3-(N-hydroxycarbamimidoyl)-piperidine-1-carboxylic acid tert-butyl ester (204 mg, 0.84 mmol) (prepared as described in Example 10(C)) was added and stirring at RT was maintained overnight. The reaction mixture was diluted with DCM and washed with water, then with 5% citric acid (aq) and $NaHCO_3$ satd. solution (aq). The organic layer was separated, dried over $Na_2SO_4$ and concentrated to dryness affording a beige solid (261 mg). This solid (250 mg) was suspended in $CH_3CN$ (3 ml) and heated at 100°C under microwaves irradiation for 3 hours, in a sealed tube. Then, the solution was concentrated *in vacuo* and the residue purified by flash chromatography (silica gel, eluent: petroleum ether/ethyl acetate 60:40) affording 192 mg of a white solid.
Yield: 55% (over 4 steps); LCMS (RT): 8.2 min (Method M); MS (ES+) gave m/z: 409.0 (M+23), 287.0 (M-99).

76(C) 3-[5-(4-Trifluoromethyl-1H-pyrrol-2-yl)-[1,2,4]oxadiazol-3-yl]-piperidine hydrochloride

(S)-3-[5-(4-Trifluoromethyl-1H-pyrrol-2-yl)-[1,2,4]oxadiazol-3-yl]-piperidine-1-carboxylic acid tert-butyl ester (192 mg, 0.5 mmol) was dissolved in 4M HCl in dioxane (2 mL), and the reaction mixture was stirred at room temperature for 1 h. The solvent was evaporated under reduced pressure to give the title compound, which was used for the next step without further purification.
Yield: quantitative; LCMS (RT): 1.39 min (Method L); MS (ES+) gave m/z: 287.0 (M+1).

76(D) (4-Fluoro-phenyl)-{(S)-3-[5-(4-trifluoromethyl-1H-pyrrol-2-yl)-[1,2,4]oxadiazol-3-yl]-piperidin-1-yl}-methanone

A mixture of 3-[5-(4-trifluoromethyl-1H-pyrrol-2-yl)-[1,2,4]oxadiazol-3-yl]-piperidine hydrochloride (73 mg, 0.22 mmol), 4-fluorobenzoyl chloride (26 $\mu$l, 0.22 mmol) and triethylamine (68 $\mu$l, 0.48 mmol) in DCM (7 ml), was stirred at room temperature overnight. The reaction mixture was concentrated and the residue was purified by flash chromatography (silica gel, eluent: petroleum ether/ethyl acetate 60:40) affording 71 mg of a white solid.
Yield: 79% (white solid); $[\alpha]_D^{20}$ = +94.3 (c=1.0, MeOH); mp= 183.5°C; LCMS (RT): 2.49 min (Method S); MS (ES+) gave m/z: 408.9 (MH+).
$^1$H-NMR (DMSO-$d_6$ 353K), $\delta$ (ppm): 12.83 (s br, 1H); 7.62 (m, 1H); 7.47 (dd, 2H); 7.22 (dd, 2H); 7.21 (m, 1H); 4.28 (m, 1H); 3.83 (m, 1H); 3.35 (dd, 1H); 3.22 (ddd, 1H); 3.13 (ddd, 1H); 2.21 (m, 1H); 1.97-1.78 (m, 2H); 1.63 (m, 1H).

Example 77

(3-Fluoro-pyridin-4-yl)-{(S)-3-[5-(4-trifluoromethyl-1H-pyrrol-2-yl)[1,2,4]oxadiazol-3-yl]-piperidin-1-yl}-methanone

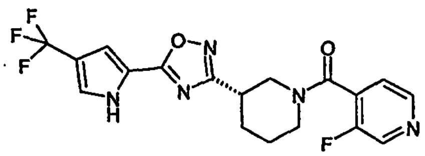

A mixture of 3-fluoro-isonicotinic acid (43 mg, 0.30 mmol) HOAT (50 mg, 0.37 mmol), EDCI.HCl (71 mg, 0.37 mmol) in dry DCM (8 mL) was kept under stirring at ambient temperature for 2 hours under nitrogen atmosphere. The reaction mixture was added to a solution of 3-[5-(4-trifluoromethyl-1H-pyrrol-2-yl)-[1,2,4]oxadiazol-3-yl]-piperidine hydrochloride (93 mg, 0.28 mmol), prepared as described in Example 76(C), and triethylamine (50 μL, 0.37 mmol) in DCM (2 mL) and the solution was kept under stirring at ambient temperature overnight. Then the reaction mixture was diluted with DCM and washed with water. The organic layer was separated, dried over $Na_2SO_4$ and concentrated. Flash chromatography purification of the crude (silica gel, eluent: petroleum ether/ethyl acetate 15: 85) afforded 66 mg of a white foam.
Yield: 57% (white foam); $[\alpha]_D^{20}$ = +76.4 (c=0.5, MeOH); LCMS (RT): 2.15 min (Method S); MS (ES+) gave m/z: 410.1 (MH+).
$^1$H-NMR (DMSO-$d_6$, 373 K), δ (ppm): 12.70 (s br, 1H); 8.61 (s, 1H); 8.50 (dd, 1H); 7.59 (m, 1H); 7.43 (dd, 1H); 7.19 (s br, 1H); 4.86-3.65 (m br, 2H); 3.42 (m, 1H); 3.28 (m, 1H); 3.13 (m, 1H); 2.22 (m, 1H); 2.01-1.80 (m, 2H); 1.65 (m, 1H).

Example 78

(6-Fluoro-pyridin-3-yl)-{(S)-3-[5-(4-trifluoromethyl-1H-pyrrol-2-yl)-[1,2,4]oxadiazol-3-yl]-piperidin-1-yl}-methanone

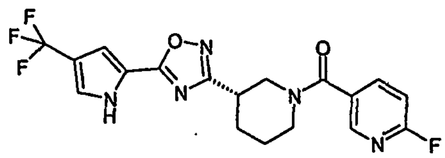

The compound was prepared following the procedure described in the Example 77, starting from 3-[5-(4-trifluoromethyl-1H-pyrrol-2-yl)-[1,2,4]oxadiazol-3-yl]-piperidine hydrochloride (93 mg, 0.28 mmol), prepared as described in Example 76(C), and using 6-fluoro-nicotinic acid (43 mg, 0.30 mmol) as the acid of choice. The final compound was purified by flash chromatography (silica gel, eluent: petroleum ether/ethyl acetate 30:70).
Yield: 38% (off-white solid); $[\alpha]_D^{20}$ = +124.0 (c=0.5, MeOH); mp= 165.7°C; LCMS (RT): 2.26 min (Method S); MS (ES+) gave m/z: 410.1 (MH+).
$^1$H-NMR (DMSO-$d_6$, 353K), δ (ppm): 12.80 (s br, 1H); 8.31 (ddd, 1H); 8.03 (ddd, 1H); 7.62 (m, 1H); 7.22 (m, 1H); 7.21 (ddd, 1H); 4.26 (m, 1H); 3.81 (m, 1H); 3.41 (dd, 1H); 3.28 (ddd, 1H); 3.17 (ddd, 1H); 2.22 (m, 1H); 2.00-1.78 (m, 2H); 1.68 (m, 1H).

Example 79

(3,4-Difluoro-phenyl)-{(S)-3-[3-(4-methyl-1H-imidazol-2-yl)-[1,2,4]oxadiazol-5-yl]-piperidin-1-yl}-methanone

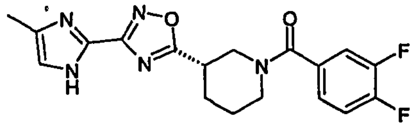

79(A) N-Hydroxy-4-methyl-1H-imidazole-2-carboxamidine

A solution of 4-methyl-1H-imidazole-2-carbonitrile (83 mg, 0.776 mmol), prepared according to Helvetica Chimica Acta, 2005, 88, 2454-2469, and $NH_2OH$ (50% water, 0.191 ml, 3.104 mmol) in absolute ethanol (2 ml) was heated at reflux for 1.5 h. The solvent was evaporated to give 110 mg of amorphous solid that was used in the next step without further purification.
Yield: quantitative; LC-MS (RT): 0.31 min (Method H), MS (ES+) gave m/z: 140.9 (MH+).

79(B) (S)-3-[3-(4-Methyl-1H-imidazol-2-yl)-[1,2,4]oxadiazol-5-yl]-piperidine-1-carboxylic acid tert-butyl ester

HOBT (118 mg, 0.776 mmol) and EDC (222 mg, 1.164 mmol) were added to a stirred solution of (S)-N-Boc-nipecotic acid (177 mg, 0.776 mmol) in dioxane (1.5 ml) at room temperature. After 1 h, a solution of N-hydroxy-4-methyl-1H-imidazole-2-carboxamidine (0.776 mmol) in dioxane (3 ml) was added and the mixture stirred at RT for 24 h. Ethyl acetate was added and the mixture was washed with 5% $NaHCO_3$ (aq); the organic phase was dried over $Na_2SO_4$ and concentrated. The crude was purified by flash chromatography (silica gel cartridge, eluent: ethyl acetate/petroleum ether 2:1) to give 240 mg of pure product.

A mixture of the obtained product (240 mg, 0.683 mmol) and molecular sieves (4A, 50 mg) in acetonitrile (3 ml) was heated at 130°C for 3 h in a sealed tube, under microwave irradiation. Molecular sieves were filtered off and the solution was concentrated. The crude was purified by flash chromatography (silica gel cartridge, eluent: ethyl acetate/petroleum ether 2:1) to give 152 mg of title compound (transparent viscous oil).
Yield: 67%; LC-MS (RT): 1.05 min (Method H), MS (ES+) gave m/z: 334.0 (MH+).

79(C) (S)-3-[3-(4-Methyl-1H-imidazol-2-yl)-[1,2,4]oxadiazol-5-yl]-piperidine dihydrochloride

A mixture of S)-3-[3-(4-methyl-1H-imidazol-2-yl)-[1,2,4]oxadiazol-5-yl]-piperidine-1-carboxylic acid tert-butyl ester (152 mg, 0.456 mmol) and HCl (4M dioxane solution, 0.57 ml) in dichloromethane (3 ml) was stirred at room temperature for 20 h. The solvent was evaporated to give a white solid (140 mg) that was used in the next step without further purification.
Yield: quantitative; LC-MS (RT): 0.32 min (Method H ), MS (ES+) gave m/z: 234.1 (MH+).

79(D) (3,4-Difluoro-phenyl)-{(S)-3-[3-(4-methyl-1H-imidazol-2-yl)-[1,2,4]oxadiazol-5-yl]-piperidin-1-yl}-methanone

A mixture of 3,4-difluoro-benzoyl chloride (0.057 ml, 0.456 mmol) in 2 ml of dichloromethane was added to a stirred solution of (S)-3-[3-(4-methyl-1H-imidazol-2-yl)-[1,2,4]oxadiazol-5-yl]-piperidine dihydrochloride (140 mg, 0.456 mmol) and triethylamine (0.255 ml, 1.824 mmol) in 2 ml of dichloromethane at 0°C. After 30 min the solvent was evaporated, the residue was partitioned between ethyl acetate and 5% $NaHCO_3$ (aq). The aqueous phase was separated and extracted twice with ethyl acetate; the combined organic layers were dried over $Na_2SO_4$ and concentrated. The crude was purified by flash chromatography (silica gel cartridge, eluent: dichloromethane/methanol 20/0.8) to give 118 mg of title compound (amorphous solid).
Yield: 69%. LCMS (RT): 1.92 min (Method N); MS (ES+) gave m/z: 374.3 (MH+). $^1$H-NMR (DMSO-$d_6$, 353 K), δ (ppm): 12.58 (s br, 1H); 7.53-7.40 (m, 2H); 7.28 (m, 1H); 6.93 (s, 1H); 4.22 (m, 1H); 3.76 (m, 1H); 3.53 (dd, 1H); 3.42 (ddd, 1H); 3.29 (ddd, 1H); 2.27 (m, 1H); 2.24 (s, 3H); 1.98 (m, 1H); 1.83 (m, 1H); 1.66 (m, 1H).

Example 80

{(S)-3-[5-(4-Chloro-1H-pyrrol-2-yl)-[1,2,4]oxadiazol-3-yl]-piperidin-1-yl}-pyridin-4-yl-methanone

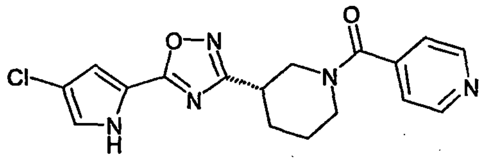

The title compound was prepared following the experimental procedure described in Example 28(C), starting from (S)-3-[5-(4-chloro-1H-pyrrol-2-yl)-[1,2,4]oxadiazol-3-yl]-piperidine hydrochloride, prepared as described in Example 39 (C), and using isonicotinic acid as the acid of choice.
Purification was performed by flash chromatography (silica gel, eluent: petroleum ether/ethyl acetate 2:8 + 1%$NH_4OH$).
Yield: 38% (gummy white solid); LCMS (RT): 1.62 min (Method S); MS (ES+) gave m/z: 358.1 (MH+).

Example 81

(6-Fluoro-pyridin-3-yl)-{(S)-3-[3-(4-trifluoromethyl-1H-pyrrol-2-yl)-[1,2,4]oxadiazol-5-yl]-piperidin-1-yl}-methanone

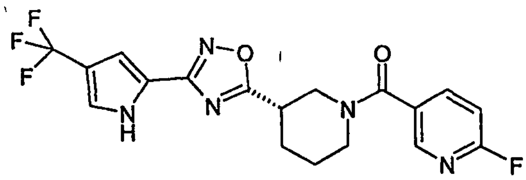

81(A) 4-Trifluoromethyl-1H-pyrrole-2- carboxylic acid amide

Carbonyl diimidazole (379 mg, 2.34 mmol) was added to a solution of 4-trifluoromethyl-1H-pyrrole-2-carboxylic acid (350 mg, 1.95 mmol) in MeCN (10 mL) and stirred for 90 min. Concentrated $NH_4OH$ solution (2 mL) was added and the resulting mixture refluxed for 90 min. The solvent was removed, 10% citric acid solution (10 mL) was added and the solution extracted three times with EtOAc. The organic extracts were combined, dried over sodium sulphate and the solvent removed to give the product as a syrup.
Yield: 100% LCMS (RT): 1.29 min (Method L); MS (ES+) gave m/z: 178.9 (MH+).

81(B) 4-Trifluoromethyl -*N*-hydroxy-1H-pyrrole-2-carboxamidine

A solution of 4-Trifluoromethyl-1H-pyrrole-2-carboxylic acid amide (347 mg, 1.95 mmol) in phosphorus oxychloride (5 mL) was heated at 100°C for 5 minutes, cooled, ice was added, basified with conc. $NH_4OH$ solution then extracted three times with EtOAc. The organic extracts were combined, dried and the solvent removed to give a pale brown oil. This product was treated with 50% Hydroxylamine solution in water (1.2 mL, 20 mmol) and heated under reflux for 1h. The solvent was removed under vacuum and the residue purified by flash chromatography (silica gel cartridge, eluent gradient: from hexane/ethyl acetate 100:0 to hexane/ethyl acetate 0:100) to give the product as a syrup.
Yield: 42% LCMS (RT): 0.93 min (Method L); MS (ES+) gave m/z: 193.9 (MH+).

81(C) (S)-3-[3-(4-Trifluoromethyl-1H-pyrrol-2-yl)-[1,2,4]oxadiazol-5-yl]-piperidine-1-carboxylic acid tert-butyl ester

A mixture of (S)-N-Boc-nipecotic acid (206 mg, 0.90 mmol), HOAT (147 mg, 1.08 mmol), EDCI.HCl (207 mg, 1.08 mmol) in dry DCM (15 mL) was stirred under $N_2$ for 45 minutes, then 4-Trifluoromethyl-*N*-hydroxy-1H-pyrrole-2-carboxamidine (160 mg, 0.83 mmol) was added and the solution stirred 3 hours. The solution was washed with water, 10% citric acid solution and 5% $NaHCO_3$ solution, dried over sodium sulphate and the solvent removed to give a residue that was purified by flash chromatography (silica gel cartridge, eluent gradient: from hexane/ethyl acetate 100:0 to hexane/ethyl acetate 80:20). The solid thus obtained was dissolved in acetonitrile (2 mL) and heated in a sealed tube at 80°C for 75 min in a microwave reactor. The solvent was removed and the crude residue was purified by flash chromatography (silica gel, petroleum ether/ethyl acetate 70:30) to give the product as a syrup.

Yield: 43 %; LCMS (RT): 2.66 min (Method L); MS (ES+) gave m/z: 408.9 (MNa+).

81(D) (S)-3-[3-(4-Trifluorometuyl-1H-pyrrol-2-yl)-[1,2,4]oxadiazol-5-yl]-piperidine hydrochloride salt

(S)-3-[3-(4-Trifluoromethyl-1H-pyrrol-2-yl)-[1,2,4]oxadiazol-5-yl]-piperidine-1-carboxylic acid tert-butyl ester (140 mg, 0.36 mmol) was dissolved in 4M HCl in dioxane (2 mL), and the reaction mixture was stirred, at room temperature for 1 h. The solvent was evaporated under reduced pressure to give the title compound, which was used for the next step without further purification.
Yield: quantitative; LCMS (RT): 1.38 min (Method L); MS (ES+) gave m/z: 286.9 (M+1).

81(E) (4-Fluoro-phenyl)-{(S)-3-[3-(4-Trifluoromethyl-1H-pyrrol-2-yl)-[1,2,4]oxadiazol-5-yl]-piperidin-1-yl}-methanone

A mixture of 6-Fluoro-nicotinic acid (37 mg, 0.26 mmol), HOAT (38 mg, 0.28 mmol), EDCI.HCl (55 mg, 0.28 mmol) in dry DCM (8 mL) was kept under stirring at ambient temperature for 1.5 hours under nitrogen atmosphere. The reaction mixture was added to a solution of (S)-3-[3-(4-Trifluoromethyl-1H-pyrrol-2-yl)-[1,2,4]oxadiazol-5-yl]-piperidine hydrochloride salt (77 mg, 0.24 mmol) and triethylamine (73 uL, 0.54 mmol) in DCM (2 mL) and the solution was kept under stirring at ambient temperature overnight. Then the reaction mixture was diluted with DCM and washed with water. The organic layer was separated, dried over $Na_2SO_4$ and concentrated. Flash chromatography purification of the crude (silica gel, petroleum ether/ethyl acetate 50: 50) afforded 72 mg of a gummy solid.
Yield: 73%; LCMS (RT): 2.12 min (Method L); MS (ES+) gave m/z: 409.8 (MH+), 431.9 (M-Na+).

PHARMACOLOGY:

The compounds provided in the present invention are positive allosteric modulators of mGluR5. As such, these compounds do not appear to bind to the orthosteric glutamate recognition site, and do not activate the mGluR5 by themselves. Instead, the response of mGluR5 to a concentration of glutamate or mGluR5 agonist is increased when compounds of Formula I are present. Compounds of Formula I are expected to have their effect at mGluR5 by virtue of their ability to enhance the function of the receptor.

**EXAMPLE A**

**mGluR5 assay on rat cultured cortical astrocytes**

Under exposure to growth factors (basic fibroblast growth factor, epidermal growth factor), rat cultured astrocytes express group I-Gq coupled mGluR transcripts, namely mGluR5, but none of the splice variants of mGluR1, and as a consequence, a functional expression of mGluR5 receptors (Miller et al. (1995) J. Neurosci. 15:6103-9): The stimulation of mGluR5 receptors with selective agonist CHPG and the full blockade of the glutamate-induced phosphoinositide (PI) hydrolysis and subsequent intracellular calcium mobilization with specific antagonist as MPEP confirm the unique expression of mGluR5 receptors in this preparation. This preparation was established and used in order to assess the properties of the compounds of the present invention to increase the $Ca^{2+}$ mobilization-induced by glutamate without showing any significant activity when applied in the absence of glutamate.

**Primary cortical astrocytes culture:**

Primary glial cultures were prepared from cortices of Sprague-Dawley 16 to 19 days old embryos using a modification of methods described by Mc Carthy and de Vellis (1980) J. Cell Biol. 85:890-902 and Miller et al. (1995) J. Neurosci. 15 (9):6103-9. The cortices were dissected and then dissociated by trituration in a sterile buffer containing 5.36 mM KCl, 0.44 mM $NaHCO_3$, 4.17 mM $KH_2PO_4$, 137 mM NaCl, 0.34 mM $NaH_2PO_4$, 1 g/L glucose. The resulting cell homogenate was plated onto poly-D-lysine precoated T175 flasks (BIOCOAT, Becton Dickinson Biosciences, Erembodegem, Belgium) in Dubelcco's Modified Eagle's Medium (D-MEM GlutaMAX™ I, Invitrogen, Basel, Switzerland) buffered with 25 mM HEPES and 22.7 mM $NaHCO_3$, and supplemented with 4.5g/L glucose, 1 mM pyruvate and 15 % fetal bovine serum (FBS, Invitrogen, Basel, Switzerland), penicillin and streptomycin and incubated at 37°C with 5% $CO_2$. For subsequent seeding, the FBS supplementation was reduced to 10 %. After 12 days, cells were subplated by trypsinisation onto poly-D-lysine precoated 384-well plates at a density of 20.000 cells per well in culture buffer.

**$Ca^{2+}$ mobilization assay using rat cortical astrocytes:**

After one day of incubation, cells were washed with assay buffer containing: 142 mM NaCl, 6 mM KCl, 1 mM

$Mg_2SO_4$, 1 mM $CaCl_2$, 20 mM HEPES, 1 g/L glucose, 0.125 mM sulfinpyrazone, pH 7.4. After 60 min of loading with 4 μM Fluo-4 (TefLabs, Austin, TX), the cells were washed three times with 50 μl of PBS Buffer and resuspended in 45 μl of assay Buffer. The plates were then transferred to a Fluorometric Imaging Plate Reader (FLIPR, Molecular Devices, Sunnyvale, CA) for the assessment of intracellular calcium flux. After monitoring the baseline fluorescence for 10 s, a solution containing 10μM of representative compound of the present invention diluted in Assay Buffer (15 μl of 4X dilutions) was added to the cell plate in the absence or in the presence of 300 nM of glutamate. Under these experimental conditions, this concentration induces less than 20 % of the maximal response of glutamate and was the concentration used to detect the positive allosteric modulator properties of the compounds from the present invention. The final DMSO concentration in the assay was 0.3 %. In each experiment, fluorescence was then monitored as a function of time for 3 minutes and the data analyzed using Microsoft Excel and GraphPad Prism. Each data point was also measured two times.

The results in Figure 1 represent the effect of 10 μM of Example # 1 on primary cortical mGluR5-expressing cell cultures in the absence or in the presence of 300 nM glutamate. Data are expressed as the percentage of maximal response observed with 30 μM glutamate applied to the cells. Each bar graph is the mean and S.E.M of duplicate data points and is representative of three independent experiments

The results shown in Example A demonstrate that the compounds described in the present invention do not have an effect per se on mGluR5. Instead, when compounds are added together with an mGluR5 agonist such as glutamate, the effect measured is significantly potentiated compared to the effect of the agonist alone at the same concentration. This data indicates that the compounds of the present invention are positive allosteric modulators of mGluR5 receptors in native preparations.

**EXAMPLE B**

**<u>mGluR5 assay on HEK-expressine: rat mGluR5</u>**

**Cell culture**

Positive functional expression of HEK-293 cells stably expressing rat mGluR5 receptor was determined by measuring intracellular $Ca^{2+}$ changes using a Fluorometric Imaging Plate Reader (FLIPR, Molecular Devices, Sunnyvale, CA) in response to glutamate or selective known mGluR5 agonists and antagonists. Rat mGluR5 RT-PCR products in HEK-293 cells were sequenced and found 100% identical to rat mGluR5 Genbank reference sequence (NM_017012). HEK-293 cells expressing rmGluR5 were maintained in media containing DMEM, dialyzed Fetal Bovine Serum (10 %), Glutamax™ (2 mM), Penicillin (100 units/ml), Streptomycin (100 μg/ml), Geneticin (100 μg/ml) and Hygromycin-B (40 μg/ml) at 37°C/5%CO2.

**Fluorescent cell based- $Ca^{2+}$ mobilization assay**

After one day of incubation, cells were washed with assay buffer containing: 142 mM NaCl, 6 mM KCl, 1 mM $Mg_2SO_4$, 1 mM $CaCl_2$, 20 mM HEPES, 1 g/L glucose, 0.125 mM sulfinpyrazone, pH 7.4. After 60 min of loading with 4 uM Fluo-4 (TefLabs, Austin, TX), the cells were washed three times with 50 μl of PBS Buffer and resuspended in 45 μl of assay Buffer. The plates were then transferred to a Fluorometric Imaging Plate Reader (FLIPR, Molecular Devices, Sunnyvale, CA) for the assessment of intracellular calcium flux. After monitoring the baseline fluorescence for 10 seconds, increasing concentrations of representative compound (from 0.01 to 60 μM) of the present invention diluted in Assay Buffer (15 μl of 4X dilutions) was added to the cell. The final DMSO concentration in the assay was 0.3 %. In each experiment, fluorescence was then monitored as a function of time for 3 minutes and the data analyzed using Microsoft Excel and GraphPad Prism. Each data point was also measured two times.

Under these experimental conditions, this HEK-rat mGluR5 cell line is able to directly detect positive allosteric modulators without the need of co-addition of glutamate or mGluR5 agonist. Thus, DFB, CPPHA and CDPPB, published reference positive allosteric modulators that are inactive in rat cortical astrocytes culture in the absence of added glutamate (Liu et al (2006) Eur. J. Pharmacol. 536:262-268; Zhang et al (2005); J. Pharmacol. Exp. Ther. 315:1212-1219) are activating, in this system, rat mGluR5 receptors.

The concentration-response curves of representative compounds of the present invention were generated using the Prism GraphPad software (Graph Pad Inc, San Diego, USA). The curves were fitted to a four-parameter logistic equation:

$$(Y=Bottom + (Top-Bottom)/(1+10^\wedge((LogEC_{50}-X)*Hill\ Slope)$$

allowing determination of $EC_{50}$ values.

The Table 1 below represents the mean $EC_{50}$ obtained from at least three independent experiments of selected molecules performed in duplicate.

**Table 1:**

| EXAMPLE | Ca++ Flux* | EXAMPLE | Ca++ Flux* | EXAMPLE | Ca++ Flux* |
|---|---|---|---|---|---|
| 1 | +++ | 25 | ++ | 49 | ++ |
| 2 | +++ | 26 | ++ | 50 | +++ |
| 3 | +++ | 27 | +++ | 51 | +++ |
| 4 | ++ | 28 | +++ | 52 | +++ |
| 5 | +++ | 29 | +++ | 53 | +++ |
| 6 | +++ | 30 | +++ | 54 | +++ |
| 7 | +++ | 31 | +++ | 55 | ++ |
| 8 | ++ | 32 | +++ | 57 | ++ |
| 9 | ++ | 33 | +++ | 58 | +++ |
| 10 | +++ | 34 | +++ | 60 | +++ |
| 11 | ++ | 35 | +++ | 64 | ++ |
| 12 | ++ | 36 | +++ | 65 | ++ |
| 13 | + | 37 | ++ | 66 | ++ |
| 14 | + | 38 | ++ | 67 | ++ |
| 15 | + | 39 | +++ | 68 | ++ |
| 16 | ++ | 40 | +++ | 69 | ++ |
| 17 | ++ | 41 | +++ | 70 | ++ |
| 18 | ++ | 42 | +++ | 71 | ++ |
| 19 | ++ | 43 | +++ | 72 | ++ |
| 20 | + | 44 | +++ | 76 | + |
| 21 | +++ | 44 | + | 77 | ++ |
| 22 | +++ | 45 | +++ | 79 | ++ |
| 23 | ++ | 46 | +++ | | |
| 24 | ++ | 47 | ++ | | |

***Table legend:**
(+) : $EC_{50} > 10\ \mu M$
(++): $1\ \mu M < EC_{50} < 10\ \mu M$
(+++): $EC_{50} < 1\ \mu M$

**EXAMPLE C**

**mGluR5 binding assay**

Activity of compounds of the invention was examined following a radioligand binding technique using whole rat brain and tritiated 2-methyl-6-(phenylethynyl)-pyridine ( $[^3H]$-MPEP) as a ligand following similar methods than those described in Gasparini et al. (2002) Bioorg. Med. Chem. Lett. 12:407-409 and in Anderson et al. (2002) J. Pharmacol. Exp. Ther. 303 (3) 1044-1051.

**Membrane preparation:**

Cortices were dissected out from brains of 200-300g Sprague-Dawley rats (Charles River Laboratories, L'Arbresle, France). Tissues were homogenized in 10 volumes (vol/wt) of ice-cold 50 mM HEPES-NaOH (pH 7.4) using a Polytron disrupter (Kinematica AG, Luzern, Switzerland) and centrifuged for 30 min at 40,000 g. (4°C). The supernatant was discarded and the pellet washed twice by resuspension in 10 volumes 50 mM HEPES-NaOH. Membranes were then collected by centrifugation and washed before final resuspension in 10 volumes of 20 mM HEPES-NaOH, pH 7.4. Protein concentration was determined by the Bradford method (Bio-Rad protein assay, Reinach, Switzerland) with bovine serum albumin as standard.

**[$^3$H]-MPEP binding experiments:**

Membranes were thawed and resuspended in binding buffer containing 20 mM HEPES-NaOH, 3 mM $MgCl_2$, 3 mM $CaCl_2$, 100 mM NaCl, pH 7.4. Competition studies were carried out by incubating for 1h at 4°C: 3 nM [$^3$H]-MPEP (39 Ci/mmol, Tocris, Cookson Ltd, Bristol, U.K.), 50 μg membrane and a concentration range of 0.003 nM- 30 μM of compounds, for a total reaction volume of 300 μl. The nonspecific binding was defined using 30 μM MPEP. Reaction was terminated by rapid filtration over glass-fiber filter plates (Unifilter 96-well GF/B filter plates, Perkin-Elmer, Schwerzenbach, Switzerland) using 4 x 400 μl ice cold buffer using cell harvester (Filtermate, Perkin-Elmer, Downers Grove, USA). Radioactivity was determined by liquid scintillation spectrometry using a 96-well plate reader (TopCount, Perkin-Elmer, Downers Grove, USA).

**Data analysis:**

The inhibition curves were generated using the Prism GraphPad program (Graph Pad Software Inc, San Diego, USA). $IC_{50}$ determinations were made from data obtained from 8 point-concentration response curves using a non linear regression analysis. The mean of $IC_{50}$ obtained from at least three independent experiments of selected molecules performed in duplicate were calculated.

The compounds of this application have $IC_{50}$ values in the range of less than 100 μM. Example # 1 has $IC_{50}$ value of less than 30 μM.

The results shown in Examples A, B and C demonstrate that the compounds described in the present invention are positive allosteric modulators of rat mGluR5 receptors. These compounds are active in native systems and are able to inhibit the binding of the prototype mGluR5 allosteric modulator [$^3$H]-MPEP known to bind remotely from the glutamate binding site into the transmembrane domains of mGluR5 receptors (Malherbe et al (2003) Mol. Pharmacol. 64(4):823-32)

Thus, the positive allosteric modulators provided in the present invention are expected to increase the effectiveness of glutamate or mGluR5 agonists at mGluR5 receptor. Therefore, these positive allosteric modulators are expected to be useful for treatment of various neurological and psychiatric disorders associated with glutamate dysfunction described to be treated herein and others that can be treated by such positive allosteric modulators.

**EXAMPLE D**

**Amphetamine model of schizophrenia**

Amphetamine-induced increases in locomotor ambulation are well known and are widely used as a model of the positive symptoms of schizophrenia. This model is based on evidence that amphetamine increases motor behaviors and can induce a psychotic state in humans (Yui et al. (2000) Ann. N.Y. Acad. Sci. 914:1-12). Further, it is well known that amphetamine-induced increases in locomotor activity are blocked by antipsychotics drugs that are effective in the treatment of schizophrenia (Arnt (1995) Eur. J. Pharmacol. 283:55-62). These results demonstrate that locomotor activation induced by amphetamine is a useful model for screening of compounds which may be useful in the treatment of schizophrenia.

Subjects: The present studies were performed in accordance with the animal care and use policies of Addex Pharmaceuticals and the laws and directives of Switzerland governing the care and use of animals. Male C57BL6/j mice (20-30 g) 7 weeks of age at the time of delivery were group housed in a temperature and humidity controlled facility on a 12 hour light /dark cycle for at least 7 days before use. Mice had access to food and water ad libitum except during locomotor activity experiments.

**Assessment of locomotor (ambulatory) activity:** The effects of compounds on amphetamine-induced locomotor activation in mice were tested. Locomotor activity of mice was tested in white plastic boxes 35 cm X 35 cm square with walls 40 cm in height. Locomotor activity (ambulations) was monitored by a videotracking system (VideoTrack, Viewpoint, Champagne au Mont d'Or, France) that recorded the ambulatory movements of mice. Mice were naïve to

the apparatus prior to testing. On test days, test compounds (10, 30, 50 or 100 mg/kg i.p. (intraperitoneal)) or vehicle were administered 120 minutes before amphetamine (3.0 mg/kg s.c.) or saline injection. Mice were placed into the locomotor boxes immediately after amphetamine or saline vehicle injection and their locomotor activity, defined as the distance traveled in centimeters (cm), was measured for 60 minutes.

**Compound administration:** Compounds were prepared as a microsuspension in sterile water (60% of final volume) and Labrafil M1944 CS (apricot kernel oil - Gattefossé, Saint Priest, France) (40% of final volume) and administered in a volume of 10 ml/kg. Compound-vehicle-treated mice received the equivalent volume of vehicle solution i.p. in the absence of added compound. D-amphetamine sulfate (Amino AG, Neuenhof, Switzerland) was dissolved in saline and administered at a dose of 3.0 mg/kg s.c. in a volume of 10 ml/kg. D-amphetamine-vehicle-treated mice received an equivalent volume of saline vehicle injected s.c.

Statistical analyses: Statistical analyses were performed using GraphPad PRISM statistical software (GraphPad, San Diego, CA, USA). Data were analyzed using one-way analysis of variance (ANOVA) followed by post-hoc Bonferroni-corrected multiple comparisons, where appropriate. The significance level was set at $p<0.05$.

**Effect of compounds on amphetamine-induced locomotor activity in mice**

Representative compound of the invention significantly attenuated the increase in locomotor activity induced by amphetamine.

The compounds of the present invention are allosteric modulators of mGluR5 receptors, they are useful for the production of medications, especially for the prevention or treatment of central nervous system disorders as well as other disorders modulated by this receptor.

The compounds of the invention can be administered either alone, or in combination with other pharmaceutical agents effective in the treatment of conditions mentioned above.

FORMULATION EXAMPLES

Typical examples of recipes for the formulation of the invention are as follows:

1) Tablets

| | |
|---|---|
| Compound of the example 1 | 5 to 50 mg |
| Di-calcium phosphate | 20 mg |
| Lactose | 30 mg |
| Talcum | 10 mg |
| Magnesium stearate | 5 mg |
| Potato starch | ad 200 mg |

In this example, the compound of the example 1 can be replaced by the same amount of any of the described examples according to the invention.

2) Suspension

An aqueous suspension is prepared for oral administration so that each 1 milliliter contains 1 to 5 mg of one of the described example, 50 mg of sodium carboxymethyl cellulose, 1 mg of sodium benzoate, 500 mg of sorbitol and water ad 1 ml.

3) Injectable

A parenteral composition is prepared by stirring 1.5 % by weight of active ingredient of the invention in 10% by volume propylene glycol and water.

4) Ointment

| | |
|---|---|
| Compound of the example 1 | 5 to 1000 mg |
| Stearyl alcohol | 3 g |
| Lanoline | 5 g |
| White petroleum | 15 g |
| Water | ad 100 g |

In this example, the compound 1 can be replaced by the same amount of any of the described examples according to the invention.

## Claims

1. A compound having the formula I-B

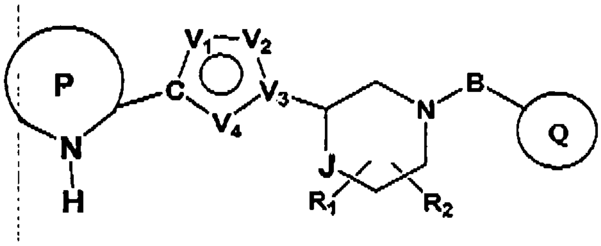

I-B

Wherein

$R_1$ and $R_2$ represent independently hydrogen, $-(C_1-C_6)$alkyl, $-(C_2-C_6)$alkenyl, - $(C_2-C_6)$alkynyl, arylalkyl, heteroarylalkyl, hydroxy, amino, aminoalkyl, hydroxyalkyl, $-(C_1-C_6)$alkoxy or $R_1$ and $R_2$ together can form a $(C_3-C_7)$ cycloalkyl ring, a carbonyl bond C=O or a carbon double bond;
P represents a $(C_5-C_7)$heterocycloalkyl, $(C_5-C_7)$heterocycloalkenyl ring or a heteroaryl group of formula

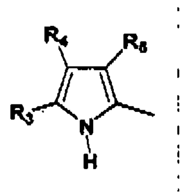 , 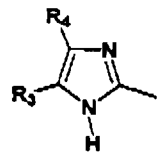 , 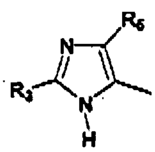 , 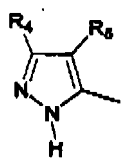 , 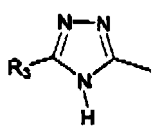 ,

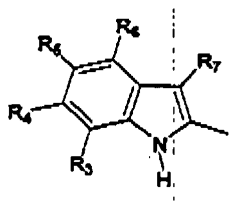 , 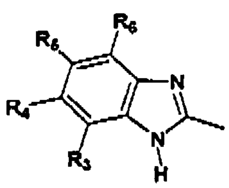 , 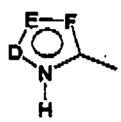 , 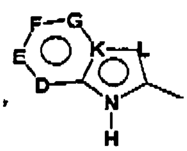 .

$R_3$, $R_4$, $R_5$, $R_6$, and $R_7$ independently are hydrogen, halogen, $-NO_2$, - $(C_1-C_6)$alkyl, $-(C_3-C_6)$cycloalkyl, $-(C_3-C_7)$ cycloalkylalkyl, $-(C_2-C_6)$alkenyl, $-(C_2-C_6)$alkynyl, halo-$(C_1-C_6)$alkyl, heteroaryl, heteroaxylalkyl, arylalkyl, aryl, $-OR_8$, $-NR_8R_9$, $-C(=NR_{10})NR_8R_9$, - $NR_8COR_9$, $NR_8CO_2R_9$, $NR_8SO_2R_9$, $-NR_{10}CO\ NR_8R_9$, $-SR_8$, $-S(=O)R_8$, -S $(=O)_2R_8$, $-S(=O)_2NR_8R_9$, $-C(=O)R_8$, $-C(O)-O-R_8$, $-C(=O)NR_8R_9$, - $C(=NR_8)R_9$, or $C(=NOR_8)R_9$ substituents; wherein optionally two substituents are combined to the intervening atoms to form a bicyclic heterocycloalkyl, aryl or heteroaryl ring; wherein each ring is optionally further substituted with 1-5 independent halogen, -CN, $-(C_1-C_6)$alkyl, $-O-(C_0-C_6)$alkyl, $-O-(C_3-C_7)$cycloalkylalkyl, -O(aryl), - O(heteroaryl), $-O-(-C_1-C_3)$alkylaryl, $-O-(C_1-C_3)$alkylheteroaryl, $-N((-C_0-C_6)$alkyl$)((C_0-C_3)$alkylaryl) or $-N((C_0-C_6)$alkyl$)((C_0-C_3-)$alkylheteroaryl)

groups;

$R_8$, $R_9$, $R_{10}$ each independently is hydrogen, $(C_1-C_6)$alkyl, $(C_3-C_6)$cycloalkyl, $(C_3-C_7)$cycloalkylalkyl, $(C_2-C_6)$ alkenyl, $(C_2-C_6)$alkynyl, halo-$(C_1-C_6)$alkyl, heterocycloalkyl, heteroaryl, heteroarylalkyl, axylalkyl or aryl; any of which is optionally substituted with 1-5 independent halogen, -CN, -$(C_1-C_6)$alkyl, -O-$(C_0-C_6)$alkyl, -O-$(C_3-C_7)$ cycloalkylalkyl, -O(aryl), -O(heteroaryl), -N($C_0$-$C_6$-alkyl)$_2$,-N(($C_0$-$C_6$)alkyl)(($C_3$-$C_7$-)cycloalkyl) or N(($C_0$-$C_6$)alkyl) (aryl) substituents;

D, E, F, G, K and L in P independently represent -C($R_3$)=, -C($R_3$)=C($R_4$)-,-C(=O)-, -C(=S)-, -O-, -N=, -N($R_3$)- or -S-;

Q denotes a $(C_5-C_6)$cycloalkyl, an aryl or heteroaryl group of formula

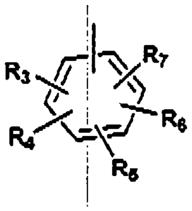 , 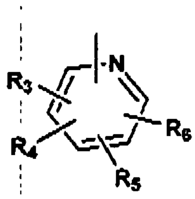 , 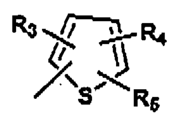 , 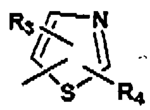 , 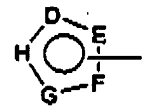

$R_3$, $R_4$, $R_5$, $R_6$, and $R_7$ independently are as defined above;

D, E, F, G and H in Q independently represent -C($R_3$)=, -C($R_3$)=C($R_4$)-,-C(=O)-, -C(=S)-, -O-, -N=, -N($R_3$)- or -S-;

$V_1$, $V_2$ and $V_4$ represent independently -0- or -N= and $V_3$ is C or $V_1$, $V_2$ and $V_4$ represent -N= and $V_3$ is -N-;

B represents a single bond, -C(=O)-$(C_0-C_2)$alkyl-, -C(=O)-$(C_2-C_6)$alkenyl-, -C(=O)-$(C_2-C_6)$alkynyl-, -C(=O)-O-, C(=O)N$R_8$-$(C_0-C_2)$alkyl-, -C(=N$R_8$)N$R_9$, -S(=O)-$(C_0-C_2)$alkyl-, -S(=O)$_2$-$(C_0-C_2)$alkyl-, -S(=O)$_2$N$R_8$-$(C_0-C_2)$ alkyl-, C(=N$R_8$)-$(C_0-C_2)$alkyl-, -C(=NO$R_8$)-$(C_0-C_2)$alkyl- or -C(=NO$R_8$)N$R_9$-$(C_0-C_2)$alkyl-;

$R_8$ and $R_9$, independently are as defined above;

J represents -C($R_{10}$, $R_{11}$), -O-, -N($R_{10}$)- or -S-;

$R_{10}$, $R_{11}$ independently are hydrogen, -$(C_1-C_6)$alkyl, -$(C_3-C_6)$cycloalkyl, -$(C_3-C_7)$cycloalkylalkyl, -$(C_2-C_6)$alkenyl, -$(C_2-C_6)$alkynyl, halo$(C_1-C_6)$alkyl, heteroaryl, heteroarylalkyl, arylalkyl or aryl; any of which is optionally substituted with 1-5 independent halogen, -CN, -$(C_1-C_6)$alkyl -O$(C_0-C_6)$alkyl, -O$(C_3-C_7)$cycloalkylalkyl, -O(aryl), -O (heteroaryl), -N(($C_0$-$C_6$)alkyl)(($C_0$-$C_6$)alkyl),-N(($C_0$-$C_6$)alkyl)(($C_3$-$C_7$)cycloalkyl) or N(($C_0$-$C_6$)alkyl)(aryl) substituents;

Any N may be an N-oxide;

or pharmaceutically acceptable salts, hydrates or solvates of such compounds.

**2.** A compound according to claim 1 having the formula I-C

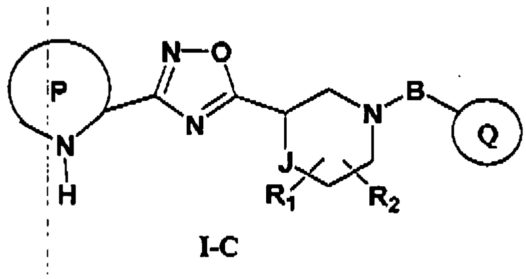

I-C

Wherein

$R_1$ and $R_2$ represent independently hydrogen, -$(C_1-C_6)$alkyl, -$(C_2-C_6)$akenyl, - $(C_2-C_6)$alkynyl, arylalkyl, heteroarylalkyl, hydroxy, amino, aminoalkyl, hydroxyalkyl, -$(C_1-C_6)$alkoxy or $R_1$ and $R_2$ together can form a $(C_3-C_7)$ cycloalkyl ring, a carbonyl bond C=O or a carbon double bond;

P represents a $(C_5-C_7)$heterocycloalkyl, $(C_5-C_7)$heterocycloalkenyl ring or a heteroaryl group of formula

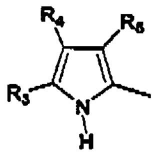, 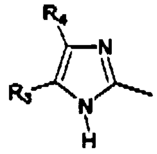, 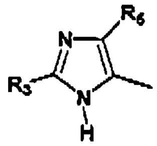, 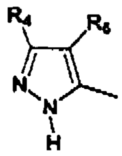, 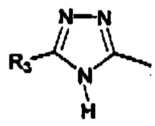,

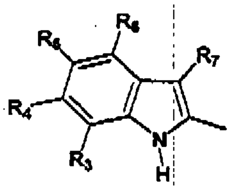, 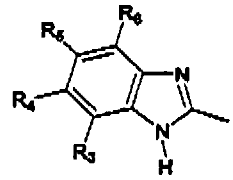, 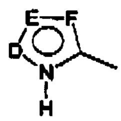, 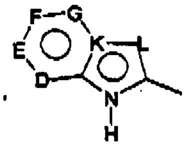.

$R_3$, $R_4$, $R_5$, $R_6$, and $R_7$ independently are hydrogen, halogen, $-NO_2$, - $(C_1-C_6)$alkyl, $-(C_3-C_6)$cycloalkyl, $-(C_3-C_7)$cycloalkylalkyl, $-(C_2-C_6)$alkenyl, $-(C_2-C_6)$alkynyl, halo-$(C_1-C_6)$alkyl, heteroaryl, heteroarylalkyl, arylalkyl, aryl, $-OR_8$, $-NR_8R_9$, $-C(=NR_{10})NR_8R_9$, - $NR_8COR_9$, $NR_8CO_2R_9$, $NR_8SO_2R_9$, $-NR_{10}CO$ $NR_8R_9$, $-SR_8$, $-S(=O)R_8$, -S $(=O)_2R_8$, $-S(=O)_2NR_8R_9$, $-C(=O)R_8$, $-C(O)-O-R_8$, $-C(=O)NR_8R_9$, - $C(=NR_8)R_9$, or $C(=NOR_8)R_9$ substituents; wherein optionally two substituents are combined to the intervening atoms to form a bicyclic heterocycloalkyl, aryl or heteroaryl, ring; wherein each ring is optionally further substituted with 1-5 independent halogen, -CN, $-(C_1$ - $C_6)$alkyl. $-O-(C_0-C_6)$alkyl, $-O-(C_3-C_7)$cycloalkylalkyl, -O(acryl), - O(heteroaryl), $-O-(-C_1-C_3)$alkylaryl, $-O-(C_1-C_3)$alkylheteroaryl, $-N((-C_0-C_6)$alkyl$)((C_0-C_3)$alkylaryl) or $N((C_0-C_6)$alkyl$)((C_0-C_3-)$alkylheteroaryl) groups;

$R_8$, $R_9$, $R_{10}$ each independently is hydrogen, $(C_1-C_6)$alkyl, $(C_3-C_6)$cycloalkyl, $(C_3-C_7)$cycloalkylalkyl, $(C_2-C_6)$alkenyl, $(C_2-C_6)$alkynyl, halo-$(C_1-C_6)$alkyl, heterocycloalkyl, heteroaryl, heteroarylalkyl, arylalkyl or aryl; any of which is optionally substituted with 1-5 independent halogen, -CN, $-(C_1-C_6)$alkyl $-O-(C_0-C_6)$alkyl, $-O-(C_3-C_7)$cycloalkylalkyl, -O(aryl), -O(heteroaryl), $-N(C_0-C_6$-alkyl$)_2$,$-N((C_0-C_6)$alkyl$)((C_3-C_7-)$cycloalkyl) or $-N((C_0-C_6)$alkyl)(aryl) substituents;

D, E, F, G, K and L in P independently represent $-C(R_3)=$, $-C(R3)=C(R_4)-$,$-C(=O)-$, $-C(=S)-$ -O-, -N=, $-N(R_3)-$ or -S-;

Q denotes an aryl or heteroaryl group of formula

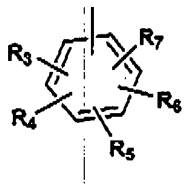 . 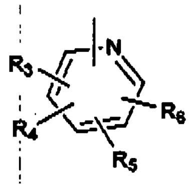 . 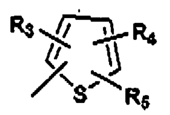 , 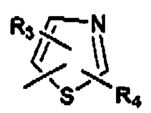 , 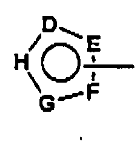

$R_3$, $R_4$, $R_5$, $R_6$, and $R_7$ independently are as defined above;

D, E, F, G and H in Q independently represent $-C(R_3)=$, $-C(R_3)=C(R_4)-$,$-C(=O)-$, $-C(=S)-$,-O-, -N=, $-N(R_3)-$ or -S-;

B represents a single bond, $-C(=O)-(C_0-C_2)$alkyl-, $-C(=O)-(C_2-C_6)$alkenyl-, $-C(=O)-(C_2-C_6)$alkynyl-, $-C(=O)-O-$, $-C(=O)NR_8-(C_0-C_2)$alkyl-, $-C(=NR_8)NR_9$, $-S(=O)-(C_0-C_2)$alkyl-, $-S(=O)_2-(C_0-C_2)$alkyl-, $-S(=O)_2NR_8-(C_0-C_2)$alkyl-, $C(=NR_8)-(C_0-C_2)$alkyl-, $-C(=NOR_8)-(C_0-C_2)$alkyl- or -C(=NORg)$NR_9$-(Co-Cz)alkyl-;

$R_8$ and $R_9$, independently are as defined above;

J represents $-C(R_{10}$ o, $R_{11})$, -O-, $-N(R_{10})-$ or -S-;

$R_{10}$, $R_{11}$ independently are hydrogen, $-(C_1-C_6)$alkyl, $-(C_3-C_6)$cycloalkyl, $-(C_3-C_7)$cycloalkylalkyl, $-(C_2-C_6)$alkenyl, $-(C_2-C_6)$alkynyl, halo$(C_1-C_6)$alkyl, heteroaryl, heteroarylalkyl, arylalkyl or aryl; any of which is optionally substituted with 1-5 independent halogen, -CN, $-(C_1-C_6)$alkyl, $-O(C_0-C_6)$alkyl, $-O(C_3-C_7)$cycloalkylalkyl, -O(aryl), -O (heteroaryl), $-N((C_0-C_6)$alkyl$)((C_0-C_6)$alkyl),$-N((C_0-C_6)$alkyl$)((C_3-C_7)$cycloalkyl) or $-N((C_0-C_6)$akyl)(aryl) substituents;

Any N may be an N-oxide;

or pharmaceutically acceptable salts, hydrates or solvates of such compounds.

**3.** A compound according to claim 1 or 2 having the formula I-D

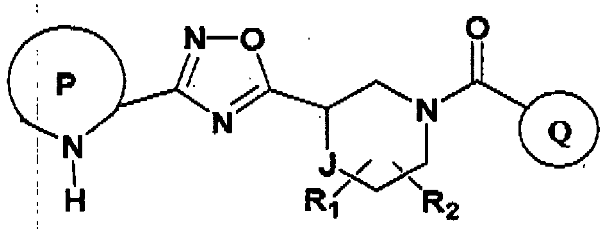

I-D

Wherein

$R_1$ and $R_2$ represent independently hydrogen, $-(C_1-C_6)$alkyl, $-(C_2-C_6)$alkenyl, - $(C_2-C_6)$alkynyl, arylalkyl, heteroarylalkyl, hydroxy, amino, aminoakyl, hydroxyalkyl, $-(C_1-C_6)$alkoxy or $R_1$ and $R_2$ together can form a $(C_3-C_7)$ cycloalkyl ring, a carbonyl bond C=O or a carbon double bond;

P represents a $(C_5-C_7)$heterocycloalkyl, $(C_5-C_7)$heterocycloalkenyl ring or a heteroaryl group of formula

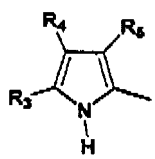 , 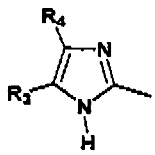 , 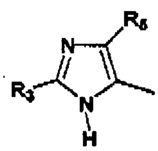 , 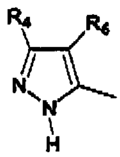 , 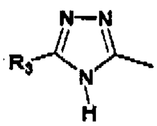 ,

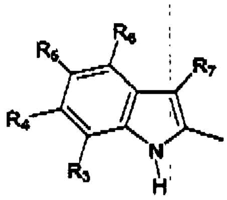 , 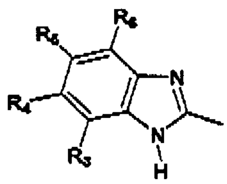 , 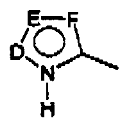 , 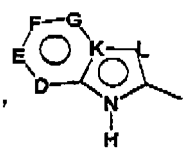 .

$R_3$, $R_4$, $R_5$, $R_6$, and $R_7$ independently are hydrogen, halogen, $-NO_2$,$(C_1-C_4)$alkyl, $-(C_3-C_6)$cycloalkyl, $-(C_3-C_7)$ cycloalkylalkyl, $-(C_2-C_6)$alkenyl, $-(C_2-C_6)$alkynyl, halo-$(C_1-C_6)$alkyl, heteroaryl, heteroarylalkyl, arylalkyl, aryl, $-OR_8$, $-NR_8R_9$, $-C(=NR_{10})NR_8R_9$, - $NR_8COR_9$, $NR_8CO_2R_9$, $NR_8SO_2R_9$, $-NR_{10}CO$ $NR_8R_9$, $-SR_8$, $-S(=O)R_8$, -S $(=O)_2R_8$, $-S(=O)_2NR_8R_9$, $-C(=O)R_8$, $-C(O)-O-R_8$, $-C(=O)NR_8R_9$,$C(=NR_8)R_9$, or $C(=NOR_8)R_9$ substituents; wherein optionally two substituents are combined to the intervening atoms to form a bicyclic heterocycloalkyl, aryl or heteroaryl ring; wherein each ring is optionally further substituted with 1-5 independent halogen, -CN, $-(C_1-C_6)$alkyl, $-O-(C_0-C_6)$alkyl, $-O-(C_3-C_7)$cycloalkylalkyl, -O(aryl), - O(heteroaryl), $-O-(-C_1-C_3)$alkylaryl, $-O-(C_1-C_3)$alkylheteroaryl, $-N((-C_0-C_6)$alkyl)$((C_0-C_3)$alkylaryl) or $-N((C_0-C_6)$alkyl)$((C_0-C_3-)$alkylheteroaryl) groups;

$R_8$, $R_9$, $R_{10}$ each independently is hydrogen, $(C_1-C_6)$alkyl, $(C_3-C_6)$cycloalkyl, $(C_3-C_7)$cycloalkylalkyl, $(C_2-C_6)$ alkenyl, $(C_2-C_6)$alkynyl, halo-$(C_1-C_6)$alkyl, heterocycloalkyl, heteroaryl, heteroarylalkyl, arylalkyl or aryl; any of which is optionally substituted with 1-5 independent halogen, -CN, $-(C_1-C_6)$alkyl, $-O-(C_0-C_6)$alkyl, $-O-(C_3-C_7)$ cycloalkylalkyl, -O(aryl), -O(heteroaryl,), $-N(C_0-C_6$-alkyl$)_2$,$-N((C_0-C_6)$alkyl)$((C_3-C_7-)$cycloalkyl) or $-N((C_0-C_6)$ alkyl)(aryl) substituents;

D, E, F, G, K and L in P independently represent $-C(R_3)=$, $-C(R_3)=C(R_4)-$,-C(=O)-, -C(=S)-, -O-, -N=, $-N(R_3)-$ or -S-;

Q denotes an aryl or heteroaryl group of formula

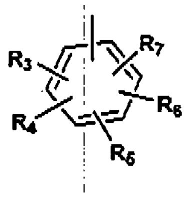, 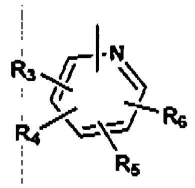, 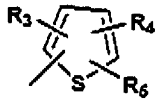, 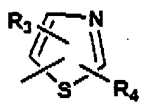, 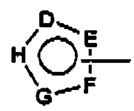

$R_3$, $R_4$, $R_5$, $R_6$, and $R_7$ independently are as defined above;
D, E, F, G and H in Q independently represent -C($R_3$)=, -C($R_3$)=C($R_4$)-,-C(=O)-, -C(=S)-, -O-, -N=, -N($R_3$)- or -S-;
J represents -C($R_{10}$, $R_{11}$), -O-, -N($R_{10}$)- or -S-;
$R_{10}$, $R_{11}$ independently are hydrogen, -($C_1$-$C_6$)alkyl, -($C_3$-$C_6$)cycloalkyl, -($C_3$-$C_7$)cycloalkylakyl, -($C_2$-$C_6$)alkenyl, -($C_2$-$C_6$)alkynyl, halo($C_1$-$C_6$)alkyl, heteroaryl, heteroarylalkyl, arylalkyl or aryl; any of which is optionally substituted with 1-5 independent halogen, -CN, -($C_1$-$C_6$)alkyl -O($C_0$-$C_6$)alkyl, -O($C_3$-$C_7$)cycloalkylalkyl, -O(aryl), O (heteroaryl), -N(($C_0$-$C_6$alkyl)(($C_0$-$C_6$)alkyl),-N(($C_0$-$C_6$)alkyl)(($C_3$-$C_7$)cycloakyl) or -N(($C_0$-$C_6$)alkyl)(aryl) substituents;
Any N may be an N-oxide;

or pharmaceutically acceptable salts, hydrates or solvates of such compounds.

**4.** A compound according to claim 1 having the formula II-A

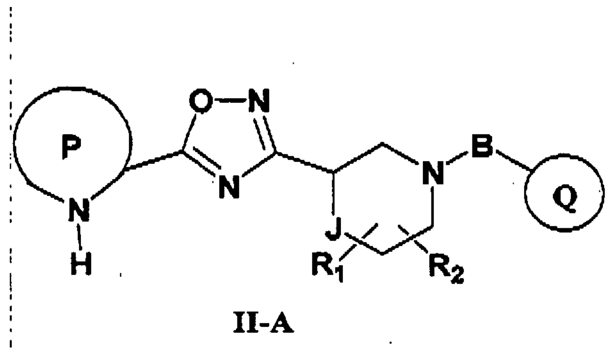

II-A

Wherein

$R_1$ and $R_2$ represent independently hydrogen, -($C_1$-$C_6$)alkyl, -($C_2$-$C_6$)alkenyl, - ($C_2$-$C_6$)alkynyl, arylalkyl, heteroarylalkyl, hydroxy, amino, aminoalkyl, hydroxyalkyl, -($C_1$-$C_6$)alkoxy or $R_1$ and $R_2$ together can form a ($C_3$-$C_7$) cycloalkyl ring, a carbonyl bond C=O or a carbon double bond;
P represents a ($C_5$-$C_7$)heterocycloalkyl, ($C_5$-$C_7$)heterocycloalkenyl ring or a heteroaryl group of formula

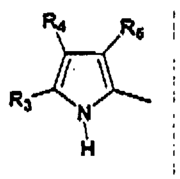, 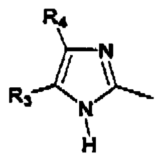, 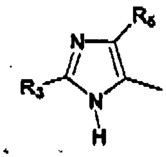, 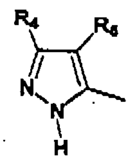, 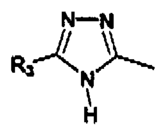,

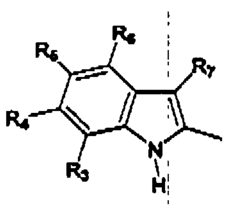, 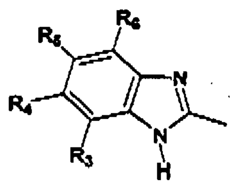, 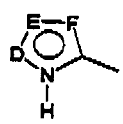, 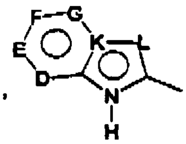.

$R_3$, $R_4$, $R_5$, $R_6$, and $R_7$ independently are hydrogen, halogen, $-NO_2$, - ($C_1$-$C_6$)alkyl, ($C_3$-$C_6$)cycloalkyl, -($C_3$-$C_7$)

cycloalkylalkyl, -($C_2$-$C_6$)alkenyl, -($C_2$-$C_6$)alkynyl, halo-($C_1$-$C_6$)alkyl, heteroaryl, heteroarylalkyl, arylalkyl, aryl, -$OR_8$, -$NR_8R_9$, -C(=$NR_{10}$)$NR_8R_9$, - $NR_8COR_9$, $NR_8CO_2R_9$, $NR_8SO_2R_9$, -$NR_{10}$CO $NR_8R_9$, -$SR_8$, -S(=O)$R_8$, -S (=O)$_2R_8$, .S(=O)$_2NR_8R_9$, -C(=O)$R_8$, -C(O)-O-$R_8$, -C(=O)$NR_8R_9$, - C(=$NR_8$)$R_9$, or C(=$NOR_8$)$R_9$ substituents; wherein optionally two substituents are combined to the intervening atoms to form a bicyclic heterocycloalkyl, aryl or heteroaryl ring; wherein each ring is optionally further substituted with 1-5 independent halogen, -CN, -($C_1$-$C_6$)alkyl, -O-($C_0$-$C_6$)alkyl, -O-$C_3$-$C_7$)cycloalkylalkyl, -O(aryl), - O(heteroaryl), -O-(-$C_1$-$C_3$)alkylaryl, -O-($C_1$-$C_3$)alkylheteroaryl, -N((-$C_0$-$C_6$)alkyl)(($C_0$-$C_6$)alkylaryl) or -N(($C_0$-$C_6$)alkyl)(($C_0$-$C_3$-)alkylheteroaryl) groups;

$R_8$, $R_9$, $R_{10}$ each independently is hydrogen, ($C_1$-$C_6$)alkyl, ($C_3$-$C_6$)cycloalkyl, ($C_3$-$C_7$)cycloalkylalkyl, ($C_2$-$C_6$) alkenyl, ($C_2$-$C_6$)alkynyl, halo-($C_1$-$C_6$)alkyl, heterocycloalkyl, heteroaryl, heteroarylalkyl, arylalkyl or aryl; any of which is optionally substituted with 1-5 independent halogen, -CN, -($C_1$-$C_6$)alkyl, -O-($C_0$-$C_6$)alkyl, -O-($C_3$-$C_7$) cycloalkylalkyl, -O(aryl), -O(heteroaryl), -N($C_0$-$C_6$-alkyl)$_2$,-N(($C_0$-$C_6$)alkyl)(($C_3$-$C_7$-)cycloalkyl) or -N(($C_O$-$C_6$) alkyl)(aryl) substituents;

D, E, F, G, K and L in P independently represent -C($R_3$)=, -C($R_3$)=C($R_4$)-,-C(=O)-, -C(=S)-, -O-, -N=, -N($R_3$)- or -S-;

Q denotes an aryl or heteroaryl group of formula

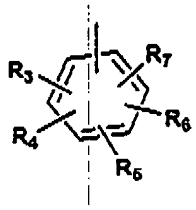 , 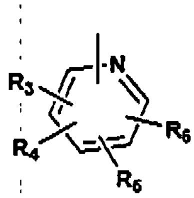 , 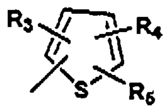 . 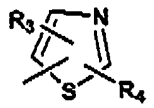 . 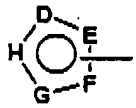

$R_3$, $R_4$, $R_5$, $R_6$, and $R_7$ independently are as defined above;

D, E, F, G and H in Q independently represent -C($R_3$)=, -C($R_3$)=C($R_4$)-,-C(=O)-, -C(=S)-, -O-, -N=, -N($R_3$)- or -S-;

B represents a single bond, -C(=O)-($C_0$-$C_2$)alkyl-, -C(=O)-($C_2$-$C_6$)alkenyl-, -C(=O)-($C_2$-$C_6$)alkynyl-, -C(=O)-O-, -C(=O)$NR_8$-($C_0$-$C_2$)alkyl-, -C(=$NR_8$)$NR_9$, -S(=O)-($C_0$-$C_2$)alkyl-, -S(=O)$_2$-($C_0$-$C_2$)alkyl-, -S(=O)$_2NR_8$-($C_0$-$C_2$) alkyl-, C(=$NR_8$)-($C_0$-$C_2$)alkyl-, -C(=$NOR_8$)-($C_0$-$C_2$)alkyl- or -C(-$NOR_8$)$NR_9$-($C_0$-$C_2$)alkyl-;

$R_8$ and independently are as defined above;

J represents -C($R_{10}$, $R_{11}$), -O-, -N($R_{10}$)- or -S-;

$R_{10}$, $R_{11}$ independently are hydrogen, -($C_1$-$C_6$)alkyl, -($C_3$-$C_6$)cycloalkyl, -($C_3$-$C_7$)cycloalkylalkyl, -($C_2$-$C_6$)alkenyl, -($C_2$-$C_6$)akynyl, halo($C_1$-$C_6$)alkyl, heteroaryl, heteroarylalkyl, arylalkyl or aryl; any of which is optionally substituted with 1-5 independent halogen, -CN, -($C_1$-$C_6$)alkyl, -O($C_0$-$C_6$)alkyl, -O($C_3$-$C_7$)cycloalkylalkyl, -O(aryl), - O (heteroaryl), -N(($C_0$-$C_6$)alkyl)(($C_0$-$C_6$)alkyl),-N(($C_0$-$C_6$)alkyl)(($C_3$-$C_7$)cycloalkyl) or -N(($C_0$-$C_6$)alkyl)(aryl) substituents;

Any N may be an N-oxide;

or pharmaceutically acceptable salts, hydrates or solvates of such compounds.

**5.** A compound according to claim 1 having the formula II-B

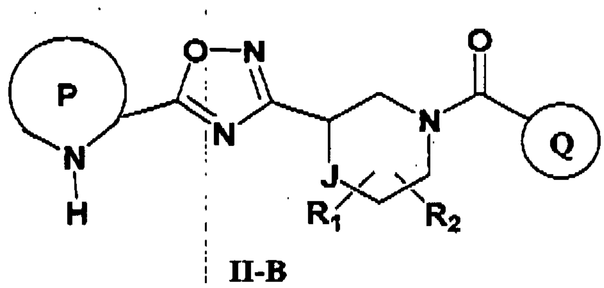

II-B

Wherein

$R_1$ and $R_2$ represent independently hydrogen, -($C_1$-$C_6$)alkyl, -($C_2$-$C_6$)alkenyl,($C_2$-$C_6$)alkynyl, arylalkyl, heter-

oarylalkyl, hydroxy, amino, aminoalkyl, hydroxyalkyl, -($C_1$-$C_6$)alkoxy or $R_1$ and $R_2$ together can form a ($C_3$-$C_7$) cycloalkyl ring, a carbonyl bond C=O or a carbon double bond;

P represents a ($C_5$-$C_7$)heterocycloalkyl, ($C_5$-$C_7$)heterocycloalkenyl ring or a heteroaryl group of formula

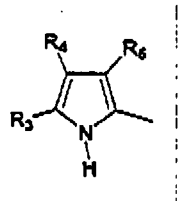 , 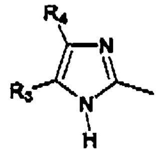 , 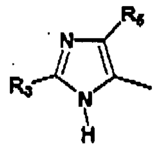 , 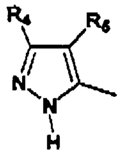 , 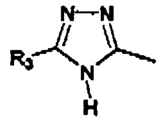 ,

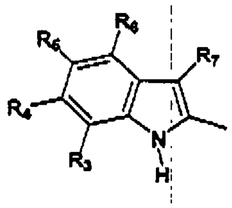 , 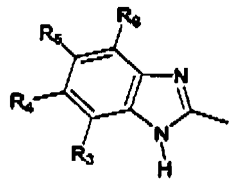 , 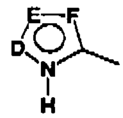 , 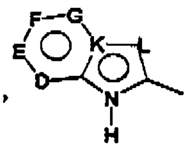

$R_3$, $R_4$, $R_5$, $R_6$, and $R_7$ independently are hydrogen, halogen, -$NO_2$,($C_1$-$C_6$)alkyl, -($C_3$-$C_6$)cycloalkyl, -($C_3$-$C_7$cycloalkylalkyl, -($C_2$-$C_6$)alkenyl, -($C_2$-$C_6$)alkynyl, halo-($C_1$-$C_6$)alkyl, heteroaryl, heteroarylalkyl, arylalkyl, aryl, -$OR_8$, -$NR_8R_9$, -C(=$NR_{10}$)$NR_8R_9$, - $NR_8COR_9$, $NR_8CO_2R_9$, $NR_8SO_2R_9$, -$NR_{10}$CO $NR_8R_9$, -$SR_8$, -S(=O) $R_8$, -S(=O)$_2R_8$, -S(-O)$_2NR_8R_9$, -C(=O)$R_8$, -C(O)-O-$R_8$, -C(=O)$NR_8R_9$, - C(-$NR_8$)$R_9$, or C(=$NOR_8$)$R_9$ substituents; wherein optionally two substituents are combined to the intervening atoms to form a bicyclic heterocycloalkyl, aryl or heteroaryl ring; wherein each ring is optionally further substituted with 1-5 independent halogen, -CN, -($C_1$-$C_6$)alkyl, -O-($C_0$-$C_6$)alkyl, -O-($C_3$-$C_7$)cycloalkylalkyl, -O(aryl), O(heteroaryl), -O-(-$C_1$-$C_3$)alkylaryl, -O-($C_1$-$C_3$)alkylheteroaryl, -N((-$C_0$-$C_6$)alkyl(($C_0$-$C_3$)alkylaryl) or -N(($C_0$-$C_6$)alkyl)(($C_0$-$C_3$-)alkylheteroaryl) groups;

$R_8$, $R_9$, $R_{10}$ each independently is hydrogen, ($C_1$-$C_6$)alkyl, ($C_3$-$C_6$)cycloalkyl, ($C_3$-$C_7$)cycloalkylalkyl, ($C_2$-$C_6$) alkenyl, ($C_2$-$C_6$)alkynyl, halo-($C_1$-$C_6$)alkyl, heterocycloalkyl, heteroaryl, heteroarylalkyl, arylalkyl or aryl; any of which is optionally substituted with 1-5 independent halogen, -CN, -($C_1$-$C_5$)alkyl, -O-($C_0$-$C_6$)alkyl, -O-($c_3$-$C_7$) cycloalkylalkyl, -O(aryl), -O(heteroaryl), -N($C_0$-$C_6$-alkyl)$_2$, -N(($C_0$-$C_6$)alkyl)(($C_3$-$C_7$-)cycloalkyl) or N(($C_0$-$C_6$) alkyl)(aryl) substituents;

D, E, F, G, K and L in P independently represent -C($R_3$)=, -C($R_3$)=C($R_4$)-, C(=O)-, -C(=S)-, -O-, -N=, -N(R3)- or -S-;

Q denotes an aryl or heteroaryl group of formula

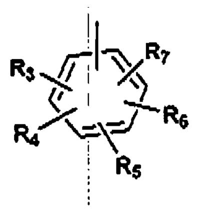 , 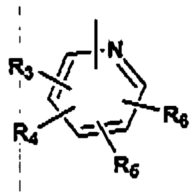 , 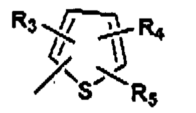 , 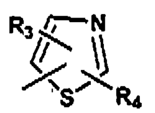 , 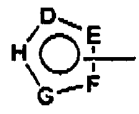

$R_3$, $R_4$, $R_5$, $R_6$, and $R_7$ independently are as defined above;

D, E, F, G and H in Q independently represent -C($R_3$)=, -C($R_3$)=C($R_4$)-,-C(=O)-, -C(=S)-, -O-, -N=, -N($R_3$)- or -S-;

J represents -C($R_{10}$, $R_{11}$), -O-, -N($R_{10}$)- or -S-;

$R_{10}$, $R_{11}$ independently are hydrogen, -($C_1$-$C_6$)alkyl, -($C_3$-$C_6$)cycloalkyl, -($C_3$-$C_7$)cycloalkylalkyl, -($C_2$-$C_6$)alkenyl, -($C_2$-$C_6$)alkynyl, halo($C_1$-$C_6$)alkyl, heteroaryl, heteroarylalkyl, arylalkyl or aryl,; any of which is optionally substituted with 1-5 independent halogen, -CN, -($C_1$-$C_6$)alkyl, -O($C_0$-$C_6$)alkyl, -O($C_3$-$C_7$)cycloalkylalkyl, -O(aryl), -O(heteroaryl), -N(($C_0$-$C_6$)alkyl)(($C_0$-$C_6$)alkyl),-N(($C_0$-$C_6$)alkyl)(($C_3$-$C_7$)cycloalkyl) or -N(($C_0$-$C_6$)alkyl)(aryl) substituents;

Any N may be an N-oxide;

or pharmaceutically acceptable salts, hydrates or solvates of such compounds.

**6.** A compound according to claim 1 having the formula II-B

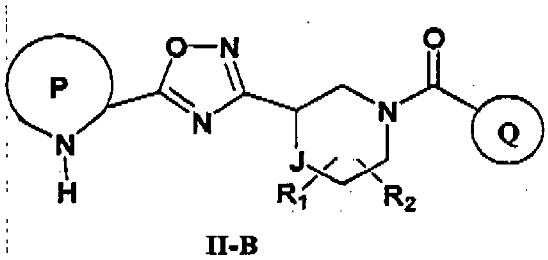

II-B

Wherein

$R_1$ and $R_2$ represent independently hydrogen, -($C_1$-$C_6$)alkyl, -($C_2$-$C_6$)alkenyl, - ($C_2$-$C_6$)alkynyl, arylalkyl, heteroarylalkyl, hydroxy, amino, aminoalkyl, hydroxyalkyl, -($C_1$-$C_6$)alkoxy or $R_1$ and $R_2$ together can form a ($C_3$-$C_7$) cycloalkyl ring, a carbonyl bond C=O or a carbon double bond;

P represents a ($C_5$-$C_7$)heterocycloalkyl, ($C_5$-$C_7$)heterocycloalkenyl ring or a heteroaryl group of formula

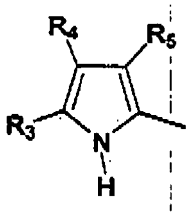 , 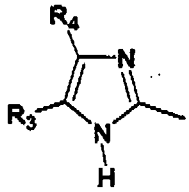 , 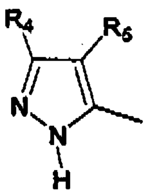 , 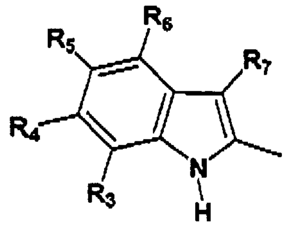

$R_3$, $R_4$, $R_5$, $R_6$, and $R_7$ independently are hydrogen, halogen, -$NO_2$, - ($C_1$-$C_6$)alkyl, -($C_3$-$C_6$)cycloalkyl, -($C_3$-$C_7$) cycloalkylalkyl, -($C_2$-$C_6$)alkenyl, -($C_2$-$C_6$)alkynyl, halo-($C_1$-$C_6$)alkyl, heteroaryl, heteroarylalkyl, arylalkyl, aryl, -$OR_8$, -$NR_8R_9$, -C(=$NR_{10}$)$NR_8R_9$, - $NR_8COR_9$, $NR_8CO_2R_9$, $NR_8SO_2R_9$, -$NR_{10}$CO $NR_8R_9$, -$SR_8$, -S(=O)$R_8$, -S (=O)$_2R_8$, -S(=O)$_2NR_8R_9$, -C(=O)$R_8$, -C(O)-O-$R_8$, -C(-O)$NR_8R_9$, - C(=$NR_8$)$R_9$, or C(=$NOR_8$)$R_9$ substituents; wherein optionally two substituents are combined to the intervening atoms to form a bicyclic heterocycloalkyl, aryl or heteroaryl ring; wherein each ring is optionally further substituted with 1-5 independent halogen, -CN, -($C_1$-$C_6$)alkyl. -O-($C_0$-$C_6$)alkyl, -O-($C_3$-$C_7$)cycloalkylalkyl, -O(aryl), - O(heteroaryl), -O-(-$C_1$-$C_3$)alkylaryl, -O-($C_1$-$C_3$)alkylheteroaryl, -N((-$C_0$-$C_6$)alkyl)(($C_0$-$C_3$)alkylaryl) or -N(($C_0$-$C_6$)alkyl)(($C_0$-$C_3$-)alkylheteroaryl) groups;

$R_8$, $R_9$, $R_{10}$ each independently is hydrogen, ($C_1$-$C_6$)alkyl, ($C_3$-$C_6$)cycloalkyl, ($C_3$-$C_7$)cycloalkylalkyl, ($C_2$-$C_6$) alkenyl, ($C_2$-$C_6$)alkynyl, halo-($C_1$-$C_6$)alkyl, heterocycloalkyl, heteroaryl, heteroarylalkyl, arylalkyl or aryl; any of which is optionally substituted with 1-5 independent halogen, -CN, -($C_1$-$C_6$)alkyl, -O-($C_0$-$C_6$)alkyl, -O-($C_3$-$C_7$) cycloalkylalkyl, -O(aryl), -O(heteroaryl), -N($C_0$-$C_6$-alkyl)$_2$,-N(($C_0$-$G_6$)alkyl)(($C_3$-$C_7$)cycloalkyl) or -N(($C_0$-$C_6$)alkyl) (aryl) substituents;

Q denotes an aryl or heteroaryl group of formula

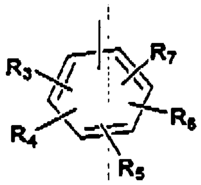 . 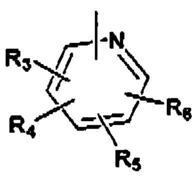 . 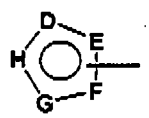

$R_3$, $R_4$, R5, $R_6$, and $R_7$ independently are as defined above;

D, E, F, G and H in Q independently represent -C($R_3$)=, -C($R_3$)=C($R_4$)-,-O- or -N=;

J represents -C($R_{10}$, $R_{11}$), -O-, -N($R_{10}$)- or -S-;

$R_{10}$, $R_{11}$ independently are hydrogen, -($C_1$-$C_6$)alkyl, -($C_3$-$C_6$)cycloalkyl, -($C_3$-$C_7$)cycloalkylalkyl, -($C_2$-$C_4$)alkenyl, -($C_2$-$C_6$)alkynyl, halo($C_1$-$C_6$)alkyl, heteroaryl, heteroarylalkyl, arylalkyl or aryl; any of which is optionally substituted with 1-5 independent halogen, -CN, -($C_1$-$C_6$)alkyl, -O($C_0$-$C_6$)alkyl, -O($C_3$-$C_7$)cycloalkylalkyl, -O(aryl), - O

(heteroaryl), $-N((C_0-C_6)alkyl)((C_0-C_6)alkyl)$,$-N((C_0-C_6)alkyl)((C_3-C_7)cycloalkyl)$ or $-N((C_0-C_6)alkyl)(aryl)$ substituents;
Any N may be an N-oxide;

or pharmaceutically acceptable salts, hydrates or solvates of such compounds.

**7.** A compound according to claim 1 having the formula II-C

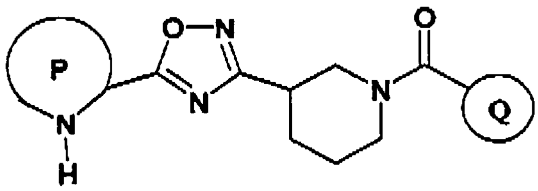

II-C

Wherein

P represents a $(C_5-C_7)$heterocycloalkyl, $(C_5-C_7)$heterocycloalkenyl ring or a heteroaryl, group of formula

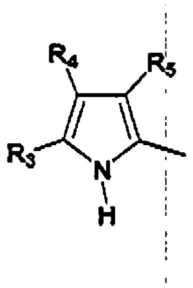 , 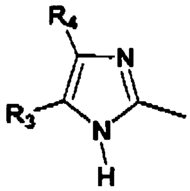 , 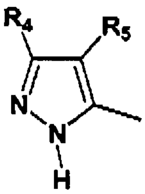 , 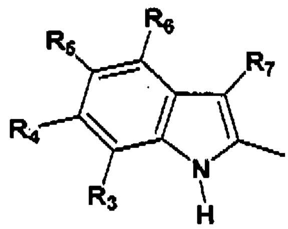

$R_3$, $R_4$, $R_5$, $R_6$, and $R_7$ independently are hydrogen, halogen, $-NO_2$, - $(C_1C_6)$alkyl, halo-$(C_1-C_6)$alkyl or -CN;
Q denotes an aryl or heteroaryl group of formula

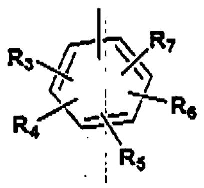 , 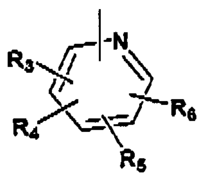 , 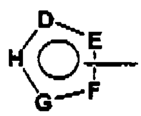

$R_3$, $R_4$, $R_5$, $R_6$, and $R_7$ independently are as defined above;
D, E, F, G and H in Q independently represent $-C(R_3)-$, $-C(R_3)=C(R_4)-$,-O- or -N=;
Any N may be an N-oxide;

or pharmaceutically acceptable salts, hydrates or solvates of such compounds.

**8.** A compound according to claim 1 having the formula I-D

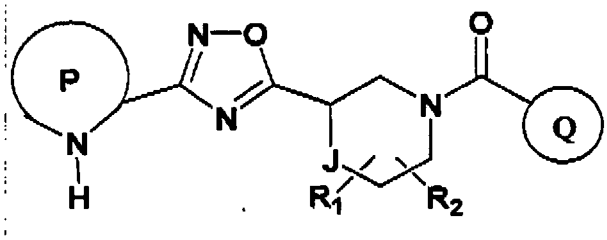

I-D

Wherein

$R_1$ and $R_2$ represent independently hydrogen, -($C_1$-$C_6$)alky), -($C_2$-$C_6$)alkenyl, - ($C_2$-$C_6$)alkynyl, arylalkyl, heteroarylalkyl, hydroxy, amino, aminoalkyl, hydroxyalkyl, -($C_1$-$C_6$)alkoxy or $R_1$ and $R_2$ together can form a ($C_3$-$C_7$) cycloalkyl ring, a carbonyl bond C=O or a carbon double bond;

P represents a ($C_5$-$C_7$)heterocycloalkyl, ($C_5$-$C_7$)heterocydoalkenyl ring or a heteroaryl group of formula

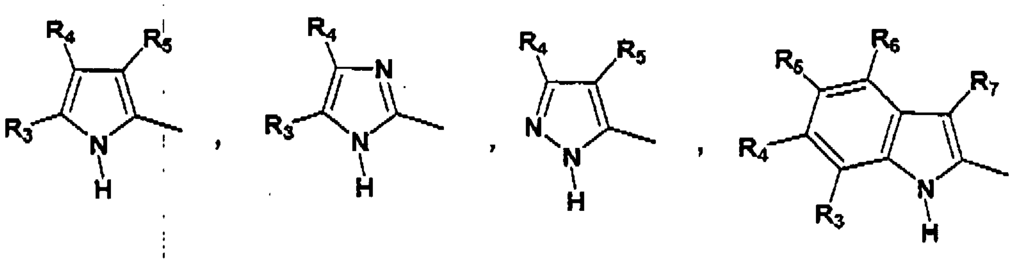

$R_3$, $R_4$, $R_5$, $R_6$, and $R_7$ independently are hydrogen, halogen, -$NO_2$, - ($C_1$-$C_6$)alkyl, -($C_3$-$C_6$)cycloalkyl, ($C_3$-$C_7$) cycloalkylalkyl, -($C_2$-$C_6$)alkenyl, -($C_2$-$C_6$)alkynyl, halo-($C_1$-$C_6$)alkyl, heteroaryl, heteroarylalkyl, arylalkyl, aryl, -$OR_8$, $NR_8R_9$, -C(=$NR_{10}$)$NR_8R_9$, - $NR_8COR_9$, $NR_8CO_2R_9$, $NR_8SO_2R_9$, -$NR_{10}$CO $NR_8R_9$, -$SR_8$, -S(=O)$R_{18}$;- S (=O)$_2R_8$, -S(=O)$_2NR_8R_9$, -C(=O)$R_8$, -C(O)-O-$R_8$, -C(=O)$NR_8R_9$, - C(=$NR_8$)$R_9$, or C(=$NOR_8$)$R_9$ substituents; wherein optionally two substituents are combined to the intervening atoms to form a bicyclic heterocycloalkyl, aryl or heteroaryl ring; wherein each ring is optionally further substituted with 1-5 independent halogen, -CN, -($C_1$-$C_6$)alkyl, -O-($C_0$-$C_6$)alkyl, -O($C_3$-$C_7$)cycloalkylalkyl, -O(aryl),O(heteroaryl), -O-(-$C_1$-$C_3$)alkylaryl, -O-($C_1$-$C_3$)alkylheteroaryl, -N((-$C_0$-$C_6$)alkyl)(($C_0$-$C_3$)alkylaryl) or -N(($C_0$-$C_6$)alkyl)(($C_0$-$C_3$-)alkylheteroaryl) groups;

$R_8$, $R_9$, $R_{10}$ each independently is hydrogen, ($C_1$-$C_6$)alkyl, ($C_3$-$C_6$)cycloalkyl, ($C_3$-$C_7$)cycloalkylalkyl, ($C_2$-$C_6$) alkenyl, ($C_2$-$C_6$)akynyl, halo-($C_1$-$C_6$)alkyl, heterocycloalkyl, heteroaryl, heteroarylalkyl, arylalkyl or aryl; any of which is optionally substituted with 1-5 independent halogen, -CN, -($C_1$-$C_6$)alkyl, -O-($C_0$-$C_6$)alkyl, -O-($C_3$-$C_7$) cycloalkylalkyl, -O(aryl), -O(heteroaryl), -N($C_0$-$C_6$-alkyl)$_2$,-N(($C_0$-$C_6$)alkyl)(($C_3$-$C_7$-)cycloalkyl) or N(($C_0$-$C_6$)alkyl) (aryl) substituents;

Q denotes' an aryl or heteroaryl group of formula

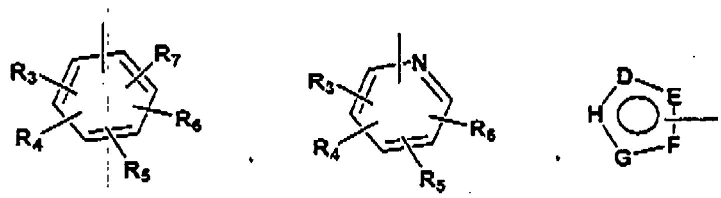

$R_3$, $R_4$, $R_5$, $R_6$, and $R_7$ independently are as defined above;

D, E, F, G and H in Q independently represent -C($R_3$)=, -C($R_3$)=C($R_4$)-,-O- or -N=;

J represents -C($R_{10}$, $R_{11}$), -O-, -N($R_{10}$)- or -S-;

$R_{10}$, $R_{11}$ independently are hydrogen, -($C_1$-$C_6$)alkyl, -($C_3$-$C_6$)cycloalkyl, -($C_3$-$C_7$)cycloalkylalkyl, -($C_2$-$C_6$)alkenyl, -($C_2$-$C_6$)alkynyl, halo($C_1$-$C_6$)alkyl, heteroaryl, heteroarylalkyl, arylalkyl or aryl; any of which is optionally substituted with 1-5 independent halogen, -CN, -($C_1$-$C_6$)alkyln -O($C_0$-$C_6$)alkyl, -O($C_3$-$C_7$)cycloakylalkyl, -O(aryl), - O

(heteroaryl), N(($C_0$-$C_6$)alkyl)(($C_0$-$C_6$)alkyl),-N(($C_0$-$C_6$)alkyl)(($C_3$-$C_7$)cycloalkyl) or -N(($C_0$-$C_6$)alkyl)(aryl) substituents;
Any N may be an N-oxide;

or pharmaceutically acceptable salts, hydrates or solvates of such compounds.

**9.** A compound according to claim 1 having the formula I-E

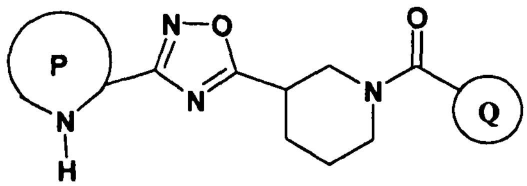

I-E

Wherein

P represents a ($C_5$-$C_7$)heterocycloalkyl, ($C_5$-$C_7$)heterocycloalkenyl ring or a heteroaryl group of formula

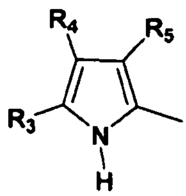 , 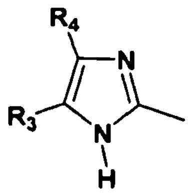 , 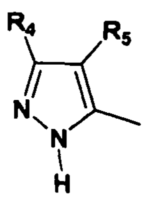 , 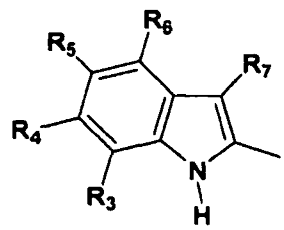

$R_3$, $R_4$, $R_5$, $R_6$, and $R_7$ independently are hydrogen, halogen, -$NO_2$, - ($C_1$-$C_6$)alkyl, halo-($C_1$-$C_6$)alkyl or -CN;
Q denotes an aryl or heteroaryl group of formula

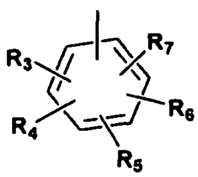 , 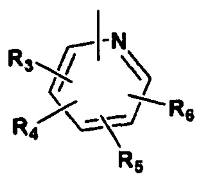 , 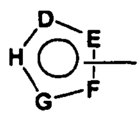

$R_3$, $R_4$, $R_5$, $R_6$, and $R_7$ independently are as defined above;
D, E, F, G and H in Q independently represent -C($R_3$)=, -C($R_3$)=C($R_4$)-,-O- or -N=;
Any N may be an N-oxide;

or pharmaceutically acceptable salts, hydrates or solvates of such compounds.

**10.** A compound according to claims 1 to 9, which can exist as optical isomers, wherein said compound is either the racemic mixture or an individual optical isomer.

**11.** A compound according to claims 1 to 10, wherein said compound is selected from:

(4-Fluoro-phenyl)-{(S)-3-[3-(1H-pyrrol-2-yl)-[1,2,4]oxadiazol-5-yl]-piperidin-1-yl}-methanone

(2,4-Difluoro-phenyl)-{(S)-3-[3-(1H-pyrrol-2-yl)-[1,2,4]oxadiazol-5-yl]-piperidin-1-yl}-methanone
(3,4-Difluoro-phenyl)-{(S)-3-[3-(1H-pyrrol-2-yl)-[1,2,4]oxadiazol-5-yl]-piperidin-1-yl}-methanone
(6-Fluoro-pyridin-3-yl)-{(S)-3-[3-(1H-pyrrol-2-yl)-[1,2,4]oxadiazol-5-yl]-piperidin-1-yl}-methanone
(3,4-Difluoro-phenyl)- {3-[5-(1H-pyrrol-2-yl)-[1,2,4]oxadiazol-3-yl]-piperidin-1-yl}-methanone
(2,4-Difluoro-phenyl)-{3-[5-(1H-pyrrol-2-yl)-[1,2,4]oxadiazol-3-yl]-piperidin-1-yl}-methanone
(4-Fluoro-phenyl)-{3-[5-(1H-pyrrol-2-yl)-[1,2,4]oxadiazol-3-yl]-piperidin-1-yl}-methanone
(6-Fluoro-pyridin-3-yl)-(3-[5-(1H-pyrrol-2-yl)-[1,2,4]oxadiazol-3-yl]-piperidin-1-yl)-methanone
(4-Fluoro-2-methyl-phenyl)-{3-[5-(1H-pyrrol-2-yl)-[1,2,4]oxadiazol-3-yl]-piperidin-1-yl}-methanone
(3,4-Difluoro-phenyl)-{(S)-3-[5-(1H-pyrrol-2-yl)-[1,2,4]oxadiazol-3-yl]-piperidin-1-yl}-methanone
(4-Fluoro-phenyl)-{3-[5-(1H-indol-2-yl)-[1,2,4]oxadiazol-3-yl]-piperidin-1-yl}-methanone
(2,4-Difluoro-phenyl)-{(S)-3-[3-(1H-indol-2-yl)-[1,2,4]oxadiazol-5-yl]-piperidin-1-yl}-methanone
(4-Fluoro-phenyl)-(3-[5-(2H-pyrazol-3 -yl)-[1,2,4]oxadiazol-3-yl]-piperidin-1-yl}-methanone
(3,4-Difluoro-phenyl)-{3-[5-(2H-pyrazol-3-yl)-[1,2,4]oxadiazol-3-yl]-piperidin-1-yl}-methanone
(4-Fluoro-phenyl)-{3-[5-(1H-imidazol-2-yl)-[1,2,4]oxadiazol-3-yl]-piperidin-1-yl}-methanone
(3,4-Difluoro-phenyl)-{(S)-3-[3-(1H-indol-2-yl)-[1,2,4]oxadiazol-5-yl]-piperidin-1-yl}-methanone
(4-Fluoro-phenyl)-{(S)-3-[3-(1H-indol-2-yl)-[1,2,4]oxadiazol-5-yl]-piperidin-1-yl}-methanone (3,4-diFluoro-phenyl)-{3-[5-(1H-imidazol-2-yl)-[1,2,4]oxadiazol-3-yl]-piperidin-1-yl}-methanone
{(S)-3-[3-(1H-Indol-2-yl)-[1,2,4]oxadiazol-5-yl]-piperidin-1-yl}-(5-methyl-isoxazol-4-yl)-methanone
(5-Methyl-isoxazol-4-yl)-{(S)-3-[3-(1H-pyrrol-2-yl)-[1,2,4]oxadiazol-5-yl]-piperidin-1-yl}-methanone
(6-Fluoro-pyridin-3-yl)-{(S)-3-[5-(1H-pyrrol-2-yl)-[1,2,4]oxadiazol-3-yl]-piperidin-1-yl}-methanone
(4-Fluoro-phenyl)-{(S)-3-[5-(1H-pyrrol-2-yl)-[1,2,4]oxadiazol-3-yl]-piperidin-1-yl}-methanone
(6-Fluoro-pyridin-3-yl)-{3-[5-(1H-indol-2-yl)-[1,2,4]oxadiazol-3-yl]-piperidin-1-yl}-methanone
(4-Fluoro-phenyl)-{(S)-3-[3-(1H-imidazol-2-yl)-[1,2,4]oxadiazol-5-yl]-piperidin-1-yl}-methanone
(3,4-Difluoro-phenyl)-{(S)-3-[3-(1H-imidazol-2-yl)-[1,2,4]oxadiazol-5-yl]-piperidin-1-yl}-methanone
{3-[5-(1H-Indol-2-yl)-[1,2,4]oxadiazol-3-yl]-piperidin-1-yl-(5-methyl-isoxazol-4-yl)-methanone
(4-Fluoro-phenyl)-{(S)-3-[5-(4-methyl-1H-pyrrol-2-yl)-[1,2,4]oxadiazol-3-yl]-piperidin-1-yl-methanone
(6-Fluoro-pyridin-3-yl)-{{(S)-3-[5-(4-methyl-1H-pyrrol-2-yl)-[1,2,4]oxadiazol-3-yl]-piperidin-1-yl}-methanone
(5-Methyl-isoxazol-4-yl)-{(S)-3-[5-(4-methyl-1H-pyrrol-2-yl)-[1,2,4]oxadiazol-3-yl]-piperidin-1-yl-methanone
(2-Fluoro-pyridin-4-yl)-{(S)-3-[5-(4-methyl-1H-pyrrol-2-yl)-[1,2,4]oxadiazol-3-yl]-piperidin-1-yl}-methanone
(4-Fluoro-phenyl)-{(S)-3-[3-(4-methyl-1H-pyrrol-2-yl)-[1,2,4]oxadiazol-5-yl]-piperidin-1-yl}-methanone
(3,4-Difluoro-phenyl)-{(S)-3-[3-(4-methyl-1H-pyrrol-2-yl)-[1,2,4]oxadiazol-5-yl]-piperidin-1-yl}-methanone
(6-Fluoro-pyridin-3-yl)-{(S)-3-[3-(4-methyl-1H-pyrrol-2-yl)-[1,2,4]oxadiazol-5-yl]-piperidin-1-yl}-methanone
(2-Fluoro-pyridin-4-yl)-{(S)-3-[3-(4-methyl-1H-pyrrol-2-yl)-[1,2,4]oxadiazol-5-yl]-piperidin-1-yl}-methanone
(5-Methyl-isoxazol-4-yl)-{(S)-3-[3-(4-methyl-1H-pyrrol-2-yl)-[1,2,4]oxadiazol-5-yl]-piperidin-1-yl}-methanone
(4-Fluoro-phenyl)-{(S)-3-[5-(4-nitro-1H-pyrrol-2-yl)-[1,2,4]oxadiazol-3-yl]-piperidin-1-yl}-methanone
(4-Fluoro-phenyl)-{(R)-3-[3-(1H-pyrrol-2-yl)-[1,2,4]oxadiazol-5-yl]-piperidin-1-yl}-methanone
(4-Fluoro-phenyl)-{(S)-3-[5-(5-methyl-1H-pyrrol-2-yl)-[1,2,4]oxadiazol-3-yl]-piperidin-1-yl}-methanone
{(S)-3-[5-(4-Chloro-1H-pyrrol-2-yl)-[1,2,4]oxadiazol-3-yl]-piperidin-1-yl}-(4-fluorophenyl)-methanone
{(S)-3-[5-(4-Chloro-1H-pyrrol-2-yl)-[1,2,4]oxadiazol-3-yl]-piperidin-1-yl}-(6-fluoro-pyridin-3-yl)-methanone
{(S)-3-[5-(4-Chloro-1H-pyrrol-2-yl)-[1,2,4]oxadiazol-3-yl]-piperidin-1-yl}-(2-fluoro-pyridin-4-yl)-methanone
{(S)-3-[5-(4-Chloro-1H-pyrrol-2-yl)-[1,2,4]oxadiazol-3-yl]-piperidin-1-yl}-(5-methyl-isoxazol-4-yl)-methanone
{(S)-3-[3-(4-Chloro-1H-pyrrol-2-yl)-[1,2,4]oxadiazol-5-yl]-piperidin-1-yl}-(4-fluorophenyl)-methanone
{(S)-3-[5-(4-Bromo-1H-pyrrol-2-yl)-[1,2,4]oxadiazol-3-yl]-piperidin-1-yl}-(6-fluoro-pyridin-3-yl)-methanone
{(S)-3-[3-(4-Bromo-1H-pyrrol-2-yl)-[1,2,4]oxadiazol-5-yl]-piperidin-1-yl}-(4-fluorophenyl)-methanone
{(S)-3-[3-(4-Bromo-1H-pyrrol-2-yl)-[1,2,4]oxadiazol-5-yl]-piperidin-1-yl}-(6-fluoro-pyridin-3-yl)-methanone
{3,3-Dimethyl-5-[3-(1H-pyrrol-2-yl)-[1,2,4]oxadiazol-5-yl]-piperidin-1-yl}-(4-fluorophenyl)-methanone
(4-Fluoro-phenyl)-{(S)-3-[3-(4-fluoro-1H-pyrrol-2-yl)-[1,2,4]oxadiazol-5-yl]-piperidin-1-yl}-methanone
(3,4-Difluoro-phenyl)-{(S)-3-[3-(4-fluoro-1H-pyrrol-2-yl)-[1,2,4]oxadiazol-5-yl]-piperidin-1-yl -methanone
(6-Fluoro-pyridin-3-yl)-{(S)-3-[3-(4-fluoro-1H-pyrrol-2-yl)-[1,2,4]oxadiazol-5-yl]-piperidin-1-yl}-methanone
(2-Fluoro-pyridin-4-yl)-{(S)-3-[3-(4-fluoro-1H-pyrrol-2-yl)-[1,2,4]oxadiazol-5-yl]-piperidin-1-yl}-methanone
(4-Fluoro-phenyl)-{(S)-3-[5-(1H-pyrrol-2-yl)-tetrazol-2-yl]-piperidin-1-yl}-methanone
(4-Fluoro-phenyl)-{(S)-3-[5-(4-trifluoromethyl-1H-imidazol-2-yl)-[1,2,4]oxadiazol-3-yl]-piperidin-1-yl}-methanone
(6-Fluoro-pyridin-3-yl)-{(S)-3-[5-(4-isopropyl-1H-pyrrol-2-yl)-[1,2,4]oxadiazol-3-yl]-piperidin-1-yl}-methanone
(3-Fluoro-pyridin-4-yl)-{(S)-3-[5-(4-methyl-1H-pyrrol-2-yl)-[1,2,4]oxadiazol-3-yl]-piperidin-1-yl}-methanone
{(S)-3-[5-(4-Chloro-1H-pyrrol-2-yl)-[1,2,4]oxadiazol-3-yl]-piperidin-1-yl}-(3-fluoro-pyridin-4-yl)-methanone
(2-Fluoro-pyridin-4-yl)-{(S)-3-[5-(4-fluoro-1H-pyrrol-2-yl)-[1,2,4]oxadiazol-3-yl]-piperidin-1-yl}-methanone
{(S)-3-[5-(4-Bromo-1H-pyrrol-2-yl)-[1,2,4]oxadiazol-3-yl]-piperidin-1-yl}-(3-fluoro-pyridin-4-yl)-methanone

(3-Fluoro-pyridin-4-yl)-{(S)-3-[5-(4-fluoro-1H-pyrrol-2-yl)-[1,2,4]oxadiazol-3-yl]-piperidin-1-yl}-methanone
(4-Fluoro-phenyl)-{(S)-3-[5-(4-fluoro-1H-pyrrol-2-yl)-[1,2,4]oxadiazol-3-yl]-piperidin-1-yl}-methanone
(6-Fluoro-pyridin-3-yl)-{(S)-3-[5-(4-fluoro-1H-pyrrol-2-yl)-[1,2,4]oxadiazol-3-yl]-piperidin-1-yl}-methanone
{(S)-3-[3-(4-Chloro-1H-pyrrol-2-yl)-[1,2,4]oxadiazol-5-yl]-piperidin-1-yl}-(6-fluoro-pyridin-3-yl)-methanone
{(S)-3-[3-(4-Chloro-1H-pyrrol-2-yl)-[1,2,4]oxadiazol-5-yl]-piperidin-1-yl}-(2-fluoro-pyridin-4-yl)-methanone
{(S)-3-[3-(4-Chloro-1H-pyrrol-2-yl)-[1,2,4]oxadiazol-5-yl]-piperidin-1-yl}-(3-fluoro-pyridin-4-yl)-methanone
{(S)-3-[3-(4-Chloro-1H-pyrrol-2-yl)-[1,2,4]oxadiazol-5-yl]-piperidin-1-yl}-(5-methyl-isoxazol-4-yl)-methanone
{(S)-3-[3-(4-Bromo-1H-pyrrol-2-yl)-[1,2,4]oxadiazol-5-yl]-piperidin-1-yl}-(3-fluoro-pyridin-4-yl)-methanone
(3-Fluoro-pyridin-4-yl)-{(S)-3-[3-(4-fluoro-1H-pyrrol-2-yl)-[1,2,4]oxadiazol-5-yl]-piperidin-1-yl}-methanone
(3-Fluoro-pyridin-4-yl)-{(S)-3-[3-(4-methyl-1H-pyrrol-2-yl)-[1,2,4]oxadiazol-5-yl]-piperidin-1-yl}-methanone
(4-Fluoro-phenyl)-{(S)-3-[5-(4-trifluoromethyl-1H-pyrrol-2-yl)-[1,2,4]oxadiazol-3-yl]-piperidin-1-yl-methanone
(3-Fluoro-pyridin-4-yl)-{(S)-3-[5-(4-trifluoromethyl-1H-pyrrol-2-yl)[1,2,4]oxadiazol-3-yl]-piperidin-1-yl}-methanone
(6-Fluoro-pyridin-3-yl)-{(S)-3-[5-(4-trifluoromethyl-1H-pyrrol-2-yl)-[1,2,4]oxadiazol-3-yl]-piperidin-1-yl}-methanone
(3,4-Difluoro-phenyl)-{(S)-3-[3-(4-methyl-1H-imidazol-2-yl)-[1,2,4]oxadiazol-5-yl]-piperidin-1-yl}-methanone
{(S)-3-[5-(4-Chloro-1H-pyrrol-2-yl)-[1,2,4]oxadiazol-3-yl]-piperidin-1-yl}-pyridin-4-yl-methanone
(6-Fluoro-pyridin-3-yl)-{(S)-3-[3-(4-trifluoromethyl-1H-pyrrol-2-yl)-[1,2,4]oxadiazol-5-yl]-piperidin-1-yl}-methanone;

or pharmaceutically acceptable salts, hydrates or solvates of such compounds.

**12.** A pharmaceutical composition comprising a therapeutically effective amount of a compound according to claims 1 to 11 and a pharmaceutically acceptable carrier and/or excipient.

**13.** A compound/composition according to claims 1 to 12 for treating or preventing a condition in a mammal, including a human, the treatment or prevention of which is affected or facilitated by the neuromodulatory effect of mGluR5 allosteric modulators.

**14.** A compound/composition according to claims 1 to 12 for treating or preventing a condition in a mammal, including a human, the treatment or prevention of which is affected or facilitated by the neuromodulatory effect of mGluRS positive allosteric modulators (enhancer).

**15.** A compound/composition according to claims 1 to 12 for treating or preventing central nervous system disorders selected from the group consisting of anxiety disorders: Agoraphobia, Generalized Anxiety Disorder (GAD), Obsessive-Compulsive Disorder (OCD), Panic Disorder, Posttraumatic Stress Disorder (PTSD), Social Phobia, Other Phobias, Substance-Induced Anxiety Disorder.

**16.** A compound/composition according to claims 1 to 12 for treating or preventing central nervous system disorders selected from the group consisting of childhood disorders: Attention-Deficit/Hyperactivity Disorder).

**17.** A compound/composition according to claims 1 to 12 for treating or preventing central nervous system disorders selected from the group consisting of eating Disorders (Anorexia Nervosa, Bulimia Nervosa).

**18.** A compound/composition according to claims 1 to 12 for treating or preventing central nervous system disorders selected from the group consisting of mood disorders: Bipolar Disorders (I & II), Cyclothymic Disorder, Depression, Dysthymic Disorder, Major Depressive Disorder, Substance-Induced Mood Disorder.

**19.** A compound/composition according to claims 1 to 12 for treating or preventing central nervous system disorders selected from the group consisting of psychotic disorders: Schizophrenia, Delusional Disorder, Schizoaffective Disorder, Schizophreniform Disorder, Substance-Induced Psychotic Disorder.

**20.** A compound/composition according to claims 1 to 12 for treating or preventing central nervous system disorders selected from the group consisting of cognitive disorders: Delirium, Substance-Induced Persisting Delirium, Dementia, Dementia Due to HIV Disease, Dementia Due to Huntington's Disease, Dementia Due to Parkinson's Disease, Dementia of the Alzheimer's Type, Substance-Induced Persisting Dementia, Mild Cognitive Impairment.

**21.** A compound/composition according to claims 1 to 12 for treating or preventing central nervous system disorders

selected from the group consisting of personality disorders: Obsessive-Compulsive Personality Disorder, Schizoid, Schizotypal disorder.

**22.** A compound/composition according to claims 1 to 12 for treating or preventing central nervous system disorders selected from the group consisting of substance-related disorders: Alcohol abuse, Alcohol dependence, Alcohol withdrawal, Alcohol withdrawal delirium, Alcohol-induced psychotic disorder, Amphetamine dependence, Amphetamine withdrawal, Cocaine dependence, Cocaine withdrawal, Nicotine dependence, Nicotine withdrawal, Opioid dependence, Opioid withdrawal.

**23.** A compound/composition according to claims 1 to 12 for treating or preventing inflammatory central nervous system disorders selected from multiple sclerosis form such as benign multiple sclerosis, relapsing-remitting multiple sclerosis, secondary progressive multiple sclerosis, primary progressive multiple sclerosis.

**24.** Use of a compound/composition according to claims 1 to 12 in the manufacture of a medicament for a treatment or prevention as defined in any of claims 15 to 23.

**25.** The use of the compounds according to claim 1 to prepare tracers for imaging metabotropic glutamate receptors.

## Patentansprüche

**1.** Verbindung mit der Formel I-B

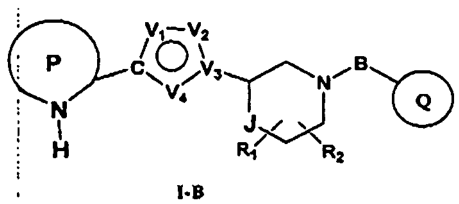

I-B

wobei

$R_1$ und $R_2$ unabhängig Wasserstoff, $(C_1-C_6)$Alkyl, $-(C_2-C_6)$Alkenyl, $-(C_2-C_6)$Alkynyl, Arylalkyl, Heteroarylalkyl, Hydroxy, Amino, Aminoalkyl, Hydroxyalkyl, $-(C_1-C_6)$Alkoxy repräsentieren oder $R_1$ und $R_2$ zusammen einen $(C_3-C_7)$ Cycloalkylring, eine Carbonylbindung C=O oder eine Kohlenstoffdoppelbindung bilden können;

P einen $(C_5-C_7)$Heterocycloalkyl-, $(C_5-C_7)$Heterocycloalkenylring oder eine Heteroarylgruppe der folgenden Formel repräsentiert

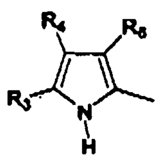 , 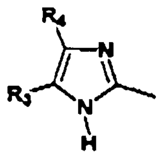 , 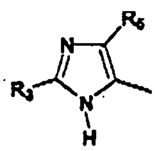 , 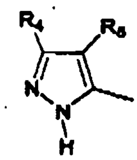 , 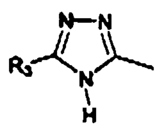

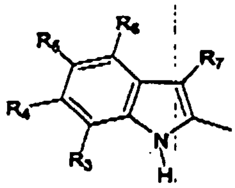 , 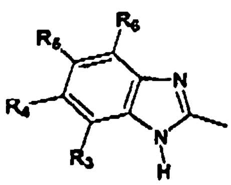 , 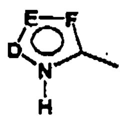 , 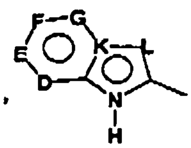 .

$R_3$, $R_4$, $R_5$, $R_6$ und $R_7$ unabhängig Wasserstoff, Halogen, - $NO_2$, - $(C_1-C_6)$Alkyl, - $(C_3-C_6)$Cycloalkyl, $(C_3-C_7)$Cycloalkylalkyl, $(C_2-C_6)$Alkenyl, - $-(C_2-C_6)$Alkynyl, Halo-$(C_1-C_6)$alkyl, Heteroaryl, Heteroarylalkyl, Arylalkyl, Aryl, $-OR_8$, $-NR_8R_9$, $-C(=NR_{10})NR_8R_9$, $-NR_8COR_9$, $NR_8CO_2R_9$, $NR_8SO_2R_9$, - $NR_{10}CO$ $NR_8R_9$, $-SR_8$, $-S(=O)R_8$, $-S(=O)_2Ra$, -S

$(=O)_2NR_8R_9$, - $C(=O)R_8$, $-C(O)-O-R_8$, $-C(=O)NR_8R_9$, $-C(=NR_8)R_9$ oder $C(=NOR_8)R_9$ Substituenten sind; wobei optional zwei Substituenten mit den dazwischen liegenden Atomen kombiniert sind, um einen bicyclischen Heterocycloalkyl-, Aryl- oder Heteroarylring zu bilden; wobei jeder Ring optional weiter substituiert ist durch 1-5 unabhängige Halogen, -CN, $-(C_1-C_6)$Alkyl, $-O-(C_0-C_6)$Alkyl-, $-O-(C_3-C$-7)Cycloalkylalkyl, -O(Aryl), - O(Heteroaryl), $-O-(-C_1-C_3)$ Alkylaryl, -O- $(C_1-C_3)$Alkylheteroaryl $-N((-C_0-C_6)$Alkyl)$((C_0-C_3)$alkylaryl)oder $-N((C_0-C_6)$Alkyl)$((C_0-C_3$-)alkylheteroaryl) Gruppen;

$R_8$, $R_9$, $R_{10}$ jeweils unabhängig Wasserstoff, $(C_1-C_6)$ Alkyl, $(C_3-C_6)$Cycloalkyl, $(C_3-C_7)$Cycloalkylalkyl, $(C_2-C_6)$Alkenyl, $(C_2-C_6)$Alkynyl, Halo-$(C_1-C_6)$alkyl, Heterocycloalkyl, Heteroaryl, Heteroarylalkyl, Arylalkyl oder Aryl sind; wobei beliebige davon optional substituiert sind durch 1-5 unabhängige Halogen, -CN, $-(C_1-C_6)$Alkyl, $-O-(C_0-C_6)$Alkyl, $-O-(C_3-C_7)$Cycloalkylalkyl, -O(Aryl), - O(Heteroaryl), $-N(C_0-C_6$-Alkyl)2, $-N((C_0-C_6)$Alkyl) $((C_3-C_7$-)cycloalkyl) oder $-N((C_0-C_6)$Alkyl)(aryl) Substituenten;

D, E, F, G, K und L in P unabhängig $-C(R_3)=$, - $C(R_3)=C(R_4)-$, -C(=O)-, -C(=S)-, -O-, -N=, $-N(R_3)-$ oder -S-repräsentieren;

Q eine $(C_5-C_6)$Cycloalkyl-, eine Aryl- oder Heteroarylgruppe der folgenden Formel kennzeichnet

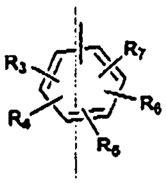 , 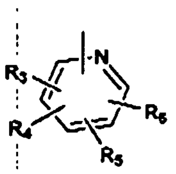 , 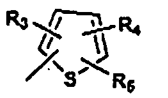 , 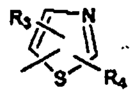 , 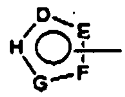

$R_3$, $R_4$, $R_5$, $R_6$ und $R_7$ unabhängig der obigen Definition entsprechen;

D, E, F, G und H in Q unabhängig $-C(R_3)=$, $-C(R_3)=C(R_4)-$, -C(=O)-, -C(=S)-, -O-, -N=, $-N(R_3)-$ oder -S-repräsentieren;

$V_1$, $V_2$ und $V_4$ unabhängig -O- oder -N= repräsentieren und $V_3$ C ist oder $V_1$, $V_2$ und $V_4$ -N= repräsentieren und $V_3$ -N-ist;

B Folgendes repräsentiert: eine Einfachbindung, - $C(=O)-(C_0-C_2$Alkyl-, $-C(=)-(C_2-C_6)$Alkenyl-, $-C(=O)-(C_2-C_6)$Alkynyl-, -C(=O)-O-, $-C(=O)NR_8-(C_0-C_2)$Alkyl-, $-C(=NR_8)NR_9$, $-S(=O)-(C_0-C_2)$Alkyl-, $-S(=O)_2-(C_0-C_2)$Alkyl-, -S $(=O)_2NR_8-(C_0-C_2)$Alkyl-, $C(=NR_8)-(C_0-C_2)$Alkyl-, $-C(=NOR_8)-(C_0-C_2)$Alkyl- oder $-C(=NOR_8)NR_9$ - $(C_0-C_2)$Alkyl-;

$R_8$ und $R_9$ unabhängig der obigen Definition entsprechen;

J $-C(R_{10}, R_{11})$, -O-, $-N(R_{10})$ - oder -S- repräsentiert;

$R_{10}$, $R_{11}$ unabhängig Folgendes sind: Wasserstoff, - $(C_1-C_6)$Alkyl, $-(C_3-C_6)$Cycloalkyl, $-(C_3-C_7)$Cycloalkylalkyl, $-(C_2-C_6)$ Alkenyl, - $(C_2-C_6)$Alkynyl, Halo$(C_1-C_6)$alkyl, Heteroaryl, Heteroarylalkyl, Arylalkyl oder Aryl; wobei beliebige davon optional substituiert sind durch 1-5 unabhängige Halogen, - CN, - $(C_1-C_6)$Alkyl, $-O(C_0-C_6)$Alkyl, $-O(C_3-C_7)$ Cycloalkylalkyl, -O(Aryl), -O(Heteroaryl), $-N((C_0-C_6)$Alkyl)$((C_0-C_6)$alkyl, - $N((C_0-C_6)$Alkyl)$((C_3-C_7)$cycloalkyl) oder $-N((C_0-C_6)$Alkyl)(aryl) Substituenten;

jedes N ein N-Oxid sein kann;

oder pharmazeutisch akzeptable Salze, Hydrate oder Solvate solcher Verbindungen.

**2.** Verbindung nach Anspruch 1 mit der Formel I-C

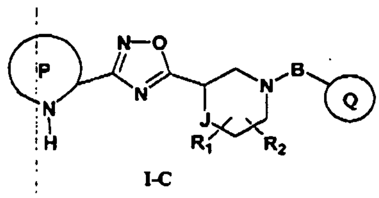

I-C

wobei

$R_1$ und $R_2$ unabhängig Wasserstoff, $-(C_1-C_6)$Alkyl, $-(C_2-C_6)$Alkenyl, $-(C_2-C_6)$Alkynyl, Arylalkyl, Heteroarylalkyl, Hydroxy, Amino, Aminoalkyl, Hydroxyalkyl, $-(C_1-C_6)$Alkoxy repräsentieren oder $R_1$ und $R_2$ zusammen einen $(C_3-C_7)$ Cycloalkylring, eine Carbonylbindung C=O oder eine Kohlenstoffdoppelbindung bilden können;

P einen $(C_5-C_7)$Heterocycloalkyl-, $(C_5-C_7)$Heterocycloalkenylring oder eine Heteroarylgruppe der folgenden Formel repräsentiert

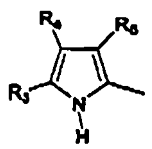 , 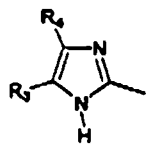 , 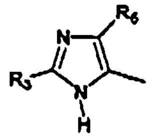 , 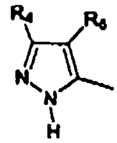 , 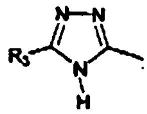 ,

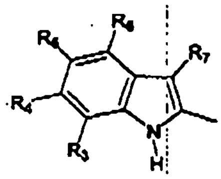 , 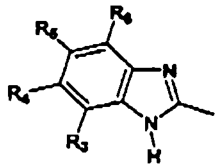 , 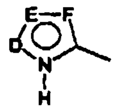 , 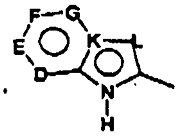 .

$R_3$, $R_4$, $R_5$, $R_6$ und $R_7$ unabhängig Wasserstoff, Halogen, - $NO_2$, - ($C_1$-$C_6$)Alkyl, - ($C_3$-$C_6$) Cycloalkyl, - ($C_3$-$C_7$) Cycloalkylalkyl, ($C_2$-$C_6$)Alkenyl, - -($C_2$-$C_6$)Alkynyl, Halo-($C_1$-$C_6$)alkyl, Heteroaryl, Heteroarylalkyl, Arylalkyl, Aryl, -$OR_8$, -$NR_8R_9$, -C(=$NR_{10}$)$NR_8R_9$, -$NR_8COR_9$, $NR_8CO_2R_9$, $NR_8SO_2R_9$, - $NR_{10}$CO $NR_8R_9$, -$SR_8$, -S(=O)$R_8$, -S (=O)$_2R_8$, -S(=O)$_2NR_8R_9$, - C(=O)$R_8$, -C(O)-O-$R_8$, -C(=O)$NR_8R_9$, -C(=$NR_8$)$R_9$ oder C(=$NOR_8$)$R_9$ Substituenten sind; wobei optional zwei Substituenten mit den dazwischen liegenden Atomen kombiniert sind, um einen bicyclischen Heterocycloalkyl-, Aryl- oder Heteroarylring zu bilden; wobei jeder Ring optional weiter substituiert ist durch 1-5 unabhängige Halogen, -CN, -($C_1$-$C_6$)Alkyl, -O-($C_0$-$C_6$)Alkyl, -O-($C_3$-C-$_7$)Cycloalkylalkyl, -O(Aryl), - O(Heteroaryl), -O-(-$C_1$-$C_3$)Alkylaryl, -O-($C_1$-$C_3$)Alkylheteroaryl, -N((-$C_0$-$C_6$)Alkyl) (($C_0$-$C_3$)alkylaryl) oder -N(($C_0$-$C_6$)Alkyl) (($C_0$-$C_3$-) alkylheteroaryl) Gruppen;

$R_8$, $R_9$, $R_{10}$ jeweils unabhängig Wasserstoff, ($C_1$-$C_6$)Alkyl, ($C_3$-$C_6$)Cycloalkyl, ($C_3$-$C_7$)Cycloalkylalkyl, ($C_2$-$C_6$)Alkenyl, ($C_2$-$C_6$)Alkynyl, Halo-($C_1$-$C_6$)alkyl, Heterocycloalkyl, Heteroaryl, Heteroarylalkyl, Arylalkyl oder Aryl sind; wobei beliebige davon optional substituiert sind durch 1-5 unabhängige Halogen, -CN, -($C_1$-$C_6$)Alkyl, -O-($C_0$-$C_6$)Alkyl, -O-($C_3$-$C_7$)Cycloalkylalkyl, -O(Aryl), - O(Heteroaryl), -N($C_0$-$C_6$-Alkyl)$_2$, -N(($C_0$-$C_6$)Alkyl) (($C_3$-$C_7$-)cycloalkyl) oder -N(($C_0$-$C_6$)Alkyl)(aryl) Substituenten;

D, E, F, G, K und L in P unabhängig -C($R_3$)=, - C($R_3$)=C($R_4$) -, -C(=O)-, -C(=S)-, -O-, -N=, -N($R_3$) - oder -S-repräsentieren;

Q eine Aryl- oder Heteroarylgruppe der folgenden Formel kennzeichnet

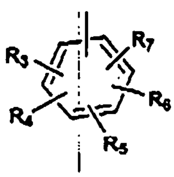 , 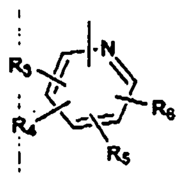 , 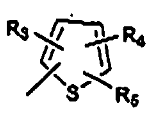 , 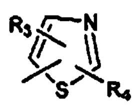 , 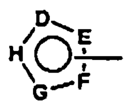 .

$R_3$, $R_4$, $R_5$, $R_6$ und $R_7$ unabhängig der obigen Definition entsprechen;

D, E, F, G und H in Q unabhängig -C($R_3$)=, -C($R_3$)=C($R_4$)-, -C(=O)-, -C(=S)-, -O-, -N=, -N($R_3$)- oder -S-repräsentieren;

B Folgendes repräsentiert: eine Einfachbindung, - C(=O)-($C_0$-$C_2$)Alkyl-, -C(=O)-($C_2$-$C_6$)Alkenyl-, -C(=O) - ($C_2$ - $C_6$) Alkynyl-, -C(=O)-O-, -C(=O)$NR_8$-($C_0$-$C_2$)Alkyl-, -C(=$NR_8$)$NR_9$, -S(=O)-($C_0$-$C_2$)Alkyl-, -S(=O)$_2$-($C_0$-$C_2$)Alkyl-, -S (=O)$_2NR_8$-($C_0$-$C_2$)Alkyl-, C(=$NR_8$)-($C_0$-$C_2$)Alkyl-, -C(=$NOR_8$)-($C_0$-$C_2$)Alkyl- oder -C(=$NOR_8$)$NR_9$- ($C_0$-$C_2$)Alkyl-;

$R_8$ und $R_9$ unabhängig der obigen Definition entsprechen; J -C($R_{10}$, $R_{11}$), -O-, -N($R_{10}$) - oder -S- repräsentiert; $R_{10}$, $R_{11}$ unabhängig Folgendes sind: Wasserstoff, - -($C_1$-$C_6$)Alkyl, -($C_3$-$C_6$)Cycloalkyl, -($C_3$-$C_7$)Cycloalkylalkyl, -($C_2$-$C_6$) Alkenyl, -($C_2$-$C_6$)Alkynyl, Halo($C_1$-$C_6$)alkyl, Heteroaryl, Heteroarylalkyl, Arylalkyl oder Aryl; wobei beliebige davon optional substituiert sind durch 1-5 unabhängige Halogen, - CN, -($C_1$-$C_6$)Alkyl, -O($C_0$-$C_6$)Alkyl, -O($C_3$-$C_7$)Cycloalkylalkyl, -O(Aryl), -O(Heteroaryl), -N(($C_0$-$C_6$)Alkyl) (($C_0$-$C_6$)alkyl, - N(($C_0$-$C_6$)Alkyl) (($C_3$-$C_7$)cycloalkyl) oder -N(($C_0$-$C_6$)Alkyl) (aryl) Substituenten;

jedes N ein N-Oxid sein kann;

oder pharmazeutisch akzeptable Salze, Hydrate oder Solvate solcher Verbindungen.

**3.** Verbindung nach Anspruch 1 oder 2 mit der Formel I-D

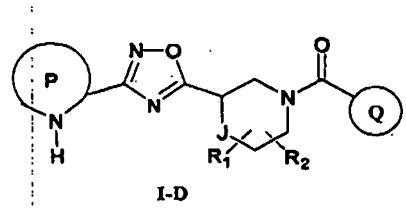

I-D

wobei

$R_1$ und $R_2$ unabhängig Wasserstoff, -$(C_1$-$C_6)$Alkyl, $(C_2$-$C_6)$Alkenyl, - $(C_2$-$C_6)$Alkynyl, Arylalkyl, Heteroarylalkyl, Hydroxy, Amino, Aminoalkyl, Hydroxyalkyl, -$(C_1$-$C_6)$Alkoxy repräsentieren oder $R_1$ und $R_2$ zusammen einen $(C_3$-$C_7)$ Cycloalkylring, eine Carbonylbindung C=O oder eine Kohlenstoffdoppelbindung bilden können;

P einen $(C_5$-$C_7)$Heterocycloalkyl-, $(C_5$-$C_7)$Heterocycloalkenylring oder eine Heteroarylgruppe der folgenden Formel repräsentiert

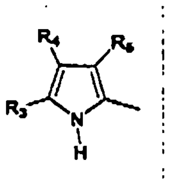 , 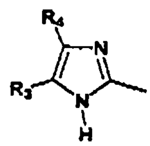 , 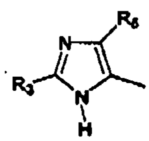 , 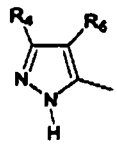 , 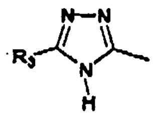 ,

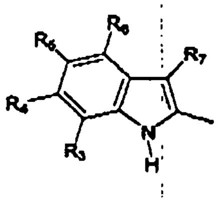 , 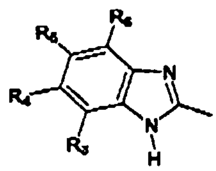 , 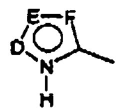 , 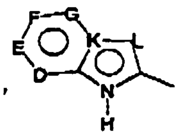 .

$R_3$, $R_4$, $R_5$, $R_6$ und $R_7$ unabhängig Wasserstoff, Halogen, - $NO_2$, - $(C_1$-$C_6)$Alkyl, - $(C_3$-$C_6)$Cycloalkyl, - $(C_3$-$C_7)$ Cycloalkylalkyl, -$(C_2$-$C_6)$Alkenyl, -$(C_2$-$C_6)$Alkynyl, Halo-$(C_1$-$C_6)$alkyl, Heteroaryl, Heteroarylalkyl, Arylalkyl, Aryl, -$OR_8$, -$NR_8R_9$, -$C(=NR_{10})NR_8R_9$, -$NR_8COR_9$, $NR_8CO_2R_9$, $NR_8SO_2R_9$, - $NR_{10}CO$ $NR_8R_9$, -$SR_8$, $S(=O)R_8$, -S $(=O)_2R_8$, -$S(=O)_2NR_8R_9$, - $C(=O)R_8$, -C(O)-O-$R_8$, -$C(=O)NR_8R_9$, -$C(=NR_8)R_9$ oder $C(=NOR_8)R_9$ Substituenten sind; wobei optional zwei Substituenten mit den dazwischen liegenden Atomen kombiniert sind, um einen bicyclischen Heterocycloalkyl-, Aryl- oder Heteroarylring zu bilden; wobei jeder Ring optional weiter substituiert ist durch 1-5 unabhängige Halogen, -CN, -$(C_1$-$C_6)$Alkyl, -O-$(C_0$-$C_6)$Alkyl, -O-$(C_3$-$C_7)$Cycloalkylalkyl, -O(Aryl), - O(Heteroaryl), -O-(-$C_1$-$C_3)$Alkylaryl, -O- $(C_1$-$C_3)$Alkylheteroaryl, -N((-$C_0$-$C_6)$Alkyl) (($C_0$-$C_3)$alkylaryl) oder -N(($C_0$-$C_6)$Alkyl) (($C_0$-$C_3$-) alkylheteroaryl) Gruppen;

$R_8$, $R_9$, $R_{10}$ jeweils unabhängig Wasserstoff, $(C_1$-$C_6)$ Alkyl, $(C_3$-$C_6)$ Cycloalkyl, $(C_3$-$C_7)$Cycloalkylalkyl, $(C_2$-$C_6)$Alkenyl, $(C_2$-$C_6)$ Alkynyl, Halo-$(C_1$-$C_6)$alkyl, Heterocycloalkyl, Heteroaryl, Heteroarylalkyl, Arylalkyl oder Aryl sind; wobei beliebige davon optional substituiert sind durch 1-5 unabhängige Halogen, -CN, -$(C_1$-$C_6)$Alkyl, -O-$(C_0$-$C_6)$ Alkyl, -O-$(C_3$-$C_7)$Cycloalkylalkyl, -O(Aryl), - O(Heteroaryl), -N($C_0$-$C_6$-Alkyl$)_2$, -N(($C_0$-$C_6)$Alkyl) (($C_3$-$C_7$-)cycloalkyl) oder -N(($C_0$-$C_6)$Alkyl) (aryl) Substituenten;

D, E, F, G, K und L in P unabhängig -$C(R_3)$=, - $C(R_3)=C(R_4)$-, -C(=O)-, -C(=S)-, -O-, -N=, -$N(R_3)$- oder -S-repräsentieren;

Q eine Aryl- oder Heteroarylgruppe der folgenden Formel kennzeichnet

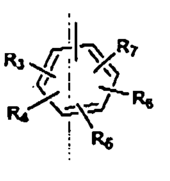 , 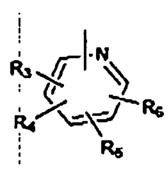 , 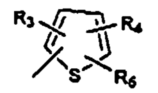 , 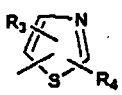 , 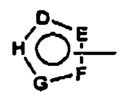

$R_3$, $R_4$, $R_5$, $R_6$ und $R_7$ unabhängig der obigen Definition entsprechen;

D, E, F, G und H in Q unabhängig -C($R_3$)=, -C($R_3$)=C($R_4$) - , -C(=O)-, -C(=S)-, -O-, -N=, -N($R_3$)- oder -S-repräsentieren;

J -C($R_{10}$, $R_{11}$), -O-, -N($R_{10}$) - oder -S- repräsentiert;

$R_{10}$, $R_{11}$ unabhängig Folgendes sind: Wasserstoff, -($C_1$-$C_6$)Alkyl, - ($C_3$-$C_6$)Cycloalkyl, - ($C_3$-$C_7$)Cycloalkylalkyl, -($C_2$-$C_6$)Alkenyl,- ($C_2$-$C_6$)Alkynyl, Halo($C_1$-$C_6$)alkyl, Heteroaryl, Heteroarylalkyl, Arylalkyl oder Aryl; wobei beliebige davon optional substituiert sind durch 1-5 unabhängige Halogen, - CN, -($C_1$-$C_6$)Alkyl, -O($C_0$-$C_6$)Alkyl, -O($C_3$-$C_7$) Cycloalkylalkyl, -O(Aryl), -O(Heteroaryl), -N(($C_0$-$C_6$)Alkyl) (($C_0$-$C_6$)alkyl, - N(($C_0$-$C_6$)Alkyl) (($C_3$-$C_7$)cycloalkyl) oder -N(($C_0$-$C_6$)Alkyl) (aryl) Substituenten;

jedes N ein N-Oxid sein kann;

oder pharmazeutisch akzeptable Salze, Hydrate oder Solvate solcher Verbindungen.

**4.** Verbindung nach Anspruch 1 mit der Formel II-A

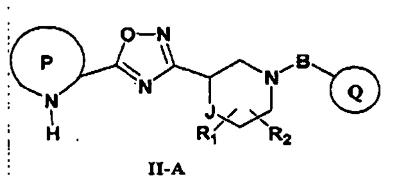

II-A

wobei

$R_1$ und $R_2$ unabhängig Wasserstoff, - ($C_1$-$C_6$)Alkyl, -($C_2$-$C_6$)Alkenyl, - ($C_2$-$C_6$)Alkynyl, Arylalkyl, Heteroarylalkyl, Hydroxy, Amino, Aminoalkyl, Hydroxyalkyl, -($C_1$-$C_6$)Alkoxy repräsentieren oder $R_1$ und $R_2$ zusammen einen ($C_3$-$C_7$) Cycloalkylring, eine Carbonylbindung C=O oder eine Kohlenstoffdoppelbindung bilden können;

P einen (C-$_5$-$C_7$)Heterocycloalkyl-, ($C_5$-$C_7$)Heterocycloalkenylring oder eine Heteroarylgruppe der folgenden Formel repräsentiert

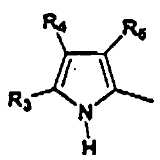 , 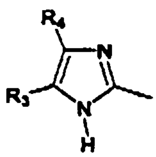 , 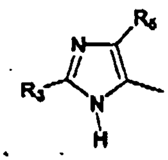 , 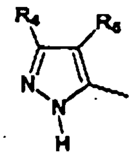 , 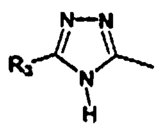 ,

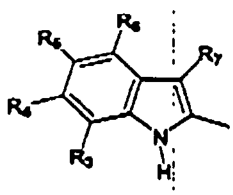 , 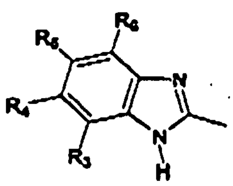 , 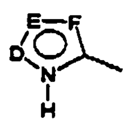 , 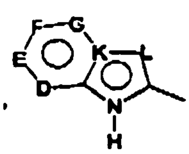 .

$R_3$ , $R_4$, $R_5$, $R_6$ und $R_7$ unabhängig Wasserstoff, Halogen, - $NO_2$, - ($C_1$-$C_6$)Alkyl, - ($C_3$-$C_6$)Cycloalkyl, - ($C_3$-$C_7$) Cycloalkylalkyl, -($C_2$-$C_6$)Alkenyl, -($C_2$-$C_6$)Alkynyl, Halo-($C_1$-$C_6$)alkyl, Heteroaryl, Heteroarylalkyl, Arylalkyl, Aryl, -$OR_8$, -$NR_8R_9$, -C(=$NR_{10}$)$NR_8R_9$, -$NR_8COR_9$, $NR_8CO_2R_9$, $NR_8SO_2R_9$, - $NR_{10}$CO $NR_8R_9$, -$SR_8$, -S(=O)$R_8$, -S (=O)$_2R_8$, -S(=O)$_2NR_8R_9$, - C(=O)$R_8$, -C(O)-O-$R_8$, -C(=O)$NR_8R_9$, -C(=$NR_8$)$R_9$ oder C(=$NOR_8$)$R_9$ Substituenten sind; wobei optional zwei Substituenten mit den dazwischen liegenden Atomen kombiniert sind, um einen bicyclischen Heterocycloalkyl-, Aryl- oder Heteroarylring zu bilden; wobei jeder Ring optional weiter substituiert ist durch 1-5 unabhängige Halogen, -CN, -($C_1$-$C_6$)Alkyl, -O-($C_0$-$C_6$)Alkyl, -O-($C_3$-$C_7$)Cycloalkylalkyl, -O(Aryl), - O(Heteroaryl), -O-(-$C_1$-$C_3$)Alkylaryl, -O-($C_1$-$C_3$)Alkylheteroaryl, -N((-$C_0$-$C_6$)Alkyl) (($C_0$-$C_3$)alkylaryl) oder -N(($C_0$-$C_6$)Alkyl) (($C_0$-$C_3$-) alkylheteroaryl) Gruppen;

$R_8$, $R_9$, $R_{10}$ jeweils unabhängig Wasserstoff, ($C_1$-$C_6$)Alkyl, ($C_3$-$C_6$)Cycloalkyl, ($C_3$-$C_7$)Cycloalkylalkyl, ($C_2$-$C_6$)Alkenyl, ($C_2$-$C_6$)Alkynyl, Halo-($C_1$-$C_6$)alkyl, Heterocycloalkyl, Heteroaryl, Heteroarylalkyl, Arylalkyl oder Aryl sind; wobei beliebige davon optional substituiert sind durch 1-5 unabhängige Halogen, -CN, -($C_1$-$C_6$)Alkyl, -O-($C_0$-$C_6$)Alkyl, -O-($C_3$-$C_7$)Cycloalkylalkyl, -O(Aryl), - O(Heteroaryl), -N($C_0$-$C_6$-Alkyl)$_2$, -N(($C_0$-$C_6$)Alkyl) (($C_3$-$C_7$-)cycloalkyl) oder

-N(($C_0$-$C_6$)Alkyl) (aryl) Substituenten;

D, E, F, G, K und L in P unabhängig -C($R_3$)=, - C($R_3$)=C($R_4$) -, -C(=O) -, -C(=S) -, -O-, -N=, -N($R_3$) - oder -S- repräsentieren;

Q eine Aryl- oder Heteroarylgruppe der folgenden Formel kennzeichnet

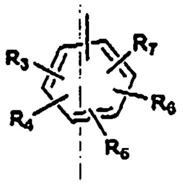 , 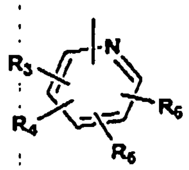 , 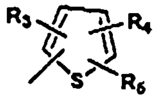 . 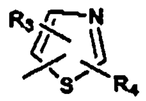 . 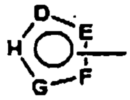

$R_3$, $R_4$, $R_5$, $R_6$ und $R_7$ unabhängig der obigen Definition entsprechen;

D, E, F, G und H in Q unabhängig -C($R_3$)=, -C($R_3$)=C($R_4$) - , -C(=O)-, -C(=S)-, -O-, -N=, -N($R_3$)- oder -S-repräsentieren;

B Folgendes repräsentiert: eine Einfachbindung, - C(=O) - ($C_0$-$C_2$)Alkyl-, -C(=O) - ($C_2$-$C_6$)Alkenyl-, -C(=O)-($C_2$-$C_6$) Alkynyl-, -C(=O)-O-, -C(=O)N$R_8$-($C_0$-$C_2$)Alkyl-, -C(=N$R_8$)N$R_9$, -S(=O) - ($C_0$-$C_2$)Alkyl-, -S(=O)$_2$- ($C_0$-$C_2$)Alkyl-, -S (=O)$_2$N$R_8$-($C_0$-$C_2$)Alkyl-, C(=N$R_8$)-($C_0$-$C_2$)Alkyl-, -C(=NO$R_8$)-($C_0$-$C_2$)Alkyl- oder -C(=NO$R_8$)N$R_9$-($C_0$-$C_2$)Alkyl-;

$R_8$ und $R_9$ unabhängig der obigen Definition entsprechen;

J -C($R_{10}$, $R_{11}$), -O-, -N($R_{10}$)- oder -S- repräsentiert;

$R_{10}$, $R_{11}$ unabhängig Folgendes sind: wasserstoff, - -($C_1$-$C_6$)Alkyl, -($C_3$-$C_6$)Cycloalkyl, -($C_3$-$C_7$)Cycloalkylalkyl, -($C_2$-$C_6$)Alkenyl, -($C_2$-$C_6$)Alkynyl, Halo($C_1$-$C_6$)alkyl, Heteroaryl, Heteroarylalkyl, Arylalkyl oder Aryl; wobei beliebige davon optional substituiert sind durch 1-5 unabhängige Halogen, - CN, -($C_1$-$C_6$)Alkyl, -O($C_0$-$C_6$)Alkyl, -O($C_3$-$C_7$) Cycloalkylalkyl, -O(Aryl), -O(Heteroaryl), -N(($C_0$-$C_6$)Alkyl) (($C_0$-$C_6$)alkyl, - N(($C_0$-$C_6$)Alkyl)(($C_3$-$C_7$)cycloalkyl) oder -N(($C_0$-$C_6$)Alkyl)(aryl) Substituenten;

jedes N ein N-Oxid sein kann;

oder pharmazeutisch akzeptable Salze, Hydrate oder Solvate solcher Verbindungen.

**5.** Verbindung nach Anspruch 1 mit der Formel II-B

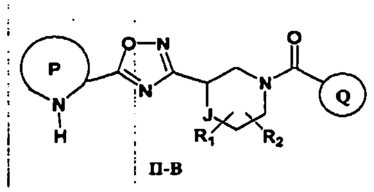

II-B

wobei

$R_1$ und $R_2$ unabhängig Wasserstoff, -($C_1$-$C_6$)Alkyl, -($C_2$-$C_6$)Alkenyl, -($C_2$-$C_6$)Alkynyl, Arylalkyl, Heteroarylalkyl, Hydroxy, Amino, Aminoalkyl, Hydroxyalkyl, -($C_1$-$C_6$)Alkoxy repräsentieren oder $R_1$ und $R_2$ zusammen einen ($C_3$-$C_7$) Cycloalkylring, eine Carbonylbindung C=O oder eine Kohlenstoffdoppelbindung bilden können;

P einen ($C_5$-$C_7$)Heterocycloalkyl-, ($C_5$-$C_7$)Heterocycloalkenylring oder eine Heteroarylgruppe der folgenden Formel repräsentiert

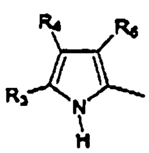 , 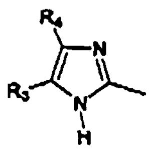 , 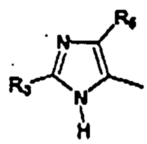 , 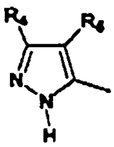 , 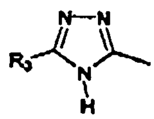 ,

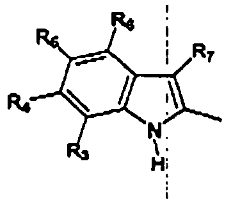, 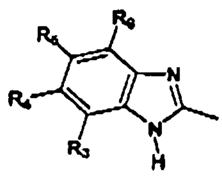, 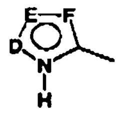, 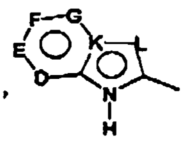

$R_3$, $R_4$ , $R_5$, $R_6$ und $R_7$ unabhängig Wasserstoff, Halogen, - $NO_2$, - $(C_1-C_6)$Alkyl, - $(C_3-C_6)$Cycloalkyl, - $(C_3-C_7)$ Cycloalkylalkyl, -$(C_2-C_6)$Alkenyl, $(C_2-C_6)$Alkynyl, Halo-$(C_1-C_6)$alkyl, Heteroaryl, Heteroarylalkyl, Arylalkyl, Aryl, -$OR_8$, -$NR_8R_9$, -C(=$NR_{10}$)$NR_8R_9$, -$NR_8COR_9$, $NR_8CO_2R_9$, $NR_8SO_2R_9$, - $NR_{10}$CO $NR_8R_9$, -$SR_8$, -S(=O)$R_8$, -S(=O)$_2R_8$, -S (=O)$_2NR_8R_9$, - C(=O)$R_8$, -C(O)-O-$R_8$, -C(=O)$NR_8R_9$, -C(=$NR_8$)$R_9$ oder C(=$NOR_8$)$R_9$ Substituenten sind; wobei optional zwei Substituenten mit den dazwischen liegenden Atomen kombiniert sind, um einen bicyclischen Heterocycloalkyl-, Aryl- oder Heteroarylring zu bilden; wobei jeder Ring optional weiter substituiert ist durch 1-5 unabhängige Halogen, -CN, -$(C_1-C_6)$Alkyl, -O-$(C_0-C_6)$Alkyl, -O-$(C_3-C_7)$Cycloalkylalkyl, -O(Aryl), - O(Heteroaryl), -O-(-$C_1-C_3$)Alkylaryl, -O-$(C_1-C_3)$Alkylheteroaryl, -N((-$C_0-C_6$)Alkyl) (($C_0-C_3$)alkylaryl) oder -N(($C_0-C_6$)Alkyl) (($C_0-C_3$-)alkylheteroaryl) Gruppen;

$R_8$, $R_9$, $R_{10}$ jeweils unabhängig Wasserstoff, $(C_1-C_6)$Alkyl, $(C_3-C_6)$Cycloalkyl, $(C_3-C_7)$Cycloalkylalkyl, $(C_2-C_6)$Alkenyl, $(C_2-C_6)$Alkynyl, Halo-$(C_1-C_6)$alkyl, Heterocycloalkyl, Heteroaryl, Heteroarylalkyl, Arylalkyl oder Aryl sind; wobei beliebige davon optional substituiert sind durch 1-5 unabhängige Halogen, -CN, -$(C_1-C_6)$Alkyl, -O-$(C_0-C_6)$Alkyl, -O-$(C_3-C_7)$Cycloalkylalkyl, -O(Aryl), - O(Heteroaryl), -N($C_0$-$C_6$-Alkyl)$_2$, -N(($C_0-C_6$)Alkyl) (($C_3-C_7$-)cycloalkyl) oder -N(($C_0-C_6$)Alkyl) (aryl) Substituenten;

D, E, F, G, K und L in P unabhängig -C($R_3$)=, - C($R_3$)=C($R_4$) -, -C(=O) -, -C(=S) -, -O-, -N=, -N($R_3$) - oder -S-repräsentieren;

Q eine Aryl- oder Heteroarylgruppe der folgenden Formel kennzeichnet

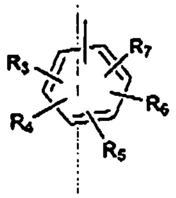, 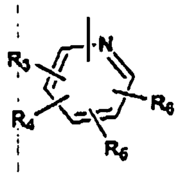, 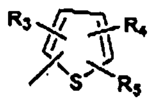, 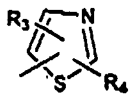, 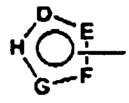

$R_3$, $R_4$, $R_5$, $R_6$ und $R_7$ unabhängig der obigen Definition entsprechen;

D, E, F, G und H in Q unabhängig -C($R_3$))=, -C($R_3$)=C($R_4$) - , -C(=O)-, -C(=S)-, -O-, -N=, -N($R_3$)- oder -S-repräsentieren;

J -C($R_{10}$, $R_{11}$), -O-, -N($R_{10}$)- oder -S- repräsentiert; $R_{10}$, $R_{11}$ unabhängig Folgendes sind: Wasserstoff, -$(C_1-C_6)$ Alkyl, - $(C_3-C_6)$ Cycloalkyl, - $(C_3-C_7)$ Cycloalkylalkyl, - $(C_2-C_6)$ Alkenyl, - $(C_2-C_6)$ Alkynyl, Halo $(C_1-C_6)$ alkyl, Heteroaryl, Heteroarylalkyl, Arylalkyl oder Aryl; wobei beliebige davon optional substituiert sind durch 1-5 unabhängige Halogen, - CN, -$(C_1-C_6)$Alkyl, -O$(C_0-C_6)$Alkyl, -O$(C_3-C_7)$Cycloalkylalkyl, -O(Aryl), -O(Heteroaryl), -N(($C_0-C_6$)Alkyl) (($C_0-C_6$)alkyl, - N(($C_0-C_6$)Alkyl) (($C_3-C_7$) cycloalkyl) oder -N(($C_0-C_6$)Alkyl) (aryl) Substituenten;

jedes N ein N-Oxid sein kann;

oder pharmazeutisch akzeptable Salze, Hydrate oder Solvate solcher Verbindungen.

**6.** Verbindung nach Anspruch 1 mit der Formel II-B

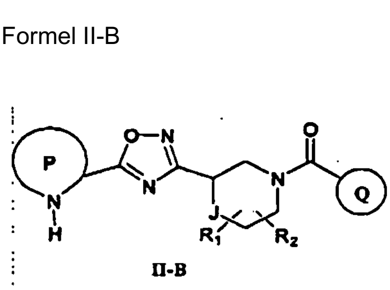

II-B

wobei

$R_1$ und $R_2$ unabhängig Wasserstoff, $(C_1-C_6)$Alkyl, - -$(C_2-C_6)$Alkenyl, - -$(C_2-C_6)$Alkynyl, Arylalkyl, Heteroarylalkyl, Hydroxy, Amino, Aminoalkyl, Hydroxyalkyl, -$(C_1-C_6)$Alkoxy repräsentieren oder $R_1$ und $R_2$ zusammen einen $(C_3-C_7)$

Cycloalkylring, eine Carbonylbindung C=O oder eine Kohlenstoffdoppelbindung bilden können;
P einen $(C_5-C_7)$Heterocycloalkyl-, $(C_5-C_7)$ Heterocycloalkenylring oder eine Heteroarylgruppe der folgenden Formel repräsentiert

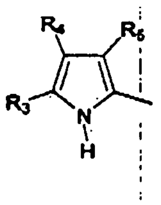 , 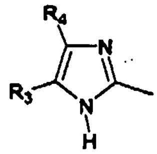 , 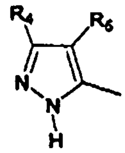 , 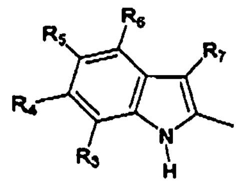

$R_3$, $R_4$, $R_5$, $R_6$ und $R_7$ unabhängig Wasserstoff, Halogen, - $NO_2$, - $(C_1-C_6)$Alkyl, - $(C_3-C_6)$ Cycloalkyl, - $(C_3-C_7)$ Cycloalkylalkyl, -$(C_2-C_6)$Alkenyl, $(C_2-C_6)$Alkynyl, Halo-$(C_1-C_6)$alkyl, Heteroaryl, Heteroarylalkyl, Arylalkyl, Aryl, -$OR_8$, -$NR_8R_9$, -C(=$NR_{10}$)$NR_8R_9$, -$NR_8COR_9$, $NR_8CO_2R_9$, $NR_8SO_2R_9$, - $NR_{10}CO$ $NR_8R_9$, -$SR_8$, -S(=O)$R_8$, -S(=O)$_2R_8$, -S (=O)$_2NR_8R_9$, - C(=O)$R_8$, -C(O)-O-$R_8$, -C(=O)$NR_8R_9$, - C(=$NR_8$)$R_9$ oder C(=$NOR_8$)$R_9$ Substituenten sind; wobei optional zwei Substituenten mit den dazwischen liegenden Atomen kombiniert sind, um einen bicyclischen Heterocycloalkyl-, Aryl- oder Heteroarylring zu bilden; wobei jeder Ring optional weiter substituiert ist durch 1-5 unabhängige Halogen, -CN, -$(C_1-C_6)$Alkyl, -O-$(C_0-C_6)$Alkyl, -O-$(C_3-C_{-7})$Cycloalkylalkyl, -O(Aryl), - O(Heteroaryl), -O-(-$C_1-C_3$)Alkylaryl, -O- $(C_1-C_3)$Alkylheteroaryl, -N((-$C_0-C_6$)Alkyl) (($C_0-C_3$)alkylaryl) oder -N(($C_0-C_6$)Alkyl) (($C_0-C_3$-) alkylheteroaryl) Gruppen;
$R_8$, $R_9$, $R_{10}$ jeweils unabhängig Wasserstoff, $(C_1-C_6)$Alkyl, $(C_3-C_6)$Cycloalkyl, $(C_3-C_7)$Cycloalkylalkyl, $(C_2-C_6)$Alkenyl, $(C_2-C_6)$Alkynyl, Halo-$(C_1-C_6)$alkyl, Heterocycloalkyl, Heteroaryl, Heteroarylalkyl, Arylalkyl oder Aryl sind; wobei beliebige davon optional substituiert sind durch 1-5 unabhängige Halogen, -CN, -$(C_1-C_6)$Alkyl, -O-$(C_0-C_6)$Alkyl, -O-$(C_3-C_7)$Cycloalkylalkyl, -O(Aryl), - O(Heteroaryl), -N($C_0-C_6$-Alkyl)$_2$, -N(($C_0-C_6$)Alkyl) (($C_3-C_7$-)cycloalkyl) oder -N(($C_0-C_6$)Alkyl) (aryl) Substituenten;
Q eine Aryl- oder Heteroarylgruppe der folgenden Formel kennzeichnet

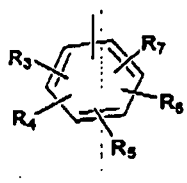 , 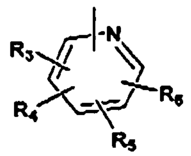 , 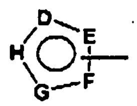

$R_3$, $R_4$, $R_5$, $R_6$ und $R_7$ unabhängig der obigen Definition entsprechen;
D, E, F, G und H in Q unabhängig -C(R3)=, -C($R_3$)=C($R_4$)-, -O-oder -N= repräsentieren;
J -C($R_{10}$, $R_{11}$), -O-, -N($R_{10}$)- oder -S- repräsentiert; $R_{10}$, $R_{11}$ unabhängig Folgendes sind: Wasserstoff, -$(C_1-C_6)$ Alkyl, -$(C_3-C_6)$Cycloalkyl, -$(C_3-C_7)$Cycloalkylalkyl, -$(C_2-C_6)$Alkenyl, -$(C_2-C_6)$Alkynyl, Halo$(C_1-C_6)$ alkyl, Heteroaryl, Heteroarylalkyl, Arylalkyl oder Aryl; wobei beliebige davon optional substituiert sind durch 1-5 unabhängige Halogen, - CN, -$(C_1-C_6)$Alkyl, -O$(C_0-C_6)$Alkyl, -O$(C_3-C_7)$Cycloalkylalkyl, -O(Aryl), -O(Heteroaryl), -N(($C_0-C_6$)Alkyl) (($C_0-C_6$) alkyl, - N(($C_0-C_6$)Alkyl) (($C_3-C_7$)cycloalkyl) oder -N(($C_0-C_6$)Alkyl)(aryl) Substituenten;
jedes N ein N-Oxid sein kann;
oder pharmazeutisch akzeptable Salze, Hydrate oder Solvate solcher Verbindungen.

**7.** Verbindung nach Anspruch 1 mit der Formel II-C

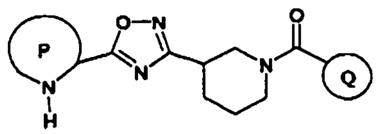

II-C

wobei
P einen $(C_5-C_7)$Heterocycloalkyl-, $(C_5-C_7)$Heterocycloalkenylring oder eine Heteroarylgruppe der folgenden Formel

repräsentiert

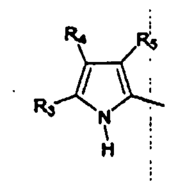 , 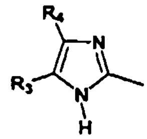 , 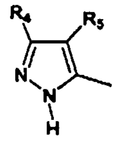 , 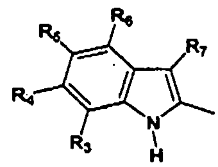

$R_3$, $R_4$, $R_5$, $R_6$ und $R_7$ unabhängig Wasserstoff, Halogen, - $NO_2$, ($C_1$-$C_6$)Alkyl, Halo-($C_1$-$C_6$)Alkyl oder -CN sind;
Q eine Aryl- oder Heteroarylgruppe der folgenden Formel kennzeichnet

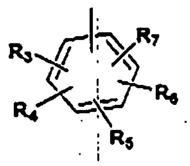 , 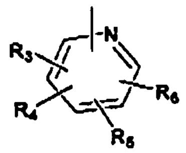 , 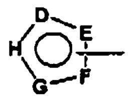

$R_3$, $R_4$, $R_5$, $R_6$ und $R_7$ unabhängig der obigen Definition entsprechen;
D, E, F, G und H in Q unabhängig -C($R_3$)=, -C($R_3$)=C($R_4$)-, -O-oder -N= repräsentieren;
jedes N ein N-Oxid sein kann;
oder pharmazeutisch akzeptable Salze, Hydrate oder Solvate solcher Verbindungen.

**8.** Verbindung nach Anspruch 1 mit der Formel I-D

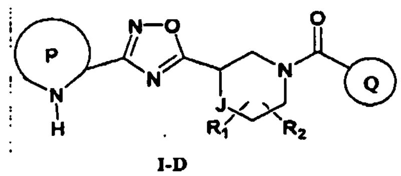

**I-D**

wobei
$R_1$ und $R_2$ unabhängig Wasserstoff, -($C_1$-$C_6$) Alkyl, -($C_2$-$C_6$)Alkenyl, -($C_2$-$C_6$)Alkynyl, Arylalkyl, Heteroarylalkyl, Hydroxy, Amino, Aminoalkyl, Hydroxyalkyl, -($C_1$-$C_6$)Alkoxy repräsentieren oder $R_1$ und $R_2$ zusammen einen ($C_3$-$C_7$) Cycloalkylring, eine Carbonylbindung C=O oder eine Kohlenstoffdoppelbindung bilden können;
P einen ($C_5$-$C_7$)Heterocycloalkyl-, ($C_5$-$C_7$)Heterocycloalkenylring oder eine Heteroarylgruppe der folgenden Formel repräsentiert

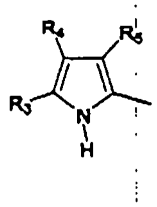 , 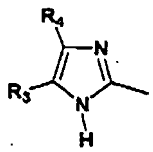 , 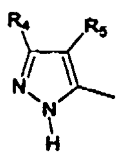 , 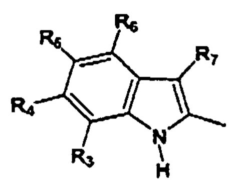

$R_3$, $R_4$, $R_5$, $R_6$ und $R_7$ unabhängig Wasserstoff, Halogen, - $NO_2$, -($C_1$-$C_6$)Alkyl, -($C_3$-$C_6$)Cycloalkyl, - ($C_3$-$C_7$)Cycloalkylalkyl, -($C_2$-$C_6$)Alkenyl, -($C_2$-$C_6$)Alkynyl, Halo-($C_1$-$C_6$)alkyl, Heteroaryl, Heteroarylalkyl, Arylalkyl, Aryl, -$OR_8$, -$NR_8R9$, -C(=$NR_{10}$)$NR_8R_9$, -$NR_8COR_9$, $NR_8CO_2R_9$, $NR_8SO_2R_9$, - $NR_{10}CO$ $NR_8R_9$, -$SR_8$, S(=O)$R_8$, -S(=O)$_2R_8$, -S (=O)$_2NR_8R_9$, - C(=O)$R_8$, -C(O)-O-$R_8$, -C(=O)$NR_8R_9$, -C(=$NR_8$)$R_9$ oder C(=$NOR_8$)$R_9$ Substituenten sind; wobei optional zwei Substituenten mit den dazwischen liegenden Atomen kombiniert sind, um einen bicyclischen Heterocycloalkyl-, Aryl- oder Heteroarylring zu bilden; wobei jeder Ring optional weiter substituiert ist durch 1-5 unabhängige Halogen, -CN, -($C_1$-$C_6$)Alkyl, -O-($C_0$-$C_6$)Alkyl, -O-($C_3$-$C_7$)Cycloalkylalkyl, -O(Aryl), - O(Heteroaryl), -O-(-$C_1$-$C_3$)Alkylaryl, -O-($C_1$-$C_3$)Alkylheteroaryl, -N((-$C_0$-$C_6$)Alkyl) (($C_0$-$C_3$)alkylaryl) oder -N(($C_0$-$C_6$)Alkyl) (($C_0$-$C_3$-)alkylhete-

roaryl) Gruppen;

$R_8$, $R_9$, $R_{10}$ jeweils unabhängig Wasserstoff, ($C_1$-$C_6$)Alkyl, ($C_3$-$C_6$)Cycloalkyl, ($C_3$-$C_7$)Cycloalkylalkyl, ($C_2$-$C_6$)Alkenyl, ($C_2$-$C_6$)Alkynyl, Halo-($C_1$-$C_6$)alkyl, Heterocycloalkyl, Heteroaryl, Heteroarylalkyl, Arylalkyl oder Aryl sind; wobei beliebige davon optional substituiert sind durch 1-5 unabhängige Halogen, -CN, -($C_1$-$C_6$)Alkyl, -O-($C_0$-$C_6$)Alkyl, -O-($C_3$-$C_7$)Cycloalkylalkyl, -O(Aryl), - O(Heteroaryl), -N($C_0$-$C_6$-Alkyl)$_2$, -N(($C_0$-$C_6$)Alkyl) (($C_3$-$C_7$-)cycloalkyl) oder -N(($C_0$-$C_6$)Alkyl)(aryl) Substituenten;

Q eine Aryl- oder Heteroarylgruppe der folgenden Formel kennzeichnet

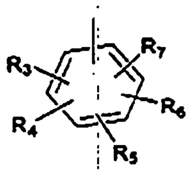 , 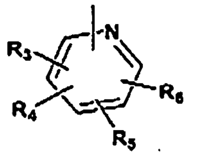 , 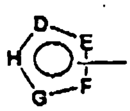

$R_3$, $R_4$, $R_5$, $R_6$ und $R_7$ unabhängig der obigen Definition entsprechen;

D, E, F, G und H in Q unabhängig -C($R_3$)=, -C($R_3$)=C($R_4$)-, -O-oder -N= repräsentieren;

J -C($R_{10}$, $R_{11}$), -O-, -N($R_{10}$)- oder -S- repräsentiert; $R_{10}$, $R_{11}$ unabhängig Folgendes sind: Wasserstoff, ($C_1$-$C_6$)Alkyl, -($C_3$-$C_6$)Cycloalkyl, -($C_3$-$C_7$)Cycloalkylalkyl, -($C_2$-$C_6$)Alkenyl, -($C_2$-$C_6$)Alkynyl, Halo($C_1$-$C_6$)alkyl, Heteroaryl, Heteroarylalkyl, Arylalkyl oder Aryl; wobei beliebige davon optional substituiert sind durch 1-5 unabhängige Halogen, -CN, -($C_1$-$C_6$)Alkyl, -O($C_0$-$C_6$)Alkyl, -O($C_3$-$C_7$)Cycloalkylalkyl, -O(Aryl), -O(Heteroaryl), -N(($C_0$-$C_6$)Alkyl) (($C_0$-$C_6$)alkyl, - N(($C_0$-$C_6$)Alkyl) (($C_3$-$C_7$)cycloalkyl) oder -N(($C_0$-$C_6$)Alkyl)(aryl) Substituenten;

jedes N ein N-Oxid sein kann;

oder pharmazeutisch akzeptable Salze, Hydrate oder Solvate solcher Verbindungen.

**9.** Verbindung nach Anspruch 1 mit der Formel I-E

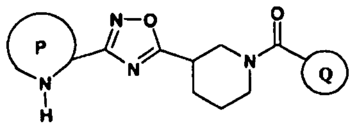

I-E

wobei

P einen ($C_5$-$C_7$)Heterocycloalkyl-, ($C_5$-$C_7$)Heterocycloalkenylring oder eine Heteroarylgruppe der folgenden Formel repräsentiert

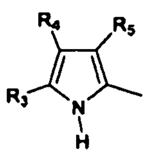 , 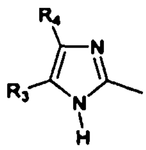 , 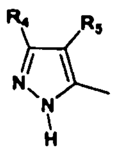 , 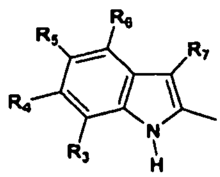

$R_3$, $R_4$, $R_5$, $R_6$ und $R_7$ unabhängig Wasserstoff, Halogen, - $NO_2$, -($C_1$-$C_6$)Alkyl, Halo-($C_1$-$C_6$)alkyl oder -CN sind;

Q eine Aryl- oder Heteroarylgruppe der folgenden Formel kennzeichnet

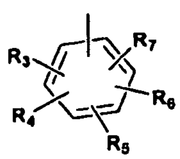 , 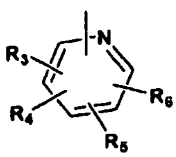 , 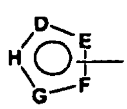

$R_3$, $R_4$, $R_5$, $R_6$ und $R_7$ unabhängig der obigen Definition entsprechen;
D, E, F, G und H in Q unabhängig -C($R_3$))=, -C($R_3$)=C($R_4$)-, -O-oder -N= repräsentieren;
jedes N ein N-Oxid sein kann;
oder pharmazeutisch akzeptable Salze, Hydrate oder Solvate solcher Verbindungen.

**10.** Verbindung nach den Ansprüchen 1 bis 9, die als optische Isomere vorliegen kann, wobei die genannte Verbindung entweder das racemische Gemisch oder ein individuelles optisches Isomer ist.

**11.** Verbindung nach den Ansprüchen 1 bis 10, wobei die genannte Verbindung ausgewählt ist aus:

(4-Fluor-phenyl)-{(S)-3-[3-(1H-pyrrol-2-yl)-[1,2,4]oxadiazol-5-yl]-piperidin-1-yl}-methanon
(2,4-Difluor-phenyl)-{(S)-3-[3-(1H-pyrrol-2-yl)-[1,2,4]oxadiazol-5-yl]-piperidin-1-yl}-methanon
(3,4-Difluor-phenyl)-{(S)-3-[3-(1H-pyrrol-2-yl)-[1,2,4]oxadiazol-5-yl]-piperidin-1-yl}-methanon
(6-Fluor-pyridin-3-yl)-{(S)-3-[3-(1H-pyrrol-2-yl)-[1,2,4]oxadiazol-5-yl]-piperidin-1-yl}-methanon
(3,4-Difluor-phenyl)-{3-[5-(1H-pyrrol-2-yl)-[1,2,4]oxadiazol-3-yl]-piperidin-1-yl}-methanon
(2,4-Difluor-phenyl)-{3-[5-(1H-pyrrol-2-yl)-[1,2,4]oxadiazol-3-yl]-piperidin-1-yl}-methanon
(4-Fluor-phenyl)-{3-[5-(1H-pyrrol-2-yl)-[1,2,4]oxadiazol-3-yl]-piperidin-1-yl}-methanon
(6-Fluor-pyridin-3-yl)-{3-[5-(1H-pyrrol-2-yl)-[1,2,4]oxadiazol-3-yl]-piperidin-1-yl}-methanon
(4-Fluor-2-methyl-phenyl)-{3-[5-(1H-pyrrol-2-yl)-[1,2,4]oxadiazol-3-yl]-piperidin-1-yl}-methanon
(3,4-Difluor-phenyl)-{(S)-3-[5-(1H-pyrrol-2-yl)-[1,2,4]oxadiazol-3-yl]-piperidin-1-yl}-methanon
(4-Fluor-phenyl)-{3-[5-(1H-indol-2-yl)-[1,2,4]oxadiazol-3-yl]-piperidin-1-yl}-methanon
(2,4-Difluor-phenyl)-{(S)-3-[3-(1H-indol-2-yl)-[1,2,4]oxadiazol-5-yl]-piperidin-1-yl}-methanon
(4-Fluor-phenyl)-{3-[5-(2H-pyrazol-3-yl)-[1,2,4]oxadiazol-3-yl]-piperidin-1-yl}-methanon
(3,4-Difluor-phenyl)-{3-[5-(2H-pyrazol-3-yl)-[1,2,4]oxadiazol-3-yl]-piperidin-1-yl}-methanon
(4-Fluor-phenyl)-{3-[5-(1H-imidazol-2-yl)-[1,2,4]oxadiazol-3-yl]-piperidin-1-yl}-methanon
(3,4-Difluor-phenyl)-{(S)-3-[3-(1H-indol-2-yl)-[1,2,4]oxadiazol-5-yl]-piperidin-1-yl}-methanon
(4-Fluor-phenyl)-{(S)-3-[3-(1H-indol-2-yl)-[1,2,4]oxadiazol-5-yl]-piperidin-1-yl}-methanon
(3,4-Difluor-phenyl)-{3-[5-(1H-imidazol-2-yl)-[1,2,4]oxadiazol-3-yl]-piperidin-1-yl}-methanon
{(S)-3-[3-(1H-Indol-2-yl)-[1,2,4]oxadiazol-5-yl]-piperidin-1-yl}-(5-methyl-isoxazol-4-yl)-methanon
(5-Methyl-isoxazol-4-yl)-{(S)-3-[3-(1H-pyrrol-2-yl)-[1,2,4]oxadiazol-5-yl]-piperidin-1-yl}-methanon
(6-Fluor-pyridin-3-yl)-{(S)-3-[5-(1H-pyrrol-2-yl)-[1,2,4]oxadiazol-3-yl]-piperidin-1-yl}-methanon
(4-Fluor-phenyl)-{(S)-3-[5-(1H-pyrrol-2-yl)-[1,2,4]oxadiazol-3-yl]-piperidin-1-yl}-methanon
(6-Fluor-pyridin-3-yl)-{3[5-(1H-indol-2-yl)-[1,2,4]oxadiazol-3-yl]-piperidin-1-yl}-methanon
(4-Fluor-phenyl)-{(S)-3-[3-(1H-imidazol-2-yl)-[1,2,4]oxadiazol-5-yl]-piperidin-1-yl}-methanon
(3,4-Difluor-phenyl)-{(S)-3-[3-(1H-imidazol-2-yl)-[1,2,4]oxadiazol-5-yl]-piperidin-1-yl}-methanon
{3-[5-(1H-Indol-2-yl)-[1,2,4]oxadiazol-3-yl]-piperidin-1-yl}-(5-methyl-isoxazol-4-yl)-methanon
(4-Fluor-phenyl)-{(S)-3-[5-(4-methyl-1H-pyrrol-2-yl)-[1,2,4]oxadiazol-3-yl]-piperidin-1-yl}-methanon
(6-Fluor-pyridin-3-yl)-{(S)-3-[5-(4-methyl-1H-pyrrol-2-yl)-[1,2,4]oxadiazol-3-yl]-piperidin-1-yl}-methanon
(5-Methyl-isoxazol-4-yl)-{(S)-3-[5-(4-methyl-1H-pyrrol-2-yl)-[1,2,4]oxadiazol-3-yl]-piperidin-1-yl}-methanon
(2-Fluor-pyridin-4-yl)-{(S)-3-[5-(4-methyl-1H-pyrrol-2-yl)-[1,2,4]oxadiazol-3-yl]-piperidin-1-yl}-methanon
(4-Fluor-phenyl)-{(S)-3-[3-(4-methyl-1H-pyrrol-2-yl)-[1,2,4]oxadiazol-5-yl]-piperidin-1-yl}-methanon
(3,4-Difluor-phenyl)-{(S)-3-[3-(4-methyl-1H-pyrrol-2-yl)-{1,2,4]oxadiazol-5-yl]-piperidin-1-yl}-methanon
(6-Fluor-pyridin-3-yl)-{(S)-3-[3-(4-methyl-1H-pyrrol-2-yl)-[1,2,4]oxadiazol-5-yl]-piperidin-1-yl}-methanon
(2-Fluor-pyridin-4-yl)-{(S)-3-[3-(4-methyl-1H-pyrrol-2-yl)-[1,2,4]oxadiazol-5-yl]-piperidin-1-yl}-methanon
(5-Methyl-isoxazol-4-yl)-{(S)-3-[3-(4-methyl-1H-pyrrol-2-yl)-[1,2,4]oxadiazol-5-yl]-piperidin-1-yl}-methanon
(4-Fluor-phenyl)-{(S)-3-[5-(4-nitro-1H-pyrrol-2-yl)-[1,2,4]oxadiazol-3-yl]-piperidin-1-yl}-methanon
(4-Fluor-phenyl)-{(R)-3-[3-(1H-pyrrol-2-yl)-[1,2,4]oxadiazol-5-yl]-piperidin-1-yl}-methanon
(4-Fluor-phenyl)-{(S)-3-[5-(5-methyl-1H-pyrrol-2-yl)-[1,2,4]oxadiazol-3-yl]-piperidin-1-yl}-methanon
{(S)-3-[5-(4-Chlor-1H-pyrrol-2-yl)-[1,2,4]oxadiazol-3-yl]-piperidin-1-yl}-(4-fluor-phenyl)-methanon
{(S)-3-[5-(4-Chlor-1H-pyrrol-2-yl)-[1,2,4]oxadiazol-3-yl]-piperidin-1-yl}-(6-fluor-pyridin-3-yl)-methanon
{(S)-3-[5-(4-Chlor-1H-pyrrol-2-yl)-[1,2,4]oxadiazol-3-yl]-piperidin-1-yl}-(2-fluor-pyridin-4-yl)-methanon
{(S)-3-[5-(4-Chlor-1H-pyrrol-2-yl)-[1,2,4]oxadiazol-3-yl]-piperidin-1-yl}-(5-methyl-isoxazol-4-yl)-methanon
{(S)-3-[3-(4-Chlor-1H-pyrrol-2-yl)-[1,2,4]oxadiazol-5-yl]-piperidin-1-yl}-(4-fluor-phenyl)-methanon
{(S)-3-[5-(4-Brom-1H-pyrrol-2-yl)-[1,2,4]oxadiazol-3-yl]-piperidin-1-yl}-(6-fluor-pyridin-3-yl)-methanon
{(S)-3-[3-(4-Brom-1H-pyrrol-2-yl)-[1,2,4]oxadiazol-5-yl]-piperidin-1-yl}-(4-fluor-phenyl)-methanon
{(S)-3-[3-(4-Brom-1H-pyrrol-2-yl)-[1,2,4]oxadiazol-5-yl]-piperidin-1-yl}-(6-fluor-pyridin-3-yl)-methanon
{3,3-Dimethyl-5-[3-(1H-pyrrol-2-yl)-[1,2,4]oxadiazol-5-yl]-piperidin-1-yl}-(4-fluor-phenyl)-methanon
(4-Fluor-phenyl)-{(S)-3-[3-(4-fluor-1H-pyrrol-2-yl)-[1,2,4]oxadiazol-5-yl]-piperidin-1-yl}-methanon

(3,4-Difluor-phenyl)-{(S)-3-[3-(4-fluor-1H-pyrrol-2-yl)-[1,2,4]oxadiazol-5-yl]-piperidin-1-yl}-methanon
(6-Fluor-pyridin-3-yl)-{(S)-3-[3-(4-fluor-1H-pyrrol-2-yl)-[1,2,4]oxadiazol-5-yl]-piperidin-1-yl}-methanon
(2-Fluor-pyridin-4-yl)-{(S)-3-[3-(4-fluor-1H-pyrrol-2-yl)-[1,2,4]oxadiazol-5-yl]-piperidin-1-yl}-methanon
(4-Fluor-phenyl)-{(S)-3-[5-(1H-pyrrol-2-yl)-tetrazol-2-yl]-piperidin-1-yl}-methanon
(4-Fluor-phenyl)-{(S)-3-[5-(4-trifluormethyl-1H-imidazol-2-yl)-[1,2,4]oxadiazol-3-yl]-piperidin-1-yl}-methanon
(6-Fluor-pyridin-3-yl)-{(S)-3-[5-(4-isopropyl-1H-pyrrol-2-yl)-[1,2,4]oxadiazol-3-yl]-piperidin-1-yl}-methanon
(3-Fluor-pyridin-4-yl)-{(S)-3-[5-(4-methyl-1H-pyrrol-2-yl)-[1,2,4]oxadiazol-3-yl]-piperidin-1-yl}-methanon
{(S)-3-[5-(4-Chlor-1H-pyrrol-2-yl)-[1,2,4]oxadiazol-3-yl]-piperidin-1-yl}-(3-fluor-pyridin-4-yl)-methanon
(2-Fluor-pyridin-4-yl)-{(S)-3-[5-(4-fluor-1H-pyrrol-2-yl)-[1,2,4]oxadiazol-3-yl]-piperidin-1-yl}-methanon
{(S)-3-[5-(4-Brom-1H-pyrrol-2-yl)-[1,2,4]oxadiazol-3-yl]-piperidin-1-yl}-(3-fluor-pyridin-4-yl)-methanon
(3-Fluor-pyridin-4-yl)-{(S)-3-[5-(4-fluor-1H-pyrrol-2-yl)-[1,2,4]oxadiazol-3-yl]-piperidin-1-yl}-methanon
(4-Fluor-phenyl)-{(S)-3-[5-(4-fluor-1H-pyrrol-2-yl)-[1,2,4]oxadiazol-3-yl]-piperidin-1-yl}-methanon
(6-Fluor-pyridin-3-yl)-{(S)-3-[5-(4-fluor-1H-pyrrol-2-yl)-[1,2,4]oxadiazol-3-yl]-piperidin-1-yl}-methanon
{(S)-3-[3-(4-Chlor-1H-pyrrol-2-yl)-[1,2,4]oxadiazol-5-yl]-piperidin-1-yl}-(6-fluor-pyridin-3-yl)-methanon
{(S)-3-[3-(4-Chlor-1H-pyrrol-2-yl)-[1,2,4]oxadiazol-5-yl]-piperidin-1-yl}-(2-fluor-pyridin-4-yl)-methanon
{(S)-3-[3-(4-Chlor-1H-pyrrol-2-yl)-[1,2,4]oxadiazol-5-yl]-piperidin-1-yl}-(3-fluor-pyridin-4-yl)-methanon
{(S)-3-[3-(4-Chlor-1H-pyrrol-2-yl)-[1,2,4]oxadiazol-5-yl]-piperidin-1-yl}-(5-methyl-isoxazol-4-yl)-methanon
{(S)-3-[3-(4-Brom-1H-pyrrol-2-yl)-[1,2,4]oxadiazol-5-yl]-piperidin-1-yl}-(3-fluor-pyridin-4-yl)-methanon
(3-Fluor-pyridin-4-yl)-{(S)-3-[3-(4-fluor-1H-pyrrol-2-yl)-[1,2,4]oxadiazol-5-yl]-piperidin-1-yl}-methanon
(3-Fluor-pyridin-4-yl)-{(S)-3-[3-(4-methyl-1H-pyrrol-2-yl)-[1,2,4]oxadiazol-5-yl]-piperidin-1-yl}-methanon
(4-Fluor-phenyl)-{(S)-3-[5-(4-trifluormethyl-1H-pyrrol-2-yl)-[1,2,4]oxadiazol-3-yl]-piperidin-1-yl}-methanon
(3-Fluor-pyridin-4-yl)-{(S)-3-[5-(4-trifluormethyl-1H-pyrrol-2-yl)[1,2,4]oxadiazol-3-yl]-piperidin-1-yl}-methanon
(6-Fluor-pyridin-3-yl)-{(S)-3-[5-(4-trifluormethyl-1H-pyrrol-2-yl)-[1,2,4]oxadiazol-3-yl]-piperidin-1-yl}-methanon
(3,4-Difluor-phenyl)-{(S)-3-[3-(4-methyl-1H-imidazol-2-yl)-[1,2,4]oxadiazol-5-yl]-piperidin-1-yl}-methanon
{(S)-3-[5-(4-Chlor-1H-pyrrol-2-yl)-[1,2,4]oxadiazol-3-yl]-piperidin-1-yl}-pyridin-4-yl-methanon
(6-Fluor-pyridin-3-yl)-{(S)-3-[3-(4-trifluormethyl-1H-pyrrol-2-yl)-[1,2,4]oxadiazol-5-yl]-piperidin-1-yl}-methanon;
oder pharmazeutisch akzeptable Salze, Hydrate oder Solvate solcher Verbindungen.

**12.** Pharmazeutische Zusammensetzung, die eine therapeutisch wirksame Menge einer Verbindung nach den Ansprüchen 1 bis 11 und einen pharmazeutisch akzeptablen Träger und/oder Exzipienten umfasst.

**13.** Verbindung/Zusammensetzung nach den Ansprüchen 1 bis 12 zur Behandlung oder Verhütung eines Zustands bei einem Säugetier, einschließlich eines Menschen, dessen Behandlung oder Verhütung durch den neuromodulatorischen Effekt von allosterischen mGluR5-Modulatoren beeinflusst oder erleichtert wird.

**14.** Verbindung/Zusammensetzung nach den Ansprüchen 1 bis 12 zur Behandlung oder Verhütung eines Zustands bei einem Säugetier, einschließlich eines Menschen, dessen Behandlung oder Verhütung durch den neuromodulatorischen Effekt von positiven allosterischen mGluR5-Modulatoren (Enhancer) beeinflusst oder erleichtert wird.

**15.** Verbindung/Zusammensetzung nach den Ansprüchen 1 bis 12 zur Behandlung oder Verhütung von Zentralnervensystemstörungen, ausgewählt aus der Gruppe bestehend aus Angststörungen: Agoraphobie, generalisierte Angststörung (GAD), obsessive Zwangsstörung (OCD), Panikstörung, posttraumatische Belastungsstörung (PTSD), soziale Phobie, andere Phobien, substanzinduzierte Angststörung.

**16.** Verbindung/Zusammensetzung nach den Ansprüchen 1 bis 12 zur Behandlung oder Verhütung von Zentralnervensystemstörungen, ausgewählt aus der Gruppe bestehend aus Kindheitsstörungen: Aufmerksamkeitsdefizit/Hyperaktivitätsstörung.

**17.** Verbindung/Zusammensetzung nach den Ansprüchen 1 bis 12 zur Behandlung oder Verhütung von Zentralnervensystemstörungen, ausgewählt aus der Gruppe bestehend aus Essstörungen (Magersucht, Ess-Brech-Sucht).

**18.** Verbindung/Zusammensetzung nach den Ansprüchen 1 bis 12 zur Behandlung oder Verhütung von Zentralnervensystemstörungen, ausgewählt aus der Gruppe bestehend aus Gemütszustandsstörungen: bipolare Störungen (I & II), zyklothyme Störung, Depressionen, dysthyme Störung, majore Depressionsstörung, substanzinduzierte Gemütszustandsstörung.

**19.** Verbindung/Zusammensetzung nach den Ansprüchen 1 bis 12 zur Behandlung oder Verhütung von Zentralnervensystemstörungen, ausgewählt aus der Gruppe bestehend aus psychotischen Störungen: Schizoprenie, Wahnstörung, schizoaffektive Störung, schizophreniforme Störung, substanzinduzierte psychotische Störung.

**20.** Verbindung/Zusammensetzung nach den Ansprüchen 1 bis 12 zur Behandlung oder Verhütung von Zentralnervensystemstörungen, ausgewählt aus der Gruppe bestehend aus kognitiven Störungen: Delirium, substanzinduziertes anhaltendes Delirium, Demenz, Demenz infolge der HIV-Krankheit, Demenz infolge der Huntington-Krankheit, Demenz infolge der Parkinson-Krankheit, Demenz vom Alzheimer-Typ, substanzinduzierte anhaltende Demenz, leichte kognitive Beeinträchtigung.

**21.** Verbindung/Zusammensetzung nach den Ansprüchen 1 bis 12 zur Behandlung oder Verhütung von Zentralnervensystemstörungen, ausgewählt aus der Gruppe bestehend aus Persönlichkeitsstörungen:

Zwangspersönlichkeitsstörung, schizoide, schizotypische Störung.

**22.** Verbindung/Zusammensetzung nach den Ansprüchen 1 bis 12 zur Behandlung oder Verhütung von Zentralnervensystemstörungen, ausgewählt aus der Gruppe bestehend aus substanzbedingten Störungen:

Alkoholmissbrauch, Alkoholabhängigkeit, Alkoholentzug, Alkoholentzugsdelirium, Alkohol-induzierte psychotische Störung, Amphetaminabhängigkeit, Amphetaminentzug, Kokainabhängigkeit, Kokainentzug, Nikotinabhängigkeit, Nikotinentzug, Opioidabhängigkeit, Opioidentzug.

**23.** Verbindung/Zusammensetzung nach den Ansprüchen 1 bis 12 zur Behandlung oder Verhütung von entzündlichen zentralnervensystemstörungen, ausgewählt aus Formen der Multiplen Sklerose wie benigne Multiple Sklerose, schubförmig remittierende Multiple Sklerose, sekundärprogressive Multiple Sklerose, primär-progressive Multiple Sklerose.

**24.** Verwendung einer Verbindung/Zusammensetzung nach den Ansprüchen 1 bis 12 zur Herstellung eines Medikaments zur Behandlung oder Verhütung wie in einem der Ansprüche 15 bis 23 definiert.

**25.** Verwendung der Verbindungen nach Anspruch 1 zur Herstellung von Tracern zur Abbildung von metabotropen Glutamatrezeptoren.

## Revendications

**1.** Composé ayant la formule I-B

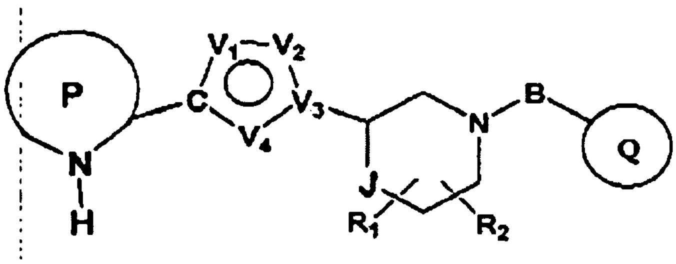

I-B
dans lequel

$R_1$ et $R_2$ représentent indépendamment hydrogène, alkyle en ($C_1$-$C_6$), alkényle en ($C_2$-$C_6$), alkynyle en ($C_2$-$C_6$), arylalkyle, hétéroarylalkyle, hydroxy, amino, aminoalkyle, hydroxyalkyle ou alkoxy en ($C_1$-$C_6$), ou $R_1$ et $R_2$ peuvent ensemble former un noyau cycloalkyle en ($C_3$-$C_7$), une liaison carbonyle C=O ou une double liaison avec un carbone ;
P représente un noyau hétérocycloalkyle en ($C_5$-$C_7$) ou hétérocycloalkényle en ($C_5$-$C_7$) ou un groupement hétéroaryle de formule

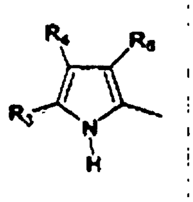 , 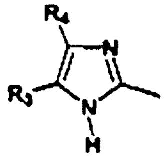 , 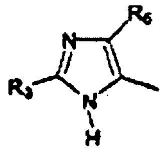 , 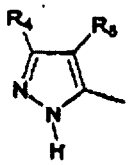 , 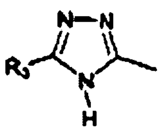 ,

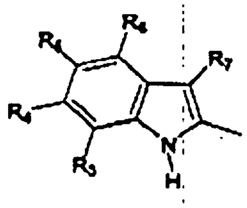 , 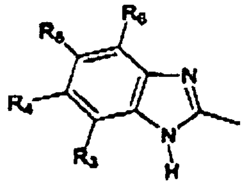 , 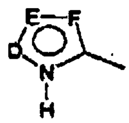 , 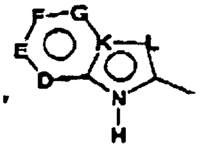 .

$R_3$, $R_4$, $R_5$, $R_6$ et $R_7$ sont chacun un substituant indépendamment sélectionné parmi hydrogène, halogène, $-NO_2$, alkyle en ($C_1$-$C_6$), cycloalkyle en ($C_3$-$C_6$), cycloalkylalkyle en ($C_3$-$C_7$), alkényle en ($C_2$-$C_6$), alkynyle en ($C_2$-$C_6$), halo-alkyle en ($C_1$-$C_6$), hétéroaryle, hétéroarylalkyle, arylalkyle, aryle, $-OR_8$, $-NR_8R_9$, $-C(=NR_{10})NR_8R_9$, $-NR_8COR_9$, $NR_8CO_2R_9$, $NR_8SO_2R_9$, $-NR_{10}CO\ NR_8R_9$, $-SR_8$, $-S(=O)R_8$, $-S(=O)_2R_8$, $-S(=O)_2NR_8R_9$, $-C(=O)R_8$, $-C(O)-O-R_8$, $-C(=O)NR_8R_9$, $-C(=NR_8)R_9$ ou $C(=NOR_8)R_9$ ; dans lequel, éventuellement, deux substituants sont combinés aux atomes intervenants pour former un noyau hétérocycloalkyle, aryle ou hétéroaryle bicyclique ; dans lequel chaque noyau est éventuellement en outre substitué par 1-5 groupements indépendamment sélectionnés parmi halogène, -CN, alkyle en ($C_1$-$C_6$), -O-alkyle en ($C_0$-$C_6$), -O-cycloalkylalkyle en ($C_3$-$C_7$), -O-aryle, -O-hétéroaryle, -O-alkyle en ($C_1$-$C_3$)aryle, -O-alkyle en ($C_1$-$C_3$)hétéroaryle, -N[alkyle en ($C_0$-$C_6$)][alkyle en ($C_0$-$C_3$)aryle] ou -N[alkyle en ($C_0$-$C_6$)][alkyle en ($C_0$-$C_3$)hétéroaryle] ;

$R_8$, $R_9$ et $R_{10}$ sont chacun indépendamment hydrogène, alkyle en ($C_1$-$C_6$), cycloalkyle en ($C_3$-$C_6$), cycloalkylalkyle en ($C_3$-$C_7$), alkényle en ($C_2$-$C_6$), alkynyle en ($C_2$-$C_6$), halo-alkyle en ($C_1$-$C_6$), hétérocycloalkyle, hétéroaryle, hétéroarylalkyle, arylalkyle ou aryle ; l'un quelconque de ceux-ci étant éventuellement substitué par 1-5 substituants indépendamment sélectionnés parmi halogène, -CN, alkyle en ($C_1$-$C_6$), -O-alkyle en ($C_0$-$C_6$), -O-cycloalkylalkyle en ($C_3$-$C_7$), -O-aryle, -O-hétéroaryle, -N-[alkyle en ($C_0$-$C_6$)]$_2$, -N-[alkyle en ($C_0$-$C_6$)][cycloalkyle en ($C_3$-$C_7$)] ou -N-[alkyle en ($C_0$-$C_6$)-aryle] ;

D, E, F, G, K et L dans P représentent indépendamment $-C(R_3)=$, $-C(R_3)=C(R_4)-$, -C(=O)-, -C(=S)-, -O-, -N=, $-N(R_3)-$ ou -S- ;

Q indique un cycloalkyle en ($C_5$-$C_6$), un aryle ou un groupement hétéroaryle de formule

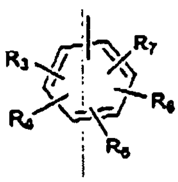 , 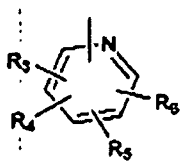 , 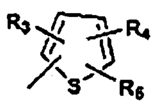 , 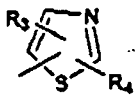 , 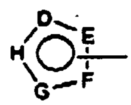

$R_3$, $R_4$, $R_5$, $R_6$ et $R_7$ sont indépendamment tels que définis ci-dessus ;

D, E, F, G et H dans Q représentent indépendamment $-C(R_3)=$, $-C(R_3)=C(R_4)-$, -C(=O)-, -C(=S)-, -O-, -N=, -N ($R_3$)- ou -S- ; $V_1$, $V_2$ et $V_4$ représentent indépendamment -O- ou -N= et $V_3$ est C, ou $V_1$, $V_2$ et $V_4$ représentent -N= et $V_3$ est -N- ;

B représente une simple liaison, -C(=O)-alkyle en ($C_0$-$C_2$), -C(=O)-alkényle en ($C_2$-$C_6$), -C(=O)-alkynyle en ($C_2$-$C_6$), -C(=O)-O-, $-C(=O)NR_8$-alkyle en ($C_0$-$C_2$), $-C(=NR_8)NR_9$, -S(=O)-alkyle en ($C_0$-$C_2$), $-S(=O)_2$-alkyle en ($C_0$-$C_2$), - $S(=O)_2NR_8$-alkyle en ($C_0$-$C_2$), $C(=NR_8)$-alkyle en ($C_0$-$C_2$), $-C(=NOR_8)$-alkyle en ($C_0$-$C_2$) ou $-C(=NOR_8)$ $NR_9$-alkyle en ($C_0$-$C_2$) ;

$R_8$ et $R_9$ sont indépendamment tels que définis ci-dessus ;

J représente $-C(R_{10}, R_{11})$, -O-, $-N(R_{10})-$ ou -S- ;

$R_{10}$, $R_{11}$ sont indépendamment hydrogène, alkyle en ($C_1$-$C_6$), cycloalkyle en ($C_3$-$C_6$), cycloalkylalkyle en ($C_3$-$C_7$), alkényle en ($C_2$-$C_6$), alkynyle en ($C_2$-$C_6$), halo-alkyle en ($C_1$-$C_6$), hétéroaryle, hétéroarylalkyle, arylalkyle ou aryle ; l'un quelconque de ceux-ci étant éventuellement substitué par 1-5 substituants indépendamment sélectionnés parmi halogène, -CN, alkyle en ($C_1$-$C_6$), -O-alkyle en ($C_0$-$C_6$), -O-cycloalkylalkyle en ($C_3$-$C_7$), -O-aryle, -O-hétéroaryle, - N[alkyle en ($C_0$-$C_6$)][alkyle en ($C_0$-$C_6$)], -N[alkyle en ($C_0$-$C_6$)][cycloalkyle en ($C_3$-$C_7$)] ou -N [alkyle en ($C_0$-$C_6$)]-aryle] ;
Tout N peut être un N-oxyde ;

ou des sels, hydrates ou solvates pharmaceutiquement acceptables de tels composés.

**2.** Composé selon la revendication 1 ayant la formule I-C

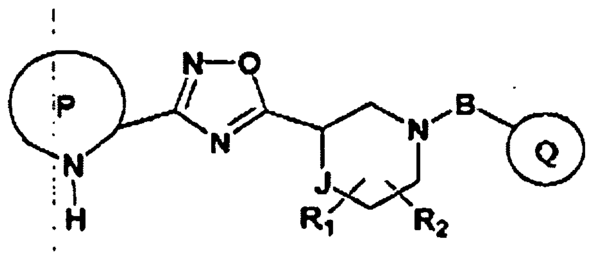

I-C

dans lequel

$R_1$ et $R_2$ représentent indépendamment hydrogène, alkyle en ($C_1$-$C_6$), alkényle en ($C_2$-$C_6$), alkynyle en ($C_2$-$C_6$), arylalkyle, hétéroarylalkyle, hydroxy, amino, aminoalkyle, hydroxyalkyle ou alkoxy en ($C_1$-$C_6$), ou $R_1$ et $R_2$ peuvent ensemble former un noyau cycloalkyle en ($C_3$-$C_7$), une liaison carbonyle C=O ou une double liaison avec un carbone ;
P représente un noyau hétérocycloalkyle en ($C_5$-$C_7$) ou hétérocycloalkényle en ($C_5$-$C_7$) ou un groupement hétéroaryle de formule

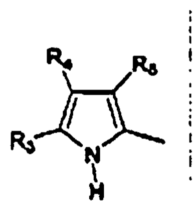 , 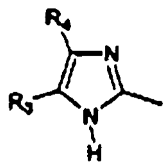 , 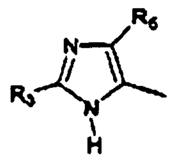 , 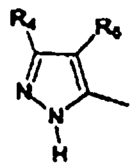 , 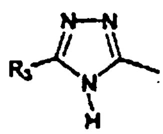 ,

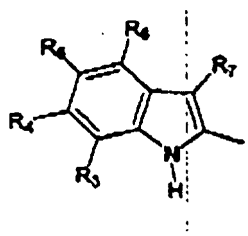 , 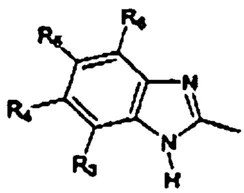 , 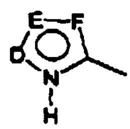 , 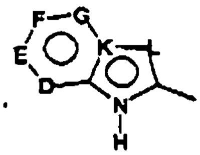 .

$R_3$, $R_4$, $R_5$, $R_6$ et $R_7$ sont chacun un substituant indépendamment sélectionné parmi hydrogène, halogène, -$NO_2$, alkyle en ($C_1$-$C_6$), cycloalkyle en ($C_3$-$C_6$), cycloalkylalkyle en ($C_3$-$C_7$), alkényle en ($C_2$-$C_6$), alkynyle en ($C_2$-$C_6$), halo-alkyle en ($C_1$-$C_6$), hétéroaryle, hétéroarylalkyle, arylalkyle, aryle, -$OR_8$, -$NR_8R_9$, -C(=$NR_{10}$)$NR_8R_9$, -$NR_8COR_9$, $NR_8CO_2R_9$, $NR_8SO_2R_9$, -$NR_{10}$CO $NR_8R_9$, -$SR_8$, -S(=O)$R_8$, -S(=O)$_2R_8$, -S(=O)$_2NR_8R_9$, -C(=O)$R_8$, -C(O)-O-$R_8$, -C(=O)$NR_8R_9$, -C(=$NR_8$)$R_9$ ou C(=$NOR_8$)$R_9$ ; dans lequel, éventuellement, deux substituants sont combinés aux atomes intervenants pour former un noyau hétérocycloalkyle, aryle ou hétéroaryle bicyclique ; dans lequel chaque noyau est éventuellement en outre substitué par 1-5 groupements indépendamment sélectionnés parmi halogène, -CN, alkyle en ($C_1$-$C_6$), -O-alkyle en ($C_0$-$C_6$), -O-cycloalkylalkyle en ($C_3$-$C_7$), -O-aryle, -O-hétéroaryle, -O-alkyle en ($C_1$-$C_3$)aryle, -O-alkyle en ($C_1$-$C_3$)hétéroaryle, -N[alkyle en ($C_0$-$C_6$)][alkyle en ($C_0$-$C_3$)aryle] ou -N[alkyle en ($C_0$-$C_6$)][alkyle en ($C_0$-$C_3$)hétéroaryle] ;
$R_8$, $R_9$ et $R_{10}$ sont chacun indépendamment hydrogène, alkyle en ($C_1$-$C_6$), cycloalkyle en ($C_3$-$C_6$), cycloalky-

lalkyle en ($C_3$-$C_7$), alkényle en ($C_2$-$C_6$), alkynyle en ($C_2$-$C_6$), halo-alkyle en ($C_1$-$C_6$), hétérocycloalkyle, hétéroaryle, hétéroarylalkyle, arylalkyle ou aryle ; l'un quelconque de ceux-ci étant éventuellement substitué par 1-5 substituants indépendamment sélectionnés parmi halogène, -CN, alkyle en ($C_1$-$C_6$), -O-alkyle en ($C_0$-$C_6$), -O-cycloalkylalkyle en ($C_3$-$C_7$), -O-aryle, -O-hétéroaryle, -N-[alkyle en ($C_0$-$C_6$)]$_2$, -N-[alkyle en ($C_0$-$C_6$)][cycloalkyle en ($C_3$-$C_7$)] ou -N-[alkyle en ($C_0$-$C_6$)-aryle] ;

D, E, F, G, K et L dans P représentent indépendamment -C($R_3$)=, -C($R_3$)=C($R_4$)-, -C(=O)-, -C(=S)-, -O-, -N=, -N($R_3$)- ou -S- ;

Q indique un aryle ou un groupement hétéroaryle de formule

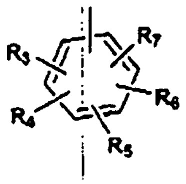 . 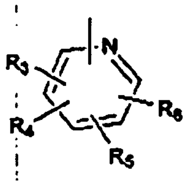 . 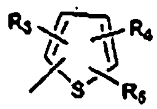 , 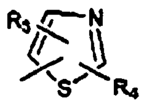 , 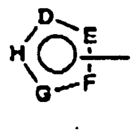

$R_3$, $R_4$, $R_5$, $R_6$ et $R_7$ sont indépendamment tels que définis ci-dessus ;

D, E, F, G et H dans Q représentent indépendamment -C($R_3$)=, -C($R_3$)=C($R_4$)-, -C(=O)-, -C(=S)-, -O-, -N=, -N($R_3$)- ou -S- ;

B représente une simple liaison, -C(=O)-alkyle en ($C_0$-$C_2$), -C(=O)-alkényle en ($C_2$-$C_6$), -C(=O)-alkynyle en ($C_2$-$C_6$), -C(=O)-O-, -C(=O)N$R_8$-alkyle en ($C_0$-$C_2$), -C(=N$R_8$)N$R_9$, -S(=O)-alkyle en ($C_0$-$C_2$), -S(=O)$_2$-alkyle en ($C_0$-$C_2$), - S(=O)$_2$N$R_8$-alkyle en ($C_0$-$C_2$), C(=N$R_8$)-alkyle en ($C_0$-$C_2$), -C(=NO$R_8$)-alkyle en ($C_0$-$C_2$) ou -C(=NO$R_8$)N$R_9$-alkyle en ($C_0$-$C_2$) ;

$R_8$ et $R_9$ sont indépendamment tels que définis ci-dessus ;

J représente -C($R_{10}$, $R_{11}$), -O-, -N($R_{10}$)- ou -S- ;

$R_{10}$, $R_{11}$ sont indépendamment hydrogène, alkyle en ($C_1$-$C_6$), cycloalkyle en ($C_3$-$C_6$), cycloalkylalkyle en ($C_3$-$C_7$), alkényle en ($C_2$-$C_6$), alkynyle en ($C_2$-$C_6$), halo-alkyle en ($C_1$-$C_6$), hétéroaryle, hétéroarylalkyle, arylalkyle ou aryle ; l'un quelconque de ceux-ci étant éventuellement substitué par 1-5 substituants indépendamment sélectionnés parmi halogène, -CN, alkyle en ($C_1$-$C_6$), -O-alkyle en ($C_0$-$C_6$), -O-cycloalkylalkyle en ($C_3$-$C_7$), -O-aryle, -O-hétéroaryle, - N[alkyle en ($C_0$-$C_6$)][alkyle en ($C_0$-$C_6$)], -N[alkyle en ($C_0$-$C_6$)][cycloalkyle en ($C_3$-$C_7$)] ou -N[alkyle en ($C_0$-$C_6$)]-aryle] ;

Tout N peut être un N-oxyde ;

ou des sels, hydrates ou solvates pharmaceutiquement acceptables de tels composés.

**3.** Composé selon la revendication 1 ou 2 ayant la formule I-D

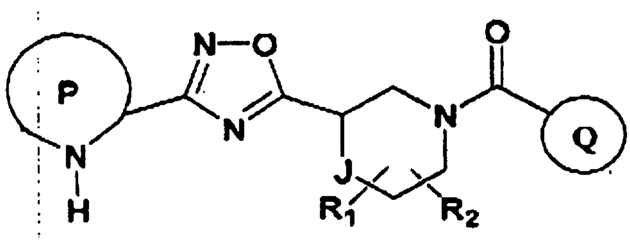

I-D

dans lequel

$R_1$ et $R_2$ représentent indépendamment hydrogène, alkyle en ($C_1$-$C_6$), alkényle en ($C_2$-$C_6$), alkynyle en ($C_2$-$C_6$), arylalkyle, hétéroarylalkyle, hydroxy, amino, aminoalkyle, hydroxyalkyle ou alkoxy en ($C_1$-$C_6$), ou $R_1$ et $R_2$ peuvent ensemble former un noyau cycloalkyle en ($C_3$-$C_7$), une liaison carbonyle C=O ou une double liaison

avec un carbone ;

P représente un noyau hétérocycloalkyle en ($C_5$-$C_7$) ou hétérocycloalkényle en ($C_5$-$C_7$) ou un groupement hétéroaryle de formule

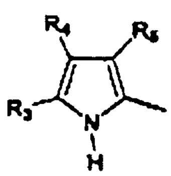 , 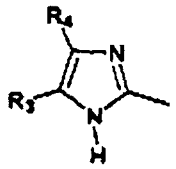 , 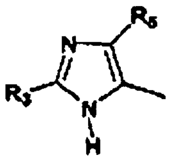 , 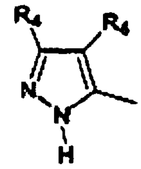 , 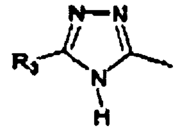 ,

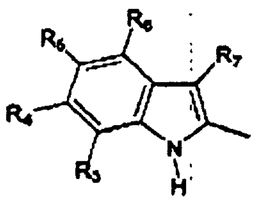 , 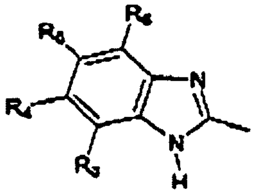 , 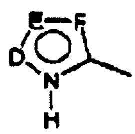 , 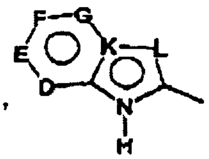 .

$R_3$, $R_4$, $R_5$, $R_6$ et $R_7$ sont chacun un substituant indépendamment sélectionné parmi hydrogène, halogène, -$NO_2$, alkyle en ($C_1$-$C_6$), cycloalkyle en ($C_3$-$C_6$), cycloalkylalkyle en ($C_3$-$C_7$), alkényle en ($C_2$-$C_6$), alkynyle en ($C_2$-$C_6$), halo-alkyle en ($C_1$-$C_6$), hétéroaryle, hétéroarylalkyle, arylalkyle, aryle, -$OR_8$, -$NR_8R_9$, -C(=$NR_{10}$)$NR_8R_9$, -$NR_8COR_9$, $NR_8CO_2R_9$, $NR_8SO_2R_9$, -$NR_{10}$CO $NR_8R_9$, -$SR_8$, -S(=O)$R_8$, -S(=O)$_2R_8$, -S(=O)$_2NR_8R_9$, -C(=O)$R_8$, -C(O)-O-$R_8$, -C(=O)$NR_8R_9$, -C(=$NR_8$)$R_9$ ou C(=$NOR_8$)$R_9$ ; dans lequel, éventuellement, deux substituants sont combinés aux atomes intervenants pour former un noyau hétérocycloalkyle, aryle ou hétéroaryle bicyclique ; dans lequel chaque noyau est éventuellement en outre substitué par 1-5 groupements indépendamment sélectionnés parmi halogène, -CN, alkyle en ($C_1$-$C_6$), -O-alkyle en ($C_0$-$C_6$), -O-cycloalkylalkyle en ($C_3$-$C_7$), -O-aryle, -O-hétéroaryle, -O-alkyle en ($C_1$-$C_3$)aryle, -O-alkyle en ($C_1$-$C_3$)hétéroaryle, -N[alkyle en ($C_0$-$C_6$)][alkyle en ($C_0$-$C_3$)aryle] ou -N[alkyle en ($C_0$-$C_6$)][alkyle en ($C_0$-$C_3$)hétéroaryle] ;

$R_8$, $R_9$ et $R_{10}$ sont chacun indépendamment hydrogène, alkyle en ($C_1$-$C_6$), cycloalkyle en ($C_3$-$C_6$), cycloalkylalkyle en ($C_3$-$C_7$), alkényle en ($C_2$-$C_6$), alkynyle en ($C_2$-$C_6$), halo-alkyle en ($C_1$-$C_6$), hétérocycloalkyle, hétéroaryle, hétéroarylalkyle, arylalkyle ou aryle ; l'un quelconque de ceux-ci étant éventuellement substitué par 1-5 substituants indépendamment sélectionnés parmi halogène, -CN, alkyle en ($C_1$-$C_6$), -O-alkyle en ($C_0$-$C_6$), -O-cycloalkylalkyle en ($C_3$-$C_7$), -O-aryle, -O-hétéroaryle, -N-[alkyle en ($C_0$-$C_6$)]$_2$, -N-[alkyle en ($C_0$-$C_6$)][cycloalkyle en ($C_3$-$C_7$)] ou -N-[alkyle en ($C_0$-$C_6$)-aryle] ;

D, E, F, G, K et L dans P représentent indépendamment -C($R_3$)=, -C($R_3$)=C($R_4$)-, -C(=O)-, -C(=S)-, -O-, -N=, -N($R_3$)- ou -S- ;

Q indique un aryle ou un groupement hétéroaryle de formule

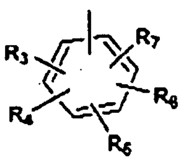 , 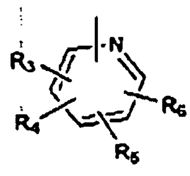 , 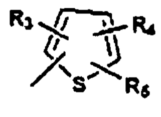 , 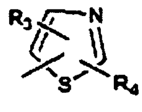 , 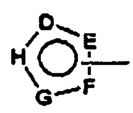

$R_3$, $R_4$, $R_5$, $R_6$ et $R_7$ sont indépendamment tels que définis ci-dessus ;

D, E, F, G et H dans Q représentent indépendamment -C($R_3$)=, -C($R_3$)=C($R_4$)-, -C(=O)-, -C(=S)-, -O-, -N=, -N ($R_3$)- ou -S- ;

J représente -C($R_{10}$, $R_{11}$), -O-, -N($R_{10}$)- ou -S- ;

$R_{10}$, $R_{11}$ sont indépendamment hydrogène, alkyle en ($C_1$-$C_6$), cycloalkyle en ($C_3$-$C_6$), cycloalkylalkyle en ($C_3$-$C_7$), alkényle en ($C_2$-$C_6$), alkynyle en ($C_2$-$C_6$), halo-alkyle en ($C_1$-$C_6$), hétéroaryle, hétéroarylalkyle, arylalkyle ou aryle ; l'un quelconque de ceux-ci étant éventuellement substitué par 1-5 substituants indépendamment sélectionnés parmi halogène, -CN, alkyle en ($C_1$-$C_6$), -O-alkyle en ($C_0$-$C_6$), -O-cycloalkylalkyle en ($C_3$-$C_7$), -O-aryle, -O-hétéroaryle, - N[alkyle en ($C_0$-$C_6$)][alkyle en ($C_0$-$C_6$)], -N[alkyle en ($C_0$-$C_6$)][cycloalkyle en ($C_3$-$C_7$)] ou -N

[alkyle en ($C_0$-$C_6$)]-aryle] ;
Tout N peut être un N-oxyde ;

ou des sels, hydrates ou solvates pharmaceutiquement acceptables de tels composés.

**4.** Composé selon la revendication 1 ayant la formule II-A

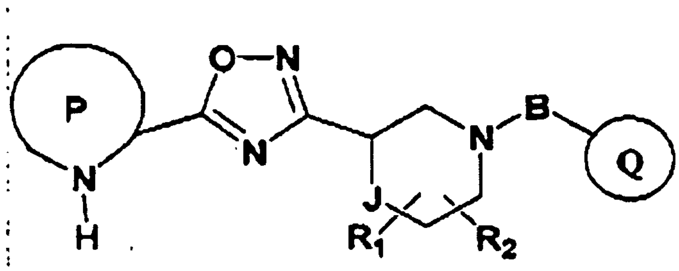

II-A

dans lequel

$R_1$ et $R_2$ représentent indépendamment hydrogène, alkyle en ($C_1$-$C_6$), alkényle en ($C_2$-$C_6$), alkynyle en ($C_2$-$C_6$), arylalkyle, hétéroarylalkyle, hydroxy, amino, aminoalkyle, hydroxyalkyle ou alkoxy en ($C_1$-$C_6$), ou $R_1$ et $R_2$ peuvent ensemble former un noyau cycloalkyle en ($C_3$-$C_7$), une liaison carbonyle C=O ou une double liaison avec un carbone ;
P représente un noyau hétérocycloalkyle en ($C_5$-$C_7$) ou hétérocycloalkényle en ($C_5$-$C_7$) ou un groupement hétéroaryle de formule

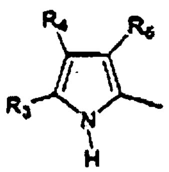 , 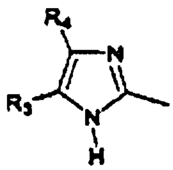 , 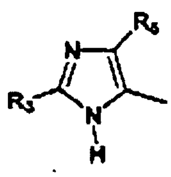 , 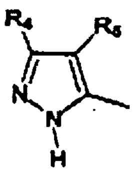 , 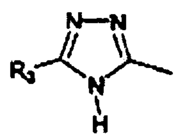 ,

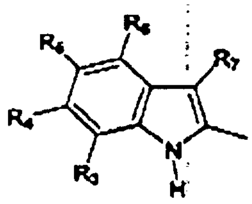 , 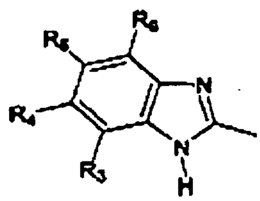 , 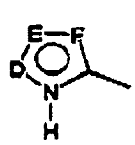 , 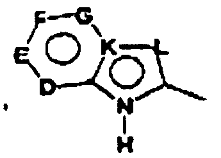 .

$R_3$, $R_4$, $R_5$, $R_6$ et $R_7$ sont chacun un substituant indépendamment sélectionné parmi hydrogène, halogène, $-NO_2$, alkyle en ($C_1$-$C_6$), cycloalkyle en ($C_3$-$C_6$), cycloalkylalkyle en ($C_3$-$C_7$), alkényle en ($C_2$-$C_6$), alkynyle en ($C_2$-$C_6$), halo-alkyle en ($C_1$-$C_6$), hétéroaryle, hétéroarylalkyle, arylalkyle, aryle, $-OR_8$, $-NR_8R_9$, $-C(=NR_{10})NR_8R_9$, $-NR_8COR_9$, $NR_8CO_2R_9$, $NR_8SO_2R_9$, $-NR_{10}CO\ NR_8R_9$, $-SR_8$, $-S(=O)R_8$, $-S(=O)_2R_8$, $-S(=O)_2NR_8R_9$, $-C(=O)R_8$, $-C(O)-O-R_8$, $-C(=O)NR_8R_9$, $-C(=NR_8)R_9$ ou $C(=NOR_8)R_9$ ; dans lequel, éventuellement, deux substituants sont combinés aux atomes intervenants pour former un noyau hétérocycloalkyle, aryle ou hétéroaryle bicyclique ; dans lequel chaque noyau est éventuellement en outre substitué par 1-5 groupements indépendamment sélectionnés parmi halogène, -CN, alkyle en ($C_1$-$C_6$),- O-alkyle en ($C_0$-$C_6$), -O-cycloalkylalkyle en ($C_3$-$C_7$), -O-aryle, -O-hétéroaryle, -O-alkyle en ($C_1$-$C_3$)aryle, -O-alkyle en ($C_1$-$C_3$)hétéroaryle, -N[alkyle en ($C_0$-$C_6$)][alkyle en ($C_0$-$C_3$)aryle] ou -N[alkyle en ($C_0$-$C_6$)][alkyle en ($C_0$-$C_3$)hétéroaryle] ;
$R_8$, $R_9$ et $R_{10}$ sont chacun indépendamment hydrogène, alkyle en ($C_1$-$C_6$), cycloalkyle en ($C_3$-$C_6$), cycloalkylalkyle en ($C_3$-$C_7$), alkényle en ($C_2$-$C_6$), alkynyle en ($C_2$-$C_6$), halo-alkyle en ($C_1$-$C_6$), hétérocycloalkyle, hétéroaryle, hétéroarylalkyle, arylalkyle ou aryle ; l'un quelconque de ceux-ci étant éventuellement substitué par 1-5 substituants indépendamment sélectionnés parmi halogène, -CN, alkyle en ($C_1$-$C_6$), -O-alkyle en ($C_0$-$C_6$), -O-

cycloalkylalkyle en ($C_3$-$C_7$), -O-aryle, -O-hétéroaryle, -N-[alkyle en ($C_0$-$C_6$)]$_2$, -N-[alkyle en ($C_0$-$C_6$)][cycloalkyle en ($C_3$-$C_7$)] ou -N-[alkyle en ($C_0$-$C_6$)-aryle] ;

D, E, F, G, K et L dans P représentent indépendamment -C($R_3$)=, -C($R_3$)=C($R_4$)-, -C(=O)-, -C(=S)-, -O-, -N=, -N($R_3$)- ou -S- ;

Q indique un aryle ou un groupement hétéroaryle de formule

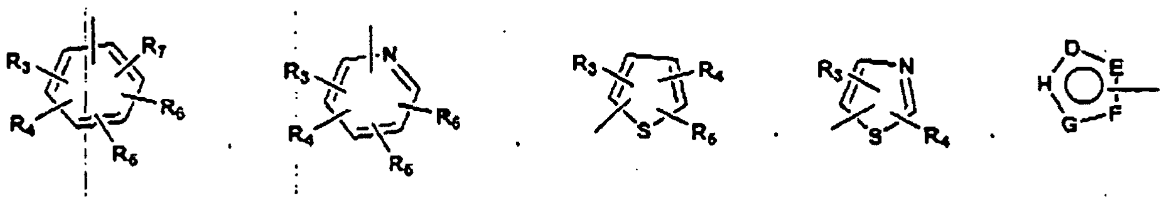

$R_3$, $R_4$, $R_5$, $R_6$ et $R_7$ sont indépendamment tels que définis ci-dessus ;

D, E, F, G et H dans Q représentent indépendamment -C($R_3$)=, -C($R_3$)=C($R_4$)-, -C(=O)-, -C(=S)-, -O-, -N=, -N ($R_3$)- ou -S- ;

B représente une simple liaison, -C(=O)-alkyle en ($C_0$-$C_2$), -C(=O)-alkényle en ($C_2$-$C_6$), -C(=O)-alkynyle en ($C_2$-$C_6$), -C(=O)-O-, -C(=O)NR$_8$-alkyle en ($C_0$-$C_2$), -C(=NR$_8$)NR$_9$, -S(=O)-alkyle en ($C_0$-$C_2$), -S(=O)$_2$-alkyle en ($C_0$-$C_2$), - S(=O)$_2$NR$_8$-alkyle en ($C_0$-$C_2$), C(=NR$_8$)-alkyle en ($C_0$-$C_2$), -C(=NOR$_8$)-alkyle en ($C_0$-$C_2$) ou -C(=NOR$_8$) NR$_9$-alkyle en ($C_0$-$C_2$) ;

$R_8$ et $R_9$ sont indépendamment tels que définis ci-dessus ;

J représente -C($R_{10}$, $R_{11}$), -O-, -N($R_{10}$)- ou -S- ;

$R_{10}$, $R_{11}$ sont indépendamment hydrogène, alkyle en ($C_1$-$C_6$), cycloalkyle en ($C_3$-$C_6$), cycloalkylalkyle en ($C_3$-$C_7$), alkényle en ($C_2$-$C_6$), alkynyle en ($C_2$-$C_6$), halo-alkyle en ($C_1$-$C_6$), hétéroaryle, hétéroarylalkyle, arylalkyle ou aryle ; l'un quelconque de ceux-ci étant éventuellement substitué par 1-5 substituants indépendamment sélectionnés parmi halogène, -CN, alkyle en ($C_1$-$C_6$), -O-alkyle en ($C_0$-$C_6$), -O-cycloalkylalkyle en ($C_3$-$C_7$), -O-aryle, -O-hétéroaryle, - N[alkyle en ($C_0$-$C_6$)][alkyle en ($C_0$-$C_6$)], -N[alkyle en ($C_0$-$C_6$)][cycloalkyle en ($C_3$-$C_7$)] ou -N [alkyle en ($C_0$-$C_6$)]-aryle] ;

Tout N peut être un N-oxyde ;

ou des sels, hydrates ou solvates pharmaceutiquement acceptables de tels composés.

**5.** Composé selon la revendication 1 ayant la formule II-B

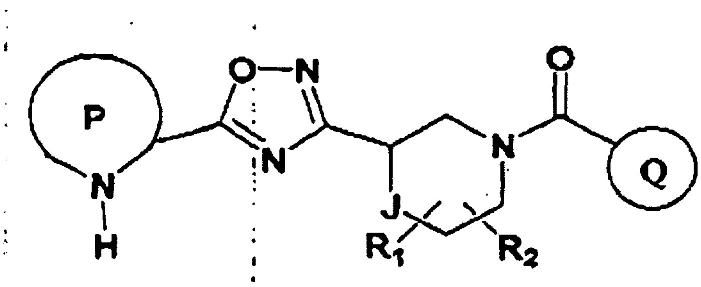

II-B

dans lequel

$R_1$ et $R_2$ représentent indépendamment hydrogène, alkyle en ($C_1$-$C_6$), alkényle en ($C_2$-$C_6$), alkynyle en ($C_2$-$C_6$), arylalkyle, hétéroarylalkyle, hydroxy, amino, aminoalkyle, hydroxyalkyle ou alkoxy en ($C_1$-$C_6$), ou $R_1$ et $R_2$ peuvent ensemble former un noyau cycloalkyle en ($C_3$-$C_7$), une liaison carbonyle C=O ou une double liaison avec un carbone ;

P représente un noyau hétérocycloalkyle en ($C_5$-$C_7$) ou hétérocycloalkényle en ($C_5$-$C_7$) ou un groupement hétéroaryle de formule

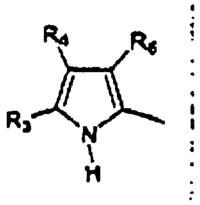, 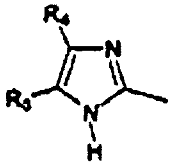, 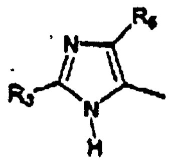, 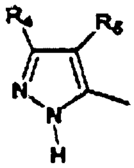, 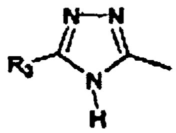,

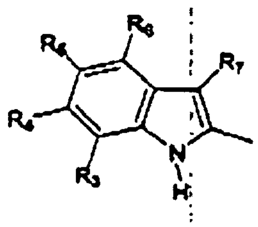, 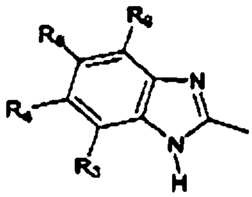, 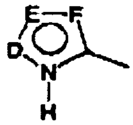, 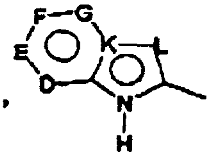.

$R_3$, $R_4$, $R_5$, $R_6$ et $R_7$ sont chacun un substituant indépendamment sélectionné parmi hydrogène, halogène, $-NO_2$, alkyle en ($C_1$-$C_6$), cycloalkyle en ($C_3$-$C_6$), cycloalkylalkyle en ($C_3$-$C_7$), alkényle en ($C_2$-$C_6$), alkynyle en ($C_2$-$C_6$), halo-alkyle en ($C_1$-$C_6$), hétéroaryle, hétéroarylalkyle, arylalkyle, aryle, $-OR_8$, $-NR_8R_9$, $-C(=NR_{10})NR_8R_9$, $-NR_8COR_9$, $NR_8CO_2R_9$, $NR_8SO_2R_9$, $-NR_{10}CO\ NR_8R_9$, $-SR_8$, $-S(=O)R_8$, $-S(=O)_2R_8$, $-S(=O)_2NR_8R_9$, $-C(=O)R_8$, $-C(O)-O-R_8$, $-C(=O)NR_8R_9$, $-C(=NR_8)R_9$ ou $C(=NOR_8)R_9$ ; dans lequel, éventuellement, deux substituants sont combinés aux atomes intervenants pour former un noyau hétérocycloalkyle, aryle ou hétéroaryle bicyclique ; dans lequel chaque noyau est éventuellement en outre substitué par 1-5 groupements indépendamment sélectionnés parmi halogène, -CN, alkyle en ($C_1$-$C_6$), -O-alkyle en ($C_0$-$C_6$), -O-cycloalkylalkyle en ($C_3$-$C_7$), -O-aryle, -O-hétéroaryle, -O-alkyle en ($C_1$-$C_3$)aryle, -O-alkyle en ($C_1$-$C_3$)hétéroaryle, -N[alkyle en ($C_0$-$C_6$)][alkyle en ($C_0$-$C_3$)aryle] ou -N[alkyle en ($C_0$-$C_6$)][alkyle en ($C_0$-$C_3$)hétéroaryle] ;

$R_8$, $R_9$ et $R_{10}$ sont chacun indépendamment hydrogène, alkyle en ($C_1$-$C_6$), cycloalkyle en ($C_3$-$C_6$), cycloalkylalkyle en ($C_3$-$C_7$), alkényle en ($C_2$-$C_6$), alkynyle en ($C_2$-$C_6$), halo-alkyle en ($C_1$-$C_6$), hétérocycloalkyle, hétéroaryle, hétéroarylalkyle, arylalkyle ou aryle ; l'un quelconque de ceux-ci étant éventuellement substitué par 1-5 substituants indépendamment sélectionnés parmi halogène, -CN, alkyle en ($C_1$-$C_6$), -O-alkyle en ($C_0$-$C_6$), -O-cycloalkylalkyle en ($C_3$-$C_7$), -O-aryle, -O-hétéroaryle, -N-[alkyle en ($C_0$-$C_6$)]$_2$, -N-[alkyle en ($C_0$-$C_6$)][cycloalkyle en ($C_3$-$C_7$)] ou -N-[alkyle en ($C_0$-$C_6$)-aryle] ;

D, E, F, G, K et L dans P représentent indépendamment $-C(R_3)=$, $-C(R_3)=C(R_4)-$, $-C(=O)-$, $-C(=S)-$, $-O-$, $-N=$, $-N(R_3)-$ ou $-S-$ ;

Q indique un aryle ou un groupement hétéroaryle de formule

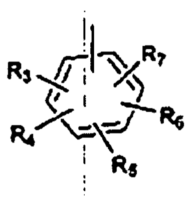, 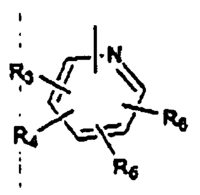, 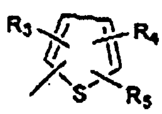, 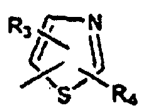, 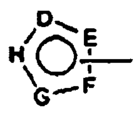

$R_3$, $R_4$, $R_5$, $R_6$ et $R_7$ sont indépendamment tels que définis ci-dessus ;

D, E, F, G et H dans Q représentent indépendamment $-C(R_3)=$, $-C(R_3)=C(R_4)-$, $-C(=O)-$, $-C(=S)-$, $-O-$, $-N=$, $-N(R_3)-$ ou $-S-$ ;

J représente $-C(R_{10}, R_{11})$, $-O-$, $-N(R_{10})-$ ou $-S-$ ;

$R_{10}$, $R_{11}$ sont indépendamment hydrogène, alkyle en ($C_1$-$C_6$), cycloalkyle en ($C_3$-$C_6$), cycloalkylalkyle en ($C_3$-$C_7$), alkényle en ($C_2$-$C_6$), alkynyle en ($C_2$-$C_6$), halo-alkyle en ($C_1$-$C_6$), hétéroaryle, hétéroarylalkyle, arylalkyle ou aryle ; l'un quelconque de ceux-ci étant éventuellement substitué par 1-5 substituants indépendamment sélectionnés parmi halogène, -CN, alkyle en ($C_1$-$C_6$), -O-alkyle en ($C_0$-$C_6$), -O-cycloalkylalkyle en ($C_3$-$C_7$), -O-aryle, -O-hétéroaryle, - N[alkyle en ($C_0$-$C_6$)][alkyle en ($C_0$-$C_6$)], -N[alkyle en ($C_0$-$C_6$)][cycloalkyle en ($C_3$-$C_7$)] ou -N [alkyle en ($C_0$-$C_6$)]-aryle] ;

Tout N peut être un N-oxyde ;

ou des sels, hydrates ou solvates pharmaceutiquement acceptables de tels composés.

**6.** Composé selon la revendication 1 ayant la formule II-B

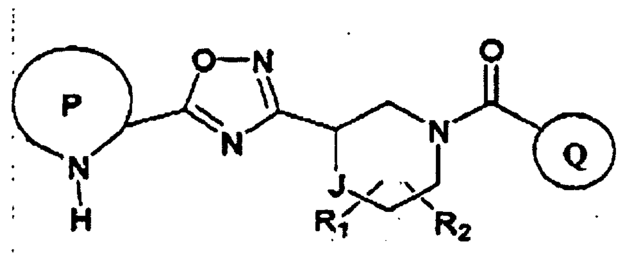

II-B

dans lequel

$R_1$ et $R_2$ représentent indépendamment hydrogène, alkyle en ($C_1$-$C_6$), alkényle en ($C_2$-$C_6$), alkynyle en ($C_2$-$C_6$), arylalkyle, hétéroarylalkyle, hydroxy, amino, aminoalkyle, hydroxyalkyle ou alkoxy en ($C_1$-$C_6$), ou $R_1$ et $R_2$ peuvent ensemble former un noyau cycloalkyle en ($C_3$-$C_7$), une liaison carbonyle C=O ou une double liaison avec un carbone ;
P représente un noyau hétérocycloalkyle en ($C_5$-$C_7$) ou hétérocycloalkényle en ($C_5$-$C_7$) ou un groupement hétéroaryle de formule

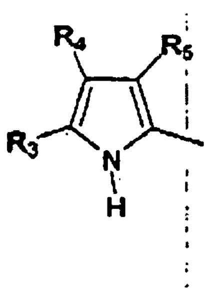 , 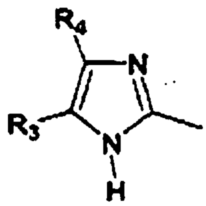 , 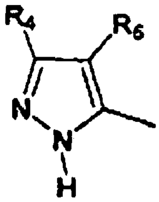 , 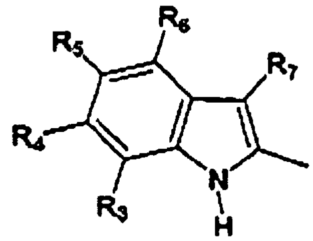

$R_3$, $R_4$, $R_5$, $R_6$ et $R_7$ sont chacun un substituant indépendamment sélectionné parmi hydrogène, halogène, $-NO_2$, alkyle en ($C_1$-$C_6$), cycloalkyle en ($C_3$-$C_6$), cycloalkylalkyle en ($C_3$-$C_7$), alkényle en ($C_2$-$C_6$), alkynyle en ($C_2$-$C_6$), halo-alkyle en ($C_1$-$C_6$), hétéroaryle, hétéroarylalkyle, arylalkyle, aryle, $-OR_8$, $-NR_8R_9$, $-C(=NR_{10})NR_8R_9$, $-NR_8COR_9$, $NR_8CO_2R_9$, $NR_8SO_2R_9$, $-NR_{10}CO\ NR_8R_9$, $-SR_8$, $-S(=O)R_8$, $-S(=O)_2R_8$, $-S(=O)_2NR_8R_9$, $-C(=O)R_8$, $-C(O)-O-R_8$, $-C(=O)NR_8R_9$, $-C(=NR_8)R_9$ ou $C(=NOR_8)R_9$ ; dans lequel, éventuellement, deux substituants sont combinés aux atomes intervenants pour former un noyau hétérocycloalkyle, aryle ou hétéroaryle bicyclique ; dans lequel chaque noyau est éventuellement en outre substitué par 1-5 groupements indépendamment sélectionnés parmi halogène, -CN, alkyle en ($C_1$-$C_6$), -O-alkyle en ($C_0$-$C_6$), -O-cycloalkylalkyle en ($C_3$-$C_7$), -O-aryle, -O-hétéroaryle, -O-alkyle en ($C_1$-$C_3$)aryle, -O-alkyle en ($C_1$-$C_3$)hétéroaryle, -N[alkyle en ($C_0$-$C_6$)][alkyle en ($C_0$-$C_3$)aryle] ou -N[alkyle en ($C_0$-$C_6$)][alkyle en ($C_0$-$C_3$)hétéroaryle] ;
$R_8$, $R_9$ et $R_{10}$ sont chacun indépendamment hydrogène, alkyle en ($C_1$-$C_6$), cycloalkyle en ($C_3$-$C_6$), cycloalkylalkyle en ($C_3$-$C_7$), alkényle en ($C_2$-$C_6$), alkynyle en ($C_2$-$C_6$), halo-alkyle en ($C_1$-$C_6$), hétérocycloalkyle, hétéroaryle, hétéroarylalkyle, arylalkyle ou aryle ; l'un quelconque de ceux-ci étant éventuellement substitué par 1-5 substituants indépendamment sélectionnés parmi halogène, -CN, alkyle en ($C_1$-$C_6$), -O-alkyle en ($C_0$-$C_6$), -O-cycloalkylalkyle en ($C_3$-$C_7$), -O-aryle, -O-hétéroaryle, -N-[alkyle en ($C_0$-$C_6$)]$_2$, -N-[alkyle en ($C_0$-$C_6$)][cycloalkyle en ($C_3$-$C_7$)] ou -N-[alkyle en ($C_0$-$C_6$)-aryle] ;
Q indique un aryle ou un groupement hétéroaryle de formule

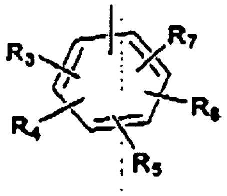 , 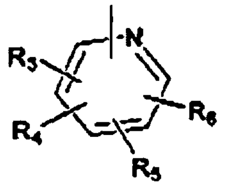 , 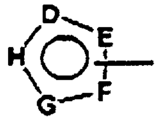

$R_3$, $R_4$, $R_5$, $R_6$ et $R_7$ sont indépendamment tels que définis ci-dessus ;
D, E, F, G et H dans Q représentent indépendamment $-C(R_3)=$, $-C(R_3)=C(R_4)-$, -O- ou -N= ;
J représente $-C(R_{10}, R_{11})$, -O-, $-N(R_{10})-$ ou -S- ;
$R_{10}$, $R_{11}$ représentent indépendamment hydrogène, alkyle en ($C_1$-$C_6$), cycloalkyle en ($C_3$-$C_6$), cycloalkylalkyle en ($C_3$-$C_7$), alkényle en ($C_2$-$C_6$), alkynyle en ($C_2$-$C_6$), halo-alkyle en ($C_1$-$C_6$), hétéroaryle, hétéroarylalkyle, arylalkyle ou aryle ; l'un quelconque de ceux-ci étant éventuellement substitué par 1-5 substituants indépendamment sélectionnés parmi halogène, -CN, alkyle en ($C_1$-$C_6$), -O-alkyle en ($C_0$-$C_6$), -O-cycloalkylalkyle en ($C_3$-$C_7$), - O-aryle, -O-hétéroaryle, -N[alkyle en ($C_0$-$C_6$)][alkyle en ($C_0$-$C_6$)], -N[alkyle en ($C_0$-$C_6$)][cycloalkyle en ($C_3$-$C_7$)] ou -N[alkyle en ($C_0$-$C_6$)]-aryle] ;
Tout N peut être un N-oxyde ;

ou des sels, hydrates ou solvates pharmaceutiquement acceptables de tels composés.

**7.** Composé selon la revendication 1 ayant la formule II-C

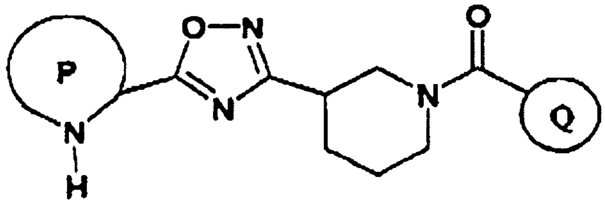

II-C

dans lequel

P représente un noyau hétérocycloalkyle en ($C_5$-$C_7$) ou hétérocycloalkényle en ($C_5$-$C_7$) ou un groupement hétéroaryle de formule

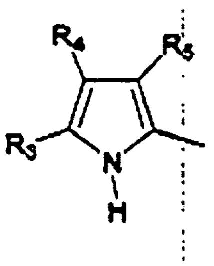 , 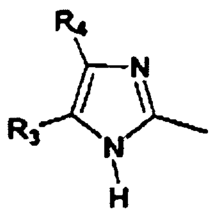 , 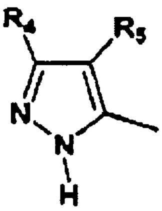 , 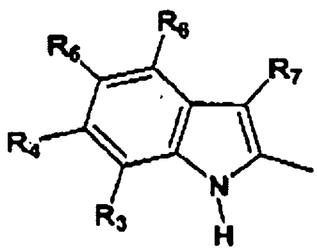

$R_3$, $R_4$, $R_5$, $R_6$ et $R_7$ représentent indépendamment hydrogène, halogène, $-NO_2$, alkyle en ($C_1$-$C_6$), halo-alkyle en ($C_1$-$C_6$) ou -CN ;
Q indique un aryle ou un groupement hétéroaryle de formule

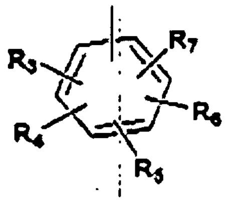 , 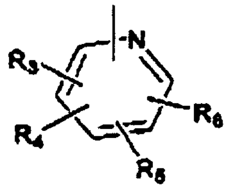 , 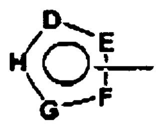

$R_3$, $R_4$, $R_5$, $R_6$ et $R_7$ sont indépendamment tels que définis ci-dessus ;
D, E, F, G et H dans Q représentent indépendamment $-C(R_3)=$, $-C(R_3)=C(R_4)-$, -O- ou -N= ;
Tout N peut être un N-oxyde ;

ou des sels, hydrates ou solvates pharmaceutiquement acceptables de tels composés.

**8.** Composé selon la revendication 1 ayant la formule I-D

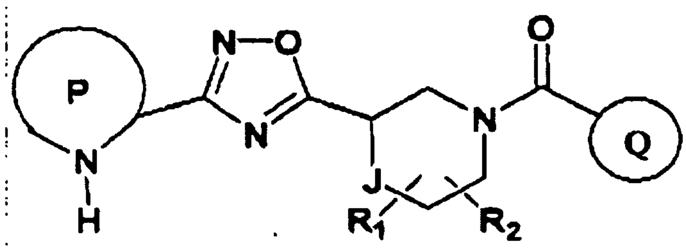 .

I-D

dans lequel

$R_1$ et $R_2$ représentent indépendamment hydrogène, alkyle en ($C_1$-$C_6$), alkényle en ($C_2$-$C_6$), alkynyle en ($C_2$-$C_6$), arylalkyle, hétéroarylalkyle, hydroxy, amino, aminoalkyle, hydroxyalkyle ou alkoxy en ($C_1$-$C_6$), ou $R_1$ et $R_2$ peuvent ensemble former un noyau cycloalkyle en ($C_3$-$C_7$), une liaison carbonyle C=O ou une double liaison avec un carbone ;
P représente un noyau hétérocycloalkyle en ($C_5$-$C_7$) ou hétérocycloalkényle en ($C_5$-$C_7$) ou un groupement hétéroaryle de formule

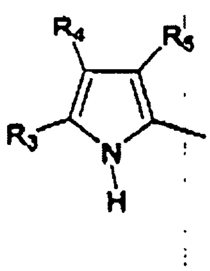 , 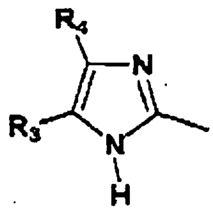 , 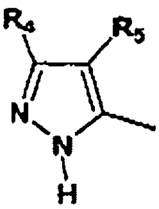 , 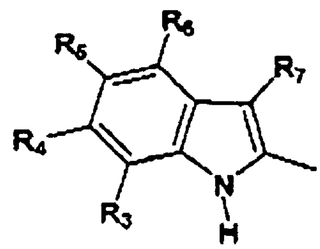

$R_3$, $R_4$, $R_5$, $R_6$ et $R_7$ sont chacun un substituant indépendamment sélectionné parmi hydrogène, halogène, $-NO_2$, alkyle en ($C_1$-$C_6$), cycloalkyle en ($C_3$-$C_6$), cycloalkylalkyle en ($C_3$-$C_7$), alkényle en ($C_2$-$C_6$), alkynyle en ($C_2$-$C_6$), halo-alkyle en ($C_1$-$C_6$), hétéroaryle, hétéroarylalkyle, arylalkyle, aryle, $-OR_8$, $-NR_8R_9$, $-C(=NR_{10})NR_8R_9$, $-NR_8COR_9$, $NR_8CO_2R_9$, $NR_8SO_2R_9$, $-NR_{10}CO\ NR_8R_9$, $-SR_8$, $-S(=O)R_8$, $-S(=O)_2R_8$, $-S(=O)_2NR_8R_9$, $-C(=O)R_8$, $-C(O)-O-R_8$, $-C(=O)NR_8R_9$, $-C(=NR_8)R_9$ ou $C(=NOR_8)R_9$ ; dans lequel, éventuellement, deux substituants sont combinés aux atomes intervenants pour former un noyau hétérocycloalkyle, aryle ou hétéroaryle bicyclique ; dans lequel chaque noyau est éventuellement en outre substitué par 1-5 groupements indépendamment sélectionnés parmi halogène, -CN, alkyle en ($C_1$-$C_6$), -O-alkyle en ($C_0$-$C_6$), -O-cycloalkylalkyle en ($C_3$-$C_7$), -O-aryle, -O-hétéroaryle, -O-alkyle en ($C_1$-$C_3$)aryle, -O-alkyle en ($C_1$-$C_3$)hétéroaryle, -N[alkyle en ($C_0$-$C_6$)][alkyle

en ($C_0$-$C_3$)aryle] ou -N[alkyle en ($C_0$-$C_6$)][alkyle en ($C_0$-$C_3$)hétéroaryle] ;

$R_8$, $R_9$ et $R_{10}$ sont chacun indépendamment hydrogène, alkyle en ($C_1$-$C_6$), cycloalkyle en ($C_3$-$C_6$), cycloalkylalkyle en ($C_3$-$C_7$), alkényle en ($C_2$-$C_6$), alkynyle en ($C_2$-$C_6$), halo-alkyle en ($C_1$-$C_6$), hétérocycloalkyle, hétéroaryle, hétéroarylalkyle, arylalkyle ou aryle ; l'un quelconque de ceux-ci étant éventuellement substitué par 1-5 substituants indépendamment sélectionnés parmi halogène, -CN, alkyle en ($C_1$-$C_6$), -O-alkyle en ($C_0$-$C_6$), -O-cycloalkylalkyle en ($C_3$-$C_7$), -O-aryle, -O-hétéroaryle, -N-[alkyle en ($C_0$-$C_6$)]$_2$, -N-[alkyle en ($C_0$-$C_6$)][cycloalkyle en ($C_3$-$C_7$)] ou -N-[alkyle en ($C_0$-$C_6$)-aryle] ;

Q indique un aryle ou un groupement hétéroaryle de formule

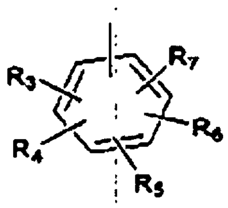
,
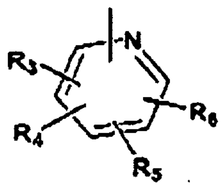
,
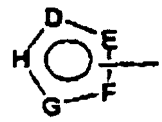

$R_3$, $R_4$, $R_5$, $R_6$ et $R_7$ sont indépendamment tels que définis ci-dessus ;

D, E, F, G et H dans Q représentent indépendamment -C($R_3$)=, -C($R_3$)=C($R_4$)-, -O- ou -N= ;

J représente -C($R_{10}$, $R_{11}$), -O-, -N($R_{10}$)- ou -S- ;

$R_{10}$, $R_{11}$ sont indépendamment hydrogène, alkyle en ($C_1$-$C_6$), cycloalkyle en ($C_3$-$C_6$), cycloalkylalkyle en ($C_3$-$C_7$), alkényle en ($C_2$-$C_6$), alkynyle en ($C_2$-$C_6$), halo-alkyle en ($C_1$-$C_6$), hétéroaryle, hétéroarylalkyle, arylalkyle ou aryle ; l'un quelconque de ceux-ci étant éventuellement substitué par 1-5 substituants indépendamment sélectionnés parmi halogène, -CN, alkyle en ($C_1$-$C_6$), -O-alkyle en ($C_0$-$C_6$), -O-cycloalkylalkyle en ($C_3$-$C_7$), -O-aryle, -O-hétéroaryle, - N[alkyle en ($C_0$-$C_6$)][alkyle en ($C_0$-$C_6$)], -N[alkyle en ($C_0$-$C_6$)][cycloalkyle en ($C_3$-$C_7$)] ou -N [alkyle en ($C_0$-$C_6$)]-aryle] ;

Tout N peut être un N-oxyde ;

ou des sels, hydrates ou solvates pharmaceutiquement acceptables de tels composés.

**9.** Composé selon la revendication 1 ayant la formule I-E

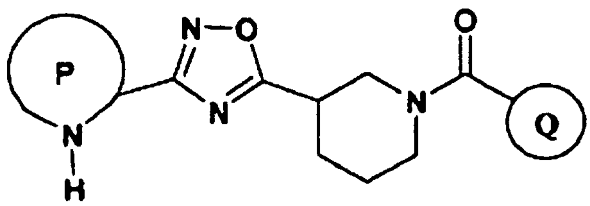

I-E

dans lequel

P représente un noyau hétérocycloalkyle en ($C_5$-$C_7$) ou hétérocycloalkényle en ($C_5$-$C_7$) ou un groupement hétéroaryle de formule

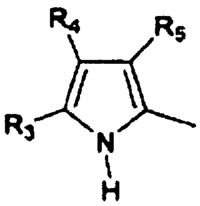 , 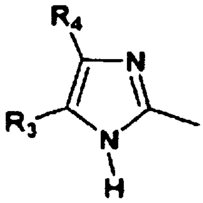 , 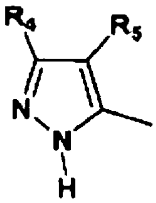 , 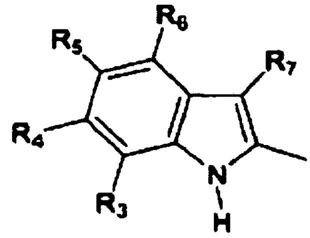

$R_3$, $R_4$, $R_5$, $R_6$ et $R_7$ sont indépendamment hydrogène, halogène, $-NO_2$, alkyle en ($C_1$-$C_6$), halo-alkyle en ($C_1$-$C_6$) ou -CN ;
Q indique un aryle ou un groupement hétéroaryle de formule

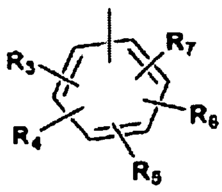 . 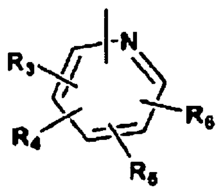 . 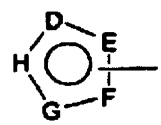

$R_3$, $R_4$, $R_5$, $R_6$ et $R_7$ sont indépendamment tels que définis ci-dessus ;
D, E, F, G et H dans Q représentent indépendamment $-C(R_3)=$, $-C(R_3)=C(R_4)-$, -O- ou -N= ;
Tout N peut être un N-oxyde ;
ou des sels, hydrates ou solvates pharmaceutiquement acceptables de tels composés.

**10.** Composé selon les revendications 1 à 9 qui peut exister sous la forme d'isomères optiques, dans lequel ledit composé est soit le mélange racémique, soit un isomère optique individuel.

**11.** Composé selon les revendications 1 à 10, dans lequel ledit composé est sélectionné parmi les suivants :

(4-Fluoro-phényl)-{(S)-3-[3-(1H-pyrrol-2-yl)-[1,2,4]oxadiazol-5-yl]-pipéridin-1-yl}-méthanone
(2,4-Difluoro-phényl)-{(S)-3-[3-(1H-pyrrol-2-yl)-[1,2,4]oxadiazol-5-yl]-pipéridin-1-yl}-méthanone
(3,4-Difluoro-phényl)-{(S)-3-[3-(1H-pyrrol-2-yl)-[1,2,4]oxadiazol-5-yl]-pipéridin-1-yl}-méthanone
(6-Fluoro-pyridin-3-yl)-{(S)-3-[3-(1H-pyrrol-2-yl)-[1,2,4]oxadiazol-5-yl]-pipéridin-1-yl}-méthanone
(3,4-Difluoro-phényl)-{3-[5-(1H-pyrrol-2-yl)-[1,2,4]oxadiazol-3-yl]-pipéridin-1-yl}-méthanone
(2,4-Difluoro-phényl)-{3-[5-(1H-pyrrol-2-yl)-[1,2,4]oxadiazol-3-yl]-pipéridin-1-yl}-méthanone
(4-Fluoro-phényl)-{3-[5-(1H-pyrrol-2-yl)-[1,2,4]oxadiazol-3-yl]-pipéridin-1-yl}-méthanone
(6-Fluoro-pyridin-3-yl)-{3-[5-(1H-pyrrol-2-yl)-[1,2,4]oxadiazol-3-yl]-pipéridin-1-yl}-méthanone
(4-Fluoro-2-méthyl-phényl)-{3-[5-(1H-pyrrol-2-yl)-[1,2,4]oxadiazol-3-yl]-pipéridin-1-y}-méthanone
(3,4-Difluoro-phényl)-{(S)-3-[5-(1H-pyrrol-2-yl)-[1,2,4]oxadiazol-3-yl]-pipéridin-1-yl}-méthanone
(4-Fluoro-phényl)-{3-[5-(1H-indol-2-yl)-[1,2,4]oxadiazol-3-yl]-pipéridin-1-yl}-méthanone
(2,4-Difluoro-phényl)-{(S)-3-[3-(1H-indol-2-yl)-[1,2,4]oxadiazol-5-yl]-pipéridin-1-yl}-méthanone
(4-Fluoro-phényl)-{3-[5-(2H-pyrazol-3-yl)-[1,2,4]oxadiazol-3-yl]-pipéridin-1-yl}-méthanone
(3,4-Difluoro-phényl)-{3-[5-(2H-pyrazol-3-yl)-[1,2,4]oxadiazol-3-yl]-pipéridin-1-yl}-méthanone
(4-Fluoro-phényl)-{3-[5-(1H-imidazol-2-yl)-[1,2,4]oxadiazol-3-yl]-pipéridin-1-yl}-méthanone
(3,4-Difluoro-phényl)-{(S)-3-[3-(1H-indol-2-yl)-[1,2,4]oxadiazol-5-yl]-pipéridin-1-yl}-méthanone
(4-Fluoro-phényl)-{(S)-3-[3-(1H-indol-2-yl)-[1,2,4]oxadiazol-5-yl]-pipéridin-1-yl}-méthanone
(3,4-Difluoro-phényl)-{3-[5-(1H-imidazol-2-yl)-[1,2,4]oxadiazol-3-yl]-pipéridin-1-yl}-méthanone
{(S)-3-[3-(1H-Indol-2-yl)-[1,2,4]oxadiazol-5-yl]-pipéridin-1-yl}-(5-méthyl-isoxazol-4-yl)-méthanone
(5-Méthyl-isoxazol-4-yl)-{(S)-3-[3-(1H-pyrrol-2-yl)-[1,2,4]oxadiazol-5-yl]-pipéridin-1-yl}-méthanone
(6-Fluoro-pyridin-3-yl)-{(S)-3-[5-(1H-pyrrol-2-yl)-[1,2,4]oxadiazol-3-yl]-pipéridin-1-yl}-méthanone
(4-Fluoro-phényl)-{(S)-3-[5-(1H-pyrrol-2-yl)-[1,2,4]oxadiazol-3-yl]-pipéridin-1-yl}-méthanone
(6-Fluoro-pyridin-3-yl)- {3-[5-(1H-indol-2-yl)-[1,2,4]oxadiazol-3-yl]-pipéridin-1-yl}-méthanone
(4-Fluoro-phényl)-{(S)-3-[3-(1H-imidazol-2-yl)-[1,2,4]oxadiazol-5-yl]-pipéridin-1-yl}-méthanone

(3,4-Difluoro-phényl)-{(S)-3-[3-(1H-imidazol-2-yl)-[1,2,4]oxadiazol-5-yl]-pipéridin-1-yl}-méthanone
{3-[5-(1H-Indol-2-yl)-[1,2,4]oxadiazol-3-yl]-pipéridin-1-yl}-(5-méthyl-isoxazol-4-yl)-méthanone
(4-Fluoro-phényl)-{(S)-3-[5-(4-méthyl-1H-pyrrol-2-yl)-[1,2,4]oxadiazol-3-yl]-pipéridin-1-yl}-méthanone
(6-Fluoro-pyridin-3-yl)-{(S)-3-[5-(4-méthyl-1H-pyrrol-2-yl)-[1,2,4]oxadiazol-3-yl]-pipéridin-1-yl}-méthanone
(5-Méthyl-isoxazol-4-yl)-{(S)-3-[5-(4-méthyl-1H-pyrrol-2-yl)-[1,2,4]oxadiazol-3-yl]-pipéridin-1-yl}-méthanone
(2-Fluoro-pyridin-4-yl)-{(S)-3-[5-(4-méthyl-1H-pyrrol-2-yl)-[1,2,4]oxadiazol-3-yl]-pipéridin-1-yl}-méthanone
(4-Fluoro-phényl)-{(S)-3-[3-(4-méthyl-1H-pyrrol-2-yl)-[1,2,4]oxadiazol-5-yl]-pipéridin-1-yl}-méthanone
(3,4-Difluoro-phényl)-{(S)-3-[3-(4-méthyl-1H-pyrrol-2-yl)-[1,2,4]oxadiazol-5-yl]-pipéridin-1-yl}-méthanone
(6-Fluoro-pyridin-3-yl)-{(S)-3-[3-(4-méthyl-1H-pyrrol-2-yl)-[1,2,4]oxadiazol-5-yl]-pipéridin-1-yl}-méthanone
(2-Fluoro-pyridin-4-yl)-{(S)-3-[3-(4-méthyl-1H-pyrrol-2-yl)-[1,2,4]oxadiazol-5-yl]-pipéridin-1-yl}-méthanone
(5-Méthyl-isoxazol-4-yl)-{(S)-3-[3-(4-méthyl-1H-pyrrol-2-yl)-[1,2,4]oxadiazol-5-yl]-pipéridin-1-yl}-méthanone
(4-Fluoro-phényl)-{(S)-3-[5-(4-nitro-1H-pyrrol-2-yl)-[1,2,4]oxadiazol-3-yl]-pipéridin-1-yl}-méthanone
(4-Fluoro-phényl)-{(R)-3-[3-(1H-pyrrol-2-yl)-[1,2,4]oxadiazol-5-yl]-pipéridin-1-yl}-méthanone (4-Fluoro-phényl)-{(S)-3-[5-(5-méthyl-1H-pyrrol-2-yl)-[1,2,4]oxadiazol-3-yl]-pipéridin-1-yl}-méthanone
{(S)-3-[5-(4-Chloro-1H-pyrrol-2-yl)-[1,2,4]oxadiazol-3-yl]-pipéridin-1-yl}-(4-fluoro-phényl)-méthanone
{(S)-3-[5-(4-Chloro-1H-pyrrol-2-yl)-[1,2,4]oxadiazol-3-yl]-pipéridin-1-yl}-(6-fluoro-pyridin-3-yl)-méthanone
{(S)-3-[5-(4-Chloro-1H-pyrrol-2-yl)-[1,2,4]oxadiazol-3-yl]-pipéridin-1-yl}-(2-fluoro-pyridin-4-yl)-méthanone
{(S)-3-[5-(4-Chloro-1H-pyrrol-2-yl)-[1,2,4]oxadiazol-3-yl]-pipéridin-1-yl}-(5-méthyl-isoxazol-4-yl)-méthanone
{(S)-3-[3-(4-Chloro-1H-pyrrol-2-yl)-[1,2,4]oxadiazol-5-yl]-pipéridin-1-yl}-(4-fluoro-phényl)-méthanone
{(S)-3-[5-(4-Bromo-1H-pyrrol-2-yl)-[1,2,4]oxadiazol-3-yl]-pipéridin-1-yl}-(6-fluoro-pyridin-3-yl)-méthanone
{(S)-3-[3-(4-Bromo-1H-pyrrol-2-yl)-[1,2,4]oxadiazol-5-yl]-pipéridin-1-yl}-(4-fluoro-phényl)-méthanone
{(S)-3-[3-(4-Bromo-1H-pyrrol-2-yl)-[1,2,4]oxadiazol-5-yl]-pipéridin-1-yl}-(6-fluoro-pyridin-3-yl)-méthanone
{3,3-Diméthyl-5-[3-(1H-pyrrol-2-yl)-[1,2,4]oxadiazol-5-yl]-pipéridin-1-yl}-(4-fluoro-phényl)-méthanone
(4-Fluoro-phényl)-{(S)-3-[3-(4-fluoro-1H-pyrrol-2-yl)-[1,2,4]oxadiazol-5-yl]-pipéridin-1-yl}-méthanone
(3,4-Difluoro-phényl)-{(S)-3-[3-(4-fluoro-1H-pyrrol-2-yl)-[1,2,4]oxadiazol-5-yl]-pipéridin-1-yl}-méthanone
(6-Fluoro-pyridin-3-yl)-{(S)-3-[3-(4-fluoro-1H-pyrrol-2-yl)-[1,2,4]oxadiazol-5-yl]-pipéridin-1-yl}-méthanone
(2-Fluoro-pyridin-4-yl)-{(S)-3-[3-(4-fluoro-1H-pyrrol-2-yl)-[1,2,4]oxadiazol-5-yl]-pipéridin-1-yl}-méthanone
(4-Fluoro-phényl)-{(S)-3-[5-(1H-pyrrol-2-yl)-tétrazol-2-yl]-pipéridin-1-yl}-méthanone
(4-Fluoro-phényl)-{(S)-3-[5-(4-trifluorométhyl-1H-imidazol-2-yl)-[1,2,4]oxadiazol-3-yl]-pipéridin-1-yl}-méthanone
(6-Fluoro-pyridin-3-yl)-{(S)-3-[5-(4-isopropyl-1H-pyrrol-2-yl)-[1,2,4]oxadiazol-3-yl]-pipéridin-1-yl}-méthanone
(3-Fluoro-pyridin-4-yl)-{(S)-3-[5-(4-méthyl-1H-pyrrol-2-yl)-[1,2,4]oxadiazol-3-yl]-pipéridin-1-yl}-méthanone
{(S)-3-[5-(4-Chloro-1H-pyrrol-2-yl)-[1,2,4]oxadiazol-3-yl]-pipéridin-1-yl}-(3-fluoro-pyridin-4-yl)-méthanone
(2-Fluoro-pyridin-4-yl)-{(S)-3-[5-(4-fluoro-1H-pyrrol-2-yl)-[1,2,4]oxadiazol-3-yl]-pipéridin-1-yl}-méthanone
{(S)-3-[5-(4-Bromo-1H-pyrrol-2-yl)-[1,2,4]oxadiazol-3-yl]-pipéridin-1-yl}-(3-fluoro-pyridin-4-yl)-méthanone
(3-Fluoro-pyridin-4-yl)-{(S)-3-[5-(4-fluoro-1H-pyrrol-2-yl)-[1,2,4]oxadiazol-3-yl]-pipéridin-1-yl}-méthanone
(4-Fluoro-phényl)-{(S)-3-[5-(4-fluoro-1H-pyrrol-2-yl)-[1,2,4]oxadiazol-3-yl]-pipéridin-1-yl}-méthanone
(6-Fluoro-pyridin-3-y1)-{(S)-3-[5-(4-fluoro-1H-pyrrol-2-yl)-[1,2,4]oxadiazol-3-yl]-pipéridin-1-yl}-méthanone
{(S)-3-[3-(4-Chloro-1H-pyrrol-2-yl)-[1,2,4]oxadiazol-5-yl]-pipéridin-1-yl}-(6-fluoro-pyridin-3-yl)-méthanone
{(S)-3-[3-(4-Chloro-1H-pyrrol-2-yl)-[1,2,4]oxadiazol-5-yl]-pipéridin-1-yl}-(2-fluoro-pyridin-4-yl)-méthanone
{(S)-3-[3-(4-Chloro-1H-pyrrol-2-yl)-[1,2,4]oxadiazol-5-yl]-pipéridin-1-yl}-(3-fluoro-pyridin-4-yl)-méthanone
{(S)-3-[3-(4-Chloro-1H-pyrrol-2-yl)-[1,2,4]oxadiazol-5-yl]-pipéridin-1-yl}-(5-méthyl-isoxazol-4-yl)-méthanone
{(S)-3-[3-(4-Bromo-1H-pyrrol-2-yl)-[1,2,4]oxadiazol-5-yl]-pipéridin-1-yl}-(3-fluoro-pyridin-4-yl)-méthanone
(3-Fluoro-pyridin-4-yl)-{(S)-3-[3-(4-fluoro-1H-pyrrol-2-yl)-[1,2,4]oxadiazol-5-yl]-pipéridin-1-yl}-méthanone
(3-Fluoro-pyridin-4-yl)-{(S)-3-[3-(4-méthyl-1H-pyrrol-2-yl)-[1,2,4]oxadiazol-5-yl]-pipéridin-1 - yl}-méthanone
(4-Fluoro-phényl)-{(S)-3-[5-(4-trifluorométhyl-1H-pyrrol-2-yl)-[1,2,4]oxadiazol-3-yl]-pipéridin-1-yl}-méthanone
(3-Fluoro-pyridin-4-yl)-{(S)-3-[5-(4-trifluorométhyl-1H-pyrrol-2-yl)[1,2,4]oxadiazol-3-yl]-pipéridin-1-yl}-méthanone
(6-Fluoro-pyridin-3-yl)-{(S)-3-[5-(4-trifluorométhyl-1H-pyrrol-2-yl)-[1,2,4]oxadiazol-3-yl]-pipéridin-1-yl}-méthanone
(3,4-Difluoro-phényl)-{(S)-3-[3-(4-méthyl-1H-imidazol-2-yl)-[1,2,4]oxadiazol-5-yl]-pipéridin-1-yl}-méthanone
{(S)-3-[5-(4-Chloro-1H-pyrrol-2-yl)-[1,2,4]oxadiazol-3-yl]-pipéridin-1-yl}-pyridin-4-yl-méthanone
(6- Fluoro- pyridin- 3- yl)- { (S)- 3-[3-(4- trifluorométhyl- 1H- pyrrol- 2- yl)-[1,2,4] oxadiazol- 5- yl]-pipéridin- 1-yl}-méthanone ;

ou des sels, hydrates ou solvates pharmaceutiquement acceptables de tels composés.

**12.** Composition pharmaceutique comprenant une quantité thérapeutiquement efficace d'un composé selon les revendications 1 à 11 et un véhicule et/ou excipient pharmaceutiquement acceptable.

**13.** Composé/composition selon les revendications 1 à 12, pour le traitement ou la prévention chez un mammifère, y compris un être humain, d'une maladie dont le traitement ou la prévention est influencé ou facilité par l'effet neuromodulateur de modulateurs allostériques du récepteur mGluR5.

**14.** Composé/composition selon les revendications 1 à 12, pour le traitement ou la prévention chez un mammifère, y compris un être humain, d'une maladie dont le traitement ou la prévention est influencé ou facilité par l'effet neuromodulateur de modulateurs allostériques positifs (amplificateur) du récepteur mGluR5.

**15.** Composé/composition selon les revendications 1 à 12, pour le traitement ou la prévention de maladies du système nerveux central sélectionnées dans le groupe consistant en des troubles anxieux : agoraphobie, trouble anxieux généralisé (TAG), trouble obsessionnel compulsif (TOC), trouble panique, syndrome de stress post-traumatique (SSPT), phobie sociale, autres phobies, trouble anxieux induit par une substance.

**16.** Composé/composition selon les revendications 1 à 12, pour le traitement ou la prévention de maladies du système nerveux central sélectionnées dans le groupe consistant en des maladies infantiles : trouble de déficit de l'attention et hyperactivité.

**17.** Composé/composition selon les revendications 1 à 12, pour le traitement ou la prévention de maladies du système nerveux central sélectionnées dans le groupe consistant en des troubles du comportement alimentaire : anorexie mentale, boulimie.

**18.** Composé/composition selon les revendications 1 à 12, pour le traitement ou la prévention de maladies du système nerveux central sélectionnées dans le groupe consistant en des troubles de l'humeur : maladies à forme bipolaire (I & II), trouble cyclothymique, dépression, trouble dysthymique, trouble dépressif majeur, trouble de l'humeur induit par une substance.

**19.** Composé/composition selon les revendications 1 à 12, pour le traitement ou la prévention de maladies du système nerveux central sélectionnées dans le groupe consistant en des maladies psychotiques : schizophrénie, trouble délirant, trouble schizoaffectif, trouble schizophréniforme, trouble psychotique induit par une substance.

**20.** Composé/composition selon les revendications 1 à 12, pour le traitement ou la prévention de maladies du système nerveux central sélectionnées dans le groupe consistant en des troubles cognitifs : délire, délire persistant induit par une substance, démence, complexe démentiel associé au VIH, démence de la maladie de Huntington, démence de la maladie de Parkinson, démence de type Alzheimer, démence persistante induite par une substance, déclin cognitif léger.

**21.** Composé/composition selon les revendications 1 à 12, pour le traitement ou la prévention de maladies du système nerveux central sélectionnées dans le groupe consistant en des troubles de la personnalité : trouble de la personnalité obsessionnelle-compulsive, schizoïdie, trouble schizotypique.

**22.** Composé/composition selon les revendications 1 à 12, pour le traitement ou la prévention de maladies du système nerveux central sélectionnées dans le groupe consistant en des troubles liés à l'usage d'une substance : usage abusif d'alcool, alcoolodépendance, sevrage alcoolique, délirium du sevrage alcoolique, trouble psychotique d'origine alcoolique, dépendance aux amphétamines, sevrage des amphétamines, dépendance à la cocaïne, sevrage de la cocaïne, dépendance à la nicotine, sevrage de la nicotine, dépendance aux opioïdes, sevrage des opioïdes.

**23.** Composé/composition selon les revendications 1 à 12, pour le traitement ou la prévention de maladies inflammatoires du système nerveux central sélectionnées dans le , groupe consistant en une forme de sclérose en plaques comme la forme bénigne de sclérose en plaques, la forme récurrente-rémittente de sclérose en plaques, la forme progressive secondaire de sclérose en plaques et la forme progressive primaire de sclérose en plaques.

**24.** Utilisation d'un composé/d'une composition selon les revendications 1 à 12 dans la fabrication d'un médicament pour un traitement ou une prévention comme défini à l'une quelconque des revendications 15 à 23.

**25.** Utilisation des composés selon la revendication 1 pour la préparation de traceurs pour l'imagerie des récepteurs métabotropes au glutamate.

**Figure 1**

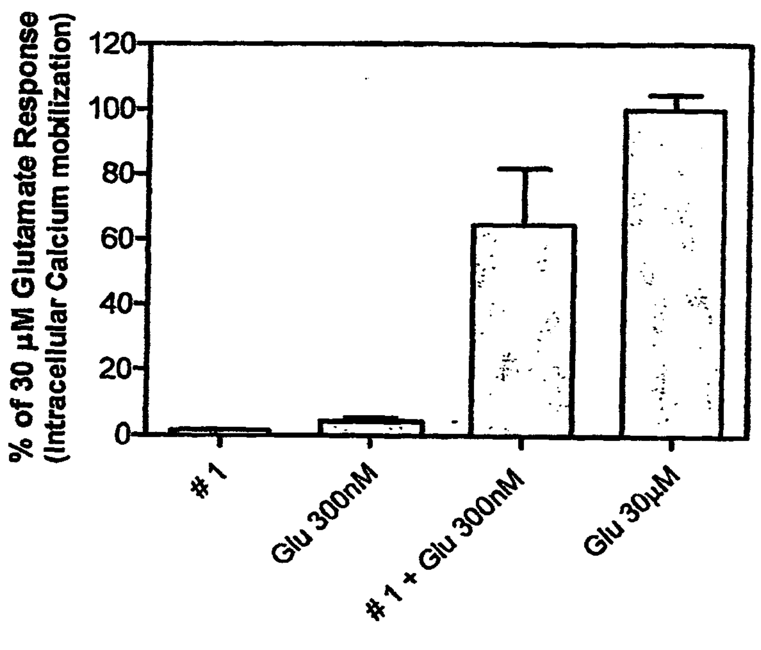
% of 30 µM Glutamate Response
(Intracellular Calcium mobilization)
120
100
80
60
40
20
0
# 1
Glu 300nM
# 1 + Glu 300nM
Glu 30µM

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2004087048 A
- WO 2005087048 A
- WO 2005044797 A
- WO 9945006 A
- US 04106607 A
- WO 03056823 A
- WO 0272570 A
- GB 1164572 A
- FR 6671
- WO 2005009988 A
- WO 2004058754 A
- WO 2004029044 A
- WO 2004014902 A2
- WO 03093236 A
- WO 2004048334 A
- WO 03093297 A2
- WO 03037888 A
- WO 03027080 A
- EP 1300396 A1
- WO 03087304 A2
- WO 2006048771 A
- WO 2006036015 A2
- WO 2005074934 A
- WO 2005115389 A2
- WO 2006044509 A2
- WO 2006123249 A
- WO 2006123255 A
- WO 2006123257 A
- WO 2006065601 A2


### Non-patent literature cited in the description


- **Nakanishi S et al.** *Brain Res Brain Res Rev.,* 1998, vol. 26, 230-235
- **Schoepp DD et al.** *Neuropharmacology,* 1999, vol. 38, 1431-1476
- **Lujan R et al.** *Eur J Neurosci.,* 1996, vol. 8, 1488-500
- **Lujan R et al.** *J Chem Neuroanat.,* 1997, vol. 13, 219-41
- **Romano C et al.** *J Comp Neurol.,* 1995, vol. 355, 455-69
- **Brauner-Osbome H et al.** *J Med Chem.,* 2000, vol. 43, 2609-45
- **Bordi F ; Ugolini A.** *Prog Neurobiol.,* 1999, vol. 59, 55-79
- **Spooren W et al.** *Behav Pharmacol,* 2003, vol. 14, 257-77
- **Goff DC ; Coyle JT.** *Am J Psychiatry,* 2001, vol. 158, 1367-1377
- **Carlsson A et al.** *Annu Rev Pharmacol Toxicol.,* 2001, vol. 41, 237-260
- **Devon RS et al.** *Mol Psychiatry,* 2001, vol. 6, 311-4
- **Ohnuma T et al.** *Brain Res Mol Brain Res.,* 1998, vol. 56, 207-17
- **Mannaioni G et al.** *Neurosci.,* 2001, vol. 21, 5925-34
- **Awad H et al.** *J Neurosci,* 2000, vol. 20, 7871-7879
- **Pisani A et al.** *Neuroscience,* 2001, vol. 106, 579-87
- **Benquet P et al.** *J Neurosci.,* 2002, vol. 22, 9679-86
- **Martin SJ et al.** *Annu. Rev. Neurosci.,* 2000, vol. 23, 649-711
- **Baudry M ; Lynch G.** *Neurobiol Learn Mem.,* 2001, vol. 76, 284-297
- **Lu et al.** *J. Neurosci.,* 1997, vol. 17, 5196-5205
- **Schulz B et al.** *Neuropharmacology,* 2001, vol. 41, 1-7
- **Jia Z et al.** *Physiol Behav.,* 2001, vol. 73, 793-802
- **Rodrigues et al.** *J Neurosci.,* 2002, vol. 22, 5219-5229
- **Knoflach F et al.** *Proc Natl Acad Sci U S A.,* 2001, vol. 98, 13402-13407
- **O'Brien JA et al.** *Mol Pharmacol.,* 2003, vol. 64, 731-40
- **Johnson K et al.** *Neuropharmacology,* 2002, vol. 43, 291
- **Johnson MP et al.** *J Med Chem.,* 2003, vol. 46, 3189-92
- **Marino MJ et al.** *Proc Natl Acad Sci U S A.,* 2003, vol. 100 (23), 13668-73
- **Mutel V.** *Expert Opin. Ther. Patents,* 2002, vol. 12, 1-8
- **Kew JN.** *Pharmacol Ther.,* 2004, vol. 104 (3), 233-44
- **Johnson MP et al.** *Biochem Soc Trans.,* 2004, vol. 32, 881-7

- **O'Brien JA et al.** *Mol. Pharmacol.,* 2003, vol. 64, 731-40
- **O'Brien JA.** *J. Pharmacol. Exp. Ther.,* 2004, vol. 309, 568-77
- **Lindsley et al.** *J. Med. Chem.,* 2004, vol. 47, 5825-8
- **Kinney GG et al.** *J Pharmacol Exp Ther,* 2005, vol. 313, 199-206
- **Vasvari-Debreczy, Lelle et al.** Nitrogen bridgehead compounds. Part 74. Cyclization of 2-[(2-pyridylamino)methylene]succinates in ethanolic sodium ethoxide. Part 2. Michael addition of pyridyldihydropyrrolones. *Chemical Abstracts Service,* 1989
- **Green T.W. ; Wuts P.G.M.** Protecting Groups in Organic Synthesis. John Wiley et Sons, 1991
- **Eliel E.L. ; Wilen S.H. ; Mander L.N.** Stereochemistry of Organic Compounds. Wiley-Interscience, 1984
- **Katrizky A.R. ; Rees C.W.** Comprehensive Heterocyclic Chemistry. Pergamon Press, 1984
- **Lucca, George V. De ; Kim, Ui T. ; Liang, Jing ; Cordova, Beverly ; Klabe, Ronald M. et al.** *J.Med.Chem.; EN,* 1998, vol. 41 (13), 2411-2423
- **Lila, Christine ; Gloanec, Philippe ; Cadet, Laurence ; Herve, Yolande ; Fournier, Jean et al.** *Synth.Commun.; EN,* 1998, vol. 28 (23), 4419-4430
- **Sendzik, Martin ; Hui, Hon C.** *Tetrahedron Lett.; EN,* 2003, vol. 44, 8697-8700
- **Suzuki, Takeshi ; Iwaoka, Kiyoshi ; Imanishi, Naoki ; Nagakura, Yukinori ; Miyata, Keiji et al.** *Chem.Pharm.Bull.; EN,* 1999, vol. 47 (1), 120-122
- **Katrizky A.R. ; Rees C.W.** *Comprehensive Heterocyclic Chemistry,* 1984
- **Lucca, George V. De ; Kim, Ui T. ; Liang, Jing ; Cordova, Beverly ; Klabe, Ronald M. et al.** *J.Med.Chem.; EN,* 1998, vol. 41, 13
- **Stahl P.H. ; Wermuth C.G.** Handbook of Pharmaceuticals Salts, Properties, Selection and Use. Wiley, 2002
- **Curran, T. ; Keaney, M.** *J. Org. Chem.,* 1996, vol. 61 (25), 9068-9069
- **Belanger.** *Tetrahedron Lett.,* 1979, 2505-2508
- *Tetrahedron,* 1996, 1231-1234
- **X. Qui ; F. Qing.** *J. Org Chem.,* 2002, vol. 67, 7162-7164
- **X. Qui ; F. Qing.** *J. Org. Chem.,* 2003, vol. 68, 3614-3617
- *Helvetica Chimica Acta,* 2005, vol. 88, 2454-2469
- **Miller et al.** *J. Neurosci.,* 1995, vol. 15, 6103-9
- **Mc Carthy ; de Vellis.** *J. Cell Biol.,* 1980, vol. 85, 890-902
- **Miller et al.** *J. Neurosci.,* 1995, vol. 15 (9), 6103-9
- **Liu et al.** *Eur. J. Pharmacol.,* 2006, vol. 536, 262-268
- **Zhang et al.** *J. Pharmacol. Exp. Ther.,* 2005, vol. 315, 1212-1219
- **Gasparini et al.** *Bioorg. Med. Chem. Lett.,* 2002, vol. 12, 407-409
- **Anderson et al.** *J. Pharmacol. Exp. Ther.,* 2002, vol. 303 (3), 1044-1051
- **Malherbe et al.** *Mol. Pharmacol.,* 2003, vol. 64 (4), 823-32
- **Yui et al.** *Ann. N.Y. Acad. Sci.,* 2000, vol. 914, 1-12
- **Arnt.** *Eur. J. Pharmacol.,* 1995, vol. 283, 55-62